# EUROPEAN PATENT SPECIFICATION

(11) **EP 3 034 580 B1**
(45) Date of publication and mention of the grant of the patent: **29.04.2020**
(21) Application number: 14836571.1
(22) Date of filing: 13.08.2014
(51) Int. Cl.: C09K 11/02, A61L 27/00, A61L 29/00, A61L 31/00, C09K 11/00, C09K 11/06, C08K 3/00, C08K 5/55, A61L 29/18, A61L 29/14, A61L 31/14, A61L 31/18, A61L 27/50, C08K 3/013, G07D 7/005

(54) **RESIN COMPOSITION AND MOLDED ARTICLE**
HARZZUSAMMENSETZUNG UND FORMARTIKEL
COMPOSITION DE RÉSINE, ET ARTICLE MOULÉ

(30) Priority: 13.08.2013 JP 2013168175; 26.12.2013 JP 2013270428
(43) Date of publication of application: 22.06.2016
(73) Proprietor: DIC Corporation, Tokyo 174-8520 (JP); National University Corporation Kochi University, Kochi-shi, Kochi 780-8520 (JP)
(72) Inventor: SAKURAI Naoto, Sakura-shi Chiba 285-8668 (JP); SAKURAI Yoshinobu, Sakura-shi Chiba 285-8668 (JP); WATANABE Yasuyuki, Sakura-shi Chiba 285-8668 (JP); IKEDA Takeo, Sakura-shi Chiba 285-8668 (JP); SATO Takayuki, Kochi-shi Kochi 780-8520 (JP)
(74) Representative: TBK
(86) International application number: PCT/JP2014/071393
(87) International publication number: WO 2015/022977

(56) References cited:
- WO-A1-2007/126052
- WO-A1-2012/073774
- JP-A- S63 281 660
- JP-A- 2008 541 987
- JP-A- 2009 263 614
- JP-A- 2010 090 313
- JP-A- 2011 162 445
- JP-A- 2013 060 399
- US-A1- 2004 236 275
- US-B1- 6 221 279
- GEORG M FISCHER ET AL: "Pyrrolopyrrole Cyanine Dyes: A New Class of Near-Infrared Dyes and Fluorophores", CHEMISTRY - A EUROPEAN JOURNAL,, vol. 15, no. 19, 4 May 2009 (2009-05-04), pages 4857-4864, XP008151231, ISSN: 0947-6539, DOI: 10.1002/CHEM.200801996 [retrieved on 2009-03-18]

## Description

### Technical Field

The present invention relates to a resin composition which is radiopaque and emits fluorescence and a molded article obtained from the resin composition.

### Background Art

A light-emitting substance and a radiopaque substance have been used in various industrial applications such as anti-counterfeiting applications of securities, certificates, credit cards, electronic equipment, and personal authentication media, product inspection applications, and medical tools, as a marking substance to identify a product, or to determine mixing of foreign materials or the internal situation. As the light-emitting substance, there are a fluorescent material and a phosphorescent material.

How to confirm the position in a living body of a medical tool used in a state of being embedded in a living body, such as a shunt tube, a catheter, or a stent, which is one of applications use, from the outside of the living body, is important. At present, as a method of visualizing a medical tool in a living body, mainly, a method in which a radiopaque substance is contained in a medical tool is used (for example, refer to PTLs 1 and 2). For example, the position of a medical tool formed of a resin in which a radiopaque substance has been contained, in a living body, can be confirmed based on an X-ray image taken by X-ray irradiation.

In addition, there is also a method in which a near-infrared fluorescent material which is one of the light-emitting substances is contained in a medical tool. In particular, as features of the near-infrared wavelength regions, since it is known that light in the near-infrared wavelength region cannot be observed with the naked human eye, the influence thereof on a living body is small, and the bio-transparency thereof with respect to the skin and the like is high. By a near-infrared fluorescent material being contained in a medical tool itself, such features can be used. For example, by a near-infrared fluorescent material being contained in a medical tool such as a shunt tube, a system in which the position of the medical tool embedded into a living body is confirmed by irradiating with near-infrared light from the outside of the living body is disclosed (for example, refer to PTL 3). Since the near-infrared light has a smaller effect on a living body than X-rays, it is possible to more safely visualize the medical tool in a living body.

To visualize a medical implant embedded subcutaneously or the like, excitation in the near-infrared light having high skin transparency is required, and the fluorescence emitted from the medical implant is also required to be in a near-infrared region having high skin transparency. That is, typically, to ensure the visibility, the near-infrared fluorescent material itself contained in the medical implant should strongly absorb light in the near-infrared region, and, in addition, is required to emit strong fluorescence. Therefore, as the near-infrared fluorescent material contained in the resin composition which is a raw material of a medical implant, it is preferable that the maximum absorption wavelength in the resin be in the near-infrared region.

In general, in a case where the fluorescence emitted from the fluorescent material is detected, the scattered light or the reflected light of the excitation light also enters a detector, and thus, typically, a filter which cuts the wavelength region of the excitation light is provided in a detector. In such a detector, there is a problem in that the wavelength regions of the excitation light and the fluorescence are overlapped, and thus, the fluorescence of the fluorescent material in the wavelength range in which fluorescence is cut by the filter cannot be detected. To distinguish the fluorescence from the excitation light and to be able to detect only the fluorescence with high sensitivity, it is desirable that the Stokes shift (a difference between the maximum absorption wavelength and the maximum fluorescence wavelength) of the near-infrared fluorescent material be sufficiently great or the fluorescence wavelength range of the material be sufficiently separated from the excitation light.

As the near-infrared fluorescent material, there are an inorganic fluorescent material and an organic fluorescent material. In general, although the inorganic near-infrared fluorescent material has a relatively long Stokes shift, rare earths such as rare earth elements which are expensive because of the rareness and nanoparticles with a uniform particle size are required. On the other hand, since the organic near-infrared fluorescent material can be relatively easily synthesized and the wavelength thereof is easily adjusted, in recent years, various organic near-infrared fluorescent materials have been developed. For example, PTL 4 is disclosed an azo-boron complex compound which exhibits excellent light absorption characteristics in the visible light region and good emission characteristics in the near-infrared region, has excellent light resistance and heat resistance, and is easy to be produced.

In addition, as the organic fluorescent material with a higher emission quantum yield, a boron complex which is a π-conjugated compound is known, and for example, BODIPY pigments having a boron dipyrromethene skeleton, in which a disubstituted boron atom and dipyrromethene (or a derivative thereof) forms a complex are known (for example, refer to NPL 1). As the BODIPY pigments which emits near-infrared fluorescence, in PTL 5, a BODIPY pigment having a heterocycle in a BODIPY skeleton is disclosed.

Furthermore, in NPL 2, a near-infrared fluorescent material which is a DPP-based boron complex having two boron complex units in the molecule, obtained by boron-complexation of a diketopyrrolopyrrole (DPP) derivative, is disclosed. These BODIPY pigments and DPP-based boron complexes are mainly used as a biomarker for labeling biological molecules such as nucleic acids or proteins, tumor tissues, or the like, and there are almost no reports regarding a resin containing BODIPY pigments or DPP-based boron complexes. As the resin composition containing the BODIPY pigments, it is disclosed in PTL 6 that a resin which emits fluoresce in the visible light region is obtained by copolymerizing a siloxane-containing BODIPY pigment introduced an organosiloxanyl group through an alkylene group in a silicone resin. In PTL 7, a composition which emits fluoresce in the visible light region obtained by mixing a BODIPY pigment and a polymer together with a solvent to increase the compatibility of the BODIPY pigment which emits the visible light is disclosed. In PTL 8, an optical filter which contains a BODIPY pigment having at least one electron-withdrawing group and a resin and has a high absorbability of light in the visible light region is disclosed, and in PTL 9, a color conversion material which contains a BODIPY pigment and a resin and converts a low wavelength light into a long wavelength light is disclosed.

In PTL 10, DPP boron complexes are exemplified as a compound which has absorbability in the infrared region and does not have absorbability in the visible light region, and in PTL 11, an infrared absorbing composition including the compound and a hydrophobic polymer is disclosed.

On the other hand, since the light-emitting substance is also used in anti-counterfeiting applications of securities, certificates, credit cards, electronic equipment, and personal authentication media, and to improve anti-counterfeiting effects, a material of a light-emitting substance having higher level of security is required.

### Citation List

### Patent Literature

[PTL 1] Japanese Unexamined Patent Application, First Publication No. 2000-060975
[PTL 2] Published Japanese Translation No. 2008-541987 of the PCT International Publication
[PTL 3] Japanese Unexamined Patent Application, First Publication No. 2012-115535
[PTL 4] Japanese Unexamined Patent Application, First Publication No. 2011-162445
[PTL 5] Japanese Patent No. 5177427
[PTL 6] Japanese Unexamined Patent Application, First Publication No. 2013-060399
[PTL 7] United States Patent Application, Publication No. 2013/0249137
[PTL 8] United States Patent Application, Publication No. 2013/0252000
[PTL 9] Japanese Unexamined Patent Application, First Publication No. 2011-241160
[PTL 10] Japanese Patent No. 5380019
[PTL 11] Japanese Unexamined Patent Application, First Publication No. 2010-090313

### Non-Patent Literature

[NPL 1] Tomimori et al., Tetrahedron, 2011, Vol. 67, pp. 3187-3193.
[NPL 2] Fischer et al., Angewandte Chemie International Edition, 2007, Vol. 46, pp. 3750-3753.

### Summary of Invention

### Technical Problem

In PTL 5, BODIPY pigments which emit near-infrared fluorescence are disclosed, but there is no description regarding whether these can be contained in a resin or not.

The siloxane-containing BODIPY pigment described in PTL 6 has good compatibility with a silicone monomer solution before being cured, and a silicone resin in which a pigment is uniformly dispersed is obtained by curing, but there is a problem in that the compatibility with other resins or resin solutions is low. In the resin composition described in PTL 7, there is a possibility that the solvent remains in the resin, and thus, there is a problem in terms of safety. In addition, in PTLs 6, 7, 8, and 9, there is no description regarding the BODIPY pigment which emits near-infrared fluorescence, and there is also no description regarding application to medical applications. Similarly, in PTLs 10 and 11, there is no description regarding the DPP-based boron complex which emits near-infrared rays, and there is also no report regarding application to medical applications.

A medical tool containing only the near-infrared fluorescent material also does not require large scale equipment, and the load thereof on a living body is small, and thus, the medical tool is expected as a navigation system during an operation, but the sensitivity to detect a position in a deep portion of a living body is not sufficient in some cases. On the other hand, a medical tool containing only an radiopaque substance can detect a deep portion, but the apparatus, the X-ray protection equipment, and the like are large, the medical tool is not easy to be applied to an operation, and there is a problem of exposure. If the medical tool can be visualized by both detection by X-ray radiation and detection by fluorescence or phosphorescence, the medical tool can be used in a wider variety of situations, and thus, the medical tool can be expected to be more useful medical tool for doctors and patients.

In addition, the anti-counterfeiting material using the light-emitting substance has a disadvantage that the anti-counterfeiting level is low, while authenticity can be easily determined by excitation light. If detection by the light-emitting substance and detection by X-rays are combined with the anti-counterfeiting material, it can be expected that the security level increases.

That is, an object of the present invention is to provide a resin composition which can be detected both by X-ray radiation and by light-emission, and a molded article obtained from the resin composition.

### Solution to Problem

A resin composition and a molded article according to the present invention are as described in the following [1] to [18].
[1] A resin composition containing a light-emitting substance, a radiopaque substance, and a resin,
   wherein the light-emitting substance is a near-infrared fluorescent material.
[2] The resin composition according to [1], in which the near-infrared fluorescent material is one or more compounds selected from the group consisting of compounds represented by the following General Formula (II₁) [In Formula (II₁), R^{a} and R^{b} form an aromatic 5-membered ring, an aromatic 6-membered ring, or a condensed aromatic ring formed by condensation of two or three 5-membered rings or 6-membered rings together with the nitrogen atom to which R^{a} is bonded and the carbon atom to which R^{b} is bonded; R^{c} and R^{d} form an aromatic 5-membered ring, an aromatic 6-membered ring, or a condensed aromatic ring formed by condensation of two or three 5-membered rings or 6-membered rings together with the nitrogen atom to which R^{c} is bonded and the carbon atom to which R^{d} is bonded; each of R^{e} and R^{f} represents a halogen atom or an oxygen atom; R^{g} represents a hydrogen atom or an electron-withdrawing group. Here, in a case where R^{e} and R^{f} are oxygen atoms, R^{e}, the boron atom bonded to R^{e}, R^{a}, and the nitrogen atom bonded to R^{a} may together form a ring, and R^{f}, the boron atom bonded to R^{f}, R^{c}, and the nitrogen atom bonded to R^{c} may together form a ring. In a case where R^{e} is an oxygen atom and does not form a ring, R^{e} is an oxygen atom having a substituent, and in a case where R^{f} is an oxygen atom and does not form a ring, R^{f} is an oxygen atom having a substituent.], compounds represented by the following General Formula (II₂) [In Formula (II₂), each of R^{a} to R^{f} is the same as that in Formula (II₁).], compounds represented by the following General Formula (II₃) [In Formula (II₃), R^{h} and R' form an aromatic 5-membered ring, an aromatic 6-membered ring, or a condensed aromatic ring formed by condensation of two or three 5-membered rings or 6-membered rings together with the nitrogen atom to which R^{h} is bonded and the carbon atom to which Rⁱ is bonded; R^{j} and R^{k} form an aromatic 5-membered ring, an aromatic 6-membered ring, or a condensed aromatic ring formed by condensation of two or three 5-membered rings or 6-membered rings together with the nitrogen atom to which R^{j} is bonded and the carbon atom to which R^{k} is bonded; each of R^{l}, R^{m}, Rⁿ, and R^{o} independently represents a halogen atom, a C₁₋₂₀ alkyl group, a C₁₋₂₀ alkoxy group, an aryl group, or a heteroaryl group; each of R^{p} and R^{q} independently represents a hydrogen atom, a halogen atom, a C₁₋₂₀ alkyl group, a C₁₋₂₀ alkoxy group, an aryl group, or a heteroaryl group; and each of R^{r} and R^{s} independently represents a hydrogen atom or an electron-withdrawing group.], or compounds represented by the following General Formula (II₄)
   [In Formula (II₄), each of R^{h} to R^{q} is the same as that in Formula (II₃).] and has a maximum fluorescence wavelength of 650 nm or longer. 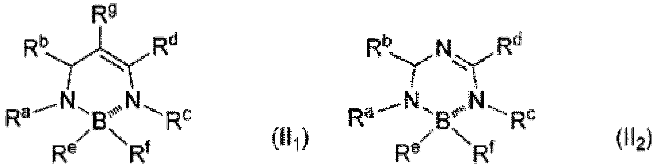 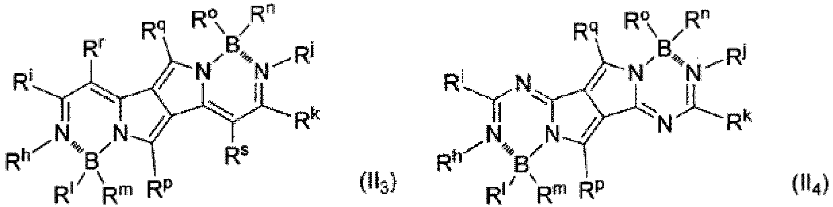
[3] The resin composition according to [2], containing one or more compounds selected from the group consisting of compounds represented by the following General Formula (II₁-0) [In Formula (II₁-0), (p1) each of R¹⁰¹, R¹⁰², and R¹⁰³ independently represents a hydrogen atom, a halogen atom, a C₁₋₂₀ alkyl group, a C₁₋₂₀ alkoxy group, an aryl group, or a heteroaryl group, (p2) R¹⁰¹ and R¹⁰² together form an aromatic 5-membered ring or an aromatic 6-membered ring, and R¹⁰³ represents a hydrogen atom, a halogen atom, a C₁₋₂₀ alkyl group, a C₁₋₂₀ alkoxy group, an aryl group, or a heteroaryl group, or (p3) R¹⁰² and R¹⁰³ together form an aromatic 5-membered ring or an aromatic 6-membered ring, and R¹⁰¹ represents a hydrogen atom, a halogen atom, a C₁₋₂₀ alkyl group, a C₁₋₂₀ alkoxy group, an aryl group, or a heteroaryl group, and (q1) each of R¹⁰⁴, R¹⁰⁵, and R¹⁰⁶ independently represents a hydrogen atom, a halogen atom, a C₁₋₂₀ alkyl group, a C₁₋₂₀ alkoxy group, an aryl group, or a heteroaryl group, (q2) R¹⁰⁴ and R¹⁰⁵ together form an aromatic 5-membered ring or an aromatic 6-membered ring, and R¹⁰⁶ represents a hydrogen atom, a halogen atom, a C₁₋₂₀ alkyl group, a C₁₋₂₀ alkoxy group, an aryl group, or a heteroaryl group, or (q3) R¹⁰⁵ and R¹⁰⁶ together form an aromatic 5-membered ring or an aromatic 6-membered ring, and R¹⁰⁴ represents a hydrogen atom, a halogen atom, a C₁₋₂₀ alkyl group, a C₁₋₂₀ alkoxy group, an aryl group, or a heteroaryl group. Each of R¹⁰⁷ and R¹⁰⁸ represents a halogen atom or an oxygen atom; R¹⁰⁹ represents a hydrogen atom or an electron-withdrawing group. Here, in a case where R¹⁰⁷ and R¹⁰⁸ are oxygen atoms, R¹⁰⁷, the boron atom bonded to R¹⁰⁷, the nitrogen atom bonded to the boron atom, R¹⁰¹, and the carbon atom bonded to R¹⁰¹may together form a ring, and R¹⁰⁸, the boron atom bonded to R¹⁰⁸, the nitrogen atom bonded to the boron atom, R¹⁰⁴, and the carbon atom bonded to R¹⁰⁴ may together form a ring. In a case where R¹⁰⁷ is an oxygen atom and does not form a ring, R¹⁰⁷ is an oxygen atom having a substituent, and in a case where R¹⁰⁸ is an oxygen atom and does not form a ring, R¹⁰⁸ is an oxygen atom having a substituent.] and compounds represented by the following General Formula (II₂-0) [In Formula (II₂-0), each of R¹⁰¹ to R¹⁰⁸ is the same as that in Formula (II₁-0).]. 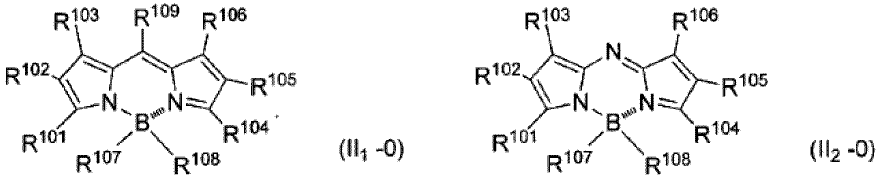
[4] The resin composition according to [3], in which, in General Formula (II₁₋₀) or (II₂₋₀), R¹⁰¹ and R¹⁰² form a ring, and R¹⁰⁴ and R¹⁰⁵ form a ring, or R¹⁰² and R¹⁰³ form a ring, and R¹⁰⁵ and R¹⁰⁶ form a ring, and the ring is represented by any one of the following General Formulas (C-1) to (C-9) 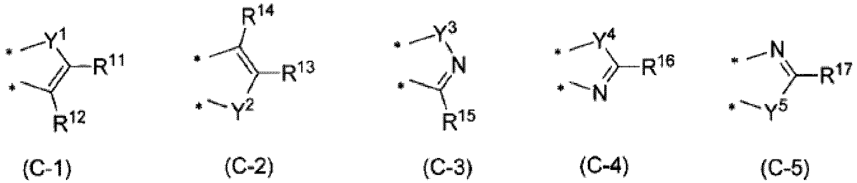 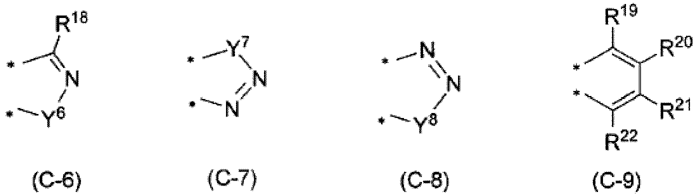
   [In Formulas (C-1) to (C-9), each of Y¹ to Y⁸ independently represents a sulfur atom, an oxygen atom, a nitrogen atom, or a phosphorus atom, and each of R¹¹ to R²² independently represents a hydrogen atom or any group which does not inhibit fluorescence of the compound.]
[5] The resin composition according to [2], containing one or more compounds selected from the group consisting of compounds represented by any one of the following General Formulas (II₁-1-1) to (II₁-1-6), (II₁-2-1) to (II₁-2-12), (II₂-1-1) to (II₂-1-6), and (II₂-2-1) to (II₂-2-12) 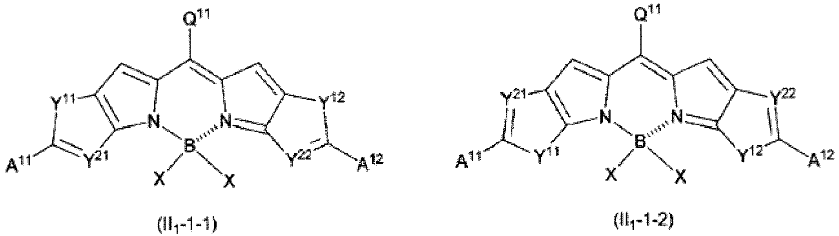 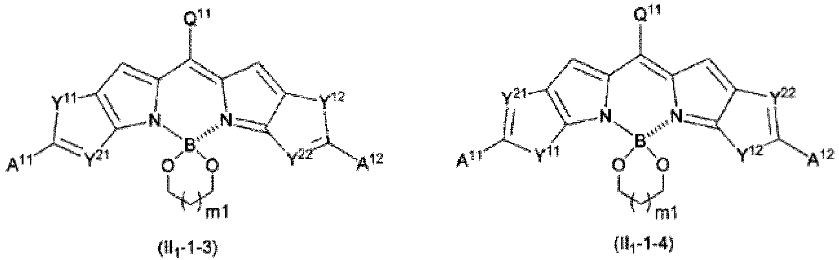 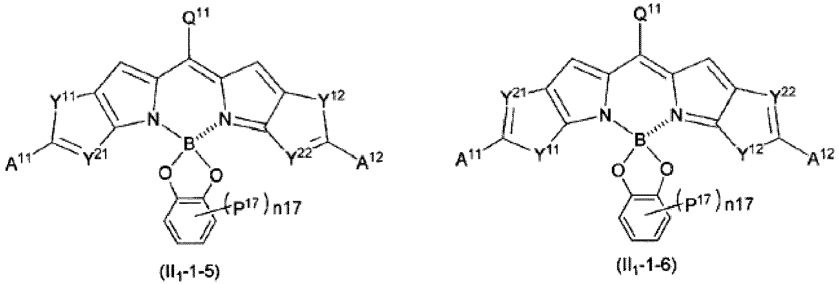 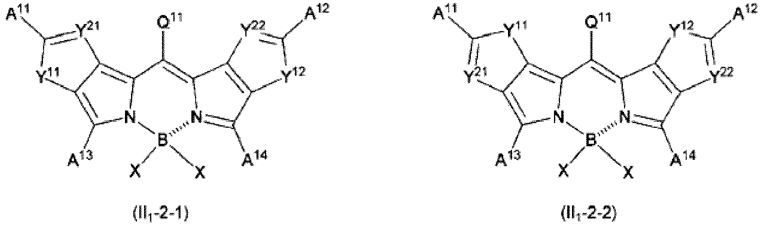 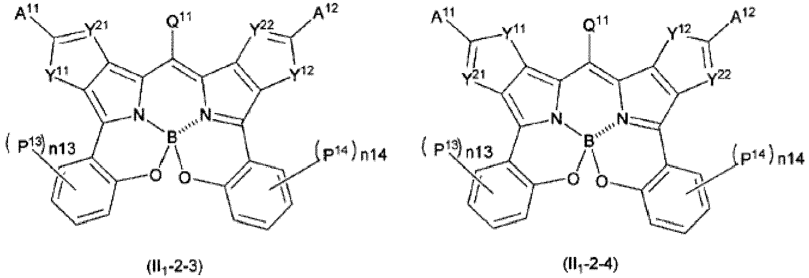 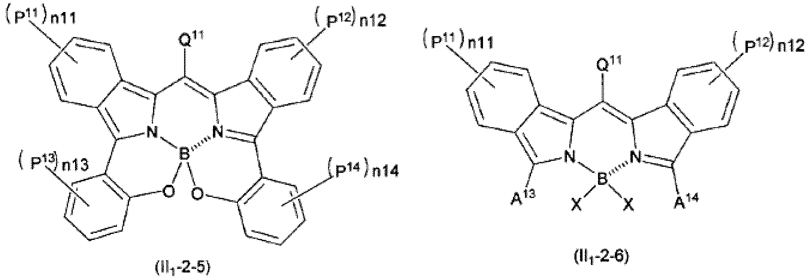 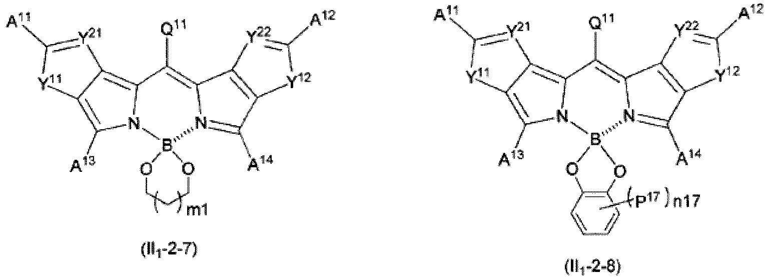 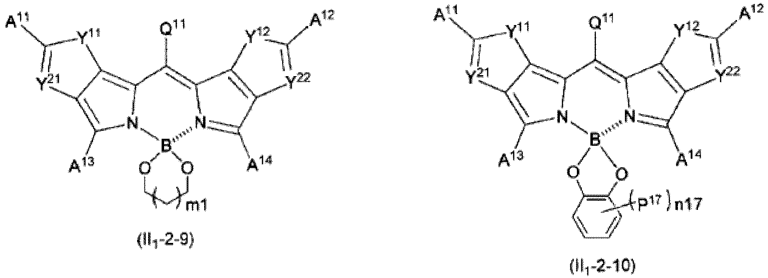 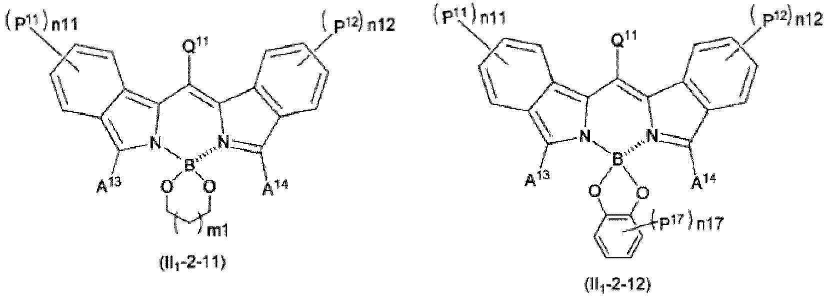 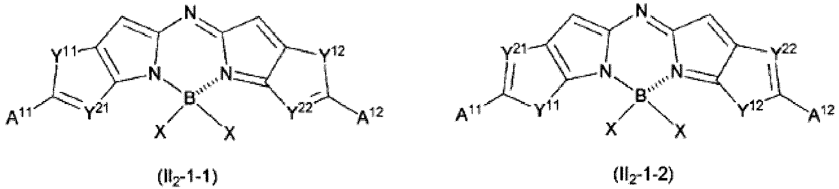 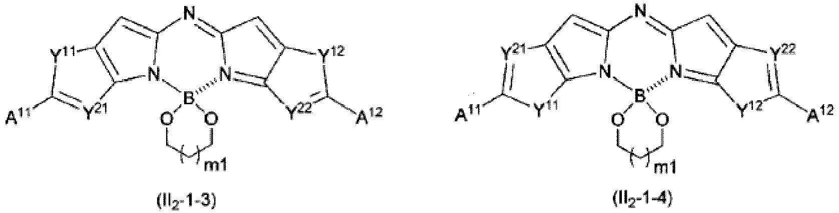 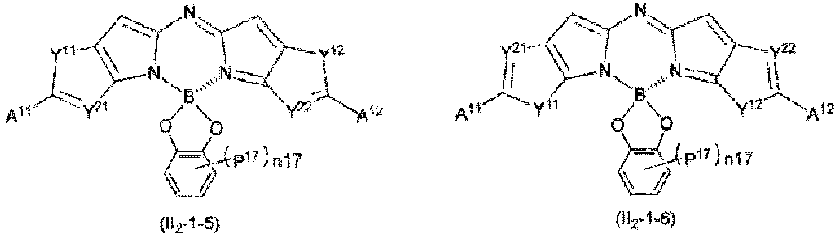 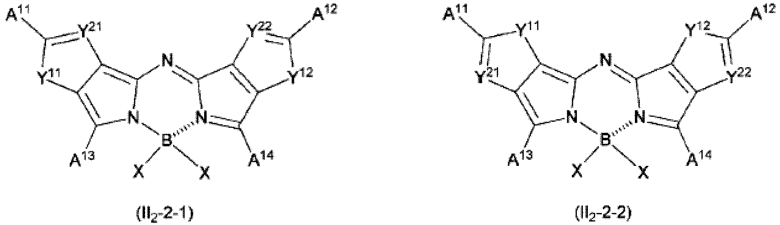 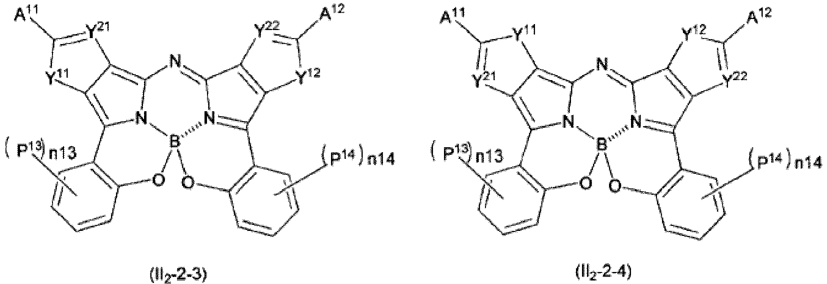 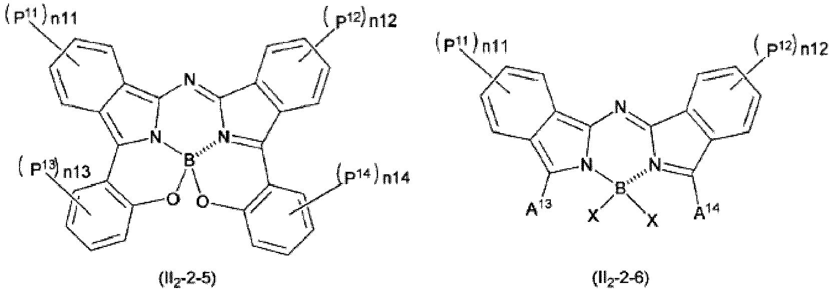 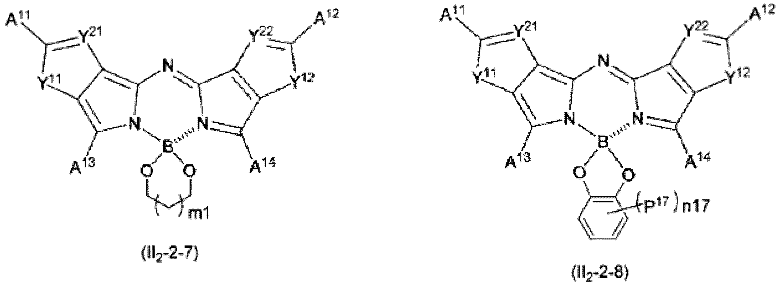 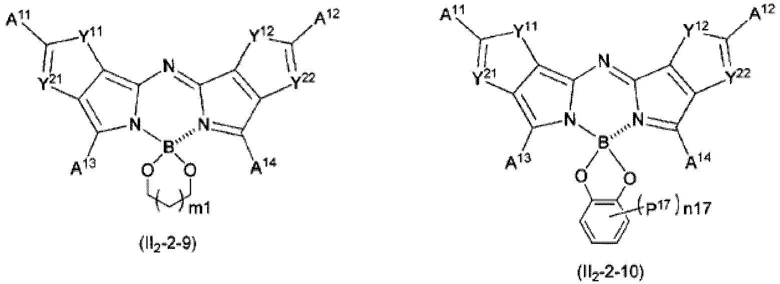 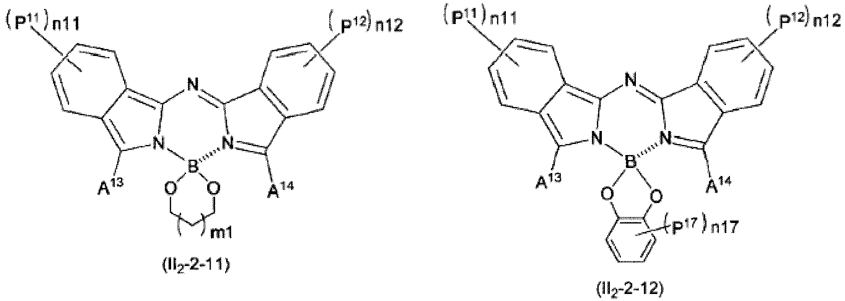
   [In the formulas, each of Y¹¹ and Y¹² independently represents an oxygen atom or a sulfur atom; each of Y²¹ and Y²² independently represents a carbon atom or a nitrogen atom; Q¹¹ represents a hydrogen atom or an electron-withdrawing group; each of Xs independently represents a halogen atom, a C₁₋₂₀ alkoxy group, an aryloxy group, or an acyloxy group; each of P¹¹ to P¹⁴ and P¹⁷ independently represents a halogen atom, a C₁₋₂₀ alkyl group, a C₁₋₂₀ alkoxy group, an amino group, a monoalkylamino group, or a dialkylamino group; each of A¹¹ to A¹⁴ independently represents a phenyl group which may have one to three substituents selected from the group consisting of a halogen atom, a C₁₋₂₀ alkyl group, a C₁₋₂₀ alkoxy group, an amino group, a monoalkylamino group, and a dialkylamino group, or a heteroaryl group which may have one to three substituents selected from the group consisting of a halogen atom, a C₁₋₂₀ alkyl group, a C₁₋₂₀ alkoxy group, an amino group, a monoalkylamino group, and a dialkylamino group; each of n11 to n14 and n17 independently represents an integer of 0 to 3; and m1 represents 0 or 1.]
[6] The resin composition according to [2], containing one or more compounds selected from the group consisting of compounds represented by any one of the following General Formulas (II₃₋₇) to (II₃₋₉) and (II₄-7) to (II₄-9). 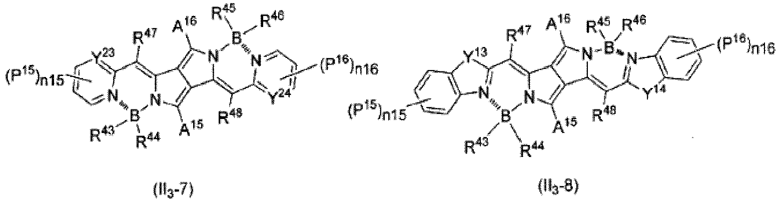 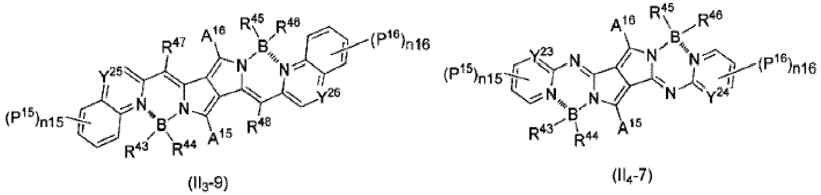 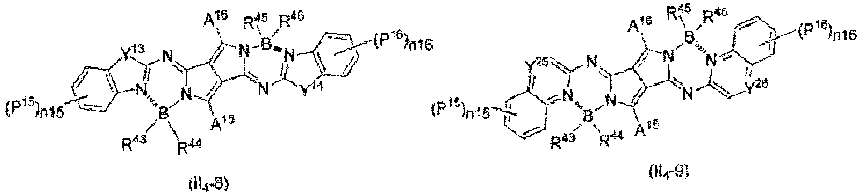
   [In the formulas, each of Y²³ and Y²⁴ independently represents a carbon atom or a nitrogen atom; each of Y¹³ and Y¹⁴ independently represents an oxygen atom or a sulfur atom; each of Y²⁵ and Y²⁶ independently represents a carbon atom or a nitrogen atom; each of R⁴⁷ and R⁴⁸ independently represents a hydrogen atom or an electron-withdrawing group; each of R⁴³, R⁴⁴, R⁴⁵, and R⁴⁶ represents a halogen atom or an aryl group which may have a substituent; each of P¹⁵ and P¹⁶ independently represents a halogen atom, a C₁₋₂₀ alkyl group, a C₁₋₂₀ alkoxy group, an amino group, a monoalkylamino group, or a dialkylamino group; each of n15 and n16 independently represents an integer of 0 to 3; and each of A¹⁵ and A¹⁶ independently represents a phenyl group which may have one to three substituents selected from the group consisting of a hydrogen atom, a halogen atom, a C₁₋₂₀ alkyl group, a C₁₋₂₀ alkoxy group, an amino group, a monoalkylamino group, or a dialkylamino group.]
[7] The resin composition according to [2], containing one or more compounds selected from the group consisting of compounds represented by any one of the following General Formulas (II₃-1) to (II₃-6) and (II₄-1) to (II₄-6).

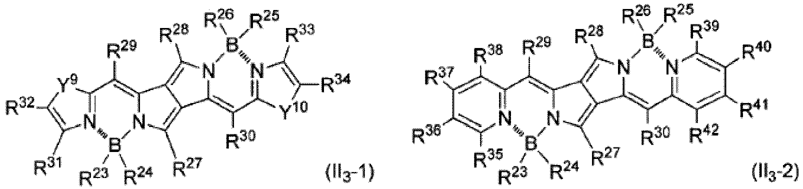

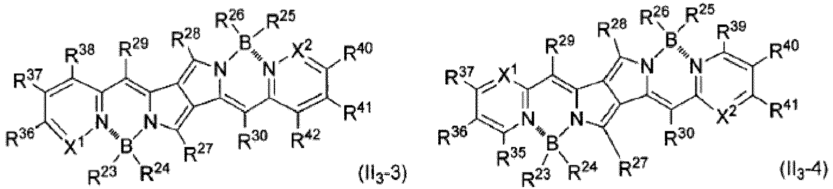

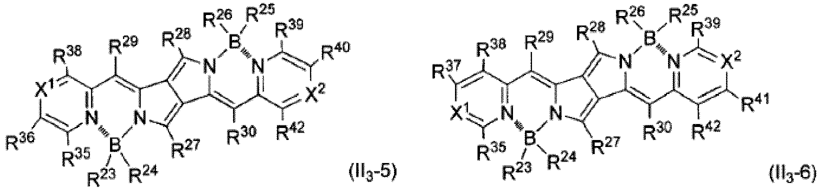

[In Formula (II₃-1), each of R²³, R²⁴, R²⁵, and R²⁶ independently represents a halogen atom, a C₁₋₂₀ alkyl group, a C₁₋₂₀ alkoxy group, an aryl group, or a heteroaryl group; each of R²⁷ and R²⁸ independently represents a hydrogen atom, a halogen atom, a C₁₋₂₀ alkyl group, a C₁₋₂₀ alkoxy group, an aryl group, or a heteroaryl group; each of R²⁹ and R³⁰ independently represents a hydrogen atom or an electron-withdrawing group; each of Y⁹ and Y¹⁰ independently represents a sulfur atom, an oxygen atom, a nitrogen atom, or a phosphorus atom; (p4) each of R³¹ and R³² independently represents a hydrogen atom, a halogen atom, a C₁₋₂₀ alkyl group, a C₁₋₂₀ alkoxy group, an aryl group, or a heteroaryl group, or (p5) R³¹ and R³² together form an aromatic 5-membered ring which may have a substituent or an aromatic 6-membered ring which may have a substituent; and (q4) each of R³³ and R³⁴ independently represents a hydrogen atom, a halogen atom, a C₁₋₂₀ alkyl group, a C₁₋₂₀ alkoxy group, an aryl group, or a heteroaryl group, or (q5) R³³ and R³⁴ together form an aromatic 5-membered ring which may have a substituent or an aromatic 6-membered ring which may have a substituent.]

[In Formulas (II₃-2) to (II₃-6), each of R²³ to R³⁰ is the same as that in Formula (II₃-1); each of X¹ and X² independently represents a nitrogen atom or a phosphorus atom; (p6) each of R³⁵, R³⁶, R³⁷, and R³⁸ independently represents a hydrogen atom, a halogen atom, a C₁₋₂₀ alkyl group, a C₁₋₂₀ alkoxy group, an aryl group, or a heteroaryl group, (p7) R³⁵ and R³⁶ together form an aromatic 5-membered ring which may have a substituent or an aromatic 6-membered ring which may have a substituent, and each of R³⁷ and R³⁸ independently represents a hydrogen atom, a halogen atom, a C₁₋₂₀ alkyl group, a C₁₋₂₀ alkoxy group, an aryl group, or a heteroaryl group, (p8) R³⁶ and R³⁷ together form an aromatic 5-membered ring which may have a substituent or an aromatic 6-membered ring which may have a substituent, and each of R³⁵ and R³⁸ independently represents a hydrogen atom, a halogen atom, a C₁₋₂₀ alkyl group, a C₁₋₂₀ alkoxy group, an aryl group, or a heteroaryl group, or (p9) R³⁷ and R³⁸ together form an aromatic 5-membered ring which may have a substituent or an aromatic 6-membered ring which may have a substituent, and each of R³⁵ and R³⁶ independently represents a hydrogen atom, a halogen atom, a C₁₋₂₀ alkyl group, a C₁₋₂₀ alkoxy group, an aryl group, or a heteroaryl group; and (q6) each of R³⁹, R⁴⁰, R⁴¹, and R⁴² independently represents a hydrogen atom, a halogen atom, a C₁₋₂₀ alkyl group, a C₁₋₂₀ alkoxy group, an aryl group, or a heteroaryl group, (q7) R³⁹ and R⁴⁰ together form an aromatic 5-membered ring which may have a substituent or an aromatic 6-membered ring which may have a substituent, and each of R⁴¹ and R⁴² independently represents a hydrogen atom, a halogen atom, a C₁₋₂₀ alkyl group, a C₁₋₂₀ alkoxy group, an aryl group, or a heteroaryl group, (q8) R⁴⁰ and R⁴¹ together form an aromatic 5-membered ring which may have a substituent or an aromatic 6-membered ring which may have a substituent, and each of R³⁹ and R⁴² independently represents a hydrogen atom, a halogen atom, a C₁₋₂₀ alkyl group, a C₁₋₂₀ alkoxy group, an aryl group, or a heteroaryl group, or (q9) R⁴¹ and R⁴² together form an aromatic 5-membered ring which may have a substituent or an aromatic 6-membered ring which may have a substituent, and each of R³⁹ and R⁴⁰ independently represents a hydrogen atom, a halogen atom, a C₁₋₂₀ alkyl group, a C₁₋₂₀ alkoxy group, an aryl group, or a heteroaryl group.]

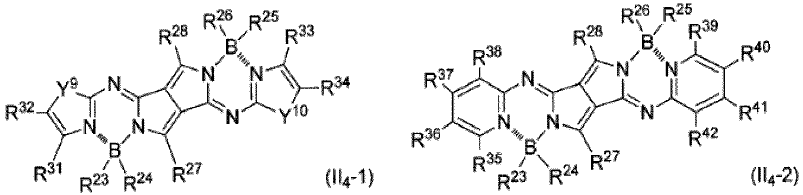

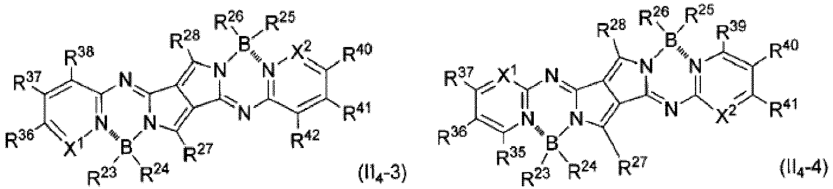

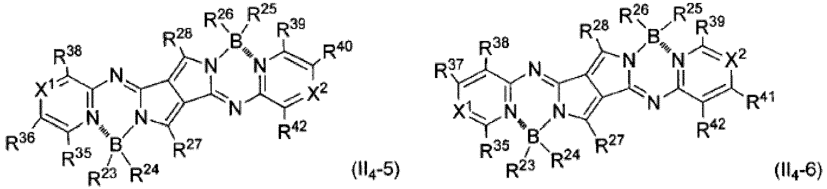

[In Formulas (II₄-1) to (II₄-6), each of R²³ to R²⁸ is the same as that in Formula (II₃-1), and in Formula (II₄-1), each of R³¹ to R³⁴, Y⁹, and Y¹⁰ is the same as that in Formula (II₃-1), in Formulas (II₄-2) to (II₄-6), each of R³⁵ to R⁴² is the same as that in Formula (II₃-2), and in Formulas (II₄-3) to (II₄-6), each of X¹ and X² is the same as that in Formula (II₃-3).]
[8] The resin composition according to [1], in which the infrared fluorescent material is formed of an azo-boron complex compound represented by the following Formula (I) and has a maximum absorption wavelength of 650 nm or longer and a Stokes shift of 50 nm or longer. 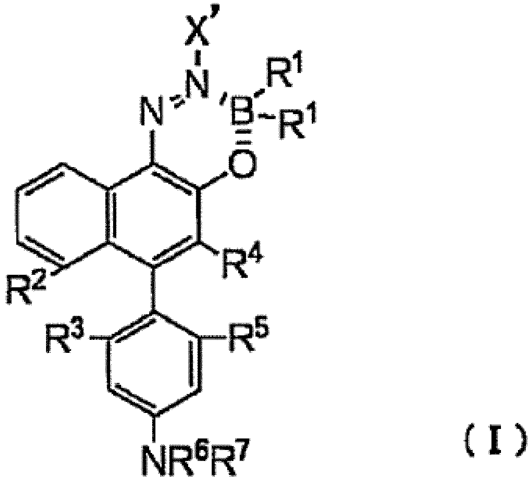
   [In Formula (I), X' represents an aryl group which may have a substituent or a heteroaryl group which may have a substituent; R¹ represents a C₁₋₁₂ alkyl group, an aryl group, an aryl ethenyl group, an aryl ethynyl group, a C₁₋₁₂ alkoxy group, an aryloxy group, or a halogen atom, or one of R¹s represents an -O-C(=O)- group which is also bonded to X', and forms a 6-membered ring, and the other R¹ independently represents a C₁₋₁₂ alkyl group, an aryl group, an aryl ethenyl group, an aryl ethynyl group, a C₁₋₁₂ alkoxy group, an aryloxy group, or a halogen atom; R² and R³ together form an -O-group, an -S- group, or an -N(R⁸)- group (here, R⁸ represents a hydrogen atom or a C₁₋₁₂ alkyl group), and each of R⁴ and R⁵ represents a hydrogen atom, or R⁴ and R⁵ together form an -O- group, an -S- group, or an -N(R⁸)- group (R⁸ has the same meaning as that described above), and each of R² and R³ represents a hydrogen atom; each of R⁶ and R⁷ independently represents a hydrogen atom, a C₁₋₁₂ alkyl group, an aryl group which may have a substituent, or a heteroaryl group which may have a substituent; and the substituent of the aryl group or the heteroaryl group represents one or more groups selected from the group consisting of a C₁₋₁₂ alkyl group, a mono (C₁₋₁₂ alkyl)amino group, a di (C₁₋₁₂ alkyl)amino group, a hydroxyl group, and a C₁₋₁₂ alkoxy group.]
[9] The resin composition according to [8], in which the azo-boron complex compound is represented by the following Formula (I₁) [in Formula (I₁), Y represents an aryl group which may have a substituent or a heteroaryl group which may have a substituent, and each of R¹ to R⁷ has the same meaning as each of R¹ to R⁷ in Formula (I)]. 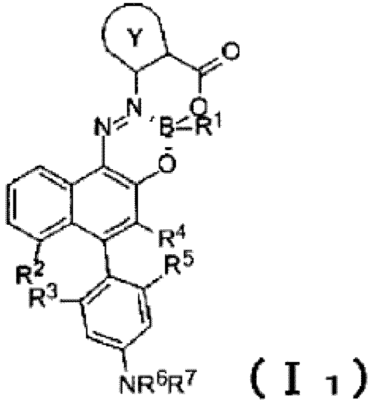
[10] The resin composition according to any one of [1] to [9], in which the radiopaque substance is one or more selected from the group consisting of barium sulfate, bismuth oxide, bismuth subcarbonate, calcium carbonate, aluminum hydroxide, tungsten, zinc oxide, zirconium oxide, zirconium, titanium, platinum, bismuth subnitrate, and bismuth.
[11] The resin composition according to any one of [1] to [10], in which the content of the radiopaque substance is 5% by mass to 50% by mass.
[12] The resin composition according to any one of [1] to [11], in which the content of the light-emitting substance is 0.001% by mass to 0.5% by mass.
[13] The resin composition according to any one of [1] to [11], in which the content of the light-emitting substance is 0.001 % by mass to 0.05% by mass.
[14] The resin composition according to any one of [1] to [13], in which the resin is a thermoplastic resin.
[15] The resin composition according to any one of [1] to [14], in which the resin is one or more selected from the group consisting of a urethane-based resin, an olefin-based resin, a polystyrene-based resin, a polyester-based resin, and a vinyl chloride-based resin.
[16] The resin composition according to any one of [1] to [15], which is used as a medical material.
[17] A molded article obtained by processing the resin composition according to any one of [1] to [16].
[18] The molded article according to [17], in which the article is a medical tool and of which at least a part is used in the body of a patient.

### Advantageous Effects of Invention

Since the resin composition according to the present invention and a molded article obtained from the composition have opaqueness to radiation and contain a light-emitting substance, both of detection by X-ray radiation and detection by light-emission are possible. In addition, since the resin composition according to the present invention has stronger emission intensity in the excitation light source direction than that of a resin composition not containing the radiopaque substance, it is possible to sensitively detect light emission even by weaker excitation light.

Therefore, the molded article obtained from the resin composition of the present invention is particularly suitable as a medical tool or a member thereof used in vivo, and, in addition, is also preferable for security applications such as an identification marker for so-called anti-counterfeiting.

### Brief Description of Drawings

FIG. 1 is a schematic diagram (a front view, a rear view, and a side view) of a film (1) partially shielded with aluminum foil (2), manufactured in Test Reference Example 1.
FIG. 2 is a graph showing emission spectra of a film obtained by partially shielding a film manufactured in Reference Example 1 and a film obtained by partially shielding a film manufactured in Comparative Example 1.
FIG. 3 is a graph showing spectra at an excitation wavelength of 740 nm of films manufactured in Example 5 and Comparative Example 5, in Test Example 6.
FIG. 4 is a photograph of films manufactured in Example 6 and Comparative Example 6 taken using a near-infrared imaging camera, in Test Example 7.
FIG. 5 is a graph showing spectra at an excitation wavelength of 740 nm of films manufactured in Example 8 and Comparative Example 7, in Test Example 8.
FIG. 6 is a photograph of the films manufactured in Example 8 and Comparative Example 7 taken using a near-infrared imaging camera, in Test Example 8.
FIG. 7 is a graph showing spectra at an excitation wavelength of 740 nm of films manufactured in Example 17, Example 18, and Comparative Example 7, in Test Example 9.
FIG. 8 is a graph showing spectra at an excitation wavelength of 740 nm of films manufactured in Example 19 and Comparative Example 8, in Test Example 10.
FIG. 9A is a photograph of the film manufactured in Example 8 over a piece of pork having a thickness of 15 mm taken using a near-infrared imaging camera without irradiation with light, in Test Example 11.
FIG. 9B is a photograph of the film manufactured in Example 8 over a piece of pork having a thickness of 2 mm taken using a near-infrared imaging camera, while being irradiated with excitation light having a center wavelength of 740 nm, in Test Example 11.
FIG. 9C is a photograph of the film manufactured in Example 8 over a piece of pork having a thickness of 15 mm taken using a near-infrared imaging camera, while being irradiated with excitation light having a center wavelength of 740 nm, in Test Example 11.

### Description of Embodiments

### <Light-emitting substance>

The light-emitting substance contained in the resin composition according to the present invention can be suitably selected and used in consideration of product quality required for a molded article obtained from the resin composition, the type of resin component to be mixed, or the like. The light-emitting substance is a fluorescent material. The fluorescent material is a fluorescent material of which the fluorescence maximum wavelength is in the near-infrared region (near-infrared fluorescent material). The light-emitting substance may be an inorganic substance or an organic substance.

Examples of the near-infrared fluorescent material include compounds such as a polymethine-based pigment, an anthraquinone-based pigment, a dithiol metal salt-based pigment, a cyanine-based pigment, a phthalocyanine-based pigment, an indophenol-based pigment, a cyamine-based pigment, a styryl-based pigment, an aluminum-based pigment, a diimonium-based pigment, an azo-based pigment, an azo-boron-based pigment, a boron dipyrromethene (BODIPY)-based pigment described in PCT International Publication No. WO2007/126052 or the like, a squarylium-based pigment, and a perylene-based pigment.

For example, in a case where the resin composition according to the present invention is used as a material for a medical tool used in vivo or a security device, the resin composition preferably contains a near-infrared fluorescent material. Since the resin composition containing the near-infrared fluorescent material and a molded article obtained from this is excited by invisible light in a near-infrared region and can be detected, excitation light and light emission can be detected without change in the color of biological tissues.

As the near-infrared fluorescent material contained in the resin composition according to the present invention, among the above-described materials, a cyanine-based pigment, an azo-boron-based pigment, a boron dipyrromethene (BODIPY)-based pigment, a diketopyrrolopyrrole (DPP)-based boron complex, a phthalocyanine-based pigment, or a squarylium-based pigment is preferable from the viewpoint of light-emitting efficiency, and an azo-boron complex compound represented by the following General Formula (I), a BODIPY pigment represented by the following General Formula (II₁) or (II₂), or a DPP-based boron complex represented by the following General Formula (II₃) or the following General Formula (II₄) is particularly preferable from the viewpoint of heat resistance. In a case where the light-emitting efficiency is low, there is a possibility that no sufficient emission intensity is obtained, and in a case where the heat resistance is low, there is a possibility that the materials are decomposed when kneaded with a resin.

### <Azo-Boron Complex Compound Represented by General Formula (I)>

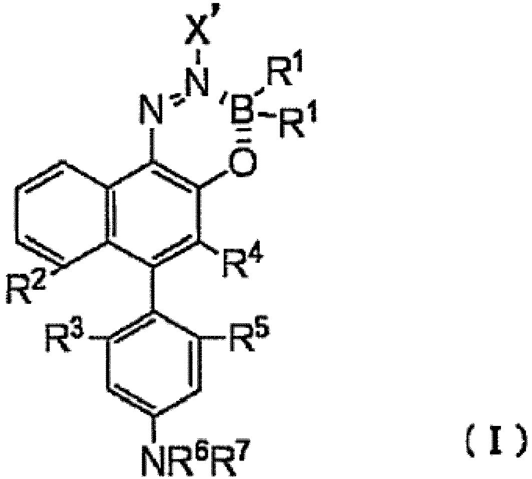

[In Formula (I), X' represents an aryl group which may have a substituent or a heteroaryl group which may have a substituent; R¹ represents a C₁₋₁₂ alkyl group, an aryl group, an aryl ethenyl group, an aryl ethynyl group, a C₁₋₁₂ alkoxy group, an aryloxy group, or a halogen atom, or one of R¹s represents an -O-C(=O)- group which is also bonded to X', and forms a 6-membered ring, and the other R¹ independently represents a C₁₋₁₂ alkyl group, an aryl group, an aryl ethenyl group, an aryl ethynyl group, a C₁₋₁₂ alkoxy group, an aryloxy group, or a halogen atom; R² and R³ together form an -O-group, an -S- group, or an -N(R⁸)- group (here, R⁸ represents a hydrogen atom or a C₁₋₁₂ alkyl group), and each of R⁴ and R⁵ represents a hydrogen atom, or R⁴ and R⁵ together form an -O- group, an -S- group, or an -N(R⁸)- group (R⁸ has the same meaning as that described above), and each of R² and R³ represents a hydrogen atom; each of R₆ and R₇ independently represents a hydrogen atom, a C₁₋₁₂ alkyl group, an aryl group which may have a substituent, or a heteroaryl group which may have a substituent; and the substituent of the aryl group or the heteroaryl group represents one or more groups selected from the group consisting of a C₁₋₁₂ alkyl group, a mono (C₁₋₁₂ alkyl)amino group, a di (C₁₋₁₂ alkyl)amino group, a hydroxyl group, and a C₁₋₁₂ alkoxy group.]

In the present invention, the "aryl group" means an aromatic hydrocarbon group. Examples thereof include a phenyl group, a naphthyl group, an indenyl group, and a biphenyl group, and a C₆₋₁₀ aryl group is preferable, and a phenyl group is more preferable.

The "heteroaryl group" means an aromatic heterocyclyl group having a 5-membered ring, a 6-membered ring, or a condensed ring having at least one heteroatom such as a nitrogen atom, an oxygen atom, or a sulfur atom. Examples of the "heteroaryl group" include 5-membered ring heteroaryl groups such as a pyrrolyl group, an imidazolyl group, a pyrazolyl group, a thienyl group, a furanyl group, an oxazolyl group, an isoxazolyl group, a thiazolyl group, an isothiazolyl group, and a thiadiazole group; 6-membered ring heteroaryl groups such as a pyridinyl group, a pyrazinyl group, a pyrimidinyl group, and a pyridazinyl group; and condensed heteroaryl groups such as an indolyl group, an isoindolyl group, an indazolyl group, a quinolizinyl group, a quinolinyl group, an isoquinolinyl group, a benzofuranyl group, an isobenzofuranyl group, a chromenyl group, a benzoxazolyl group, a benzisoxazolyl group, a benzothiazolyl group, and a benzisothiazolyl group. The heteroaryl group is preferably a heteroaryl group including a nitrogen atom, and more preferably a benzothiazolyl group.

The "C₁₋₁₂ alkyl group" means a linear or branched monovalent aliphatic hydrocarbon group having 1 to 12 carbon atoms. Examples thereof include a methyl group, an ethyl group, a propyl group, an isopropyl group, an n-butyl group, an isobutyl group, a t-butyl group, a pentyl group, an isoamyl group, a hexyl group, a heptyl group, an octyl group, a nonanyl group, a decyl group, a undecyl group, and a dodecyl group. Each of R⁶ and R⁷ is preferably a C₂₋₁₂ alkyl group, more preferably a C₂₋₁₀ alkyl group, and particularly preferably an n-C₂₋₈ alkyl group. In other cases, a C₁₋₆ alkyl group is preferable, a C₁₋₄ alkyl group is more preferable, a C₁₋₂ alkyl group is more preferable, and a methyl group is more preferable.

The "aryl ethenyl group" represents a -CH=CH- group with which the aryl group is substituted, and may be a trans type or a cis type, and the cis type is preferable from the viewpoint of stability. In addition, the "aryl ethenyl group" represents a -C≡C-group with which the aryl group is substituted.

The "C₁₋₁₂ alkoxy group" means a C₁₋₁₂ alkyloxy group, and is preferably a C₁₋₆ alkoxy group, more preferably a C₁₋₄ alkoxy group, still more preferably a C₁₋₂ alkoxy group, and still more preferably a methoxy group. In addition, in the azo-boron complex compound used in the present invention, in a case where two R¹s are alkoxy groups, the hydrocarbon groups may be bonded to each other to form a ring structure together with the boron atom.

Examples of the "halogen atom" include a fluorine atom, a chlorine atom, a bromine atom, and an iodine atom, and a fluorine atom, a chlorine atom, or a bromine atom is preferable, and a fluorine atom is more preferable.

The "mono (C₁₋₁₂ alkyl)amino group" means an amino group with which one C₁₋₁₂ alkyl group described above is substituted, and examples thereof include a methylamino group, an ethylamino group, a propylamino group, an isopropylamino group, a butylamino group, an isobutyl amino group, a t-butylamino group, a pentylamino group, and a hexylamino group, and the mono (C₁₋₁₂ alkyl)amino group is preferably a mono C₁₋₆ alkylamino group, more preferably a mono C₁₋₄ alkylamino group, and still more preferably a mono C₁₋₂ alkylamino group.

The "di (C₁₋₁₂ alkyl) amino group" means an amino group with which two C₁₋₁₂ alkyl groups described above are substituted. In the group, two alkyl groups may be the same as or different from each other. Examples of the di C₁₋₁₂ alkylamino group include a dimethylamino group, a diethylamino group, a dipropylamino group, a diisopropylamino group, a dibutylamino group, a diisobutyl amino group, a dipentylamino group, a dihexylamino group, an ethylmethylamino group, a methylpropylamino group, a butylmethylamino group, an ethylpropylamino group, and a butylethylamino group, and the di C₁₋₁₂ alkylamino group is preferably a di (C₁₋₆ alkyl)amino group, more preferably a di (C₁₋₄ alkyl)amino group, and still more preferably a di (C₁₋₂ alkyl)amino group.

As the azo-boron complex compound (I) used in the present invention, a compound in which one of R¹s represents an -O-C(=O)- group which is also bonded to X', and forms a 6-membered ring, and the other R¹ independently represents a C₁₋₁₂ alkyl group, an aryl group, an aryl ethenyl group, an aryl ethynyl group, a C₁₋₁₂ alkoxy group, an aryloxy group, or a halogen atom, or compounds represented by the following Formula (I₁) to (I₃) are suitable. Among these, the compound represented by Formula (I₁) is more preferable. In Formula (I₁), Y represents an aryl group which may have a substituent or a heteroaryl group which may have a substituent, and each of R¹ to R⁷ has the same meaning as each of R¹ to R⁷ in Formula (I). In addition, in Formulas (I₂) and (I₃), each of X' and R¹ to R⁷ has the same meaning as each of X' and R¹ to R⁷ in Formula (I).

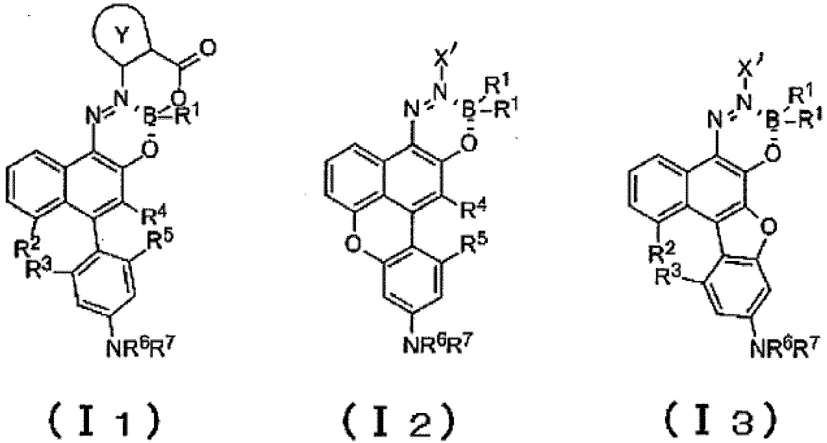

Moreover, the azo-boron complex compound represented by Formula (I) can be synthesized by reacting a boron compound with a hydrazone compound (II) represented by the following Formula (II) (for example, refer to PTL 2). In the following formulas, each of X' and R¹ to R⁷ has the same meaning as each of X' and R¹ to R⁷ in Formula (I). In addition, R⁹ represents a C₁₋₁₂ alkyl group, an aryl group, an aryl ethenyl group, an aryl ethynyl group, a C₁₋₁₂ alkoxy group, an aryloxy group, or a halogen atom, and represents the same group as R¹ or a group which is more easily left than R¹.

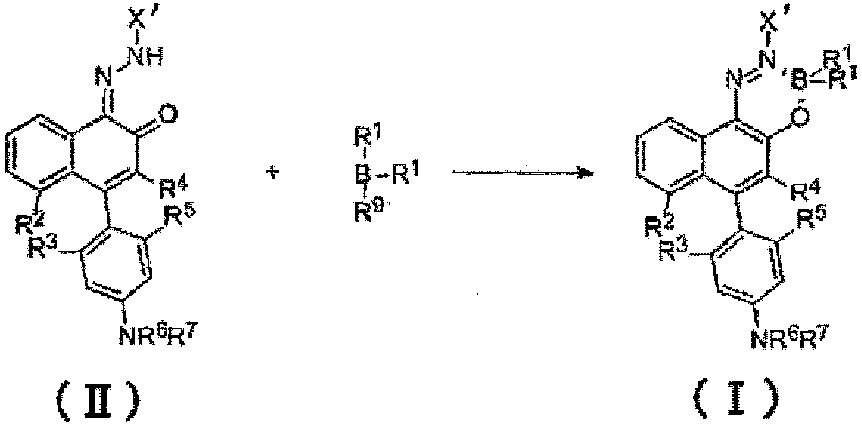

<Compound Represented by General Formula (II₁), (II₂), (II₃), or (II₄)>

As the near-infrared fluorescent material used in the present invention, the compound represented by General Formula (II₁) or (II₂) is also preferable. The compound is hereinafter referred to as a "BODIPY pigment used in the present invention" sometimes.

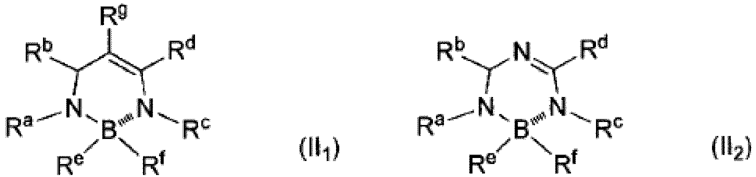

As the near-infrared fluorescent material used in the present invention, the compound represented by General Formula (II₃) or (II₄) is also preferable. The compound is hereinafter referred to as a "DPP-based boron complex used in the present invention" sometimes.

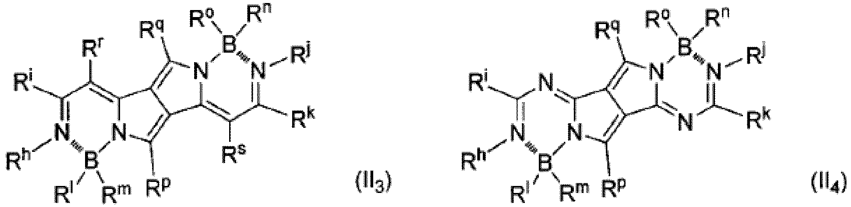

In General Formula (II₁) or (II₂), R^{a} and R^{b} form an aromatic ring consisting of one to three rings together with the nitrogen atom to which R^{a} is bonded and the carbon atom to which R^{b} is bonded. Similarly, in General Formula (II₁) or (II₂), R^{c} and R^{d} form an aromatic ring consisting of one to three rings together with the nitrogen atom to which R^{c} is bonded and the carbon atom to which R^{d} is bonded. Each ring of the ring which R^{a} and R^{b} form and the ring which R^{c} and R^{d} form is a 5-membered ring or a 6-membered ring. The compound represented by General Formula (II₁) or (II₂) has a ring structure formed by condensation of the aromatic ring which R^{a} and R^{b} form and the aromatic ring which R^{c} and R^{d} form by a ring including the boron atom bonded to two nitrogen atoms. That is, the compound represented by General Formula (II₁) or (II₂) has a rigid condensed ring structure configured of a wide conjugate plane.

In General Formula (II₃) or (II₄), R^{h} and Rⁱ form an aromatic ring consisting of one to three rings together with the nitrogen atom to which R^{h} is bonded and the carbon atom to which Rⁱ is bonded. Similarly, in General Formula (II₃) or (II₄), R^{j} and R^{k} form an aromatic ring consisting of one to three rings together with the nitrogen atom to which R^{j} is bonded and the carbon atom to which R^{k} is bonded. Each ring of the aromatic ring which R^{h} and Rⁱ form and the aromatic ring which R^{j} and R^{k} form is a 5-membered ring or a 6-membered ring. The compound represented by General Formula (II₃) or (II₄) has a ring structure formed by condensation between the 5-membered hetero ring in the condensed ring formed by condensation of three rings, the aromatic ring which R^{h} and Rⁱ form, the ring including the boron atom bonded to two nitrogen atoms, and a 5-membered hetero ring including one nitrogen atom, and the 5-membered hetero ring in the condensed ring formed by condensation of three rings, the aromatic ring which R^{j} and R^{k} form, the ring including the boron atom bonded to two nitrogen atoms, and a 5-membered hetero ring including one nitrogen atom, that is, a ring structure formed by condensation of at least 6 rings. In this manner, the compound represented by General Formula (II₃) or (II₄) has a rigid condensed ring structure configured of a very wide conjugate plane.

Each of the aromatic ring which R^{a} and R^{b} form, the aromatic ring which R^{c} and R^{d} form, the aromatic ring which R^{h} and Rⁱ form, and the aromatic ring which R^{j} and R^{k} form is not particularly limited as long as it has aromaticity. Examples of the aromatic ring include a pyrrole ring, an imidazole ring, a pyrazole ring, an oxazole ring, a thiazole ring, a pyridine ring, a pyrimidine ring, a pyridazine ring, an isoindole ring, an indole ring, an indazole ring, a purine ring, a perimidine ring, a thienopyrrole ring, a furopyrrole ring, a pyrrolothiazole ring, and a pyrrolooxazole ring. Since the maximum fluorescence wavelength becomes a longer wavelength to the near-infrared region, in particular, in the case of General Formula (II₁) or (II₃), the number of condensed rings of the aromatic ring is preferably 2 or 3, and more preferably 2 from the viewpoint of complexity of synthesis. Here, even in a case where the number of condensed rings of the aromatic ring is 1, it is also possible to make wavelengths be longer by devising the substituent on the ring or boron. In addition, in particular, in the case of General Formula (II₂) or (II₄), it is possible to make wavelengths be longer to the near-infrared region by simply bonding a substituted aryl group or a heteroaryl group thereto.

Each of the aromatic ring which R^{a} and R^{b} form, the aromatic ring which R^{c} and R^{d} form, the aromatic ring which R^{h} and Rⁱ form, and the aromatic ring which R^{j} and R^{k} form may not have a substituent or may have one or plural substituents. The substituent in the aromatic ring may be "any group which does not inhibit fluorescence of a compound".

In a case where the resin composition according to the present invention is used as a medical material (raw material for medical tools), the near-infrared fluorescent material to be contained is preferably a near-infrared fluorescent material of which mutagenicity, cytotoxicity, sensitization, skin irritation, and the like are negative in the required biological safety testing. In addition, from the viewpoint of safety, the near-infrared fluorescent material is preferably not eluted from a molded article obtained by processing the resin composition of the present invention by body fluid such as blood or tissue fluid. Thus, the near-infrared fluorescent material used in the present invention preferably has a low solubility in biological components such as blood. However, even when the near-infrared fluorescent material used in the present invention is water-soluble, in a case where the resin component itself in the resin composition according to the present invention is hardly eluted into the body fluid or the like, and where the content of the near-infrared fluorescent material itself is a very small amount, the molded article of the resin composition according to the present invention can be used while avoiding elution of the near-infrared fluorescent material even in vivo. Considering these, in the BODIPY pigment used in the present invention, as the substituent having the aromatic ring which R^{a} and R^{b} form or the aromatic ring which R^{c} and R^{d} form, a substituent which is less likely to express mutagenicity or the like or decreases water solubility is preferably selected. Similarly, in the DPP-based boron complex used in the present invention, as the substituent having the aromatic ring which R^{h} and Rⁱ form or the aromatic ring which R^{j} and R^{k} form, a substituent which is less likely to express mutagenicity or the like or decreases water solubility is preferably selected.

Examples of the substituent include a halogen atom, a nitro group, a cyano group, a hydroxy group, a carboxyl group, an aldehyde group, a sulfonic acid group, an alkylsulfonyl group, a halogenosulfonyl group, a thiol group, an alkylthio group, an isocyanate group, a thioisocyanate group, an alkyl group, an alkenyl group, an alkynyl group, an alkoxy group, an alkoxycarbonyl group, an alkylamidecarbonyl group, an alkylcarbonylamide group, an acyl group, an amino group, a monoalkylamino group, a dialkylamino group, a silyl group, a monoalkylsilyl group, a dialkylsilyl group, a trialkylsilyl group, a monoalkoxysilyl group, a dialkoxysilyl group, a trialkoxysilyl group, an aryl group, and a heteroaryl group. The aromatic ring which R^{a} and R^{b} form, the substituent which the aromatic ring which R^{c} and R^{d} form has, the aromatic ring which R^{h} and Rⁱ form, or the aromatic ring which R^{j} and R^{k} form is preferably a cyano group, a hydroxy group, a carboxyl group, an alkylthio group, an alkyl group, an alkoxy group, an alkoxycarbonyl group, an amide group, an alkylsulfonyl group, fluorine, chlorine, an aryl group, or a heteroaryl group, from the viewpoint of safety with respect to a living body, and these substituents may further have a substituent. Here, since, even in the case of a substituent other than these substituents, it is possible to improve safety by further introducing a suitable substituent, the present invention is not limited to these substituents.

Examples of the halogen atom include a fluorine atom, a chlorine atom, a bromine atom, and an iodine atom, and a fluorine atom, a chlorine atom, or a bromine atom is preferable, and a fluorine atom is more preferable.

The alkyl group, the alkenyl group, and the alkynyl group may be linear, branched, or cyclic (aliphatic cyclic group). Each of these groups preferably has 1 to 20 carbon atoms, more preferably 1 to 12 carbon atoms, still more preferably 1 to 8 carbon atoms, and still more preferably 1 to 6 carbon atoms. Examples of the alkyl group include a methyl group, an ethyl group, a propyl group, an isopropyl group, a n-butyl group, an isobutyl group, a t-butyl group (tert-butyl group), a pentyl group, an isoamyl group, a hexyl group, a heptyl group, an octyl group, a nonyl group, a decyl group, a undecyl group, and a dodecyl group. Examples of the alkenyl group include a vinyl group, an allyl group, a 1-propenyl group, an isopropenyl group, a 2-butenyl group, a 1,3-butadienyl group, a 2-pentenyl group, and a 2-hexenyl group. Examples of the alkynyl group include an ethynyl group, a 1-propynyl group, a 2-propynyl group, an isopropynyl group, a 1-butynyl group, and an isobutynyl group.

Examples of the alkyl group portion in an alkylsulfonyl group, an alkylthio group, an alkoxy group, an alkoxycarbonyl group, an alkylamidecarbonyl group, an alkylcarbonylamide group, a monoalkylamino group, a dialkylamino group, a monoalkylsilyl group, a dialkylsilyl group, a trialkylsilyl group, a monoalkoxysilyl group, a dialkoxysilyl group, and a trialkoxysilyl group include the same as the alkyl groups described above. Examples of the alkoxy group include a methoxy group, an ethoxy group, a propyloxy group, an isopropyloxy group, an n-butyloxy group, an isobutyloxy group, a t-butyloxy group, a pentyloxy group, an isoamyloxy group, a hexyloxy group, a heptyloxy group, an octyloxy group, a nonyloxy group, a decyloxy group, a undecyloxy group, and a dodecyloxy group. In addition, examples of the monoalkylamino group include a methylamino group, an ethylamino group, a propylamino group, an isopropylamino group, a butylamino group, an isobutyl amino group, a t-butylamino group, a pentylamino group, and a hexylamino group, and examples of the dialkylamino group include a dimethylamino group, a diethylamino group, a dipropylamino group, a diisopropylamino group, a dibutylamino group, a diisobutylamino group, a dipentylamino group, a dihexylamino group, an ethylmethylamino group, a methylpropylamino group, a butylmethylamino group, an ethylpropylamino group, and a butylethylamino group.

Examples of the aryl group include a phenyl group, a naphthyl group, an indenyl group, and a biphenyl group. The aryl group is preferably a phenyl group.

Examples of the heteroaryl group include 5-membered ring heteroaryl groups such as a pyrrolyl group, an imidazolyl group, a pyrazolyl group, a thienyl group, a furanyl group, an oxazolyl group, an isoxazolyl group, a thiazolyl group, an isothiazolyl group, and a thiadiazole group; 6-membered ring heteroaryl groups such as a pyridinyl group, a pyrazinyl group, a pyrimidinyl group, and a pyridazinyl group; and condensed heteroaryl groups such as an indolyl group, an isoindolyl group, an indazolyl group, a quinolizinyl group, a quinolinyl group, an isoquinolinyl group, a benzofuranyl group, an isobenzofuranyl group, a chromenyl group, a benzoxazolyl group, a benzisoxazolyl group, a benzothiazolyl group, and a benzisothiazolyl group.

Each of the alkyl group, the alkenyl group, the alkynyl group, the aryl group, and the heteroaryl group may be an unsubstituted group, or may be a group in which one or more hydrogen atoms are substituted with substituents. Examples of the substituent include a halogen atom, an alkyl group, an alkoxy group, a nitro group, a cyano group, a hydroxy group, an amino group, a thiol group, a carboxyl group, an aldehyde group, a sulfonic acid group, an isocyanate group, a thioisocyanate group, an aryl group, and a heteroaryl group.

The absorption wavelength and the fluorescence wavelength of the fluorescent material are dependent on the surrounding environment. Therefore, the absorption wavelength of the fluorescent material in the resin becomes shorter in some cases and becomes longer in some cases, than that in a solution. In a case where the absorption wavelength of the BODIPY pigment or the DPP-based boron complex used in the present invention becomes a longer wavelength, the maximum absorption wavelength becomes so as to be in the near-infrared region even in various resins, and thus, this is preferable. The maximum absorption wavelength of the fluorescent material can become a longer wavelength by narrowing the band gap between the highest occupied molecular orbital (HOMO) and the lowest unoccupied molecular orbital (LUMO) by introducing an electron-donating group and an electron-withdrawing group into a suitable position in the molecule.

For example, in the compound represented by General Formula (II₁), the maximum absorption wavelength and the maximum fluorescence wavelength of the compound can become longer wavelengths by introducing electron-donating groups into the aromatic ring which R^{a} and R^{b} form and the aromatic ring which R^{c} and R^{d} form and introducing an electron-withdrawing group into R^{g}. Similarly, in the compound represented by General Formula (II₃), the maximum absorption wavelength and the maximum fluorescence wavelength of the compound can become longer wavelengths by introducing electron-donating groups into the aromatic ring which R^{h} and Rⁱ form and the aromatic ring which Rⁱ and R^{k} form, introducing, in a case where each of R^{p} and R^{q} has an aromatic ring, an electron-donating group into the aromatic ring, or introducing an electron-withdrawing group into R^{r} and R^{s}. By suitably combining these designs, it is possible to adjust to a target wavelength.

The compound represented by General Formula (II₂) having an aza BODIPY skeleton has a skeleton having absorption at a relatively long wavelength even in a case where the aromatic ring which R^{a} and R^{b} form and the aromatic ring which R^{c} and R^{d} form are unsubstituted. In the skeleton, the crosslinking portion of the pyrrole is a nitrogen atom, and thus, it is not possible to introduce a substituent on the nitrogen, unlike the compound represented by General Formula (II₁), but the maximum absorption wavelength and the maximum fluorescence wavelength of the compound can become longer wavelengths by introducing electron-donating groups into the pyrrole portions (the aromatic ring which R^{a} and R^{b} form and the aromatic ring which R^{c} and R^{d} form),wavelength:. Similarly, in the case of the compound represented by General Formula (II₄), the maximum absorption wavelength and the maximum fluorescence wavelength of the compound can become longer wavelengths by introducing electron-donating groups into the pyrrole portions (the aromatic ring which R^{h} and Rⁱ form and the aromatic ring which R^{j} and R^{k} form), or in a case where each of R^{p} and R^{q} has an aromatic ring, introducing an electron-donating group into the aromatic ring.

Therefore, as a substituent having the aromatic ring which R^{a} and R^{b} form, the aromatic ring which R^{c} and R^{d} form, the aromatic ring which R^{h} and Rⁱ form, and the aromatic ring which R^{j} and R^{k} form, a group which functions as an electron-donating group with respect to the aromatic rings, among "any groups which does not inhibit fluorescence of a compound", is preferable. By introducing an electron-donating group into the aromatic ring, fluorescence of the compound represented by General Formula (II₁), (II₂), (II₃), or (II₄) becomes a longer wavelength. Examples of the group which functions as an electron-donating group include an alkyl group; an alkoxy group such as a methoxy group; an aryl group (aromatic ring group) such as a phenyl group, a p-alkoxyphenyl group, a p-dialkylaminophenyl group, or a dialkoxyphenyl group; and a heteroaryl group (heteroaromatic ring) such as a 2-thienyl group or a 2-furanyl group. As the alkyl group, the alkyl group in a substituent of the phenyl group, and the alkyl group portion in the alkoxy group, a linear or branched alkyl group having 1 to 10 carbon atoms is preferable. Moreover, the number of carbon atoms in the alkyl portion or the presence or absence of a branch may be suitably selected in view of the physical properties of the fluorescent material. From the viewpoint of solubility, compatibility, or the like, it is preferable in some cases that the alkyl portion have 6 or more carbon atoms or it is preferable in some cases that the alkyl portion be branched. As a substituent having the aromatic ring which R^{a} and R^{b} form, the aromatic ring which R^{c} and R^{d} form, the aromatic ring which R^{h} and Rⁱ form, and the aromatic ring which R^{j} and R^{k} form, a C₁₋₆ alkyl group, a C₁₋₆ alkoxy group, an aryl group, or a heteroaryl group is preferable, a methyl group, an ethyl group, a methoxy group, a phenyl group, a p-methoxyphenyl group, a p-ethoxyphenyl group, a p-dimethylaminophenyl group, a dimethoxyphenyl group, a thienyl group, or a furanyl group is more preferable, and a methyl group, an ethyl group, a methoxy group, a phenyl group, or a p-methoxyphenyl group is still more preferable. Since the BODIPY skeleton and the DPP skeleton have high planarity, the molecules thereof are likely to be aggregated to each other by π-π stacking. By introducing an aryl group or a heteroaryl group having a bulky substituent into the BODIPY skeleton or the DPP skeleton, it is possible to suppress aggregation of the molecules, and it is possible to increase the emission quantum yield of the resin composition according to the present invention.

In General Formula (II₁) or (II₂), the aromatic ring which R^{a} and R^{b} form and the aromatic ring which R^{c} and R^{d} form may be different from each other or the same type. In General Formula (II₃) or (II₄), the aromatic ring which R^{h} and Rⁱ form and the aromatic ring which R^{j} and R^{k} form may be different from each other or the same type. Since the BODIPY pigment or the DPP-based boron complex used in the present invention can be easily synthesized and tends to have a higher emission quantum yield, the aromatic ring which R^{a} and R^{b} form, the aromatic ring which R^{c} and R^{d} form, the aromatic ring which R^{h} and Rⁱ form, and the aromatic ring which R^{j} and R^{k} form are preferably the same type.

In General Formula (II₁) or (II₂), each of R^{e} and R^{f} independently represents a halogen atom or an oxygen atom. In a case where each of R^{e} and R^{f} is a halogen atom, a fluorine atom, a chlorine atom, a bromine atom, or an iodine atom is preferable, a fluorine atom or a chlorine atom is more preferable, and a fluorine atom is particularly preferable since it has a strong bond to the boron atom. Since a compound in which each of R^{e} and R^{f} is a fluorine atom has high heat resistance, the compound is advantageous in the case of being melt-kneaded together with a resin at a high temperature. Moreover, even in a case where the compound represented by General Formula (II₁) or (II₂) is a substituent in which each of R^{e} and R^{f} includes an atom which can bond to a boron atom rather than a halogen atom or an oxygen atom, the compound can be contained in a resin in the same manner as the BODIPY pigment used in the present invention. As the substituent, any substituent is acceptable as long as it does not inhibit fluorescence.

In General Formula (II₁) or (II₂), in a case where R^{e} and R^{f} are oxygen atoms, R^{e}, the boron atom bonded to R^{e}, R^{a}, and the nitrogen atom bonded to R^{a} may together form a ring, and R^{f}, the boron atom bonded to R^{f}, R^{c}, and the nitrogen atom bonded to R^{c} may together form a ring. That is, in the case of forming a ring structure, the ring which R^{e}, the boron atom bonded to R^{e}, and the nitrogen atom bonded to R^{a} form is condensed with the aromatic ring which R^{a} and R^{b} form, and the ring which R^{f}, the boron atom bonded to R^{f}, and the nitrogen atom bonded to R^{c} form is condensed with the aromatic ring which R^{c} and R^{d} form. The ring which R^{e} and the like forms and the ring which R^{f} and the like forms are preferably 6-membered rings.

In General Formula (II₁) or (II₂), in a case where R^{e} is an oxygen atom and a case where R^{e} does not form a ring, R^{e} is an oxygen atom having a substituent (an oxygen atom bonded to a substituent). Examples of the substituent include a C₁₋₂₀ alkyl group, an aryl group, a heteroaryl group, an alkylcarbonyl group, an arylcarbonyl group, or a heteroarylcarbonyl group. Similarly, in General Formula (II₁) or (II₂), in a case where R^{f} is an oxygen atom and a case where R^{f} does not form a ring, R^{f} is an oxygen atom having a substituent (an oxygen atom bonded to a substituent). Examples of the substituent include a C₁₋₂₀ alkyl group, an aryl group, a heteroaryl group, an alkylcarbonyl group, an arylcarbonyl group, or a heteroarylcarbonyl group. Moreover, in a case where both of R^{e} and R^{f} are oxygen atoms having a substituent, the substituent which R^{e} has and the substituent which R^{f} has may be the same as or different from each other.

In General Formula (II₁) or (II₂), in a case where each of R^{e} and R^{f} is an oxygen atom, R^{e}, R^{f}, and the boron atom bonded to R^{e}, R^{f} may together form a ring. Examples of the ring structure include a structure in which R^{e} and R^{f} are connected to the same aryl ring or heteroaryl ring and a structure in which R^{e} and R^{f} are connected by an alkylene group.

In General Formula (II₃) or (II₄), each of R^{l}, R^{m}, Rⁿ, and R^{o} independently represents a halogen atom, a C₁₋₂₀ alkyl group, a C₁₋₂₀ alkoxy group, an aryl group, or a heteroaryl group. In a case where each of R^{l}, R^{m}, Rⁿ, and R^{o} is a halogen atom, a fluorine atom, a chlorine atom, a bromine atom, or an iodine atom is preferable, a fluorine atom or a chlorine atom is more preferable, and a fluorine atom is particularly preferable since it has a strong bond to the boron atom. Since a compound in which each of R^{l}, R^{m}, Rⁿ, and R^{o} is a fluorine atom has high heat resistance, the compound is advantageous in the case of being melt-kneaded together with a resin at a high temperature.

Moreover, in the present invention and the present specification, the "C₁₋₂₀ alkyl group" means an alkyl group having 1 to 20 carbon atoms, and the "C₁₋₂₀ alkoxy group" means an alkoxy group having 1 to 20 carbon atoms.

In a case where R^{l}, R^{m}, Rⁿ, or R^{o} is a C₁₋₂₀ alkyl group, the alkyl group may be linear, branched, or cyclic (aliphatic cyclic group). Examples of the alkyl group include a methyl group, an ethyl group, a propyl group, an isopropyl group, a n-butyl group, an isobutyl group, a t-butyl group, a pentyl group, an isoamyl group, a hexyl group, a heptyl group, an octyl group, a nonyl group, a decyl group, a undecyl group, and a dodecyl group.

In a case where R^{l}, R^{m}, Rⁿ, or R^{o} is a C₁₋₂₀ alkoxy group, the alkyl group portion of the alkoxy group may be linear, branched, or cyclic (aliphatic cyclic group). Examples of the alkoxy group include a methoxy group, an ethoxy group, a propyloxy group, an isopropyloxy group, an n-butyloxy group, an isobutyloxy group, a t-butyloxy group, a pentyloxy group, an isoamyloxy group, a hexyloxy group, a heptyloxy group, an octyloxy group, a nonyloxy group, a decyloxy group, a undecyloxy group, and a dodecyloxy group.

In a case where R^{l}, R^{m}, Rⁿ, or R^{o} is an aryl group, examples of the aryl group include a phenyl group, a naphthyl group, an indenyl group, and a biphenyl group.

In a case where R^{l}, R^{m}, Rⁿ, or R^{o} is a heteroaryl group, examples of the heteroaryl group include 5-membered ring heteroaryl groups such as a pyrrolyl group, an imidazolyl group, a pyrazolyl group, a thienyl group, a furanyl group, an oxazolyl group, an isoxazolyl group, a thiazolyl group, an isothiazolyl group, and a thiadiazole group; 6-membered ring heteroaryl groups such as a pyridinyl group, a pyrazinyl group, a pyrimidinyl group, and a pyridazinyl group; and condensed heteroaryl groups such as an indolyl group, an isoindolyl group, an indazolyl group, a quinolizinyl group, a quinolinyl group, an isoquinolinyl group, a benzofuranyl group, an isobenzofuranyl group, a chromenyl group, a benzoxazolyl group, a benzisoxazolyl group, a benzothiazolyl group, and a benzisothiazolyl group.

Each of the C₁₋₂₀ alkyl group, the C₁₋₂₀ alkoxy group, the aryl group, and the heteroaryl group represented by R^{l}, R^{m}, Rⁿ, or R^{o} may be an unsubstituted group, or may be a group in which one or more hydrogen atoms are substituted with substituents. Examples of the substituent include a halogen atom, an alkyl group, an alkoxy group, a nitro group, a cyano group, a hydroxy group, an amino group, a thiol group, a carboxyl group, an aldehyde group, a sulfonic acid group, an isocyanate group, a thioisocyanate group, an aryl group, and a heteroaryl group.

As the compound represented by General Formula (II₃) or (II₄), a compound in which each of R^{l}, R^{m}, Rⁿ, and R^{o} is a halogen atom, an unsubstituted aryl group, or an aryl group having a substituent is preferable, a compound in which each of R^{l}, R^{m}, Rⁿ, and R^{o} is a fluorine atom, a chlorine atom, a bromine atom, an unsubstituted phenyl group, or a phenyl group substituted with a C₁₋₂₀ alkyl group or a C₁₋₂₀ alkoxy group is preferable, a compound in which each of R^{l}, R^{m}, Rⁿ, and R^{o} is a fluorine atom, a chlorine atom, an unsubstituted phenyl group, or a phenyl group substituted with a C₁₋₁₀ alkyl or a C₁₋₁₀ alkoxy group is more preferable, and a compound in which each of R^{l}, R^{m}, Rⁿ, and R^{o} is a fluorine atom or an unsubstituted phenyl group is particularly preferable.

In General Formula (II₃) or (II₄), each of R^{p} and R^{q} independently represents a hydrogen atom, a halogen atom, a C₁₋₂₀ alkyl group, a C₁₋₂₀ alkoxy group, an aryl group, or a heteroaryl group. Examples of the halogen atoms, the C₁₋₂₀ alkyl group, the C₁₋₂₀ alkoxy group, the aryl group, or the heteroaryl group represented by R^{p} or R^{q} include the same as those represented by R^{l}, R^{m}, Rⁿ, or R^{o} in General Formula (II₃).

As the compound represented by General Formula (II₃) or (II₄), a compound in which each of R^{p} and R^{q} is a hydrogen atom or an aryl group is preferable, a compound in which each of R^{p} and R^{q} is a hydrogen atom, an unsubstituted phenyl group, or a phenyl group substituted with a C₁₋₂₀ alkyl group or a C₁₋₂₀ alkoxy group is preferable, a compound in which each of R^{p} and R^{q} is a hydrogen atom, an unsubstituted phenyl group, or a phenyl group substituted with a C₁₋₂₀ alkoxy group is more preferable, and a compound in which each of R^{p} and R^{q} is a hydrogen atom, an unsubstituted phenyl group, or a phenyl group substituted with a C₁₋₁₀ alkoxy group is particularly preferable.

In General Formula (II₁), R^{g} represents a hydrogen atom or an electron-withdrawing group. In addition, in General Formula (II₃), each of R^{r} and R^{s} independently represents a hydrogen atom or an electron-withdrawing group. Examples of the electron-withdrawing group include a methyl halide groups such as a trifluoromethyl group; a nitro group; a cyano group; an aryl group; a heteroaryl group; an alkynyl group; an alkenyl group; a substituent having a carbonyl group such as a carboxyl group, an acyl group, a carbonyloxy group, an amide group, and an aldehyde group; a sulfoxide group; a sulfonyl group; an alkoxymethyl group; and an aminomethyl group, and an aryl group or a heteroaryl group having the electron-withdrawing group as a substituent can also be used. Among these electron-withdrawing groups, from the viewpoint of making the maximum fluorescence wavelength to be longer, a trifluoromethyl group, a nitro group, a cyano group, or a sulfonyl group which can function as a strong electron-withdrawing group is preferable.

As the BODIPY pigment used in the present invention, a compound represented by the following General Formula (II₁-0) or (II₂-0) is preferable. A compound having a boron dipyrromethene skeleton is preferably since the maximum fluorescence wavelength becomes a longer wavelength, and, in particular, a compound satisfying the following (p2), (p3), (q2), and (q3), in which the pyrrole ring is condensed with an aromatic ring or a heteroaromatic ring is preferable as the near-infrared fluorescent material used in the present invention since the maximum wavelength becomes a longer wavelength.

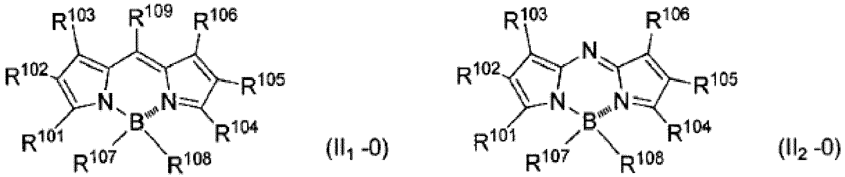

In General Formula (II₁-0) or (II₂-0), R¹⁰¹, R¹⁰², and R¹⁰³ satisfy any one of the following (p1) to (p3).
(p1) each of R¹⁰¹, R¹⁰², and R¹⁰³ independently represents a hydrogen atom, a halogen atom, a C₁₋₂₀ alkyl group, a C₁₋₂₀ alkoxy group, an aryl group, or a heteroaryl group,
(p2) R¹⁰¹ and R¹⁰² together form an aromatic 5-membered ring or an aromatic 6-membered ring, and R¹⁰³ represents a hydrogen atom, a halogen atom, a C₁₋₂₀ alkyl group, a C₁₋₂₀ alkoxy group, an aryl group, or a heteroaryl group, or
(p3) R¹⁰² and R¹⁰³ together form an aromatic 5-membered ring or an aromatic 6-membered ring, and R¹⁰¹ represents a hydrogen atom, a halogen atom, a C₁₋₂₀ alkyl group, a C₁₋₂₀ alkoxy group, an aryl group, or a heteroaryl group.

In General Formula (II₁-0) or (II₂-0), R¹⁰⁴, R¹⁰⁵, and R¹⁰⁶ satisfy any one of the following (q1) to (q3).
(q1) each of R¹⁰⁴, R¹⁰⁵, and R¹⁰⁶ independently represents a hydrogen atom, a halogen atom, a C₁₋₂₀ alkyl group, a C₁₋₂₀ alkoxy group, an aryl group, or a heteroaryl group,
(q2) R¹⁰⁴ and R¹⁰⁵ together form an aromatic 5-membered ring or an aromatic 6-membered ring, and R¹⁰⁶ represents a hydrogen atom, a halogen atom, a C₁₋₂₀ alkyl group, a C₁₋₂₀ alkoxy group, an aryl group, or a heteroaryl group, or
(q3) R¹⁰⁵ and R¹⁰⁶ together form an aromatic 5-membered ring or an aromatic 6-membered ring, and R¹⁰⁴ represents a hydrogen atom, a halogen atom, a C₁₋₂₀ alkyl group, a C₁₋₂₀ alkoxy group, an aryl group, or a heteroaryl group.

As the halogen atom, the C₁₋₂₀ alkyl group, the C₁₋₂₀ alkoxy group, the aryl group, or the heteroaryl group in (p1) to (p3) or (q1) to (q3), those exemplified as "any group which does not inhibit fluorescence of a compound" represented by each of R^{a} and R^{b} can be used.

In (p2) and (p3) or (q2) and (q3), as an aromatic 5-membered ring or an aromatic 6-membered ring which R¹⁰¹ and R¹⁰² together form, an aromatic 5-membered ring or an aromatic 6-membered ring which R¹⁰⁴ and R¹⁰⁵ together form, an aromatic 5-membered ring or an aromatic 6-membered ring which R¹⁰² and R¹⁰³ together form, or an aromatic 5-membered ring or an aromatic 6-membered ring which R¹⁰⁵ and R¹⁰⁶ together form, a ring represented by any one of the following General Formulas (C-1) to (C-9) is preferable, and a ring represented by any one of the following General Formulas (C-1), (C-2), and (C-9) is more preferable. In the following General Formulas (C-1) to (C-9), the place to which an asterisk is attached is a portion to which a boron dipyrromethene skeleton in General Formula (II₁-0) or (II₂-0) is bonded.

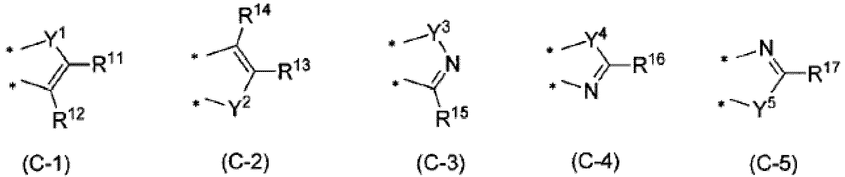

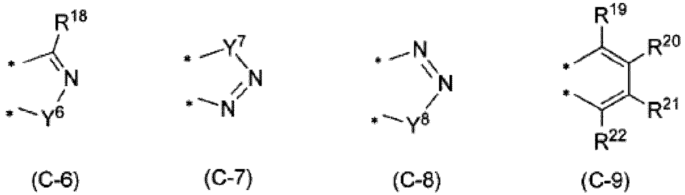

In General Formulas (C-1) to (C-8), each of Y¹ to Y⁸ independently represents a sulfur atom, an oxygen atom, a nitrogen atom, or a phosphorus atom. Each of Y¹ to Y⁸ is independently preferably a sulfur atom, an oxygen atom, or a nitrogen atom, and more preferably a sulfur atom or an oxygen atom.

In General Formulas (C-1) to (C-9), each of R¹¹ to R²² independently represents a hydrogen atom or any group which does not inhibit fluorescence of a compound described above. As "any group which does not inhibit fluorescence of a compound", those exemplified as "any group which does not inhibit fluorescence of a compound" represented by each of R^{a} and R^{b} can be used. Each of R¹¹ to R²² is independently preferably a hydrogen atom, an unsubstituted aryl group, an aryl group having a substituent, an unsubstituted heteroaryl group, or a heteroaryl group having a substituent, more preferably a hydrogen atom, an (unsubstituted) phenyl group, a p-methoxyphenyl group, a p-ethoxyphenyl group, a p-dimethylaminophenyl group, a dimethoxyphenyl group, a thienyl group, or a furanyl group, and still more preferably a hydrogen atom, an (unsubstituted) phenyl group, or a p-methoxyphenyl group. Since the electron donicity can be increased and aggregation of a BODIPY skeleton can be suppressed by a bulky substituent, the compound is particularly preferably substituted with at least one of the unsubstituted aryl group, the aryl group having a substituent, the unsubstituted heteroaryl group, and the heteroaryl group having a substituent.

In the compound of General Formula (II₁-0) or (II₂-0), R¹⁰¹ and R¹⁰⁴, R¹⁰² and R¹⁰⁵, and R¹⁰³ and R¹⁰⁶ may be different from each other, respectively, but are preferably the same group. That is, in a case where R¹⁰¹, R¹⁰², and R¹⁰³ satisfy (p1), R¹⁰⁴, R¹⁰⁵, and R¹⁰⁶ preferably satisfy (q1), in a case where R¹⁰¹, R¹⁰², and R¹⁰³ satisfy (p2), R¹⁰⁴, R¹⁰⁵, and R¹⁰⁶ preferably satisfy (q2), and in a case where R¹⁰¹, R¹⁰², and R¹⁰³ satisfy (p3), R¹⁰⁴, R¹⁰⁵, and R¹⁰⁶ preferably satisfy (q3).

As the compound of General Formula (II₁-0) or (II₂-0), a compound in which R¹⁰¹ and R¹⁰² form a ring, and R¹⁰⁴ and R¹⁰⁵ form a ring, or R¹⁰² and R¹⁰³ form a ring, and R¹⁰⁵ and R¹⁰⁶ form a ring is preferable. That is, it is preferable that R¹⁰¹, R¹⁰², and R¹⁰³ satisfy (p2) or (p3), and R¹⁰⁴, R¹⁰⁵, and R¹⁰⁶ satisfy (q2) or (q3). This is because the maximum fluorescence wavelength becomes a longer wavelength by further condensation of the aromatic ring or the heteroaromatic ring with a boron dipyrromethene skeleton.

In General Formula (II₁-0) or (II₂-0), each of R¹⁰⁷ and R¹⁰⁸ represents a halogen atom or an oxygen atom. In a case where R¹⁰⁷ and R¹⁰⁸ are oxygen atoms, R¹⁰⁷, the boron atom bonded to R¹⁰⁷, the nitrogen atom bonded to the boron atom, R¹⁰¹, and the carbon atom bonded to R¹⁰¹ may together form a ring, and R¹⁰⁸, the boron atom bonded to R¹⁰⁸, the nitrogen atom bonded to the boron atom, R¹⁰⁴, and the carbon atom bonded to R¹⁰⁴ may together form a ring. That is, each of the ring which R¹⁰⁷, a boron atom, R¹⁰¹, and the like form and the ring which R¹⁰⁸, a boron atom, R¹⁰⁴, and the like form is condensed with a boron dipyrromethene skeleton. Each of the ring which R¹⁰⁷, a boron atom, R¹⁰¹, and the like form and the ring which R¹⁰⁸, a boron atom, R¹⁰⁴, and the like form is preferably a 6-membered ring.

In General Formula (II₁-0) or (II₂-0), in a case where R¹⁰⁷ is an oxygen atom and does not form a ring, R¹⁰⁷ is an oxygen atom having a substituent (an oxygen atom bonded to a substituent). Examples of the substituent include a C₁₋₂₀ alkyl group, an aryl group, or a heteroaryl group. Similarly, in General Formula (II₁-0) or (II₂-0), in a case where R¹⁰⁸ is an oxygen atom and does not form a ring, R¹⁰⁸ is an oxygen atom having a substituent (an oxygen atom bonded to a substituent). Examples of the substituent include a C₁₋₂₀ alkyl group, an aryl group, or a heteroaryl group. Moreover, in a case where both of R¹⁰⁷ and R¹⁰⁸ are oxygen atoms having a substituent, the substituent which R¹⁰⁷ has and the substituent which R¹⁰⁸ has may be the same as or different from each other.

In General Formula (II₁-0), R¹⁰⁹ represents a hydrogen atom or an electron-withdrawing group. Examples of the electron-withdrawing group include the same as the groups exemplified as R^{g}. Among these, from the viewpoint of making the maximum fluorescence wavelength to be longer, a fluoroalkyl group, a nitro group, a cyano group, an aryl group, or a sulfonyl group which can function as a strong electron-withdrawing group is preferable, a trifluoromethyl group, a nitro group, a cyano group, a phenyl group, or a sulfonyl group is more preferable, and from the viewpoint of safety with respect to a living body, a trifluoromethyl group, a cyano group, a phenyl group, or a sulfonyl group is sill more preferable. However, the present invention is not limited to these substituents.

As the BODIPY pigment used in the present invention, among the compounds represented by General Formula (II₁-0) or (II₂-0), a compound in which R¹⁰¹ and R¹⁰² together form a ring in which, in the ring represented by General Formula (C-1), one of R¹¹ and R¹² is a hydrogen atom, and the remaining one is a phenyl group, a thienyl group, or a furanyl group in which one to three hydrogen atoms may be substituted with halogen atoms, C₁₋₂₀ alkyl groups, or C₁₋₂₀ alkoxy groups, R¹⁰⁴ and R¹⁰⁵ together form the same type of ring as the ring formed by R¹⁰¹ and R¹⁰², R¹⁰³ and R¹⁰⁶ are hydrogen atoms, and R¹⁰⁷ and R¹⁰⁸ are halogen atoms; a compound in which R¹⁰¹ and R¹⁰² together form a ring in which, in the ring represented by General Formula (C-2), one of R¹³ and R¹⁴ is a hydrogen atom, and the remaining one is a phenyl group, a thienyl group, or a furanyl group in which one to three hydrogen atoms may be substituted with halogen atoms, C₁₋₂₀ alkyl groups, or C₁₋₂₀ alkoxy groups, R¹⁰⁴ and R¹⁰⁵ together form the same type of ring as the ring formed by R¹⁰¹ and R¹⁰², R¹⁰³ and R¹⁰⁶ are hydrogen atoms, and R¹⁰⁷ and R¹⁰⁸ are halogen atoms; a compound in which R¹⁰² and R¹⁰³ together form a ring in which, in the ring represented by General Formula (C-1), one of R¹¹ and R¹² is a hydrogen atom, and the remaining one is a phenyl group, a thienyl group, or a furanyl group in which one to three hydrogen atoms may be substituted with halogen atoms, C₁₋₂₀ alkyl groups, or C₁₋₂₀ alkoxy groups, R¹⁰⁵ and R¹⁰⁶ together form the same type of ring as the ring formed by R¹⁰² and R¹⁰³, R¹⁰¹ and R¹⁰⁴ are hydrogen atoms, and R¹⁰⁷ and R¹⁰⁸ are halogen atoms; a compound in which R¹⁰² and R¹⁰³ together form a ring in which, in the ring represented by the following General Formula (C-2), one of R¹³ and R¹⁴ is a hydrogen atom, and the remaining one is a phenyl group, a thienyl group, or a furanyl group in which one to three hydrogen atoms may be substituted with halogen atoms, C₁₋₂₀ alkyl groups, or C₁₋₂₀ alkoxy groups, R¹⁰⁵ and R¹⁰⁶ together form the same type of ring as the ring formed by R¹⁰¹ and R¹⁰², R¹⁰¹ and R¹⁰⁴ are hydrogen atoms, and R¹⁰⁷ and R¹⁰⁸ are halogen atoms; or a compound in which R¹⁰² and R¹⁰³ together form a ring in which, in the ring represented by the following General Formula (C-9), one of R¹⁹ and R²² is a phenyl group, a thienyl group, or a furanyl group in which one to three hydrogen atoms may be substituted with halogen atoms, C₁₋₂₀ alkyl groups, or C₁₋₂₀ alkoxy groups, and the remaining three are hydrogen atoms, R¹⁰⁵ and R¹⁰⁶ together form the same type of ring as the ring formed by R¹⁰¹ and R¹⁰², R¹⁰¹ and R¹⁰⁴ are phenyl groups, thienyl groups, or furanyl groups in which may be substituted with hydrogen atoms, halogen atoms, C₁₋₂₀ alkyl groups, or C₁₋₂₀ alkoxy groups, and R¹⁰⁷ and R¹⁰⁸ are halogen atoms is preferable. In a case where the compound is a compound represented by General Formula (II₁-0), R¹⁰⁹ is more preferably a trifluoromethyl group, a cyano group, a nitro group, or a phenyl group, and a trifluoromethyl group or a phenyl group is particularly preferable.

Examples of a preferable compound of the BODIPY pigment used in the present invention include compounds represented by the following General Formulas (II₁-1), (II₁-2), (II₁-3), (II₂-1), (II₂-2), or (II₂-3). In the following General Formula (II₁-1), each of R¹⁰¹, R¹⁰³, R¹⁰⁴, and R¹⁰⁶ to R¹⁰⁸ has the same meaning as that described above, ED represents an electron-donating group, EW represents an electron-withdrawing group, and each of Z¹ to Z⁴ ring independently represents a 5- or 6-membered ring aryl group or a 5- or 6-membered ring heteroaryl group.

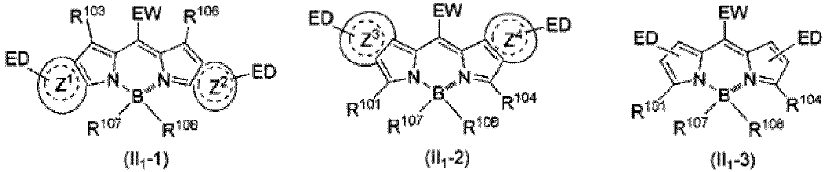

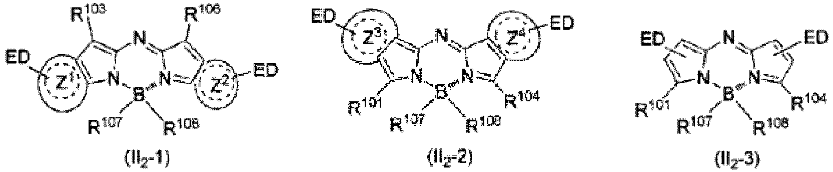

The following General Formula (II₁-1) is preferably a compound represented by each of the following General Formulas (II₁-1) to (II₁-1-6), the following General Formula (II₁-2) is preferably a compound represented by each of the following General Formulas (II₁-2-1) to (II₁-2-12), the following General Formula (II₂-1) is preferably a compound represented by each of the following General Formulas (II₂-1-1) to (II₂-I-6), and the following General Formula (II₂-2) is preferably a compound represented by each of the following General Formulas (II₂-2-1) to (II₂-2-12).

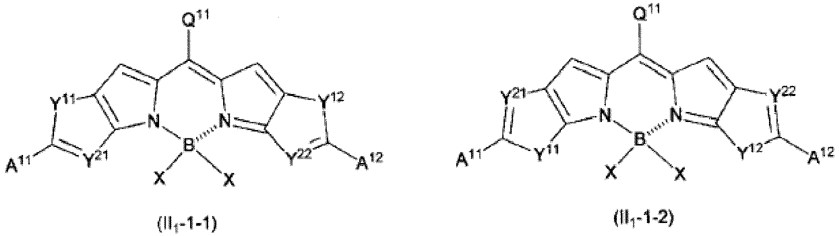

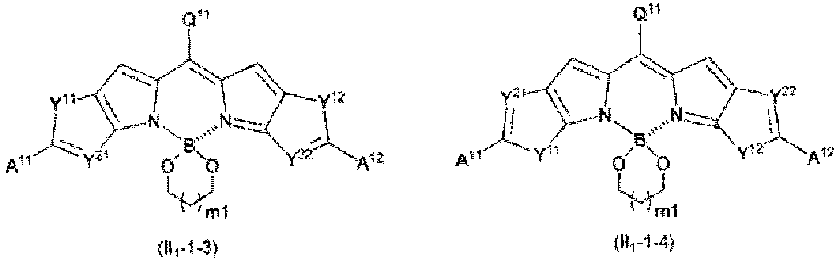

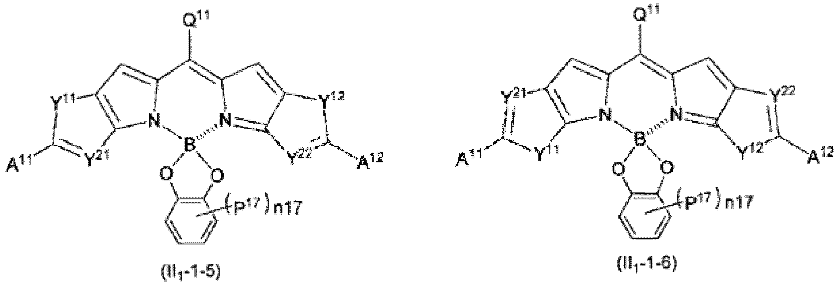

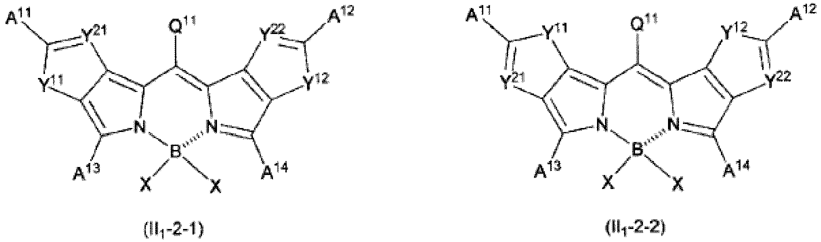

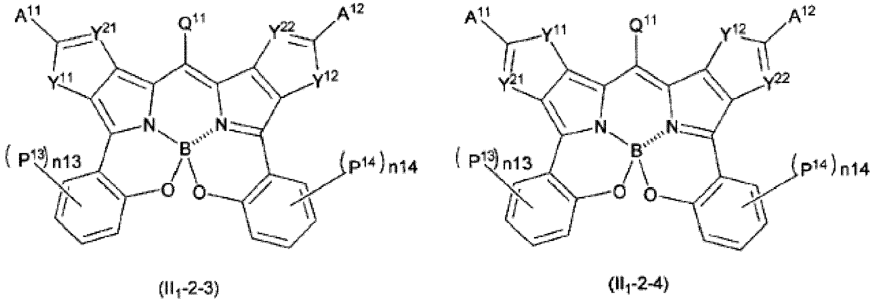

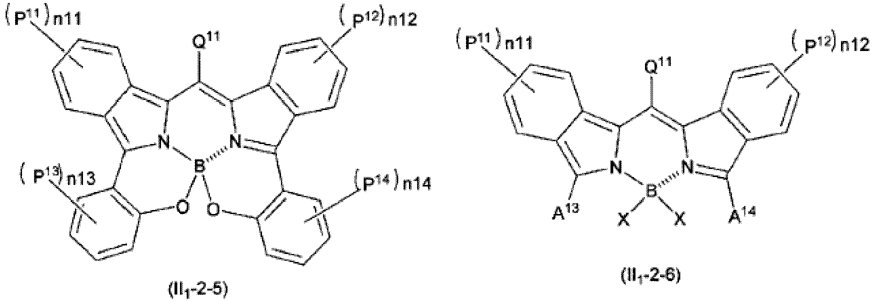

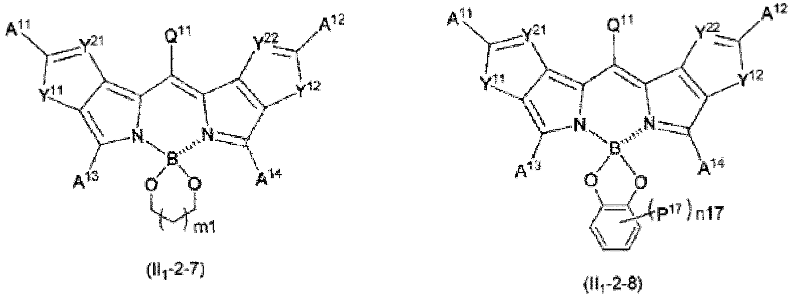

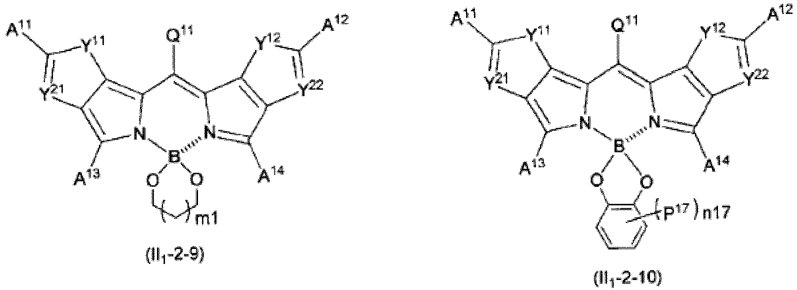

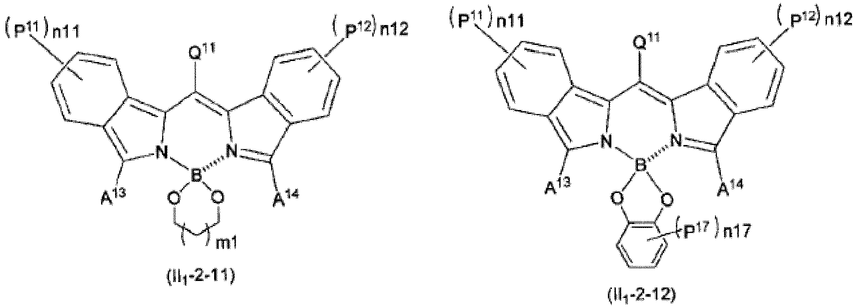

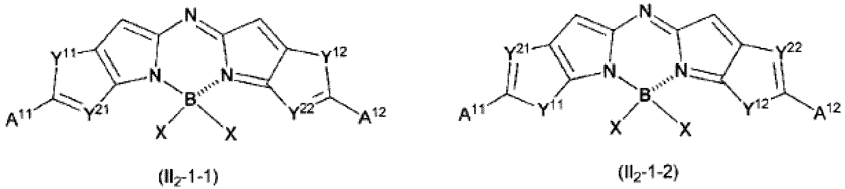

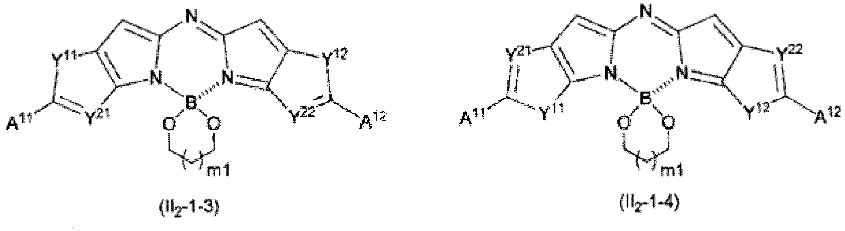

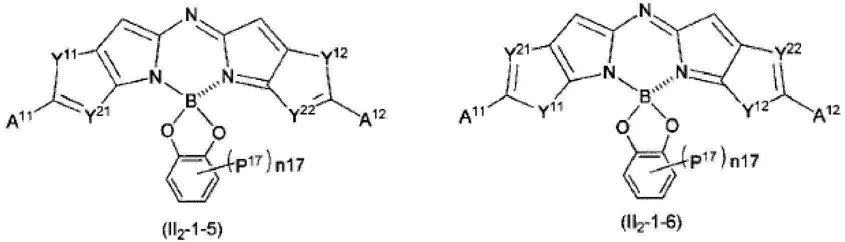

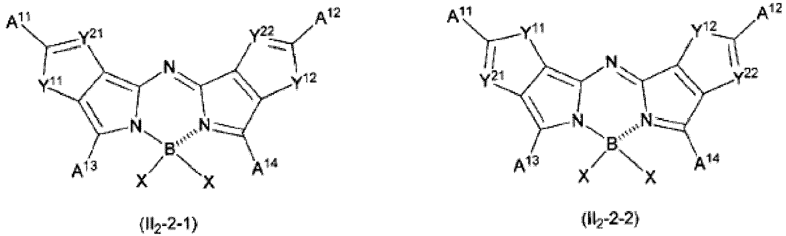

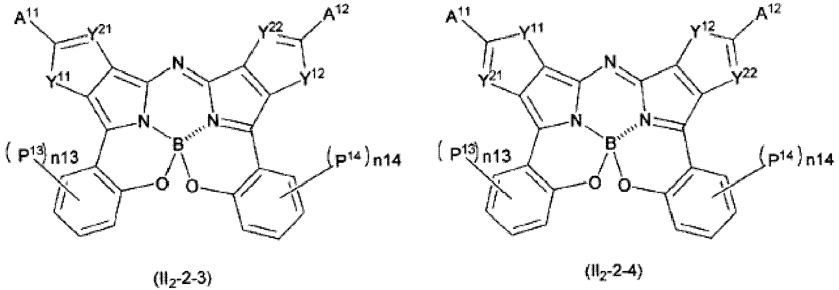

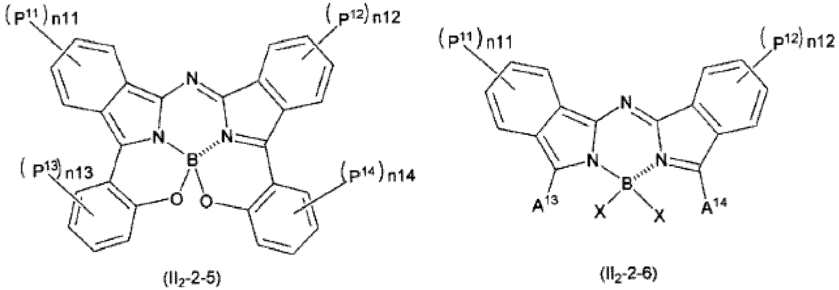

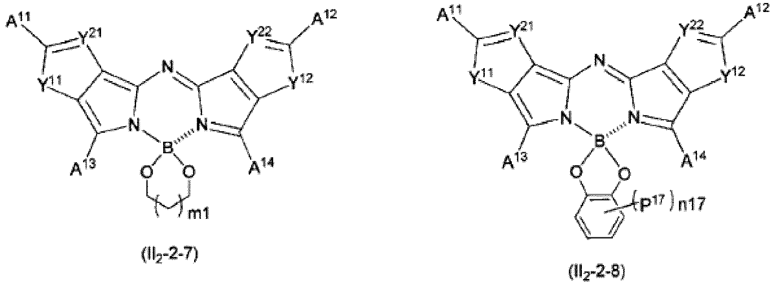

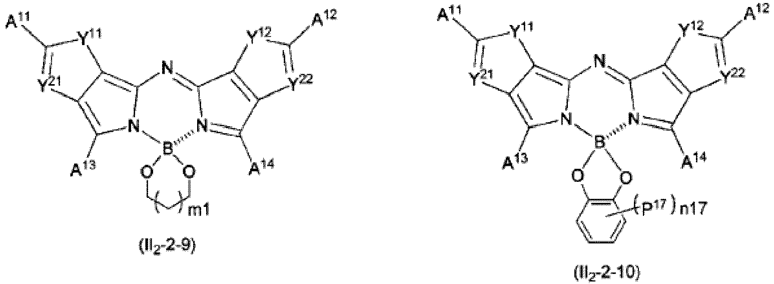

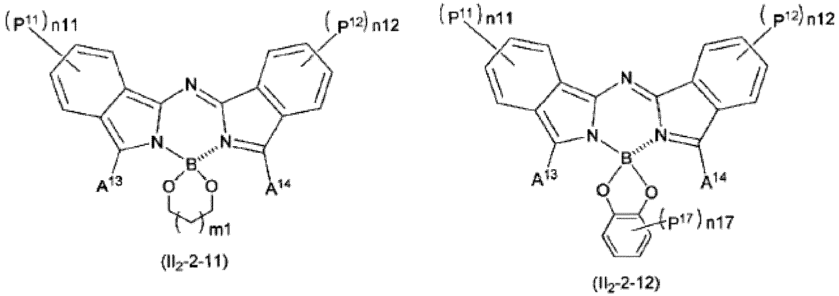

In General Formulas (II₁-1-1) to (II₁-1-6), (II₁-2-1) to (II₁-2-4), (II₁-2-7) to (II₁-2-10), (II₂-1-1) to (II₂-1-6), (II₂-2-1) to (II₂-2-4), and (II₂-2-7) to (II₂-2-10), each of Y¹¹ and Y¹² independently represents an oxygen atom or a sulfur atom, and each of Y²¹ and Y²² independently represents a carbon atom or a nitrogen atom. As the compounds represented by General Formulas (II₁-1-1) or the like, a compound in which Y¹¹ and Y¹² are the same type of atoms and Y²¹ and Y²² are the same type of atoms is preferable.

In General Formulas (II₁-1-1) to (II₁-1-6) and (II₁-2-1) to (II₁-2-12), Q¹¹ represents a hydrogen atom or an electron-withdrawing group. Examples of the electron-withdrawing group include the same as the groups exemplified as R^{g}. As the composition represented by General Formula (II₁-1-1), a compound in which Q¹¹ is a trifluoromethyl group, a cyano group, a nitro group, or a phenyl group which may have a substituent is preferable, and a compound in which Q¹¹ is a trifluoromethyl group or a phenyl group which may have a substituent is more preferable.

In General Formulas (II₁-1-1) and (II₁-1-2), (II₁-2-1) and (II₁-2-2), (II₂-1-1) and (II₂-1-2), and (II₂-2-1) and (II₂-2-2), each of Xs independently represents a halogen atom, a C₁₋₂₀ alkoxy group, an aryloxy group, or an acyloxy group.

In a case where X is a C₁₋₂₀ alkoxy group, the alkyl group portion of the alkoxy group may be linear, branched, or cyclic (aliphatic cyclic group). Examples of the alkoxy group include a methoxy group, an ethoxy group, a propyloxy group, an isopropyloxy group, an n-butyloxy group, an isobutyloxy group, a t-butyloxy group, a pentyloxy group, an isoamyloxy group, a hexyloxy group, a heptyloxy group, an octyloxy group, a nonyloxy group, a decyloxy group, a undecyloxy group, and a dodecyloxy group.

In a case where X is an aryloxy group, examples of the aryloxy group include a phenyloxy group, a naphthyloxy group, an indenyloxy group, and a biphenyloxy group.

In a case where X is an acyloxy group, as the acyloxy group, an alkylcarbonyloxy group or an arylcarbonyl group is preferable. Examples of the alkylcarbonyloxy group include a methylcarbonyloxy group (acetoxy group), an ethylcarbonyloxy group, a propylcarbonyloxy group, an isopropylcarbonyloxy group, an n-butylcarbonyloxy group, an isobutylcarbonyloxy group, a t-butylcarbonyloxy group, a pentylcarbonyloxy group, an isoamylcarbonyloxy group, a hexylcarbonyloxy group, a heptylcarbonyloxy group, an octylcarbonyloxy group, a nonylcarbonyloxy group, a decylcarbonyloxy group, a undecylcarbonyloxy group, and a dodecylcarbonyloxy group. Examples of the arylcarbonyloxy group include a phenylcarbonyloxy group (benzoyloxy group), a naphthylcarbonyloxy group, an indenylcarbonyloxy group, and a biphenylcarbonyloxy group.

As a compound represented by any one of General Formulas (II₁-1-1) to (II₁-1-6), (II₁-2-1), (II₁-2-2), (II₁-2-6), (II₂-1-1) to (II₂-1-6), (II₂-2-1), (II₂-2-2), and (II₂-2-6), a compound in which all Xs are halogen atoms is preferable, and a compound in which all Xs are fluorine atoms is particularly preferable.

In General Formulas (II₁-1-3), (II₁-1-4), (II₁-2-7), (II₁-2-9), (II₁-2-11), (II₂-1-3), (II₂-1-4), (II₂-2-7), (II₂-2-9), and (II₂-2-11), m1 represents 0 or 1.

In General Formulas (II₁-1-5), (II₁-1-6), (II₁-2-3) to (II₁-2-6), (II₁-2-8), (II₁-2-10), (II₁-2-12), (II₂-1-5), (II₂-1-6), (II₂-2-3) to (II₂-2-6), (II₂-2-8), (II₂-2-10), and (II₂-2-12), each of P¹¹ to P¹⁴ and P¹⁷ independently represents a halogen atom, a C₁₋₂₀ alkyl group, a C₁₋₂₀ alkoxy group, an amino group, a monoalkylamino group, or a dialkylamino group. Examples of the C₁₋₂₀ alkyl group, the C₁₋₂₀ alkoxy group, the monoalkylamino group, or the dialkylamino group represented by each of P¹¹ to P¹⁴ include the same as those exemplified as R^{g}, (p1) to (p3), or (q1) to (q3). Each of P¹¹ to P¹⁴ is preferably a C₁₋₂₀ alkyl group, a C₁₋₂₀ alkoxy group, an (unsubstituted) phenyl group, a p-methoxyphenyl group, a p-ethoxyphenyl group, a p-dimethylaminophenyl group, a dimethoxyphenyl group, a thienyl group, or a furanyl group, more preferably a C₁₋₂₀ alkyl group, a C₁₋₂₀ alkoxy group, a phenyl group, a p-methoxyphenyl group, a p-ethoxyphenyl group, a dimethoxyphenyl group, a thienyl group, or a furanyl group from the viewpoint of safety with respect to a living body, and these substituents may further have a substituent. Here, since, even in the case of a substituent other than these substituents, it is possible to improve safety by further introducing a suitable substituent, the present invention is not limited to these substituents.

In General Formulas (II₁-1-5), (II₁-1-6), (Ili-2-3) to (II₁-2-6), (II₁-2-8), (II₁-2-10), (II₁-2-12), (II₂-1-5), (II₂-1-6), (II₂-2-3) to (II₂-2-6), (II₂-2-8), (II₂-2-10), and (II₂-2-12), each of n11 to nl4 and n17 independently represents an integer of 0 to 3. In a case where a plurality of P¹¹s are present in one molecule (that is, in a case where n11 is 2 or 3), all of the plurality of P¹¹s may be the same type of functional groups, or may be different types of functional groups. The same applies to P¹² to P¹⁴ and P¹⁷.

In General Formulas (II₁-1-1) to (II₁-1-6), (II₁-2-1) to (II₁-2-4), (II₁-2-6) to (II₁-2-12), (II₂-1-1) to (II₂-1-6), (II₂-2-1) to (II₂-2-4), and (II₂-2-6) to (II₂-2-12), each of A¹¹ to A¹⁴ independently represents a phenyl group which may have one to three substituents selected from the group consisting of a halogen atom, a C₁₋₂₀ alkyl group, a C₁₋₂₀ alkoxy group, an amino group, a monoalkylamino group, and a dialkylamino group, or a heteroaryl group which may have one to three substituents selected from the group consisting of a halogen atom, a C₁₋₂₀ alkyl group, a C₁₋₂₀ alkoxy group, an amino group, a monoalkylamino group, and a dialkylamino group. Examples of the heteroaryl group include the same as those represented by R¹, R^{m}, Rⁿ, or R° in General Formula (II₃), and the heteroaryl group is preferably a thienyl group or a furanyl group. Examples of the C₁₋₂₀ alkyl group, the C₁₋₂₀ alkoxy group, the monoalkylamino group, or the dialkylamino group as the substituent which the phenyl group or the heteroaryl group may have are the same as those exemplified as R^{g}, (p1) to (p3), or (q1) to (q3). Each of A¹¹ to A¹⁴ is preferably an unsubstituted phenyl group, a phenyl group having one or two C₁₋₂₀ alkoxy groups as the substituent, or an unsubstituted heteroaryl group, more preferably an unsubstituted phenyl group or a phenyl group having one C₁₋₂₀ alkoxy group as the substituent, still more preferably an unsubstituted phenyl group or a phenyl group having one C₁₋₁₀ alkoxy group as the substituent, and still more preferably an unsubstituted phenyl group or a phenyl group having one C₁₋₆ alkoxy group as the substituent. In addition, the compound represented by General Formula (II₁-1-1) is preferably a compound in which all of A¹¹ to A¹⁴ are the same type of functional groups.

As the BODIPY pigments used in the present invention, in particular, a compound represented by any one of the following General Formulas (1-1) to (1-37), (2-1) to (2-7), (3-1) to (3-37), (4-1) to (4-7), (5-1), and (5-2) is preferable, a compound represented by any one of the following General Formulas (1-1) to (1-12), (1-25) to (1-31), (2-1) to (2-7), and (3-25) to (3-31) is more preferable, and a compound represented by any one of the following General Formulas (1-1), (1-3), (1-4), (1-6), (1-25), (1-27), (2-1), (3-1), (3-3), (3-4), (3-6), (3-25), (3-27), and (4-1) is still more preferable.

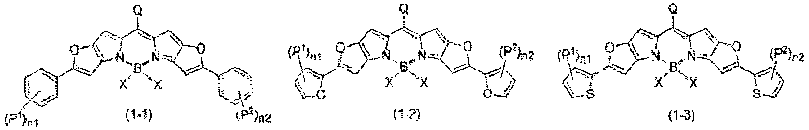

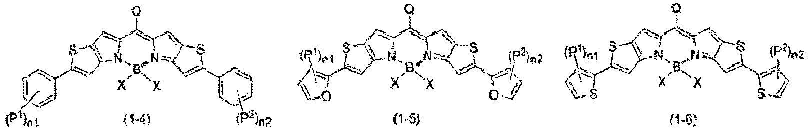

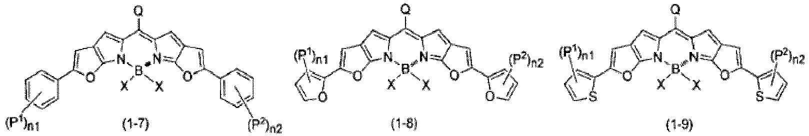

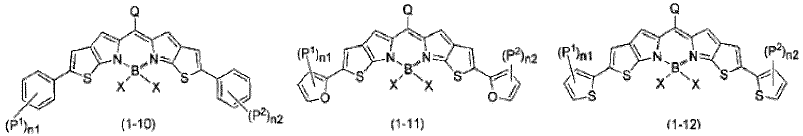

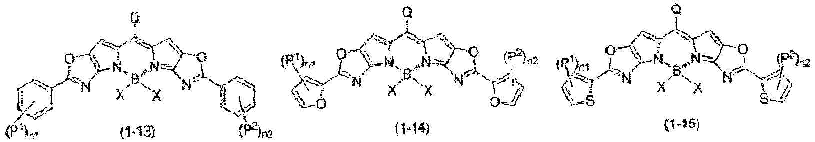

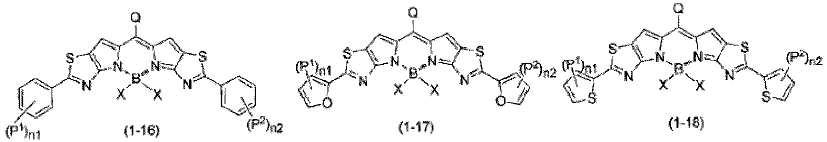

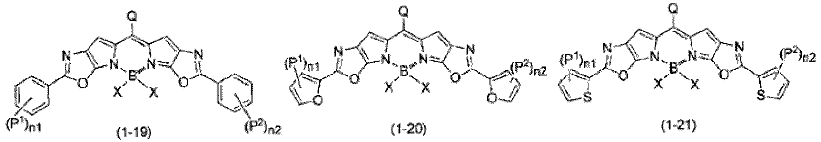

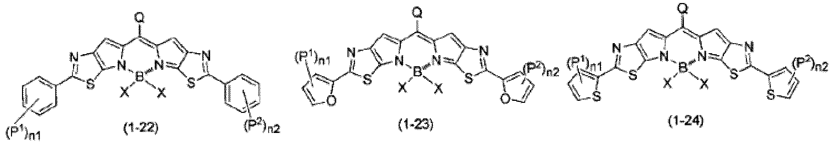

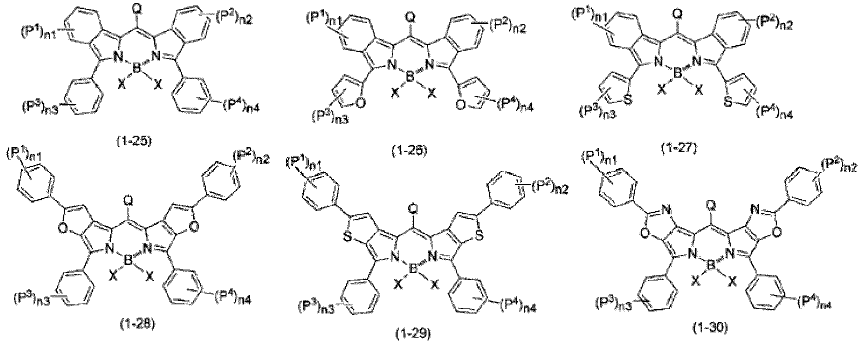

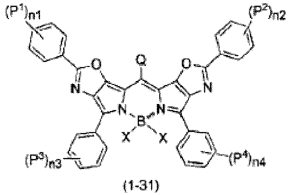

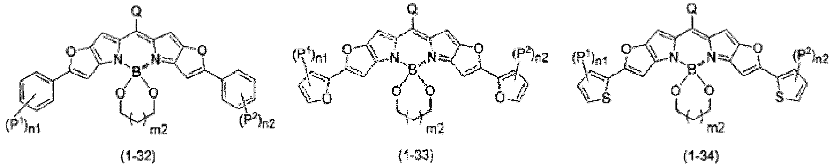

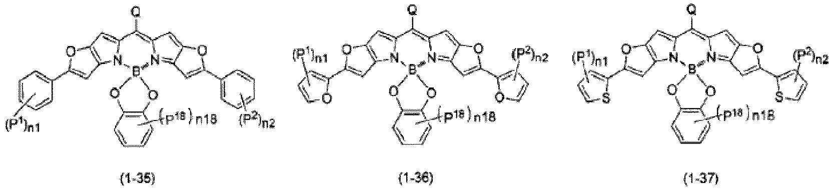

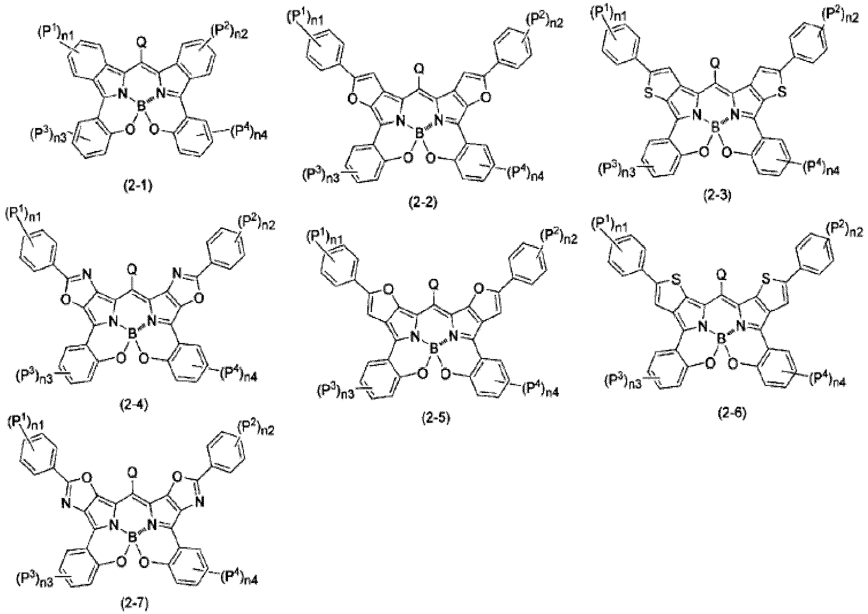

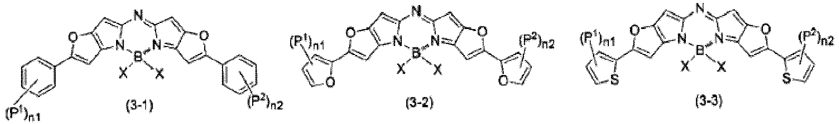

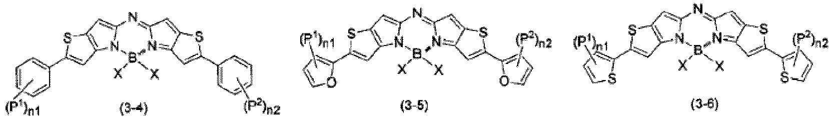

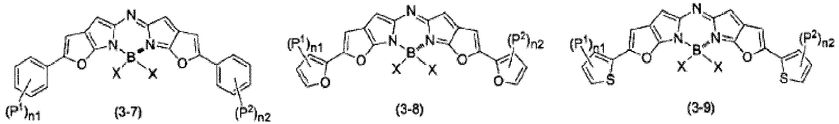

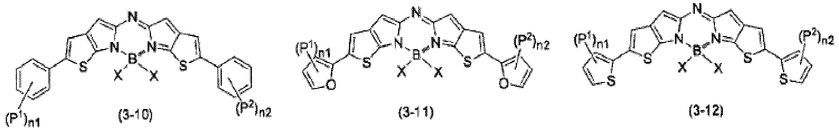

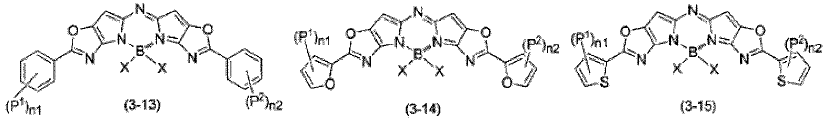

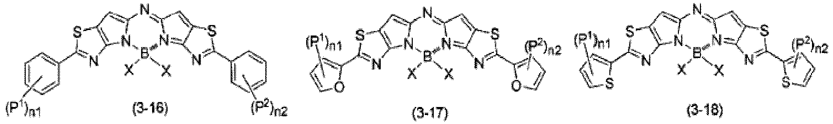

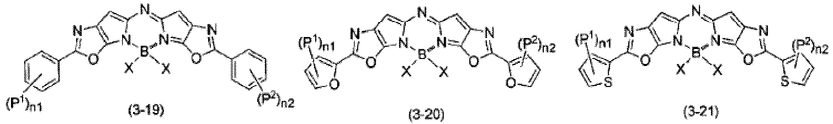

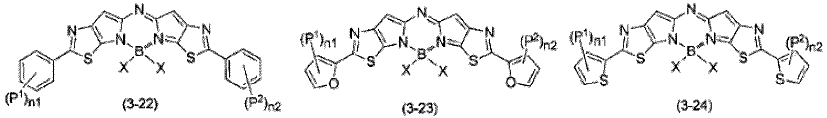

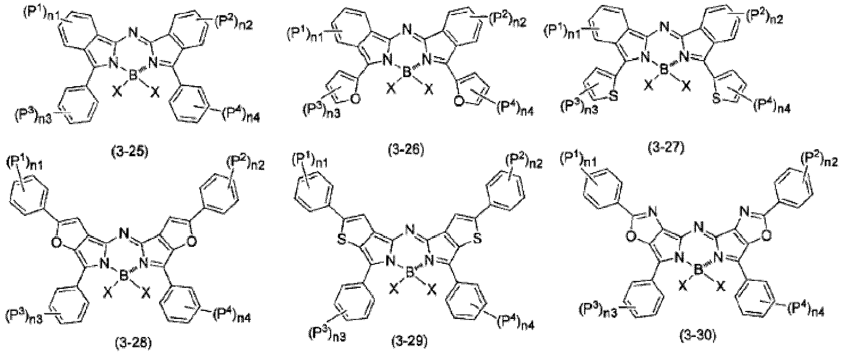

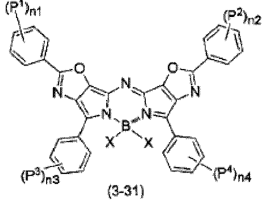

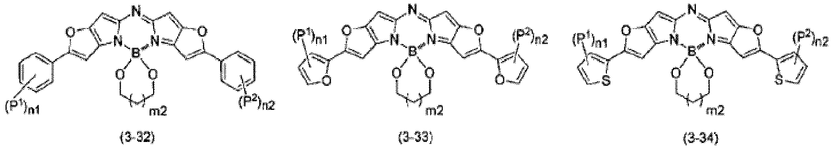

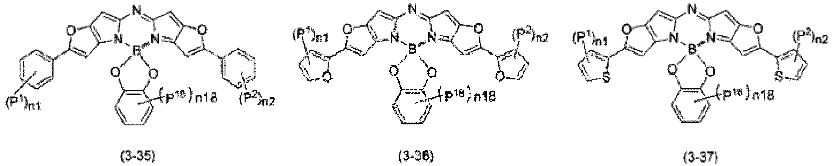

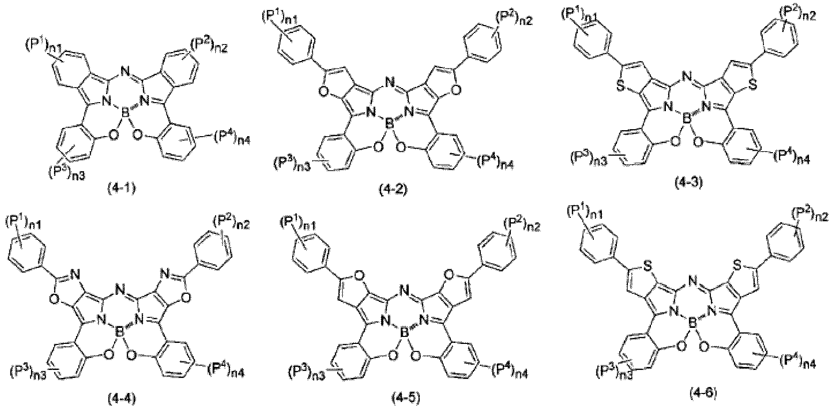

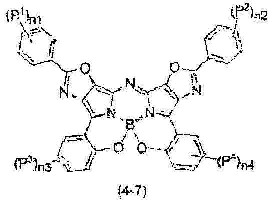

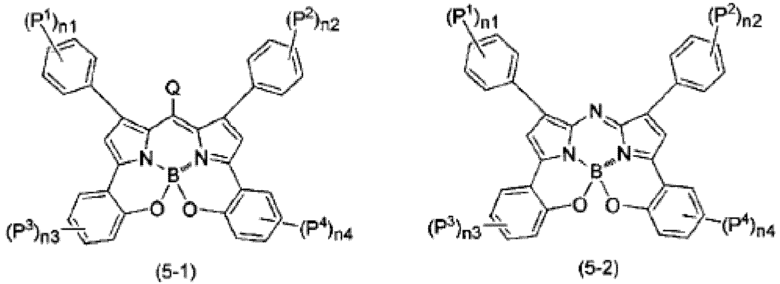

In General Formulas (1-1) to (1-37), (2-1) to (2-7), (3-1) to (3-37), (4-1) to (4-7), (5-1), and (5-2), each of P¹ to P⁴ and P¹⁸ independently represents a halogen atom, a C₁₋₂₀ alkyl group, a C₁₋₂₀ alkoxy group, an amino group, a monoalkylamino group, or a dialkylamino group. Examples of the C₁₋₂₀ alkyl group, the C₁₋₂₀ alkoxy group, the monoalkylamino group, or the dialkylamino group represented by each of P¹ to P⁴ include the same as those exemplified as R^{g}, (p1) to (p3), or (q1) to (q3). Each of P¹ to P⁴ and P¹⁸ is preferably a C₁₋₂₀ alkyl group, a C₁₋₂₀ alkoxy group, an (unsubstituted) phenyl group, a p-methoxyphenyl group, a p-ethoxyphenyl group, a p-dimethylaminophenyl group, a dimethoxyphenyl group, a thienyl group, or a furanyl group, more preferably a C₁₋₂₀ alkyl group, a C₁₋₂₀ alkoxy group, a phenyl group, a p-methoxyphenyl group, a p-ethoxyphenyl group, a dimethoxyphenyl group, a thienyl group, or a furanyl group from the viewpoint of safety with respect to a living body, and these substituents may further have a substituent. Here, since, even in the case of a substituent other than these substituents, it is possible to improve safety by further introducing a suitable substituent, the present invention is not limited to these substituents.

In General Formulas (1-1) to (1-37), (2-1) to (2-7), (3-1) to (3-37), (4-1) to (4-7), (5-1), and (5-2), each of n1 to n4 and n18 independently represents an integer of 0 to 3. In a case where a plurality of P¹s are present in one molecule (that is, in a case where n1 is 2 or 3), all of the plurality of P¹s may be the same type of functional groups, or may be different types of functional groups. The same applies to P² to P⁴ and P¹⁸.

In General Formulas (1-1) to (1-37), (2-1) to (2-7) and (5-1), Q represents a trifluoromethyl group, a cyano group, a nitro group, or a phenyl group which may have a substituent, preferably a trifluoromethyl group or a phenyl group which may have a substituent, and more preferably a trifluoromethyl group or an unsubstituted phenyl group. Examples of the substituent which the phenyl group may have include a halogen atom, a C₁₋₂₀ alkyl group, a C₁₋₂₀ alkoxy group, an amino group, a monoalkylamino group, and a dialkylamino group.

In General Formula (1-1) to (1-31), (2-1) to (2-7), (3-1) to (3-31), and (4-1) to (4-7), X is the same as that in General Formulas (II₁-1-1) and the like. As the compound represented by General Formula (1-1) or the like, a compound in which X is a halogen atom is preferable, and a compound in which X is a fluorine atom is particularly preferable.

In General Formulas (1-32) to (1-34) and (3-32) to (3-34), m2 is 0 or 1. As the compound represented by General Formula (1-32) or the like, a compound in which m2 is 1 is preferable.

As the compound represented by General Formula (1-1) to (1-37), (2-1) to (2-7), or (5-1), a compound in which each of P¹ to P⁴ and P¹⁸ is independently a C₁₋₂₀ alkyl group, a C₁₋₂₀ alkoxy group, an (unsubstituted) phenyl group, a p-methoxyphenyl group, a p-ethoxyphenyl group, a p-dimethylaminophenyl group, a dimethoxyphenyl group, a thienyl group, or a furanyl group, each of n1 to n4 and n18 is independently 0 to 2, and Q is a trifluoromethyl group or a phenyl group is preferable. Similarly, as the compound represented by General Formula (3-1) to (3-37), (4-1) to (4-7), or (5-2), a compound in which each of P¹ to P⁴ and P¹⁸ is independently a C₁₋₂₀ alkyl group, a C₁₋₂₀ alkoxy group, an (unsubstituted) phenyl group, a p-methoxyphenyl group, a p-ethoxyphenyl group, a p-dimethylaminophenyl group, a dimethoxyphenyl group, a thienyl group, or a furanyl group, and each of n1 to n4 and n18 is independently 0 to 2 is preferable.

As the near-infrared fluorescent material according to the present invention, a compound represented by any one of the following General Formulas (II₃-1) to (II₃-6) or a compound represented by any one of General Formulas (II₄-1) to (II₄-6) is also preferable since the maximum wavelength is a longer wavelength.

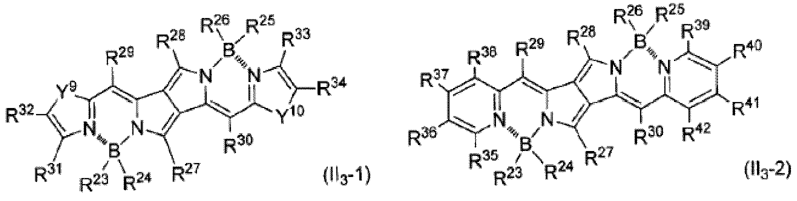

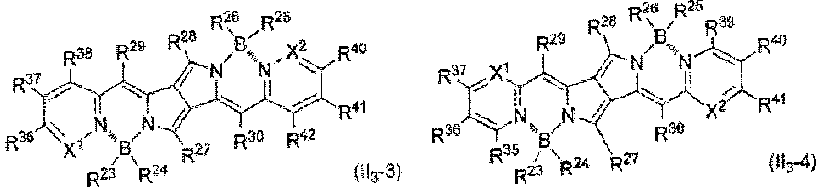

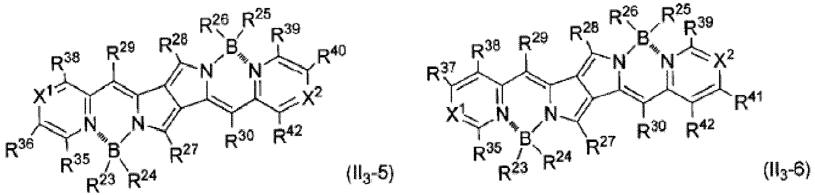

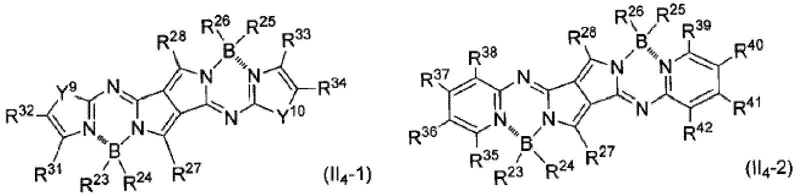

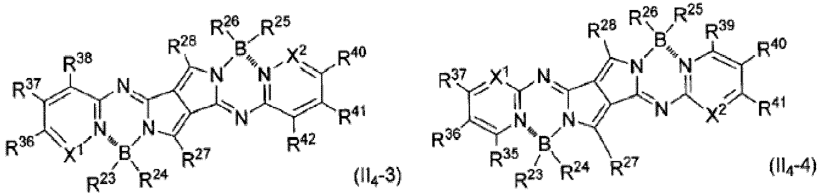

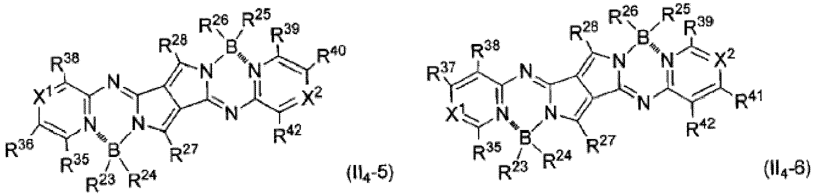

In General Formulas (II₃-1) to (II₃-6) and (II₄-1) to (II₄-6), each of R²³, R²⁴, R²⁵, and R²⁶ independently represents a halogen atom, a C₁₋₂₀ alkyl group, a C₁₋₂₀ alkoxy group, an aryl group, or a heteroaryl group. Examples of the halogen atoms, the C₁₋₂₀ alkyl group, the C₁₋₂₀ alkoxy group, the aryl group, or the heteroaryl group represented by each of R²³, R²⁴, R²⁵, and R²⁶ include the same as those represented by each of R¹, R^{m}, Rⁿ, and R^{o} in General Formula (II₃). As the compound represented by any one of General Formulas (II₃-1) to (II₃-6) or the compound represented by any one of General Formulas (II₄-1) to (II₄-6), from the viewpoint of high thermal stability of a compound, a compound in which each of R²³, R²⁴, R²⁵, and R²⁶ is a halogen atom, an unsubstituted aryl group, or an aryl group having a substituent is preferable, specifically, a compound in which each of R²³, R²⁴, R²⁵, and R²⁶ is a fluorine atom, a chlorine atom, a bromine atom, an unsubstituted phenyl group, or a phenyl group substituted with a C₁₋₂₀ alkyl group or a C₁₋₂₀ alkoxy group is preferable, a compound in which each of R²³, R²⁴, R²⁵, and R²⁶ is a fluorine atom, a chlorine atom, an unsubstituted phenyl group, or a phenyl group substituted with a C₁₋₁₀ alkyl or a C₁₋₁₀ alkoxy group is more preferable, and from the viewpoint of obtaining a compound having both high light-emitting efficiency and thermal stability, a compound in which each of R²³, R²⁴, R²⁵, and R²⁶ is a fluorine atom or an unsubstituted phenyl group is particularly preferable.

In General Formulas (II₃-1) to (II₃-6) and (II₄-1) to (II₄-6), each of R²⁷ and R²⁸ independently represents a hydrogen atom, a halogen atom, a C₁₋₂₀ alkyl group, a C₁₋₂₀ alkoxy group, an aryl group, or a heteroaryl group. Examples of the halogen atoms, the C₁₋₂₀ alkyl group, the C₁₋₂₀ alkoxy group, the aryl group, or the heteroaryl group represented by R²⁷ or R²⁸ include the same as those represented by R^{p} or R^{q} in General Formula (II₃). As the compound represented by any one of General Formulas (II₃-1) to (II₃-6) or the compound represented by any one of General Formulas (II₄-1) to (II₄-6), a compound in which each of R²⁷ and R²⁸ is a hydrogen atom or an aryl group is preferable, from the viewpoint of obtaining a compound having high light-emitting efficiency, a compound in which each of R²⁷ and R²⁸ is a hydrogen atom, an unsubstituted phenyl group, or a phenyl group substituted with a C₁₋₂₀ alkyl group or a C₁₋₂₀ alkoxy group is preferable, a compound in which each of R²⁷ and R²⁸ is a hydrogen atom, an unsubstituted phenyl group, or a phenyl group substituted with a linear or branched C₁₋₂₀ alkoxy group is more preferable, and from the viewpoint of obtaining a compound having high light-emitting efficiency and excellent compatibility with respect to a resin, a compound in which each of R²⁷ and R²⁸ is a hydrogen atom, an unsubstituted phenyl group, or a phenyl group substituted with a linear or branched C₁₋₁₀ alkoxy group is particularly preferable.

In General Formulas (II₃-1) to (II₃-6), each of R²⁹ and R³⁰ independently represents a hydrogen atom or an electron-withdrawing group. Examples of the electron-withdrawing group represented by R²⁹ or R³⁰ include the same as those represented by R^{r} or R^{s} in General Formula (II₃). As the compound represented by any one of General Formulas (II₃-1) to (II₃-6), from the viewpoint of obtaining a compound having high light-emitting efficiency, a compound in which each of R²⁹ and R³⁰ is a fluoroalkyl group, a nitro group, a cyano group, or an aryl group which can function as a strong electron-withdrawing group is preferable, a compound in which each of R²⁹ and R³⁰ is a trifluoromethyl group, a nitro group, a cyano group, or a phenyl group which may have a substituent is more preferable, and from the viewpoint of obtaining a compound having high light-emitting efficiency and excellent compatibility with respect to a resin, a compound in which each of R²⁹ and R³⁰ is a trifluoromethyl group or a cyano group is still more preferable.

In General Formulas (II₃-1) and (II₄-1), each of Y⁹ and Y¹⁰ independently represents a sulfur atom, an oxygen atom, a nitrogen atom, or a phosphorus atom. As the compound represented by General Formulas (II₃-1) or (II₄-1), from the viewpoint of obtaining a compound having high light-emitting efficiency, a compound in which each of Y⁹ and Y¹⁰ is independently a sulfur atom, an oxygen atom, or a nitrogen atom is preferable, a compound in which each of Y⁹ and Y¹⁰ is independently a sulfur atom or an oxygen atom is more preferable, and from the viewpoint of obtaining a compound having both high light-emitting efficiency and thermal stability, a compound in which Y⁹ and Y¹⁰ together are sulfur atoms or oxygen atoms is still more preferable.

In General Formulas (II₃-3) and (II₃-6) and (II₄-3) to (II₄-6), each of X¹ and X² independently represents a nitrogen atom or a phosphorus atom. As the compound represented by General Formulas (II₃-3) or (II₃-6) and (II₄-3) to (II₄-6), from the viewpoint of obtaining a compound having high light-emitting efficiency, a compound in which X¹ and X² together are nitrogen atoms or phosphorus atoms is preferable, and
and from the viewpoint of obtaining a compound having both high light-emitting efficiency and thermal stability, a compound in which X¹ and X² together are nitrogen atoms is more preferable.

In General Formulas (II₃-1) and (II₄-1), R³¹ and R³² satisfy the following (p4) or (p5).
(p4) each of R³¹ and R³² independently represents a hydrogen atom, a halogen atom, a C₁₋₂₀ alkyl group, a C₁₋₂₀ alkoxy group, an aryl group, or a heteroaryl group.
(p5) R³¹ and R³² together form an aromatic 5-membered ring which may have a substituent or an aromatic 6-membered ring which may have a substituent.

In General Formulas (II₃-1) and (II₄-1), R³³ and R³⁴ satisfy the following (q4) or (q5).
(q4) each of R³³ and R³⁴ independently represents a hydrogen atom, a halogen atom, a C₁₋₂₀ alkyl group, a C₁₋₂₀ alkoxy group, an aryl group, or a heteroaryl group, or (q5) R³³ and R³⁴ together form an aromatic 5-membered ring which may have a substituent or an aromatic 6-membered ring which may have a substituent.

In General Formulas (II₃-2) to (II₃-6) and (II₄-2) to (II₄-6), R³⁵, R³⁶, R³⁷, and R³⁸ satisfy any one of the following (p6) to (p9).
(p6) each of R³⁵, R³⁶, R³⁷, and R³⁸ independently represents a hydrogen atom, a halogen atom, a C₁₋₂₀ alkyl group, a C₁₋₂₀ alkoxy group, an aryl group, or a heteroaryl group.
(p7) R³⁵ and R³⁶ together form an aromatic 5-membered ring which may have a substituent or an aromatic 6-membered ring which may have a substituent, and each of R³⁷ and R³⁸ independently represents a hydrogen atom, a halogen atom, a C₁₋₂₀ alkyl group, a C₁₋₂₀ alkoxy group, an aryl group, or a heteroaryl group.
(p8) R³⁶ and R³⁷ together form an aromatic 5-membered ring which may have a substituent or an aromatic 6-membered ring which may have a substituent, and each of R³⁵ and R³⁸ independently represents a hydrogen atom, a halogen atom, a C₁₋₂₀ alkyl group, a C₁₋₂₀ alkoxy group, an aryl group, or a heteroaryl group.
(p9) R³⁷ and R³⁸ together form an aromatic 5-membered ring which may have a substituent or an aromatic 6-membered ring which may have a substituent, and each of R³⁵ and R³⁶ independently represents a hydrogen atom, a halogen atom, a C₁₋₂₀ alkyl group, a C₁₋₂₀ alkoxy group, an aryl group, or a heteroaryl group.

In General Formulas (II₃-2) to (II₃-6) and (II₄-2) to (II₄-6), R³⁹, R⁴⁰, R⁴¹, and R⁴² satisfy any one of the following (q6) to (q9).
(q6) each of R³⁹, R⁴⁰, R⁴¹, and R⁴² independently represents a hydrogen atom, a halogen atom, a C₁₋₂₀ alkyl group, a C₁₋₂₀ alkoxy group, an aryl group, or a heteroaryl group.
(q7) R³⁹ and R⁴⁰ together form an aromatic 5-membered ring which may have a substituent or an aromatic 6-membered ring which may have a substituent, and each of R⁴¹ and R⁴² independently represents a hydrogen atom, a halogen atom, a C₁₋₂₀ alkyl group, a C₁₋₂₀ alkoxy group, an aryl group, or a heteroaryl group.
(q8) R⁴⁰ and R⁴¹ together form an aromatic 5-membered ring which may have a substituent or an aromatic 6-membered ring which may have a substituent, and each of R³⁹ and R⁴² independently represents a hydrogen atom, a halogen atom, a C₁₋₂₀ alkyl group, a C₁₋₂₀ alkoxy group, an aryl group, or a heteroaryl group.
(q9) R⁴¹ and R⁴² together form an aromatic 5-membered ring which may have a substituent or an aromatic 6-membered ring which may have a substituent, and each of R³⁹ and R⁴⁰ independently represents a hydrogen atom, a halogen atom, a C₁₋₂₀ alkyl group, a C₁₋₂₀ alkoxy group, an aryl group, or a heteroaryl group.

As the halogen atom, the C₁₋₂₀ alkyl group, the C₁₋₂₀ alkoxy group, the aryl group, or the heteroaryl group in (p4), (p6) to (p9), (q4), or (q6) to (q9), those exemplified as "any group which does not inhibit fluorescence of a compound" represented by each of R^{a} and R^{b} can be used.

In (p5), (p7) to (p9), (q5), (q7) to (q9), as an aromatic 5-membered ring or an aromatic 6-membered ring which R³¹ and R³² together form, an aromatic 5-membered ring or an aromatic 6-membered ring which R³³ and R³⁴ together form, an aromatic 5-membered ring or an aromatic 6-membered ring which R³⁵ and R³⁶ together form, an aromatic 5-membered ring or an aromatic 6-membered ring which R³⁶ and R³⁷ together form, an aromatic 5-membered ring or an aromatic 6-membered ring which R³⁷ and R³⁸ together form, an aromatic 5-membered ring or an aromatic 6-membered ring which R³⁹ and R⁴⁰ together form, an aromatic 5-membered ring or an aromatic 6-membered ring which R⁴⁰ and R⁴¹ together form, or an aromatic 5-membered ring or an aromatic 6-membered ring which R⁴¹ and R⁴² together form, the ring represented by any one of General Formulas (C-1) to (C-9) is preferable, and the ring represented by General Formula (C-9) is more preferable since a compound having high thermal stability can be obtained.

As the compound represented by (II₃-1), a compound in which R²³, R²⁴, R²⁵, and R²⁶ together are halogen atoms, unsubstituted phenyl groups, or phenyl groups substituted with a C₁₋₁₀ alkyl group or a C₁₋₁₀ alkoxy group; R²⁷ and R²⁸ together are hydrogen atoms, unsubstituted phenyl groups, or phenyl groups substituted with a C₁₋₂₀ alkyl group or a C₁₋₂₀ alkoxy group; R²⁹ and R³⁰ together are trifluoromethyl groups, nitro groups, cyano groups, or phenyl groups; Y⁹ and Y¹⁰ together are sulfur atoms or oxygen atoms; each of R³¹ and R³² is independently a hydrogen atom or a C₁₋₂₀ alkyl group, or R³¹ and R³² together form a phenyl group which may have a substituent; and each of R³³ and R³⁴ is independently a hydrogen atom or a C₁₋₂₀ alkyl group or R³³ and R³⁴ together form a phenyl group which may have a substituent is preferable, and a compound in which R²³, R²⁴, R²⁵, and R²⁶ together are halogen atoms or unsubstituted phenyl groups; R²⁷ and R²⁸ together are hydrogen atoms, unsubstituted phenyl groups, or phenyl groups substituted with a linear or branched C₁₋₂₀ alkoxy group; R²⁹ and R³⁰ together are trifluoromethyl groups, nitro groups, or cyano groups; Y⁹ and Y¹⁰ together are sulfur atoms or oxygen atoms; each of R³¹ and R³² is independently a hydrogen atom or a C₁₋₂₀ alkyl group or R³¹ and R³² together form an unsubstituted phenyl group or a phenyl group substituted with a C₁₋₁₀ alkyl group; and each of R³³ and R³⁴ is independently a hydrogen atom or a C₁₋₂₀ alkyl group, or R³³ and R³⁴ together form a phenyl group substituted with a C₁₋₁₀ alkyl group is more preferable since the light-emitting efficiency is high and the compatibility with respect to a resin is excellent.

As the compound represented by (II₃-2), a compound in which R²³, R²⁴, R²⁵, and R²⁶ together are halogen atoms, unsubstituted phenyl groups, or phenyl groups substituted with a C₁₋₁₀ alkyl group or a C₁₋₁₀ alkoxy group; R²⁷ and R²⁸ together are hydrogen atoms, unsubstituted phenyl groups, or phenyl groups substituted with a C₁₋₂₀ alkyl group or a C₁₋₂₀ alkoxy group; R²⁹ and R³⁰ together are trifluoromethyl groups, nitro groups, cyano groups, or phenyl groups; each of R³⁵, R³⁶, R³⁷, and R³⁸ is independently a hydrogen atom or a C₁₋₂₀ alkyl group or R³⁵ and R³⁶ together form a phenyl group which may have a substituent, each of R³⁷ and R³⁸ is independently a hydrogen atom or a C₁₋₂₀ alkyl group or R³⁶ and R³⁷ together form a phenyl group which may have a substituent, each of R³⁵ and R³⁸ is independently a hydrogen atom or a C₁₋₂₀ alkyl group or R³⁷ and R³⁸ together form a phenyl group which may have a substituent, and each of R³⁵ and R³⁶ is independently a hydrogen atom or a C₁₋₂₀ alkyl group; each of R³⁹, R⁴⁰, R⁴¹, and R⁴² is independently a hydrogen atom or a C₁₋₂₀ alkyl group or R³⁹ and R⁴⁰ together form a phenyl group which may have a substituent, each of R⁴¹ and R⁴² is independently a hydrogen atom or a C₁₋₂₀ alkyl group or R⁴⁰ and R⁴¹ together form a phenyl group which may have a substituent, each of R³⁹ and R⁴² is independently a hydrogen atom or a C₁₋₂₀ alkyl group, or R⁴¹ and R⁴² together form a phenyl group which may have a substituent, and each of R³⁹ and R⁴⁰ is a hydrogen atom or a C₁₋₂₀ alkyl group is preferable, and a compound in which R²³, R²⁴, R²⁵, and R²⁶ together are halogen atoms or unsubstituted phenyl groups; R²⁷ and R²⁸ together are hydrogen atoms, unsubstituted phenyl groups, or phenyl groups substituted with a linear or branched C₁₋₂₀ alkoxy group; R²⁹ and R³⁰ together are trifluoromethyl groups, nitro groups, or cyano groups; each of R³⁵, R³⁶, R³⁷, and R³⁸ is independently a hydrogen atom or a C₁₋₂₀ alkyl group or R³⁵ and R³⁶ together form an unsubstituted phenyl group or a phenyl group substituted with a C₁₋₁₀ alkyl group; each of R³⁷ and R³⁸ is independently a hydrogen atom or a C₁₋₂₀ alkyl group or R³⁶ and R³⁷ together form an unsubstituted phenyl group or a phenyl group substituted with a C₁₋₁₀ alkyl group, each of R³⁵ and R³⁸ is independently a hydrogen atom or a C₁₋₂₀ alkyl group or R³⁷ and R³⁸ together form an unsubstituted phenyl group or a phenyl group substituted with a C₁₋₁₀ alkyl group, and each of R³⁵ and R³⁶ is independently a hydrogen atom or a C₁₋₂₀ alkyl group; each of R³⁹, R⁴⁰, R⁴¹, and R⁴² is independently a hydrogen atom or a C₁₋₂₀ alkyl group or R³⁹ and R⁴⁰ together form an unsubstituted phenyl group or a phenyl group substituted with a C₁₋₁₀ alkyl group, each of R⁴¹ and R⁴² is independently a hydrogen atom or a C₁₋₂₀ alkyl group or R⁴⁰ and R⁴¹ together form an unsubstituted phenyl group or a phenyl group substituted with a C₁₋₁₀ alkyl group, each of R³⁹ and R⁴² is independently a hydrogen atom or a C₁₋₂₀ alkyl group or R⁴¹ and R⁴² together form an unsubstituted phenyl group or a phenyl group substituted with a C₁₋₁₀ alkyl group, and each of R³⁹ and R⁴⁰ is independently a hydrogen atom or a C₁₋₂₀ alkyl group is more preferable since the light-emitting efficiency is high and the compatibility with respect to a resin is excellent.

As the compound represented by (II₃-3), a compound in which R²³, R²⁴, R²⁵, and R²⁶ together are halogen atoms, unsubstituted phenyl groups, or phenyl groups substituted with a C₁₋₁₀ alkyl group or a C₁₋₁₀ alkoxy group; R²⁷ and R²⁸ together are hydrogen atoms, unsubstituted phenyl groups, or phenyl groups substituted with a C₁₋₂₀ alkyl group or a C₁₋₂₀ alkoxy group; R²⁹ and R³⁰ together are trifluoromethyl groups, nitro groups, cyano groups, or phenyl groups; X¹ and X² together are nitrogen atoms; each of R³⁶, R³⁷, and R³⁸ is independently a hydrogen atom or a C₁₋₂₀ alkyl group or R³⁶ and R³⁷ together form a phenyl group which may have a substituent, R³⁸ is a hydrogen atom or a C₁₋₂₀ alkyl group or R³⁷ and R³⁸ together form a phenyl group which may have a substituent, and R³⁶ is a hydrogen atom or a C₁₋₂₀ alkyl group; each of R⁴⁰, R⁴¹, and R⁴² is independently a hydrogen atom or a C₁₋₂₀ alkyl group or R⁴⁰ and R⁴¹ together form a phenyl group which may have a substituent, R⁴² is a hydrogen atom or a C₁₋₂₀ alkyl group or R⁴¹ and R⁴² together form a phenyl group which may have a substituent, and R⁴⁰ is a hydrogen atom or a C₁₋₂₀ alkyl group is preferable, and a compound in which R²³, R²⁴, R²⁵, and R²⁶ together are halogen atoms or unsubstituted phenyl groups; R²⁷ and R²⁸ together are hydrogen atoms, unsubstituted phenyl groups, or phenyl groups substituted with a linear or branched C₁₋₂₀ alkoxy group; R²⁹ and R³⁰ together are trifluoromethyl groups, nitro groups, or cyano groups; X¹ and X² together are nitrogen atoms; each of R³⁶, R³⁷, and R³⁸ is independently a hydrogen atom or a C₁₋₂₀ alkyl group or R³⁶ and R³⁷ together form an unsubstituted phenyl group or a phenyl group substituted with a C₁₋₁₀ alkyl group, R³⁸ is a hydrogen atom or a C₁₋₂₀ alkyl group or R³⁷ and R³⁸ together form an unsubstituted phenyl group or a phenyl group substituted with a C₁₋₁₀ alkyl group, and R³⁶ is a hydrogen atom or a C₁₋₂₀ alkyl group; each of R⁴⁰, R⁴¹ and R⁴² is independently a hydrogen atom or a C₁₋₂₀ alkyl group or R⁴⁰ and R⁴¹ together form an unsubstituted phenyl group or a phenyl group substituted with a C₁₋₁₀ alkyl group, R⁴² is a hydrogen atom or a C₁₋₂₀ alkyl group or R⁴¹ and R⁴² together form an unsubstituted phenyl group or a phenyl group substituted with a C₁₋₁₀ alkyl group, and R⁴⁰ is a hydrogen atom or a C₁₋₂₀ alkyl group is more preferable since the light-emitting efficiency is high and the compatibility with respect to a resin is excellent.

As the compound represented by (II₃-4), a compound in which R²³, R²⁴, R²⁵, and R²⁶ together are halogen atoms, unsubstituted phenyl groups, or phenyl groups substituted with a C₁₋₁₀ alkyl group or a C₁₋₁₀ alkoxy group; R²⁷ and R²⁸ together are hydrogen atoms, unsubstituted phenyl groups, or phenyl groups substituted with a C₁₋₂₀ alkyl group or a C₁₋₂₀ alkoxy group; R²⁹ and R³⁰ together are trifluoromethyl groups, nitro groups, cyano groups, or phenyl groups; X¹ and X² together are nitrogen atoms; each of R³⁵, R³⁶, and R³⁷ is independently a hydrogen atom or a C₁₋₂₀ alkyl group or R³⁵ and R³⁶ together form a phenyl group which may have a substituent, R³⁷ is a hydrogen atom or a C₁₋₂₀ alkyl group or R³⁶ and R³⁷ together form a phenyl group which may have a substituent, and R³⁵ is a hydrogen atom or a C₁₋₂₀ alkyl group; each of R³⁹, R⁴⁰, and R⁴¹ is independently a hydrogen atom or a C₁₋₂₀ alkyl group or R³⁹ and R⁴⁰ together form a phenyl group which may have a substituent, R⁴¹ is a hydrogen atom or a C₁₋₂₀ alkyl group or R⁴⁰ and R⁴¹ together form a phenyl group which may have a substituent, and R³⁹ is a hydrogen atom or a C₁₋₂₀ alkyl group is preferable, and a compound in which R²³, R²⁴, R²⁵, and R²⁶ together are halogen atoms or unsubstituted phenyl groups; R²⁷ and R²⁸ together are hydrogen atoms, unsubstituted phenyl groups, or phenyl groups substituted with a linear or branched C₁₋₂₀ alkoxy group; R²⁹ and R³⁰ together are trifluoromethyl groups, nitro groups, or cyano groups; X¹ and X² together are nitrogen atoms; each of R³⁵, R³⁶, and R³⁷ is independently a hydrogen atom or a C₁₋₂₀ alkyl group or R³⁵ and R³⁶ together form an unsubstituted phenyl group or a phenyl group substituted with a C₁₋₁₀ alkyl group, R³⁷ is a hydrogen atom or a C₁₋₂₀ alkyl group or R³⁶ and R³⁷ together form an unsubstituted phenyl group or a phenyl group substituted with a C₁₋₁₀ alkyl group, and R³⁵ is a hydrogen atom or a C₁₋₂₀ alkyl group; each of R³⁹, R⁴⁰, and R⁴¹ is independently a hydrogen atom or a C₁₋₂₀ alkyl group or R³⁹ and R⁴⁰ together form an unsubstituted phenyl group or a phenyl group substituted with a C₁₋₁₀ alkyl group, R⁴¹ is a hydrogen atom or a C₁₋₂₀ alkyl group or R⁴⁰ and R⁴¹ together form an unsubstituted phenyl group or a phenyl group substituted with a C₁₋₁₀ alkyl group, and R³⁹ is a hydrogen atom or a C₁₋₂₀ alkyl group is more preferable since the light-emitting efficiency is high and the compatibility with respect to a resin is excellent.

As the compound represented by (II₃-5), a compound in which R²³, R²⁴, R²⁵, and R²⁶ together are halogen atoms, unsubstituted phenyl groups, or phenyl groups substituted with a C₁₋₁₀ alkyl group or a C₁₋₁₀ alkoxy group; R²⁷ and R²⁸ together are hydrogen atoms, unsubstituted phenyl groups, or phenyl groups substituted with a C₁₋₂₀ alkyl group or a C₁₋₂₀ alkoxy group; R²⁹ and R³⁰ together are trifluoromethyl groups, nitro groups, cyano groups, or phenyl groups; X¹ and X² together are nitrogen atoms; each of R³⁵, R³⁶, and R³⁸ is independently a hydrogen atom or a C₁₋₂₀ alkyl group or R³⁵ and R³⁶ together form a phenyl group which may have a substituent, and R³⁸ is a hydrogen atom or a C₁₋₂₀ alkyl group; each of R³⁹, R⁴⁰, and R⁴² is independently a hydrogen atom or a C₁₋₂₀ alkyl group or R³⁹ and R⁴⁰ together form a phenyl group which may have a substituent, and R⁴² is a hydrogen atom or a C₁₋₂₀ alkyl group is preferable, and a compound in which R²³, R²⁴, R²⁵, and R²⁶ together are halogen atoms or unsubstituted phenyl groups; R²⁷ and R²⁸ together are hydrogen atoms, unsubstituted phenyl groups, or phenyl groups substituted with a linear or branched C₁₋₂₀ alkoxy group; R²⁹ and R³⁰ together are trifluoromethyl groups, nitro groups, or cyano groups; X¹ and X² together are nitrogen atoms; each of R³⁵, R³⁶, and R³⁸ is independently a hydrogen atom or a C₁₋₂₀ alkyl group or R³⁵ and R³⁶ together form an unsubstituted phenyl group or a phenyl group substituted with a C₁₋₁₀ alkyl group, and R³⁸ is a hydrogen atom or a C₁₋₂₀ alkyl group; each of R³⁹, R⁴⁰, and R⁴² is independently a hydrogen atom or a C₁₋₂₀ alkyl group or R³⁹ and R⁴⁰ together form an unsubstituted phenyl group or a phenyl group substituted with a C1-10 alkyl group, and R⁴² is a hydrogen atom or a C₁₋₂₀ alkyl group is preferable since the light-emitting efficiency is high and the compatibility with respect to a resin is excellent.

As the compound represented by (II₃₋₆), a compound in which R²³, R²⁴, R²⁵, and R²⁶ together are halogen atoms, unsubstituted phenyl groups, or phenyl groups substituted with a C₁₋₁₀ alkyl group or a C₁₋₁₀ alkoxy group; R²⁷ and R²⁸ together are hydrogen atoms, unsubstituted phenyl groups, or phenyl groups substituted with a C₁₋₂₀ alkyl group or a C₁₋₂₀ alkoxy group; R²⁹ and R³⁰ together are trifluoromethyl groups, nitro groups, cyano groups, or phenyl groups; X¹ and X² together are nitrogen atoms; each of R³⁵, R³⁷, and R³⁸ is independently a hydrogen atom or a C₁₋₂₀ alkyl group or R³⁷ and R³⁸ together form a phenyl group which may have a substituent, and R³⁵ is a hydrogen atom or a C₁₋₂₀ alkyl group; each of R³⁹, R⁴¹, and R⁴² is independently a hydrogen atom or a C₁₋₂₀ alkyl group or R⁴¹ and R⁴² together form a phenyl group which may have a substituent, and R³⁹ is a hydrogen atom or a C₁₋₂₀ alkyl group is preferable, and a compound in which R²³, R²⁴, R²⁵, and R²⁶ together are halogen atoms or unsubstituted phenyl groups; R²⁷ and R²⁸ together are hydrogen atoms, unsubstituted phenyl groups, or phenyl groups substituted with a linear or branched C₁₋₂₀ alkoxy group; R²⁹ and R³⁰ together are trifluoromethyl groups, nitro groups, or cyano groups; X¹ and X² together are nitrogen atoms; each of R³⁵, R³⁷, and R³⁸ is independently a hydrogen atom or a C₁₋₂₀ alkyl group or R³⁷ and R³⁸ together form an unsubstituted phenyl group or a phenyl group substituted with a C₁₋₁₀ alkyl group, and R³⁵ is a hydrogen atom or a C₁₋₂₀ alkyl group; each of R³⁹, R⁴¹, and R⁴² is independently a hydrogen atom or a C₁₋₂₀ alkyl group or R⁴¹ and R⁴² together form an unsubstituted phenyl group or a phenyl group substituted with a C₁₋₁₀ alkyl group, and R³⁹ is a hydrogen atom or a C₁₋₂₀ alkyl group is more preferable since the light-emitting efficiency is high and the compatibility with respect to a resin is excellent.

As the compound represented by (II₄₋1) a compound in which R²³, R²⁴, R²⁵, and R²⁶ together are halogen atoms, unsubstituted phenyl groups, or phenyl groups substituted with a C₁₋₁₀ alkyl group or a C₁₋₁₀ alkoxy group; R²⁷ and R²⁸ together are hydrogen atoms, unsubstituted phenyl groups, or phenyl groups substituted with a C₁₋₂₀ alkyl group or a C₁₋₂₀ alkoxy group; Y⁹ and Y¹⁰ together are sulfur atoms or oxygen atoms; each of R³¹ and R³² is independently a hydrogen atom or a C₁₋₂₀ alkyl group, or R³¹ and R³² together form a phenyl group which may have a substituent; and each of R³³ and R³⁴ is independently a hydrogen atom or a C₁₋₂₀ alkyl group or R³³ and R³⁴ together form a phenyl group which may have a substituent is preferable, and a compound in which R²³, R²⁴, R²⁵, and R²⁶ together are halogen atoms or unsubstituted phenyl groups; R²⁷ and R²⁸ together are hydrogen atoms, unsubstituted phenyl groups, or phenyl groups substituted with a linear or branched C₁₋₂₀ alkoxy group; Y⁹ and Y¹⁰ together are sulfur atoms or oxygen atoms; each of R³¹ and R³² is independently a hydrogen atom or a C₁₋₂₀ alkyl group or R³¹ and R³² together form an unsubstituted phenyl group or a phenyl group substituted with a C₁₋₁₀ alkyl group; and each of R³³ and R³⁴ is independently a hydrogen atom or a C₁₋₂₀ alkyl group, or R³³ and R³⁴ together form a phenyl group substituted with a C₁₋₁₀ alkyl group is more preferable since the light-emitting efficiency is high and the compatibility with respect to a resin is excellent.

As the compound represented by (II₄-2), a compound in which R²³, R²⁴, R²⁵, and R²⁶ together are halogen atoms, unsubstituted phenyl groups, or phenyl groups substituted with a C₁₋₁₀ alkyl group or a C₁₋₁₀ alkoxy group; R²⁷ and R²⁸ together are hydrogen atoms, unsubstituted phenyl groups, or phenyl groups substituted with a C₁₋₂₀ alkyl group or a C₁₋₂₀ alkoxy group; each of R³⁵, R³⁶, R³⁷, and R³⁸ is independently a hydrogen atom or a C₁₋₂₀ alkyl group or R³⁵ and R³⁶ together form a phenyl group which may have a substituent, each of R³⁷ and R³⁸ is independently a hydrogen atom or a C₁₋₂₀ alkyl group or R³⁶ and R³⁷ together form a phenyl group which may have a substituent, each of R³⁵ and R³⁸ is independently a hydrogen atom or a C₁₋₂₀ alkyl group or R³⁷ and R³⁸ together form a phenyl group which may have a substituent, and each of R³⁵ and R³⁸ is independently a hydrogen atom or a C₁₋₂₀ alkyl group; each of R³⁹, R⁴⁰, R⁴¹, and R⁴² is independently a hydrogen atom or a C₁₋₂₀ alkyl group or R³⁹ and R⁴⁰ together form a phenyl group which may have a substituent, each of R⁴¹ and R⁴² is independently a hydrogen atom or a C₁₋₂₀ alkyl group or R⁴⁰ and R⁴¹ together form a phenyl group which may have a substituent, each of R³⁹ and R⁴² is independently a hydrogen atom or a C₁₋₂₀ alkyl group, or R⁴¹ and R⁴² together form a phenyl group which may have a substituent, and each of R³⁹ and R⁴² is independently a hydrogen atom or a C₁₋₂₀ alkyl group is preferable, and a compound in which R²³, R²⁴, R²⁵, and R²⁶ together are halogen atoms or unsubstituted phenyl groups; R²⁷ and R²⁸ together are hydrogen atoms, unsubstituted phenyl groups, or phenyl groups substituted with a linear or branched C₁₋₂₀ alkoxy group; each of R³⁵, R³⁶, R³⁷, and R³⁸ is independently a hydrogen atom or a C₁₋₂₀ alkyl group or R³⁵ and R³⁶ together form an unsubstituted phenyl group or a phenyl group substituted with a C₁₋₁₀ alkyl group; each of R³⁷ and R³⁸ is independently a hydrogen atom or a C₁₋₂₀ alkyl group or R³⁶ and R³⁷ together form an unsubstituted phenyl group or a phenyl group substituted with a C₁₋₁₀ alkyl group, each of R³⁵ and R³⁸ is independently a hydrogen atom or a C₁₋₂₀ alkyl group or R³⁷ and R³⁸ together form an unsubstituted phenyl group or a phenyl group substituted with a C₁₋₁₀ alkyl group, and each of R³⁵ and R³⁸ is independently a hydrogen atom or a C₁₋₂₀ alkyl group; each of R³⁹, R⁴⁰, R⁴¹, and R⁴² is independently a hydrogen atom or a C₁₋₂₀ alkyl group or R³⁹ and R⁴⁰ together form an unsubstituted phenyl group or a phenyl group substituted with a C₁₋₁₀ alkyl group, each of R⁴¹ and R⁴² is independently a hydrogen atom or a C₁₋₂₀ alkyl group or R⁴⁰ and R⁴¹ together form an unsubstituted phenyl group or a phenyl group substituted with a C₁₋₁₀ alkyl group, each of R³⁹ and R⁴² is independently a hydrogen atom or a C₁₋₂₀ alkyl group or R⁴¹ and R⁴² together form an unsubstituted phenyl group or a phenyl group substituted with a C₁₋₁₀ alkyl group, and each of R³⁹ and R⁴² is independently a hydrogen atom or a C₁₋₂₀ alkyl group is more preferable since the light-emitting efficiency is high and the compatibility with respect to a resin is excellent.

As the compound represented by (II₄-3), a compound in which R²³, R²⁴, R²⁵, and R²⁶ together are halogen atoms, unsubstituted phenyl groups, or phenyl groups substituted with a C₁₋₁₀ alkyl group or a C₁₋₁₀ alkoxy group; R²⁷ and R²⁸ together are hydrogen atoms, unsubstituted phenyl groups, or phenyl groups substituted with a C₁₋₂₀ alkyl group or a C₁₋₂₀ alkoxy group; X¹ and X² together are nitrogen atoms; each of R³⁶, R³⁷, and R³⁸ is independently a hydrogen atom or a C₁₋₂₀ alkyl group or R³⁶ and R³⁷ together form a phenyl group which may have a substituent, R³⁸ is a hydrogen atom or a C₁₋₂₀ alkyl group or R³⁷ and R³⁸ together form a phenyl group which may have a substituent, and R³⁶ is a hydrogen atom or a C₁₋₂₀ alkyl group; each of R⁴⁰, R⁴¹, and R⁴² is independently a hydrogen atom or a C₁₋₂₀ alkyl group or R⁴⁰ and R⁴¹ together form a phenyl group which may have a substituent, R⁴² is a hydrogen atom or a C₁₋₂₀ alkyl group or R⁴¹ and R⁴² together form a phenyl group which may have a substituent, and R⁴⁰ is a hydrogen atom or a C₁₋₂₀ alkyl group is preferable, and a compound in which R²³, R²⁴, R²⁵, and R²⁶ together are halogen atoms or unsubstituted phenyl groups; R²⁷ and R²⁸ together are hydrogen atoms, unsubstituted phenyl groups, or phenyl groups substituted with a linear or branched C₁₋₂₀ alkoxy group; each of R³⁶, R³⁷, and R³⁸ is independently a hydrogen atom or a C₁₋₂₀ alkyl group or R³⁶ and R³⁷ together form an unsubstituted phenyl group or a phenyl group substituted with a C₁₋₁₀ alkyl group, R³⁸ is a hydrogen atom or a C₁₋₂₀ alkyl group or R³⁷ and R³⁸ together form an unsubstituted phenyl group or a phenyl group substituted with a C₁₋₁₀ alkyl group, and R³⁶ is a hydrogen atom or a C₁₋₂₀ alkyl group; each of R⁴⁰, R⁴¹ and R⁴² is independently a hydrogen atom or a C₁₋₂₀ alkyl group or R⁴⁰ and R⁴¹ together form an unsubstituted phenyl group or a phenyl group substituted with a C₁₋₁₀ alkyl group, R⁴² is a hydrogen atom or a C₁₋₂₀ alkyl group or R⁴¹ and R⁴² together form an unsubstituted phenyl group or a phenyl group substituted with a C₁₋₁₀ alkyl group, and R⁴⁰ is a hydrogen atom or a C₁₋₂₀ alkyl group is more preferable since the light-emitting efficiency is high and the compatibility with respect to a resin is excellent.

As the compound represented by (II₄-4), a compound in which R²³, R²⁴, R²⁵, and R²⁶ together are halogen atoms, unsubstituted phenyl groups, or phenyl groups substituted with a C₁₋₁₀ alkyl group or a C₁₋₁₀ alkoxy group; R²⁷ and R²⁸ together are hydrogen atoms, unsubstituted phenyl groups, or phenyl groups substituted with a C₁₋₂₀ alkyl group or a C₁₋₂₀ alkoxy group; X¹ and X² together are nitrogen atoms; each of R³⁵, R³⁶, and R³⁷ is independently a hydrogen atom or a C₁₋₂₀ alkyl group or R³⁵ and R³⁶ together form a phenyl group which may have a substituent, R³⁷ is a hydrogen atom or a C₁₋₂₀ alkyl group or R³⁶ and R³⁷ together form a phenyl group which may have a substituent, and R³⁵ is a hydrogen atom or a C₁₋₂₀ alkyl group; each of R³⁹, R⁴⁰, and R⁴¹ is independently a hydrogen atom or a C₁₋₂₀ alkyl group or R³⁹ and R⁴⁰ together form a phenyl group which may have a substituent, R⁴¹ is a hydrogen atom or a C₁₋₂₀ alkyl group or R⁴⁰ and R⁴¹ together form a phenyl group which may have a substituent, and R³⁹ is a hydrogen atom or a C₁₋₂₀ alkyl group is preferable, and a compound in which R²³, R²⁴, R²⁵, and R²⁶ together are halogen atoms or unsubstituted phenyl groups; R²⁷ and R²⁸ together are hydrogen atoms, unsubstituted phenyl groups, or phenyl groups substituted with a linear or branched C₁₋₂₀ alkoxy group; X¹ and X² together are nitrogen atoms; each of R³⁵, R³⁶, and R³⁷ is independently a hydrogen atom or a C₁₋₂₀ alkyl group or R³⁵ and R³⁶ together form an unsubstituted phenyl group or a phenyl group substituted with a C₁₋₁₀ alkyl group, R³⁷ is a hydrogen atom or a C₁₋₂₀ alkyl group or R³⁶ and R³⁷ together form an unsubstituted phenyl group or a phenyl group substituted with a C₁₋₁₀ alkyl group, and R³⁵ is a hydrogen atom or a C₁₋₂₀ alkyl group; each of R³⁹, R⁴⁰, and R⁴¹ is independently a hydrogen atom or a C₁₋₂₀ alkyl group or R³⁹ and R⁴⁰ together form an unsubstituted phenyl group or a phenyl group substituted with a C₁₋₁₀ alkyl group, R⁴¹ is a hydrogen atom or a C₁₋₂₀ alkyl group or R⁴⁰ and R⁴¹ together form an unsubstituted phenyl group or a phenyl group substituted with a C₁₋₁₀ alkyl group, and R³⁹ is a hydrogen atom or a C₁₋₂₀ alkyl group is more preferable since the light-emitting efficiency is high and the compatibility with respect to a resin is excellent.

As the compound represented by (II₄-5), a compound in which R²³, R²⁴, R²⁵, and R²⁶ together are halogen atoms, unsubstituted phenyl groups, or phenyl groups substituted with a C₁₋₁₀ alkyl group or a C₁₋₁₀ alkoxy group; R²⁷ and R²⁸ together are hydrogen atoms, unsubstituted phenyl groups, or phenyl groups substituted with a C₁₋₂₀ alkyl group or a C₁₋₂₀ alkoxy group; X¹ and X² together are nitrogen atoms; each of R³⁵, R³⁶, and R³⁸ is independently a hydrogen atom or a C₁₋₂₀ alkyl group or R³⁵ and R³⁶ together form a phenyl group which may have a substituent, and R³⁸ is a hydrogen atom or a C₁₋₂₀ alkyl group; each of R³⁹, R⁴⁰, and R⁴² is independently a hydrogen atom or a C₁₋₂₀ alkyl group or R³⁹ and R⁴⁰ together form a phenyl group which may have a substituent, and R⁴² is a hydrogen atom or a C₁₋₂₀ alkyl group is preferable, and a compound in which R²³, R²⁴, R²⁵, and R²⁶ together are halogen atoms or unsubstituted phenyl groups; R²⁷ and R²⁸ together are hydrogen atoms, unsubstituted phenyl groups, or phenyl groups substituted with a linear or branched C₁₋₂₀ alkoxy group; X¹ and X² together are nitrogen atoms; each of R³⁵, R³⁶, and R³⁸ is independently a hydrogen atom or a C₁₋₂₀ alkyl group or R³⁵ and R³⁶ together form an unsubstituted phenyl group or a phenyl group substituted with a C₁₋₁₀ alkyl group, and R³⁸ is a hydrogen atom or a C₁₋₂₀ alkyl group; each of R³⁹, R⁴⁰, and R⁴² is independently a hydrogen atom or a C₁₋₂₀ alkyl group or R³⁹ and R⁴⁰ together form an unsubstituted phenyl group or a phenyl group substituted with a C₁₋₁₀ alkyl group, and R⁴² is a hydrogen atom or a C₁₋₂₀ alkyl group is more preferable since the light-emitting efficiency is high and the compatibility with respect to a resin is excellent.

As the compound represented by (II₄-6), a compound in which R²³, R²⁴, R²⁵, and R²⁶ together are halogen atoms, unsubstituted phenyl groups, or phenyl groups substituted with a C₁₋₁₀ alkyl group or a C₁₋₁₀ alkoxy group; R²⁷ and R²⁸ together are hydrogen atoms, unsubstituted phenyl groups, or phenyl groups substituted with a C₁₋₂₀ alkyl group or a C₁₋₂₀ alkoxy group; X¹ and X² together are nitrogen atoms; each of R³⁵, R³⁷, and R³⁸ is independently a hydrogen atom or a C₁₋₂₀ alkyl group or R³⁷ and R³⁸ together form a phenyl group which may have a substituent, and R³⁵ is a hydrogen atom or a C₁₋₂₀ alkyl group; each of R³⁹, R⁴¹, and R⁴² is independently a hydrogen atom or a C₁₋₂₀ alkyl group or R⁴¹ and R⁴² together form a phenyl group which may have a substituent, and R³⁹ is a hydrogen atom or a C₁₋₂₀ alkyl group is preferable, and a compound in which R²³, R²⁴, R²⁵, and R²⁶ together are halogen atoms or unsubstituted phenyl groups; R²⁷ and R²⁸ together are hydrogen atoms, unsubstituted phenyl groups, or phenyl groups substituted with a linear or branched C₁₋₂₀ alkoxy group; X¹ and X² together are nitrogen atoms; each of R³⁵, R³⁷, and R³⁸ is independently a hydrogen atom or a C₁₋₂₀ alkyl group or R³⁷ and R³⁸ together form an unsubstituted phenyl group or a phenyl group substituted with a C₁₋₁₀ alkyl group, and R³⁵ is a hydrogen atom or a C₁₋₂₀ alkyl group; each of R³⁹, R⁴¹, and R⁴² is independently a hydrogen atom or a C₁₋₂₀ alkyl group or R⁴¹ and R⁴² together form an unsubstituted phenyl group or a phenyl group substituted with a C₁₋₁₀ alkyl group, and R³⁹ is a hydrogen atom or a C₁₋₂₀ alkyl group is more preferable since the light-emitting efficiency is high and the compatibility with respect to a resin is excellent.

As the compound represented by any one of General Formulas (II₃-1) to (II₃-6), a compound represented by any one of the following General Formulas (II₃-7) to (II₃-9) is preferable, and as the compound represented by any one of General Formulas (II₄-1) to (II₄-6), a compound represented by any one of the following General Formulas (II₄-7) to (II₄-9) is preferable.

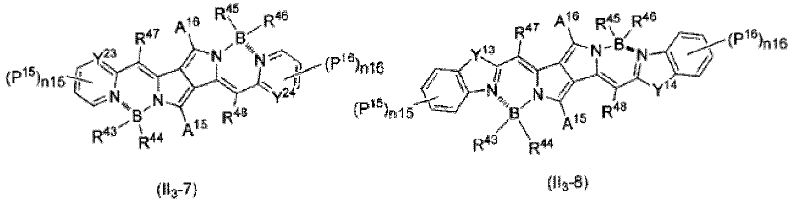

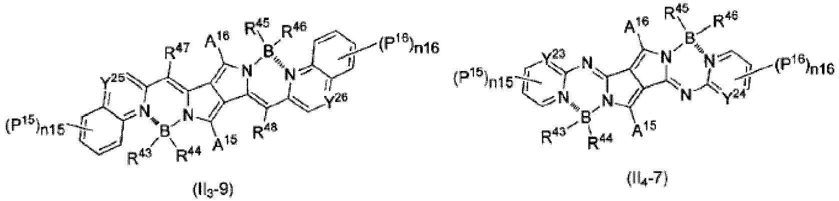

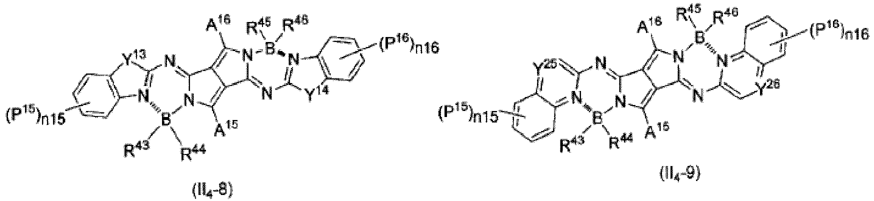

In General Formulas (II₃-7) and (II₄-7), each of Y²³ and Y²⁴ independently represents a carbon atom or a nitrogen atom. In General Formula (II₃-7), Y²³ and Y²⁴ are preferably the same type of atoms.

In General Formulas (II₃-8) and (II₄-8), each of Y¹³ and Y¹⁴ independently represents an oxygen atom or a sulfur atom. In General Formula (II₃-8), Y²³ and Y²⁴ are preferably the same type of atoms.

In General Formulas (II₃-9) and (II₄-9), each of Y²⁵ and Y²⁶ independently represents a carbon atom or a nitrogen atom. In General Formula (II₃-9), Y²⁵ and Y²⁶ are preferably the same type of atoms.

In General Formulas (II₃-7) to (II₃-9), each of Y⁴⁷ and Y⁴⁸ independently represents a hydrogen atom or an electron-withdrawing group, and since fluorescence intensity becomes high, each of Y⁴⁷ and Y⁴⁸ is preferably a trifluoromethyl group, a cyano group, a nitro group, a sulfonyl group, or a phenyl group, and particularly preferably a trifluoromethyl group or a cyano group. In General Formula (II₃-7), R⁴⁷ and R⁴⁸ are preferably the same type of functional groups.

In General Formulas (II₃-7) to (II₃-9) and (II₄-7) to (II₄-9), each of R⁴³, R⁴⁴, R⁴⁵, and R⁴⁶ represents a halogen atom or an aryl group which may have a substituent. As the aryl group, those exemplified as "any group which does not inhibit fluorescence of a compound" represented by each of R^{a} and R^{b} can be used. In addition, the substituent which the aryl group may have may be "any group which does not inhibit fluorescence of a compound", and examples thereof include a C₁₋₆ alkyl group, a C₁₋₆ alkoxy group, an aryl group, and a heteroaryl group. In General Formulas (II₃-7) to (II₃-9) and (II₄-7) to (II₄-9), all of R⁴³ to R⁴⁶ may be different groups or may be the same type of groups. As the compound represented by any one of General Formulas (II₃-7) to (II₃-9) and (II₄-7) to (II₄-9), a compound in which all of R⁴³ to R⁴⁶ are the same type of halogen atoms or phenyl groups which may have the same type of substituents is preferable, a compound in which all of R⁴³ to R⁴⁶ are fluorine atoms or unsubstituted phenyl groups is more preferable, and a compound in which all of R⁴³ to R⁴⁶ are fluorine atoms is particularly preferable.

In General Formulas (II₃-7) to (II₃-9) and (II₄-7) to (II₄-9), each of P¹⁵ and P¹⁶ independently represents a halogen atom, a C₁₋₂₀ alkyl group, a C₁₋₂₀ alkoxy group, an amino group, a monoalkylamino group, or a dialkylamino group. Examples of the C₁₋₂₀ alkyl group, the C₁₋₂₀ alkoxy group, the monoalkylamino group, or the dialkylamino group represented by each of P¹⁵ and P¹⁶ include the same as those exemplified as R^{g}, (p1) to (p3), or (q1) to (q3). Each of P¹⁵ and P¹⁶ is preferably a C₁₋₂₀ alkyl group, a C₁₋₂₀ alkoxy group, an (unsubstituted) phenyl group, a p-methoxyphenyl group, a p-ethoxyphenyl group, a p-dimethylaminophenyl group, a dimethoxyphenyl group, a thienyl group, or a furanyl group, more preferably a C₁₋₂₀ alkyl group, a C₁₋₂₀ alkoxy group, a phenyl group, a p-methoxyphenyl group, a p-ethoxyphenyl group, a dimethoxyphenyl group, a thienyl group, or a furanyl group from the viewpoint of safety with respect to a living body, and these substituents may further have a substituent. Here, since, even in the case of a substituent other than these substituents, it is possible to improve safety by further introducing a suitable substituent, the present invention is not limited to these substituents.

In General Formulas (II₃-7) to (II₃-9) and (II₄-7) to (II₄-9), each of n15 and n16 independently represents an integer of 0 to 3. In a case where a plurality of P¹⁵s are present in one molecule (that is, in a case where n15 is 2 or 3), all of the plurality of P¹⁵s may be the same type of functional groups, or may be different types of functional groups. The same applies to P¹⁶.

In General Formulas (II₃-7) to (II₃-9) and (II₄-7) to (II₄-9), each of A¹⁵ and A¹⁶ independently represents a phenyl group which may have one to three substituents selected from the group consisting of a hydrogen atom, a halogen atom, a C₁₋₂₀ alkyl group, a C₁₋₂₀ alkoxy group, an amino group, a monoalkylamino group, or a dialkylamino group. Examples of the C₁₋₂₀ alkyl group, the C₁₋₂₀ alkoxy group, the monoalkylamino group, or the dialkylamino group as the substituent which the phenyl group may have are the same as those exemplified as R^{g}, (p1) to (p3), or (q1) to (q3). Each of A¹⁵ and A¹⁶ is preferably an unsubstituted phenyl group, a phenyl group having one or two C₁₋₂₀ alkoxy groups as the substituent, more preferably an unsubstituted phenyl group or a phenyl group having one C₁₋₂₀ alkoxy group as the substituent, and still more preferably an unsubstituted phenyl group or a phenyl group having one C₁₋₁₀ alkoxy group as the substituent. In addition, the compound represented by General Formula (II₃-7) is preferably a compound in which A¹⁵ and A¹⁶ are the same type of functional groups.

As the compound represented by any one of General Formulas (II₃-1) to (II₃-6), a compound represented by any one of the following General Formulas (6-1) to (6-12) and (7-1) to (7-12) is exemplified. In General Formulas (6-7) to (6-12) and (7-7) to (7-12), Ph means an unsubstituted phenyl group. As the DPP-based boron complex used in the present invention, in particular, compounds represented by General Formulas (6-4), (6-5), (6-7), (6-8), (7-4), (7-5), (7-7), or (7-8) are preferable, and compounds represented by General Formulas (6-4), (6-5), (6-7), or (6-8) are more preferable.

In General Formulas (6-1) to (6-12) and (7-1) to (7-12), each of P⁵ and P⁸ independently represents a halogen atom, a C₁₋₂₀ alkyl group, a C₁₋₂₀ alkoxy group, an amino group, a monoalkylamino group, or a dialkylamino group. Examples of the C₁₋₂₀ alkyl group, the C₁₋₂₀ alkoxy group, the monoalkylamino group, or the dialkylamino group represented by each of P⁵ to P⁸ include the same as those exemplified as R^{g}, (p1) to (p3), or (q1) to (q3). Each of P⁵ to P⁸ is preferably a C₁₋₂₀ alkyl group, a C₁₋₂₀ alkoxy group, an (unsubstituted) phenyl group, a p-methoxyphenyl group, a p-ethoxyphenyl group, a p-dimethylaminophenyl group, a dimethoxyphenyl group, a thienyl group, or a furanyl group, from the viewpoint of safety with respect to a living body, more preferably a C₁₋₂₀ alkyl group, a C₁₋₂₀ alkoxy group, a phenyl group, a p-methoxyphenyl group, a p-ethoxyphenyl group, a dimethoxyphenyl group, a thienyl group, or a furanyl group, still more preferably a C₁₋₂₀ alkyl group or a C₁₋₂₀ alkoxy group, and still more preferably a C₁₋₁₀ alkyl group or a C₁₋₁₀ alkoxy group, and these substituents may further have a substituent. Here, since, even in the case of a substituent other than these substituents, it is possible to improve safety by further introducing a suitable substituent, the present invention is not limited to these substituents.

In General Formulas (6-1) to (6-12) and (7-1) to (7-12), each of n5 to n8 independently represents an integer of 0 to 3. In a case where a plurality of P⁵s are present in one molecule (that is, in a case where n5 is 2 or 3), all of the plurality of P⁵s may be the same type of functional groups, or may be different types of functional groups. The same applies to P⁶ to P⁸.

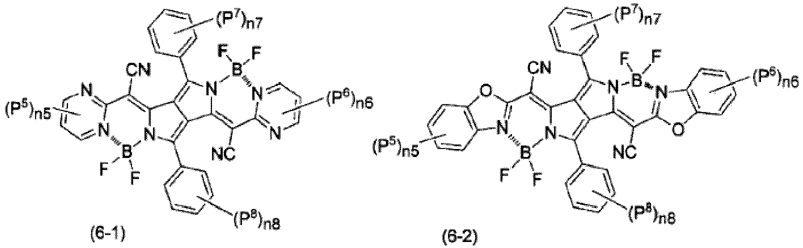

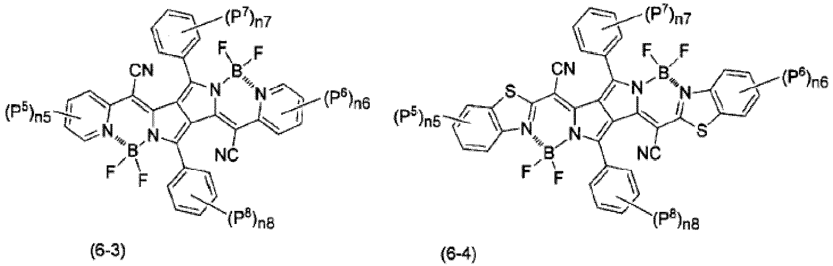

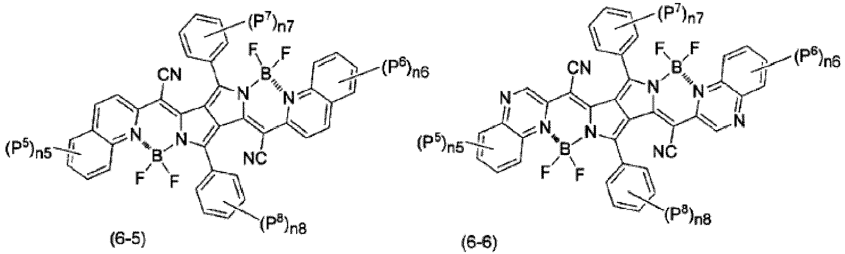

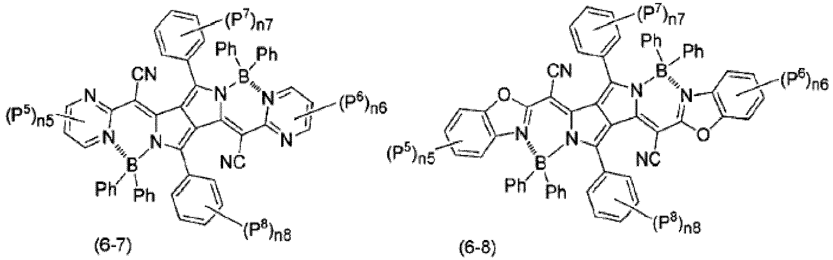

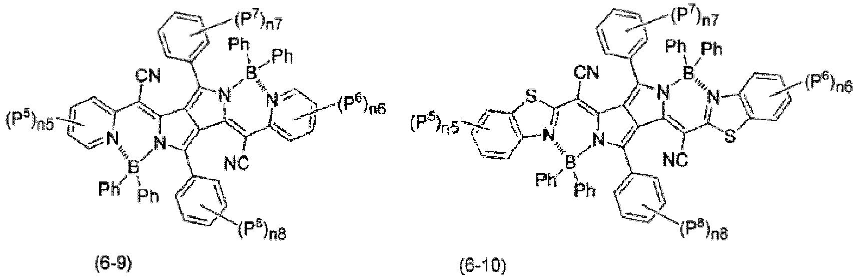

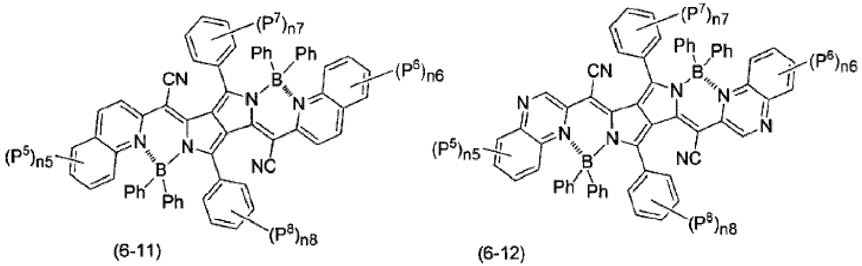

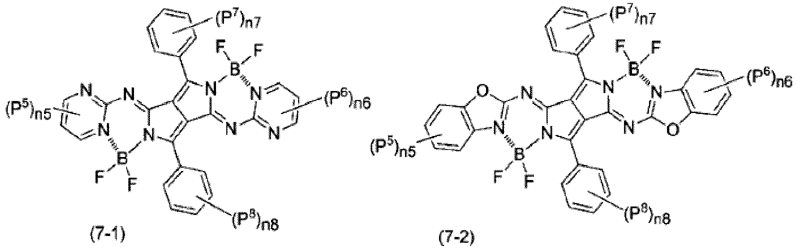

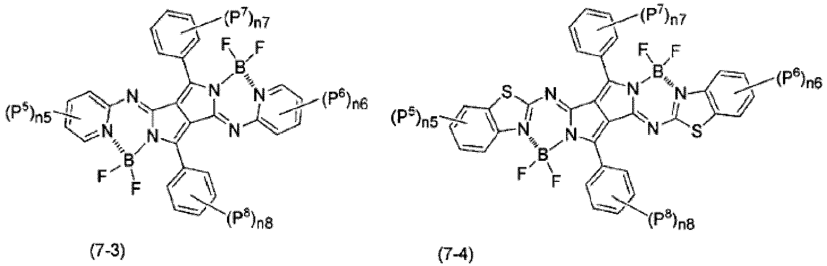

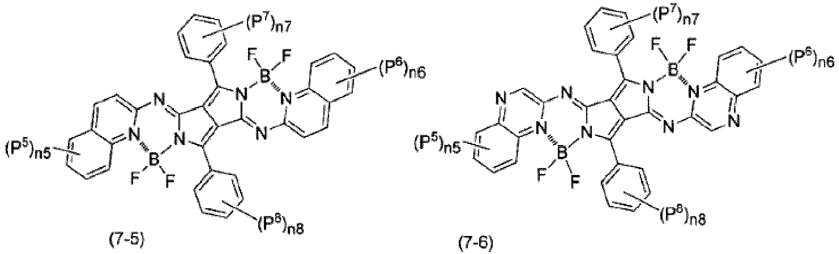

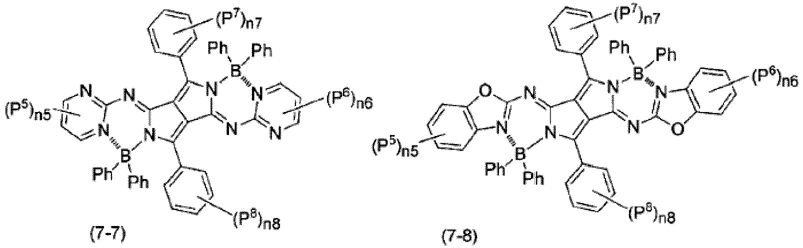

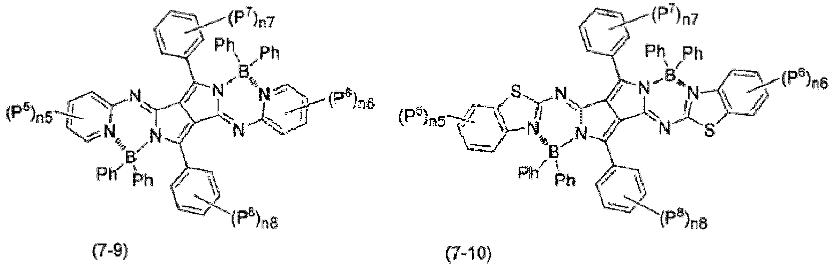

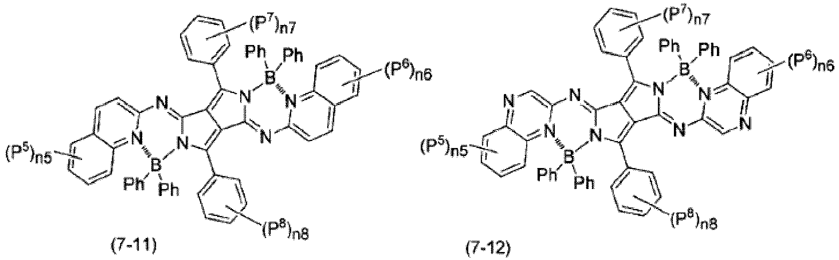

As the compounds represented by General Formulas (6-1) to (6-12) or (7-1) to (7-12), a compound in which each of P⁵ to P⁸ is independently a C₁₋₂₀ alkyl group or a C₁₋₂₀ alkoxy group and each of n5 to n8 is independently 0 to 2 is preferable, a compound in which each of P⁵ and P⁶ is independently a C1₋₂₀ alkyl group, each of n5 and n6 is independently 0 to 2, each of P⁷ and P⁸ is independently a C₁₋₂₀ alkoxy group, and each of n7 and n8 is independently 0 or 1 is more preferable, and a compound in which each of P⁵ and P⁶ is independently a C₁₋₂₀ alkyl group, each of n5 and n6 is independently 1 or 2, each of P⁷ and P⁸ is independently a C₁₋₂₀ alkoxy group, and each of n7 and n8 is independently 1 is still more preferable.

Examples of the compound represented by each of General Formulas (6-1) to (6-12) include a compound represented by each of the following General Formulas (6-1-1) to (6-12-1). "λ" is the peak wavelength of an absorption spectrum in a solution of each compound, and "Em" is the peak wavelength of a fluorescence spectrum.

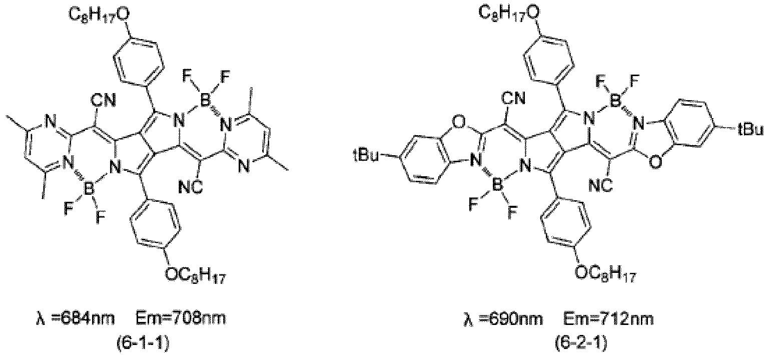

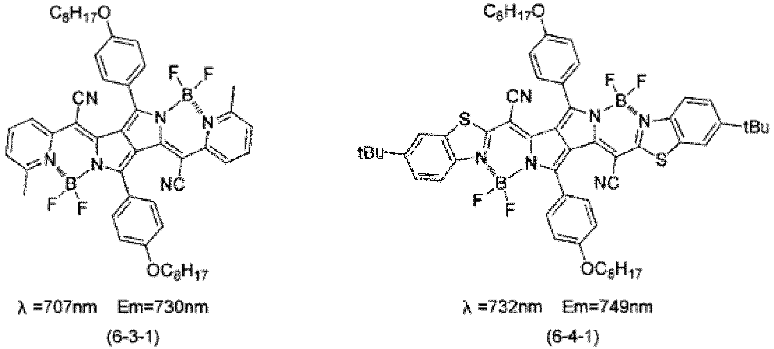

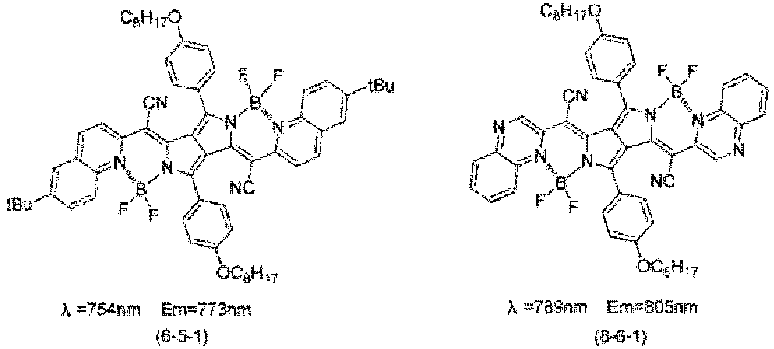

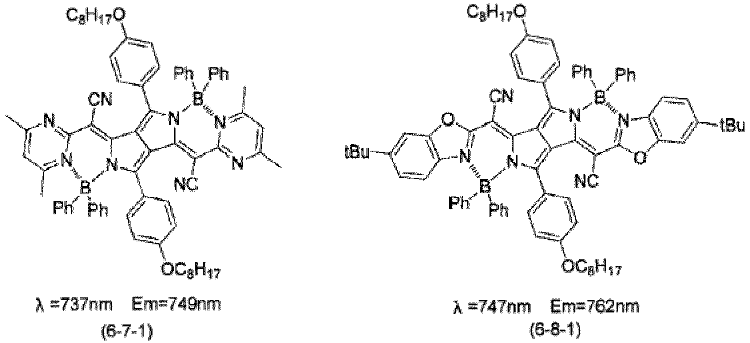

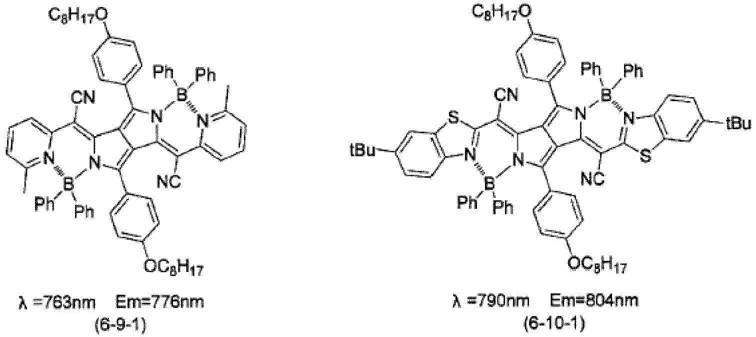

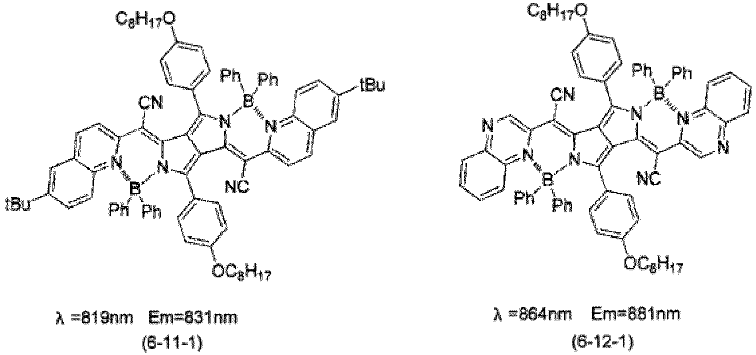

### <Radiopaque Substance>

The radiopaque substance contained in the resin composition according to the present invention preferably has lower transparency to radiation than that of skin, muscle, fat, or the like, and more preferably has lower transparency to radiation than that of bone, calcium, or the like. Examples of such a radiopaque substance include barium sulfate, calcium carbonate, aluminum hydroxide, bromine, bromide, iodine, and iodide, as a radiopaque substance formed of non-metal atoms, and metal powder or oxide of a metal such as titanium, zinc, zirconium, rhodium, palladium, silver, tin, tantalum, tungsten, rhenium, iridium, platinum, gold, or bismuth as a radiopaque substance including metal atoms. In addition, mica, talc, or the like can also be used as a radiopaque substance.

For example, in a case where the resin composition according to the present invention is used as a material for a medical tool used in vivo, the resin composition preferably contains a radiopaque substance with high biocompatibility. Examples of the radiopaque substance with high biocompatibility include barium sulfate, bismuth oxide, bismuth subcarbonate, calcium carbonate, aluminum hydroxide, tungsten, zinc oxide, zirconium oxide, zirconium, titanium, platinum, bismuth subnitrate, and bismuth. As the radiopaque substance used in the present invention, barium sulfate, bismuth subcarbonate, or bismuth oxide is particularly preferable from the viewpoint of safety or the like. The resin composition according to the present invention may contain one radiopaque substance, or may contain two or more radiopaque substances. In the resin composition according to the present invention, one or more radiopaque substances exemplified above are preferably contained.

Although the shape of the radiopaque substance used in the present invention is not particularly limited as long as it can impart radiation- opacity to the blended resin composition, the shape may be any one of a particle shape, a filament shape, and an irregular shape. The radiopaque substance used in the present invention preferably has a particle shape from the viewpoint of dispersibility in a resin, radiation transparency, and the influence on the emission intensity of the light-emitting substance described above.

### <Resin Component>

The resin component contained in the resin composition according to the present invention is not particularly limited, and light-emitting substance, the resin component can be suitably selected and used from known resin compositions or improved products thereof in consideration of the types of light-emitting substance and radiopaque substance to be blended, product quality required at the time of forming a molded article, or the like. For example, the resin component may be a thermoplastic resin or may be a thermosetting resin. In the case of being used in a molded article, as the resin component contained in the resin composition according to the present invention, a thermoplastic resin is preferable since a thermosetting resin is likely to be cured at the time of melt-kneading. The resin component used in the present invention may be used alone or in combination of two or more thereof. In a case where two or more thereof are used in combination, a combination of resins having high compatibility is preferably used.

Examples of the resin component used in the present invention include urethane resins such as polyurethane (PU) and thermoplastic polyurethane (TPU); polycarbonate (PC); vinyl chloride-based resins such as polyvinyl chloride (PVC) and a vinyl chloride-vinyl acetate copolymer resin; acrylic resins such as polyacrylic acid, polymethacrylic acid, polymethyl acrylate, polymethyl methacrylate (PMMA), and polyethyl methacrylate; polyester resins such as polyethylene terephthalate (PET), polybutylene terephthalate, polytrimethylene terephthalate, polyethylene naphthalate, and polybutylene naphthalate; polyamide-based resins such as Nylon (registered trademark); polystyrene-based resins such as polystyrene (PS), imide-modified polystyrene, an acrylonitrile-butadiene-styrene (ABS) resin, an imide-modified ABS resin, a styrene-acrylonitrile copolymer (SAN) resin, and an acrylonitrile-ethylene-propylene-diene-styrene (AES) resin; olefin-based resins such as a polyethylene (PE) resin, a polypropylene (PP) resin, and a cycloolefin resin; cellulose-based resins such as nitrocellulose and cellulose acetate; silicone-based resins; thermoplastic resins such as a fluorine-based resin; epoxy-based resins such as a bisphenol A type epoxy resin, a bisphenol F-type epoxy resin, an isocyanurate-based epoxy resin, and a hydantoin-based epoxy resin; amino-based resins such as a melamine-based resin and a urea resin; phenol-based resins; and thermosetting resins such as an unsaturated polyester-based resin.

In a case where the resin composition according to the present invention contains the azo-boron complex compound represented by Formula (I) as a light-emitting substance, since the dispersion of the azo-boron complex compound is high, as the resin component, PU, TPU, PET, PVC, PC, PMMA, or PS is preferable, and two or more thereof may be used in combination.

In a case where the resin composition according to the present invention contains the compound represented by General Formula (II₁), (II₂), (II₃), or (II₄) as a light-emitting substance, since the dispersion of the compound is high, as the resin component, a fluorine-based resin, a silicone-based resin, a urethane-based resin, an olefin-based resin, a vinyl chloride-based resin, a polyester-based resin, a polystyrene-based resin, a polycarbonate resin, a polyamide-based resin, or an acryl-based resin is preferable, and a urethane-based resin, an olefin-based resin, a polystyrene-based resin, a polyester-based resin, or a vinyl chloride-based resin is more preferable. In particular, in a case where the resin composition according to the present invention is used as a medical material, in consideration of low solubility in body fluid such as blood and difficult elution in a use environment or biocompatibility, PTFE (Teflon (registered trademark)), silicone, PU, TPU, PP, PE, PC, PET, PS, polyamide, or PVC is more preferable, and TPU, PU, PP, PE, PET, or PS is still more preferable.

Moreover, in a case where the resin composition according to the present invention contains a thermoplastic resin composition, as the resin component, a small amount of non-thermoplastic resin may be contained as long as overall the resin may be a thermoplastic resin. Similarly, in a case where the resin composition according to the present invention contains a thermosetting resin composition, as the resin component, all the resin components may be thermosetting resins, or a small amount of non-thermosetting resin may be contained.

### <Resin Composition>

The resin composition according to the present invention can be prepared by mixing and dispersing a light-emitting substance and a radiopaque substance in a resin component. The light-emitting substance according to the present invention contained in the resin composition according to the present invention may be only one or more thereof may be contained in the resin composition.

Although the content of light-emitting substance in the resin composition is not particularly limited as long as it has a concentration at which the light-emitting substance can be mixed with the resin, the content is preferably 0.0001% by mass or greater from the viewpoint of the emission intensity and the detection sensitivity thereof, and the content is preferably 1% by mass or less, more preferably within the range of 0.001% by mass to 0.5% by mass, and still more preferably within the range of 0.001% by mass to 0.05% by mass, from the viewpoint of detection sensitivity by the concentration quenching or the re-absorption of light-emission.

Although, in a case where the light-emitting substance is a near-infrared fluorescent material, the content of the near-infrared fluorescent material in the resin composition according to the present invention is not particularly limited as long as it has a concentration at which the near-infrared fluorescent material can be mixed with the resin, the content is preferably 0.0001% by mass or greater from the viewpoint of the fluorescence intensity and the detection sensitivity thereof, and the content is preferably 1% by mass or less, and more preferably within the range of 0.001% by mass to 0.5% by mass, from the viewpoint of detection sensitivity by the concentration quenching or the re-absorption of fluorescence. In addition, since the near-infrared fluorescent material used in the present invention has a high molar absorption coefficient and a high quantum yield even in the resin, even in a case where the near-infrared fluorescent material concentration in the resin is relatively low, it is possible to sufficiently observe the emission using a camera. It is desirable that the near-infrared fluorescent material concentration be low from the viewpoint of low possibility to elute, low possibility to bleed out from a molded article processed from the resin composition, and being capable of processing a molded article which requires transparency.

Although the amount of radiopaque substance added in the resin composition is not particularly limited as long as the concentration thereof is a concentration at which radiation can be shielded, the amount added is preferably 1% by mass or greater from the viewpoint of radiation shielding performance, and the amount added is preferably 80% by mass or less, more preferably within the range of 5% by mass to 50% by mass, and still more preferably within the range of 15% by mass to 45% by mass, from the viewpoint of mechanical strength of the resin composition.

A method of mixing and dispersing a light-emitting substance and a radiopaque substance in a resin component is not particularly limited, and the mixing and dispersing may be performed by any method known in the related art, and an additive may be further used in combination. For example, a light-emitting substance and a radiopaque substance may be added to a solution obtained by dissolving the resin composition in a suitable solvent and dispersed therein. In addition, even in a case where a solvent is not used, the resin composition according to the present invention can be obtained by adding a light-emitting substance and a radiopaque substance to the resin composition and melt-kneading. In this manner, a resin composition in a state in which a light-emitting substance and a radiopaque substance are evenly dispersed in the resin is obtained.

Moreover, in a case where, by melt-kneading a resin and a fluorescent material, the fluorescent material is dispersed in a thermoplastic resin, even in a case where melt-kneading is performed at a temperature lower than the decomposition point of the fluorescent material, depending on the type of the resin or the fluorescent material and the kneading conditions, fluorescence is not emitted by poor dispersion or decomposition of the fluorescent material, in some cases. Whether the fluorescent material can be dispersed in a thermoplastic resin or the like or not is difficult to predict from the thermal physical properties of the fluorescent material.

In contrast, the compound represented by General Formula (II₁), (II₂), (II₃), or (II₄) can be evenly mixed with various resin components and dispersed therein, and can emit fluorescence at a high quantum yield even in the resin. The reason for this is not clear, but it is thought to be as follows. In a case where a fluorescent material is dispersed by a method such as melt-kneading, it is thought that the quantum yield of the fluorescence is decreased by concentration quenching when aggregation or the like occurs. Therefore, for efficient emission of fluorescence by the fluorescent material, it is desired that the compatibility with a resin be high and the fluorescent material can be evenly dispersed. An SP value can be exemplified as one indicator of whether the compatibility is high or not. As the difference between the SP value of a fluorescent material and the SP value of a resin is smaller, the compatibility is high and the fluorescent material can be evenly dispersed in the resin. On the other hand, in a case where the SP values or the like are different, description by other physical property parameters is also possible. For example, calculated values such as the solubility of the fluorescent material, the partition coefficient, the relative dielectric constant, and the polarizability of the fluorescent material or the compatibility with the resin from the measured values can be explained. In addition, the compatibility between the resin and the fluorescent material varies depending on the crystallinity of the resin in some cases.

Additionally, the compatibility between the resin and the fluorescent material can be controlled by the functional group which the molecule itself of the fluorescent material has. For example, in a case where the fluorescent material is dispersed in a fat-soluble (hydrophobic) polyolefin-based resin such as polypropylene or polyethylene, the fluorescent material molecule preferably has a hydrophobic group. For example, by introducing a hydrophobic group such as an alicyclic alkyl group, a long-chain alkyl group, a halogenated alkyl group, or an aromatic ring into the fluorescent material molecule, the compatibility with the resin can be improved. However, the present invention is not limited to these functional groups. In addition, in a case where the fluorescent material is dispersed in a resin having high polarity such as polyurethane or polyamide resin, the fluorescent material molecule preferably has a hydrophilic group such as a carboxyl group, a hydroxyl group, an amino group, an alkoxy group, an aryloxy group, an alkylamino group, an ester, or an amide. However, the present invention is not limited thereto.

To increase the compatibility with a resin, it is necessary to suppress aggregation of the pigment molecules. In the case of a fluorescent material, introduction of an aromatic ring or a heterocycle into the molecule is performed to ensure extension of a conjugated system and planarity. However, by introduction of the ring, the interaction between molecules becomes stronger, and so pigment molecules are prone to stacking and aggregation. It is thought that, since the compound represented by General Formula (II₁), (II₂), (II₃), or (II₄) has a skeleton formed of a wide conjugate plane around the boron atom, and so is likely to be aggregated, but by polarizing by introducing an electron-donating group or an electron-withdrawing substituent or by introducing a bulky functional group, aggregation of a pigment is suppressed, and the compatibility with various resins can be achieved.

The partition coefficient or the SP value which is an index of compatibility can be estimated as a water/octanol partition coefficient or a SP value from "Hansen solubility parameter" obtained by calculation using a commercially available software. For example, the partition coefficients and the SP values of compounds represented by the following compounds (8-1) to (8-8), represented by General Formulas (II₁), (II₂), (II₃), or (II₄), are as follows.

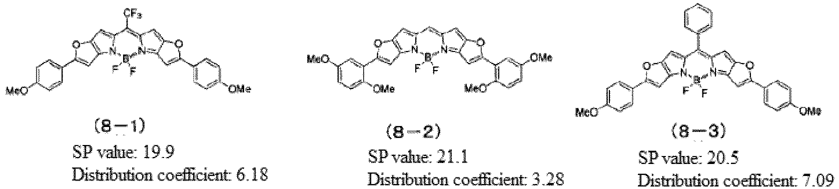

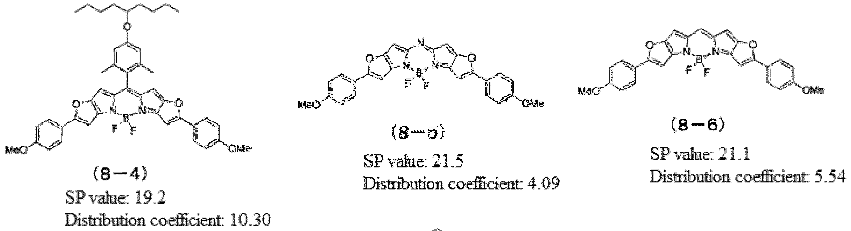

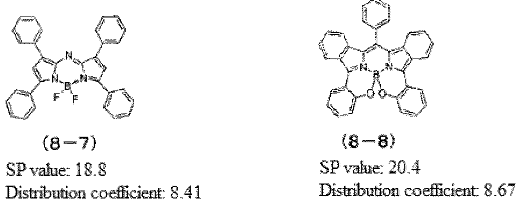

The near-infrared fluorescent material used in the present invention can be evenly dispersed and mixed by being melt-kneaded with a resin component such as PP, and the kneaded resin composition or a molded article processed from the resin composition can stably emit near-infrared fluorescence at a higher emission quantum yield. The reason why the near-infrared fluorescent material used in the present invention exhibits emission characteristics even in the case of being melt-kneaded with the resin composition unlike many other organic near-infrared fluorescent materials is not clear, but it is thought that, since the near-infrared fluorescent material used in the present invention has a rigid skeleton configured of a wide conjugate plane, the heat resistance thereof is high and the compatibility thereof with the resin is excellent. Moreover, the present inventors found for the first time that, even in a case where the BODIPY pigment or the DPP-based boron complex is subjected to a high-load treatment such as melt-kneading, fluorescence characteristics thereof are not impaired.

Since the resin composition according to the present invention includes both a light-emitting substance and a radiopaque substance, the resin composition is suitable for both emission detection and radiation detection. Furthermore, the resin composition according to the present invention has obviously stronger emission intensity in the excitation light source direction and higher sensitivity of emission detection than a resin composition containing the same type of and the same amount of light-emitting substance. For example, in the resin composition according to the present invention containing both a fluorescent material and a radiopaque substance, the maximum fluorescence wavelength and the fluorescence intensity in the vicinity thereof can be enhanced by 30% or greater, preferably 100% or greater, more preferably 150% or greater, and still more preferably 200% or greater, compared to those in a resin composition containing the same type of and the same amount of fluorescent material only. The reason why such emission intensity enhancing effects (sensitizing effects) due to the radiopaque substance are obtained is not clear, but it is thought to be as follows. For example, it is thought that it is because (1) since the resin composition contains the radiopaque substance, when excitation light hits the opaque substance, the excitation light does not pass through the resin and is scattered in the vicinity of the surface, and as a result, the excitation light is locally enhanced, (2) in the transparent smooth film, fluorescence is likely to emit light at the end surface by the law of total reflection, but the smoothness is lost due to the radiopaque substance, and thus, the total reflection is reduced, and fluorescence is scattered inside and strongly comes out in the excitation light source direction, or (3) by co-existing with the radiopaque substance, the dispersibility of the light-emitting substance is improved (the interaction between the light-emitting substances is reduced, quenching is reduced, and the emission efficiency is increased).

Although the mixing ratio of the light-emitting substance to the radiopaque substance is not particularly limited, the mixing ratio (light-emitting substance / radiopaque substance) is preferably within the range of 0.00001 to 0.1, and more preferably within the range of 0.00002 to 0.01, from the viewpoint of increasing the emission intensity.

In a case where the resin composition according to the present invention contains a light-emitting substance having a high quantum yield (the number of released photons / the number of absorbed photons) of 20% or greater, there is no particular problem, but in a case where the resin composition contains a light-emitting substance having a low quantum yield, understanding of the Stokes shift (difference between the maximum absorption wavelength and the maximum emission wavelength) of the resin composition according to the present invention is also important.

In a case where a general emission detector provided with a filter for cutting noise due to excitation light is used, when the Stokes shift of the resin composition according to the present invention is small, light emission is cut by the filter, and thus, it is difficult to detect with high sensitivity. Therefore, the Stokes shift (difference between the maximum absorption wavelength and the maximum emission wavelength) of the resin composition according to the present invention is preferably 10 nm or greater, and more preferably 20 nm or greater. As the Stokes shift is increased, even in a case where a general emission detector provided with a filter for cutting noise due to excitation light is used, it is possible to detect the emission emitted from the molded article with high sensitivity.

However, even in a case where the Stokes shift is small, under conditions as described below, it is possible to detect the near-infrared fluorescence from the resin composition according to the present invention with high sensitivity. For example, if excitation is possible at shorter-wavelength light than the maximum absorption wavelength, it is possible to detect the fluorescence even when the noise is cut. In addition, in a case where the fluorescence spectrum is broad, it is possible to sufficiently detect fluorescence even when the noise is cut. On the other hand, some fluorescent materials have a plurality of fluorescence peaks. In this case, even in a case where the Stokes shift is small, if a fluorescence peak (second peak) is present on the longer wavelength side, it is possible to detect the fluorescence peak with high sensitivity even in the case of using a detector provided with a filter for cutting noise. The difference between the fluorescence peak wavelength on the long wavelength side in a case where the resin composition of the present invention has a plurality of fluorescence and the maximum absorption wavelength may be 30 nm or longer, and is preferably 50 nm or longer. Moreover, the present invention is not limited to the above-described conditions if an excitation light source, a cut filter, or the like is suitably selected.

By containing a near-infrared fluorescent material, even when the resin composition according to the present invention is excited by excitation light in the near-infrared region, the color thereof is not changed in a visual observation state, and the resin composition emits fluorescence in the invisible near-infrared region, and thus, this can be detected by a detector. Therefore, the maximum absorption wavelength with respect to the excitation light in the near-infrared region may be 600 nm or longer, and from the viewpoint of the absorption efficiency, the maximum absorption wavelength is preferably close to the wavelength of the excitation light, more preferably 650 nm or longer, still more preferably 665 nm or longer, and particularly preferably 680 nm or longer. Furthermore, in a case where the resin composition is used as medical tools such as that of an implant, the maximum absorption wavelength is preferably 700 nm or longer.

By containing a near-infrared fluorescent material, in consideration of no change in the color of the irradiated object and detection sensitivity, the resin composition according to the present invention or a molded article obtained from the composition, having the maximum fluorescence wavelength of 650 nm or longer, has no practical problem, and the maximum fluorescence wavelength thereof is preferably 700 nm or longer, and more preferably 720 nm or longer. In a case where the resin composition or a molded article obtained from the composition has a plurality of fluorescence peaks, the resin composition or a molded article obtained from the composition may be useful if there is a fluorescence peak with a sufficient detection sensitivity at 740 nm or greater, even when the wavelength of the maximum fluorescence peak thereof is 720 nm or shorter. In this case, the intensity of the fluorescence peak on the longer wavelength side (second peak) is preferably 5% or greater and more preferably 10% or greater, with respect to the intensity of the maximum fluorescence wavelength.

The resin composition according to the present invention and a molded article obtained from the composition preferably have strong absorption in the range of 650 nm to 1500 nm and emits a strong fluorescence peak in this range. Light of 650 nm or longer is less likely to be affected by hemoglobin, and light of 1500 nm or shorter is less likely to be affected by water. That is, the light within the range of 650 nm to 1500 nm is suitable as a wavelength range of light used to visualize a medical implant embedded subcutaneously or the like because the light has a high skin transparency and is less likely to be affected by foreign substances in a living body. In a case where the maximum absorption wavelength and the maximum fluorescence wavelength are within the range of 650 nm to 1500 nm, the resin composition according to the present invention and a molded article obtained from the composition are suitable for detection by light within the range of 650 nm to 1500 nm and suitable as a medical tool or the like used in vivo.

The resin composition according to the present invention may contain components other than the resin components, the light-emitting substance, and the radiopaque substance described above, as long as the components do not impair the effect of the present invention. Examples of the other components include an ultraviolet absorber, a heat stabilizer, a light stabilizer, an antioxidant, a flame retardant, a flame retardant auxiliary agent, a crystallization accelerator, a plasticizer, an antistatic agent, a colorant, and a release agent.

### <Molded Article>

By processing the resin composition according to the present invention, a molded article to which both emission detection and radiation detection are possible is obtained. The molding method is not particularly limited, and examples thereof include a casting method, an injection molding method using a mold, a compression molding method, an extrusion molding method using a T-die, and a blow molding method.

In the production of a molded article, the molded article may be formed of only the resin composition according to the present invention, or the resin composition according to the present invention and other resin compositions may be used as the raw materials. For example, all of the molded article may be molded from the resin composition according to the present invention, or only a part of the molded article may be molded from the resin composition according to the present invention. The resin composition according to the present invention is preferably used as a raw material constituting the surface portion of the molded article. For example, in a case where a catheter is molded, by molding only the tip portion of the catheter from the resin composition according to the present invention and by molding the remaining portion from a resin composition not containing a near-infrared fluorescent material, it is possible to produce a catheter of which only the tip portion emits near-infrared fluorescence. In addition, by molding by alternately stacking the resin composition according to the present invention and a resin composition not containing a near-infrared fluorescent material, it is possible to produce a molded article which emits near-infrared fluorescence in the form of a stripe. In addition, surface coating may be performed to enhance the visibility of the molded article.

Radiation detection can be performed by using a commercially available X-ray apparatus or the like by an ordinary method. In addition, emission detection can also be performed by using a commercially available apparatus for detecting fluorescence or phosphorescence or the like by an ordinary method. As the excitation light used in fluorescence or phosphorescence detection, any light source can be used, and, in addition to a near-infrared lamp having a wide wavelength width, a laser having a narrow wavelength width, an LED, or the like can be used.

Even when a molded article obtained from the resin composition containing the near-infrared fluorescent material is irradiated with light in the near-infrared region, the color thereof is not changed and the molded article emits near-infrared fluorescence which can be detected with higher sensitivity than that in the related art, and thus, the molded article is particularly suitable for medical tools that are inserted or indwelled in the body of a patient.

In a case where fluorescence detection is performed on the molded article obtained from the resin composition containing the near-infrared fluorescent material, it is preferable to irradiate with excitation light in the near-infrared region, but in a case where the irradiated object may exhibit somewhat reddish color, the excitation light in the near-infrared region is not necessarily used. For example, in a case where fluorescence detection is used to detect the medical tool in the body by irradiating with excitation light, it is necessary to use excitation light in a wavelength region having high transparency with respect to a living body such as the skin, and in this case, excitation light of 650 nm or longer having high transparency with respect to a living body may be used.

Examples of the medical tool include a stent, a coil embolus, a catheter tube, an injection needle, an indwelling needle, a port, a shunt tube, a drain tube, and an implant. Examples

Hereinafter, the present invention will be described in more detail with reference to examples and comparative examples, but the present invention is not limited thereto.

### [Preparation Example 1] Synthesis of Azo-Boron Complex Compound

### (1) Preparation of Hydrazone Compound

An orthoquinone derivative (200 mg, 5.33 x 10⁻⁴ mol) and hydrochloride of 2-hydrazinobenzoic acid (402 mg, 2.13 x 10⁻³ mol) were put into an egg-plant shaped flask for a synthesis device, and a mixed solvent (55 mL) of methanol:water:dimethylsulfoxide = 3:4:4 was added thereto, followed by heating and stirring at 50°C. When a reaction started, crystals were precipitated in the reaction solution. 13 hours after the start of the reaction, heating of the reaction solution was stopped, and the reaction solution was allowed to cool at room temperature while stirring. The precipitated crystals were separated by filtration, and washed with a mixed solvent of methanol:water = 4:1, whereby reddish brown powder-like crystals were obtained (yield: 96 mg, yield: 35.3%). Since this compound has a low solubility, this compound was subjected to boron complexation without further purification.

### (2) Preparation of Azo-Boron Complex Compound

The reddish brown powder-like crystals (200 mg, 3.92 x 10⁻⁴ mol) obtained in the above (1) were put into a 300 mL egg-plant shaped flask and dichloromethane (70 mL) was added thereto. After a hydrazone compound was completely dissolved by adding triethylamine (137 mg, 1.37 x 10⁻³ mol) thereto, a boron trifluoride ether complex salt (334 mg, 2.35 x 10⁻³ mol) was added dropwise thereto, and the reaction was performed by stirring at room temperature. 3 days after the start of the reaction, progress of the reaction could be no longer confirmed by TLC, and thus, the reaction was stopped by adding water thereto. The dichloromethane layer was separated, washed with water, and concentrated under reduced pressure. The obtained residue was purified by silica gel column chromatography (eluent: dichloromethane/ethyl acetate = 10/1), whereby a target compound was obtained as green powder crystals (obtained amount: 62.2 mg, yield: 29.4%).
¹H-NMR(CDCl₃)δ=1.03(6H,t,J=7.46),1.40-1.49(4H,m),1.66-1.74(4H,m),3.47(4 H,t),6.78(1H,d,J=2.20),6.90(1H,dd,J=2.20,J=9.16),7.48(1H,t,J=7.44),7.66-7.78(3H,m),8. 13(1H,d,J=9.16),8.30-8.33(2H,m),8.39(1H,d,J=7.70),8.75(1H,d,J=7.70)

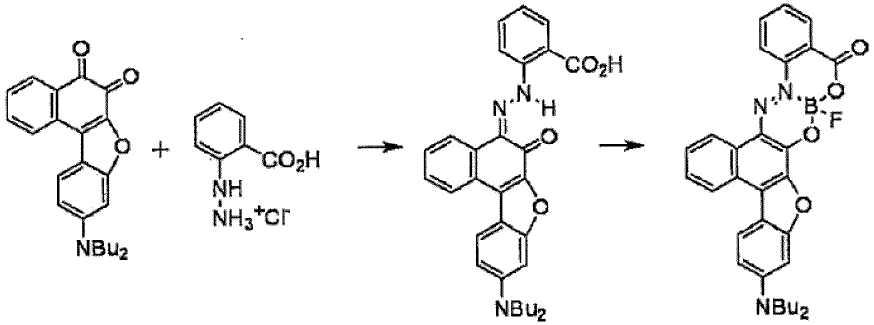

### [Preparation Example 2] Synthesis of Near-infrared Fluorescent Pigment A

Under an argon stream, 4-methoxyphenyl boronic acid (2.99 g, 19.7 mmol) was put into a 500 mL three-neck flask, then, this was dissolved in toluene (120 mL), and [1,1'-bis(diphenylphosphino)-ferrocene]palladium (II) dichloride-dichloromethane complex (1:1) (100 mg), ethanol (30 mL), 5-bromo-2-furaldehyde (3.46 g, 19.8 mmol), and a 2 mol/L sodium carbonate aqueous solution (20 mL) were added thereto, followed by stirring at 80°C for 14 hours. After the reaction ended, the organic phase was washed with water and a saturated saline solution and dried over anhydrous sodium sulfate, then, the desiccant was separated by filtration, and the solvent was concentrated under reduced pressure. The obtained crude product was separated and purified by flash silica gel chromatography (eluent: hexane/ethyl acetate = 19/1 → 4/1), whereby 5-(4-methoxyphenyl)-furan-2-carbaldehyde (a-1) was obtained as a pale yellow liquid (obtained amount: 3.39 g, yield: 84.8%).

Next, under an argon stream, the compound (a-1) (3.39 g, 16.8 mmol) and ethyl azidoacetate (8.65 g, 67.0 mmol) were dissolved in ethanol (300 mL) in a 1 L three-neck flask, and a 20% by mass sodium ethoxide ethanol solution (22.8 g, 67.0 mmol) was slowly added dropwise to the obtained solution at 0°C in an ice bath, followed by stirring for 2 hours. After the reaction ended, a saturated ammonium chloride aqueous solution was added thereto to adjust the pH to be weakly acidic, water was added thereto, suction filtration was performed, and the obtained filtered material was dried, whereby ethyl 2-azido-3-[5-(4-methoxyphenyl)-furan-2-yl] acrylate (a-2) was obtained as a yellow solid (obtained amount: 3.31 g, yield: 63.1%).

Furthermore, the compound (a-2) (3.31 g, 10.6 mmol) was put into a 200 mL egg-plant shaped flask, and this was dissolved in toluene (60 mL), followed by refluxing and stirring for 1.5 hours. After the solution after refluxing and stirring was concentrated under reduced pressure, the obtained crude product was recrystallized (solution: hexane and ethyl acetate), then, the resultant product was subjected to suction filtration, and the obtained filtered material was dried, whereby 2-(4-methoxyphenyl)-4H-furo[3.2-b]pyrrole-5-carboxylic acid ethyl ester (a-3) was obtained as a brown crystal (obtained amount: 2.32 g, yield: 76.8%).

Next, the compound (a-3) (1.90 g, 6.66 mmol) was put into a 300 mL flask, and an aqueous solution obtained by dissolving ethanol (60mL) and sodium hydroxide (3.90 g, 97.5 mmol) in water (30 mL) was added thereto, followed by refluxing and stirring for 1 hour. After the solution after refluxing and stirring was cooled, a 6 mol/L hydrochloric acid aqueous solution was added thereto to adjust the solution to be acidic, water was added thereto, suction filtration was performed, and the obtained filtered material was vacuum-dried, whereby 2-(4-methoxyphenyl)-4H-furo[3.2-b]pyrrole-5-carboxylic acid (a-4) was obtained as a gray solid (obtained amount: 1.56 g, yield: 91%).

Subsequently, the compound (a-4) (327 mg, 5.52 mmol) and trifluoroacetic acid (16.5 mL) were put into a 200 mL three-neck flask, followed by stirring at 45°C. After the compound (a-4) was dissolved, stirring was performed for 15 minutes until the bubbles subsided. Trifluoroacetic anhydride (3.3 mL) was added to the solution after stirring, and the resultant product was allowed to react at 80°C for 1 hour. After the reaction ended, a saturated sodium hydrogen carbonate aqueous solution and ice were added thereto to neutralize the solution, then, suction filtration was performed, and the filtered material was vacuum-dried, whereby a compound (a-5) was obtained as a black solid (obtained amount: 320 mg). The compound (a-5) was used in the next reaction without purification.

Under an argon stream, the compound (a-5) (320 mg) was put into a 200 mL three-neck flask, and toluene (70 mL), triethylamine (1.0 mL), and boron trifluoride diethylether complex (1.5 mL) were added dropwise thereto, followed by heating to reflux for 30 minutes. After the reaction ended, a saturated sodium hydrogen carbonate aqueous solution was added thereto, and the organic phase was collected. The organic phase was washed with water and a saturated saline solution and dried over anhydrous magnesium sulfate, then, the desiccant was separated by filtration, and the solvent was concentrated under reduced pressure. The obtained crude product was separated and purified by silica gel chromatography (eluent: toluene/ethyl acetate = 20/1 (in volume ratio)), whereby a near-infrared fluorescent pigment A was obtained as a green crystal (obtained amount: 20 mg, yield: 6%).

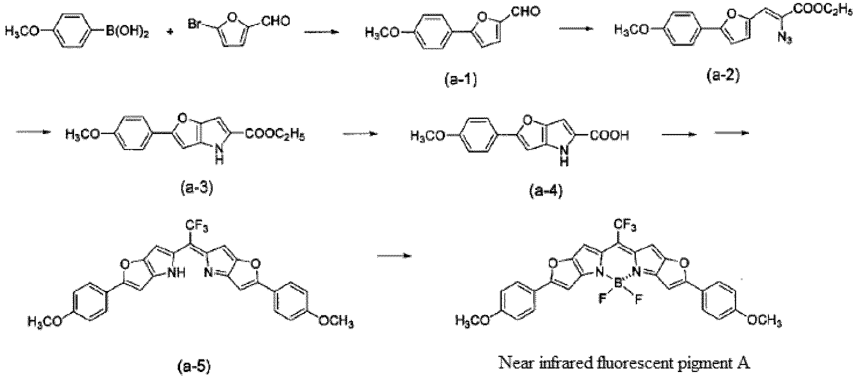

### [Preparation Example 3] Synthesis of Near-infrared Fluorescent Pigment B

Synthesis of a near-infrared fluorescent pigment B was performed in the following manner based on Organic Letters, 2012, Vol. 4, 2670-2673 and Chmestry-A European Journal, 2009, Vol. 15, 4857-4864.

4-Hydroxybenzonitrile (25.3 g, 212 mmol), acetone (800 mL), potassium carbonate (100 g, 724 mmol), and 1-bromooctane (48 g, 249 mmol) were put into a 2 L four-neck flask, followed by heating to reflux overnight. After the inorganic salt was filtered, acetone was removed under reduced pressure. Ethyl acetate was added to the obtained residues, and the organic layer was washed with water and a saturated saline solution, and treated with anhydrous magnesium sulfate. After the magnesium sulfate was separated by filtration, the solvent was removed under reduced pressure, and the residues were purified by silica gel column chromatography (eluent: hexane/ethyl acetate), whereby 4-octoxybenzonitrile (b-1) was obtained as a colorless transparent liquid (obtained amount: 45.2 g, yield: 92%).

Next, under an argon stream, tert-butyloxy potassium (25.18 g, 224.4 mmol) and tert-amyl alcohol (160 mL) were put into a 500 mL four-neck flask, and a solution obtained by mixing the compound (b-1) (14.8 g, 64 mmol) synthesized above and tert-amyl alcohol (7 mL) was added thereto, followed by heating to reflux. While heating to reflux, a solution obtained by mixing succinic acid diisopropyl ester (6.5 g, 32 mmol) and tert-amyl alcohol (10 mL) was added dropwise thereto over a period of about 3 hours, and after addition ended, the resultant product was heated to reflux for 6 hours. After the temperature was returned to room temperature, the obtained reaction liquid having viscosity was put into a solution of acetic acid:methanol:water = 1:1:1, and the resultant product was heated to reflux for several minutes, whereby a red solid was precipitated. The solid was separated by filtration, and washed with heated methanol and water, whereby 3,6-(4-octyloxyphenyl)pyrrolo[3,4-c]pyrrole-1,4(2H,5H)-dione (b-2) was obtained as a red solid (obtained amount: 5.6 g, yield: 32%).

In addition, 4-tert-butylaniline (10 g, 67 mmol), acetic acid (70 mL), and sodium thiocyanate (13 g, 160 mmol) were put into a 200 mL three-neck flask. While maintaining the inside of the system at 15°C or lower, bromine (4.5 mL, 87 mmol) was added dropwise thereto over a period of about 20 minutes, and then, the resultant product was stirred at 15°C or lower for 3.5 hours. After the reaction liquid was put into 28% ammonia water (150 mL), the resultant product was stirred for a while, the precipitated solid was separated by filtration, the solid was extracted with diethyl ether, and the organic layer was washed with water. After the diethyl ether was removed under reduced pressure, the residues were purified by silica gel column chromatography (eluent: dichloromethane/ethyl acetate), whereby 2-amino-6-tert-butyl benzothiazole (b-3) was obtained as a pale yellow solid (obtained amount: 10.32 g, yield: 69%).

Next, under water-cooling, potassium hydroxide (75.4 g, 1340 mmol) and ethylene glycol (175 mL) were put into a 1 L four-neck flask. After an argon atmosphere was established in the inside of the system, the compound (b-3) (7.8 g, 37.8 mmol) was put thereinto, and the resultant product was allowed to react at 110°C for 18 hours after bubbling was performed with argon to remove the oxygen in the system. The reaction liquid was cooled with water to 40°C or lower, and 2 mol/L hydrochloric acid which was subjected to argon bubbling in advance was added dropwise to the inside of the system to neutralize the reaction liquid (around pH 7). The precipitated white solid was separated by filtration, washed with water, and dried under reduced pressure. Thereafter, the white solid was purified by silica gel column chromatography (eluent: hexane/ethyl acetate), whereby 4-tert-butyl-2-mercaptoaniline (b-4) was obtained as a white solid (obtained amount: 2.39 g, yield: 35%).

Furthermore, acetic acid (872 mg, 14.5 mmol) and acetonitrile (30 mL) were put into a 100 mL three-neck flask, and an argon atmosphere was established in the inside of the system. Under the argon atmosphere, malononitrile (2.4 g, 36.3 mmol) and the compound (b-4) (2.39 g, 13.2 mmol) were added thereto, followed by heating to reflux for 2 hours. After the acetonitrile was removed under reduced pressure, the residues were dissolved in ethyl acetate, then, the organic layer was washed with water and a saturated saline solution, and treated with anhydrous magnesium sulfate. After the magnesium sulfate was separated by filtration, the solvent was removed under reduced pressure, and the residues were purified by silica gel column chromatography (eluent: hexane/ethyl acetate), whereby 2-(6-tert-butylbenzothiazol-2-yl) acetonitrile (b-5) was obtained as a yellow solid (obtained amount: 1.98 g, yield: 65%).

Subsequently, under an argon stream, the compound (b-2) (1.91 g, 3.5 mmol), the compound (b-5) (1.77 g, 7.68 mmol), and dehydrated toluene (68 mL) were put into a 200 mL three-neck flask, followed by heating to reflux. While heating to reflux, phosphoryl chloride (2.56 mL, 27.4 mmol) was added dropwise thereto using a syringe, followed by further heating to reflux for 2 hours. After the reaction ended, dichloromethane (40 mL) and a saturated sodium hydrogen carbonate aqueous solution (40 mL) were added thereto while ice-cooling, and the resultant product was extracted with dichloromethane. The organic layer was treated with anhydrous magnesium sulfate, the magnesium sulfate was separated by filtration, the solvent was removed under reduced pressure, and silica gel column chromatography (eluent: hexane/ethyl acetate) was used to roughly remove the impurities in the residues. The residues obtained by distilling off the solvent were purified again by silica gel column chromatography (eluent: hexane/dichloromethane), whereby a precursor (b-6) was obtained as a green solid (obtained amount: 1.56 g, yield: 46%).

Finally, under an argon stream, the precursor (b-6) (1.52 g, 1.57 mmol), toluene (45 mL), triethylamine (4.35 mL, 31.4 mmol), and boron trifluoride diethylether complex (7.88 mL, 62.7 mmol) were put into a 200 mL three-neck flask, followed by heating to reflux for 1 hour. The reaction liquid was cooled with ice, and the precipitated solid was separated by filtration, washed with water, a saturated sodium hydrogen carbonate aqueous solution, and a 50% methanol aqueous solution, and dried under reduced pressure. The obtained residues were dissolved in toluene, and methanol was added thereto to precipitate a solid, whereby a near-infrared fluorescent pigment B was obtained as a dark green solid (obtained amount: 1.25 g, yield: 75%).
¹H-NMR(300MHz,CDCl₃):δ=7.90(d,2H),7.72-7.69(m,6H),7.51(dd,2H),7.08(d,2 H),4.07(t,4H), 1.84(m,4H), 1.52(s, 18H), 1.35-1.32(m,24H),0.92(t,6H)ppm.

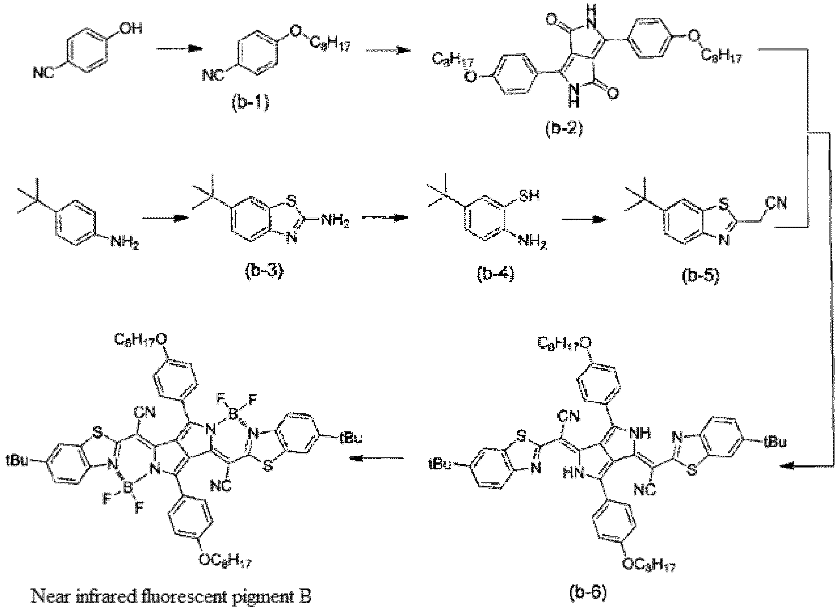

### [Preparation Example 4] Synthesis of Near-infrared Fluorescent Pigment C

Synthesis of a near-infrared fluorescent pigment C was performed in the following manner based on Organic Letters, 2012, Vol. 4, 2670-2673 and Chmestry-A European Journal, 2009, Vol. 15, 4857-4864.

4-tert-Butyl aniline (29.8 g, 0.2 mol) and 6 mol/L hydrochloric acid (100 mL) were put into a 300 mL three-neck flask, and crotonaldehyde (15.4 g, 0.22 mol) was added dropwise thereto while refluxing, followed by further refluxing for 2 hours. The refluxing was stopped, and while still being hot, zinc chloride (27.2 g, 0.2 mol) was added thereto, followed by stirring at room temperature overnight. The supernatant was removed, and isopropanol was added to the yellow syrupy residues, followed by refluxing for 2 hours. After the mixture was cooled to 70°C, petroleum ether (200 mL) was added thereto, and the precipitated crystal was collected by filtration, washed with diethyl ether, and dried, whereby zinc complex was obtained. This zinc complex was added to a mixed liquid of water/ammonia (120 mL/60 mL), and the resultant product was extracted three times with diethyl ether (80 mL). The obtained organic layer was dried over anhydrous magnesium sulfate, and concentrated, whereby 6-tert-butyl-2-methyl-quinoline (c-1) was obtained as a yellow liquid (obtained amount of 16.2 g, yield of 41%).

Next, the compound (c-1) (16.0 g, 80 mmol) and chloroform (50 mL) were put into a 200 mL two-neck flask, followed by stirring, and trichloroisocyanuric acid (6.52 g, 28 mmol) was added thereto in several aliquots. After the mixture was refluxed for 1 hour, the precipitated solid was filtered and washed with chloroform, and the obtained organic layer was extracted three times with 1mol/L sulfuric acid. The aqueous layers were combined, and the resultant product was adjusted to pH 3 with sodium carbonate aqueous solution, and extracted three times with diethyl ether. The organic layer was dried over anhydrous magnesium sulfate, and concentrated, whereby 2-chloromethyl-6-tert-butyl-quinoline (c-2) was obtained as a yellow crystal (obtained amount of 4.8 g, yield of 25.7%).

Furthermore, the compound (c-2) (4.7 g, 20 mmol), sodium cyanide (1.47 g, 30 mmol), a small amount of sodium iodide, and DMF (50 mL) were put into a 100 mL three-neck flask, and the resultant product was allowed to react at 60°C for 2 hours. The reaction liquid was cooled and extracted with water (200 mL)/ethyl acetate (300 mL), and the obtained ethyl acetate layer was further washed with water. The organic layer was dried over anhydrous magnesium sulfate and concentrated, and the resultant product was recrystallized from petroleum ether, whereby 2-(6-tert-butyl-quinolin-2-yl) acetonitrile (c-3) was obtained as a white crystal (obtained amount of 1.9 g, yield of 42.4%).

Subsequently, under an argon stream, the compound (b-2) (2.18 g, 4.0 mmol) obtained in Preparation Example 3, the compound (c-3) (1.9 g, 8.5 mmol), and dehydrated toluene (68 mL) were put into a 200 mL three-neck flask, followed by heating to reflux. While heating to reflux, phosphorus oxychloride (2.62 mL, 28 mmol) was added dropwise thereto using a syringe, followed by further heating to reflux for 2 hours. After the reaction ended, dichloromethane (40 mL) and a saturated sodium hydrogen carbonate aqueous solution (40 mL) were added thereto while ice-cooling, and the resultant product was extracted with dichloromethane. The organic layer was treated with anhydrous magnesium sulfate, the magnesium sulfate was separated by filtration, the solvent was removed under reduced pressure, and silica gel column chromatography (eluent: hexane/ethyl acetate) was used to roughly remove the impurities in the residues. The residues obtained by distilling off the solvent were purified again by silica gel column chromatography (eluent: hexane/dichloromethane), whereby a precursor (c-4) was obtained as a green solid (obtained amount: 1.84 g, yield: 48%).

Finally, under an argon stream, the precursor (c-4) (1.72 g, 1.8 mmol), toluene (45 mL), triethylamine (4.35 mL, 31.4 mmol), and boron trifluoride diethylether complex (7.88 mL, 62.7 mmol) were put into a 200 mL three-neck flask, followed by heating to reflux for 1 hours. The reaction liquid was cooled with ice, and the precipitated solid was separated by filtration, washed with water, a saturated sodium hydrogen carbonate aqueous solution, and a 50% methanol aqueous solution, and dried under reduced pressure. The obtained residues were dissolved in toluene, and methanol was added thereto to precipitate a solid, whereby a near-infrared fluorescent pigment C was obtained as a dark green solid (obtained amount: 1.10 g, yield: 58%).
¹H-NMR(300MHz,CDCl₃):δ=8.42(m,2H),8.14(d,2H),7.74(dd,2H),7.72(d,4H),7. 66(m,4H),7.06(m,4H),4.08(t,4H),1.85(m,4H),1.53(m,4H),1.45-1.2(m,16H),1.36(s, 18H),0 .91 (t,6H)ppm.

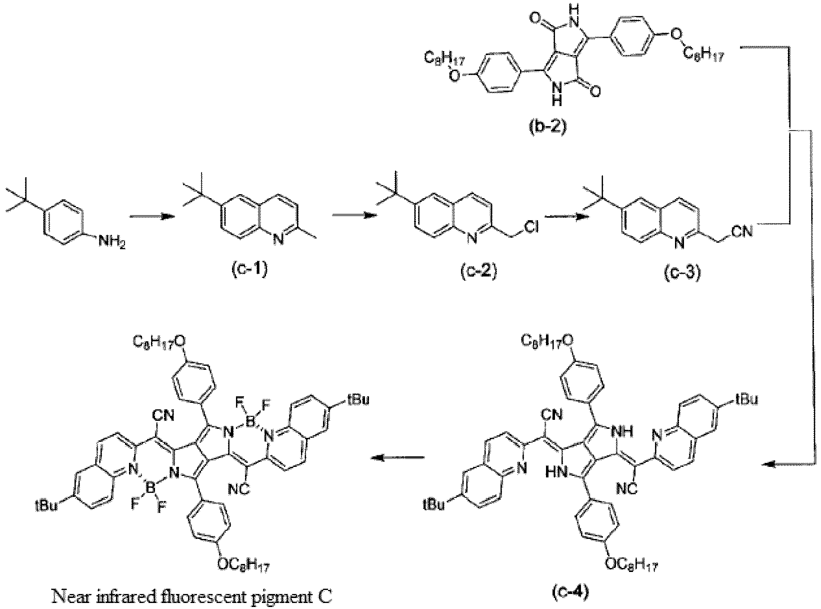

### [Preparation Example 5] Synthesis of Near-infrared Fluorescent Pigment D

Synthesis of a near-infrared fluorescent pigment D was performed in the following manner based on Organic Letters, 2012, Vol. 4, 2670-2673 and Chmestry-A European Journal, 2009, Vol. 15, 4857-4864.

Under argon stream, sodium hydride (60% dispersion, liquid paraffin) (4.0 g, 100 mmol) and dehydrated DMF and (40 mL) were put into a 200 mL three-neck flask, and the resultant product was cooled to 0°C. tert-Butyl cyanoacetate (11.9 g, 85 mmol) was slowly added thereto while stirring at the same temperature, followed by stirring at room temperature for 1 hour. Next, 2-chloro-4,6-dimethyl pyrimidine (10 g, 70 mmol) was added thereto, and the resultant product was allowed to react at 90°C for 36 hours. The reaction liquid was poured into an Erlenmeyer flask containing a 5% sodium chloride aqueous solution (200 ml), and the resultant product was neutralized with acetic acid. The precipitated yellow precipitate was collected by filtration, washed with water, and dried, whereby tert-butyl cyano-(4,6-dimethyl-pyrimidin-2-yl) acetate (d-1) was obtained (obtained amount of 9.8 g, yield of 56.9%).

Next, the compound (d-1) (9.8 g, 40 mmol), dichloromethane (60 mL), and trifluoroacetic acid (30 mL) were put into a 300 mL three-neck flask, and the resultant product was allowed to react at room temperature overnight. The reaction liquid was neutralized with a saturated sodium carbonate aqueous solution, and the dichloromethane layer was separated, and washed with water. The organic layer was dried over anhydrous magnesium sulfate and concentrated, and the obtained residues were purified by column chromatography (petroleum ether/ethyl acetate = 1/5), whereby (4,6-dimethyl-pyrimidin-2-yl) acetonitrile (d-2) was obtained as a white crystal (obtained amount of 0.85 g, yield of 14.5%).

Subsequently, under an argon stream, the compound (b-2) (1.36 g, 2.5 mmol) obtained in Preparation Example 3, the compound (d-2) (0.81 g, 5.5 mmol), and dehydrated toluene (50 mL) were put into a 200 mL three-neck flask, followed by heating to reflux. While heating to reflux, phosphoryl chloride (2.34 mL, 25 mmol) was added dropwise thereto using a syringe, followed by further heating to reflux for 2 hours. After the reaction ended, dichloromethane (40 mL) and a saturated sodium hydrogen carbonate aqueous solution (40 mL) were added thereto while ice-cooling, and the resultant product was extracted with dichloromethane. The organic layer was treated with anhydrous magnesium sulfate, the magnesium sulfate was separated by filtration, the solvent was removed under reduced pressure, and silica gel column chromatography (eluent: hexane/ethyl acetate) was used to roughly remove the impurities in the residues. The residues obtained by distilling off the solvent were purified again by silica gel column chromatography (eluent: dichloromethane/ethyl acetate = 50/1), whereby a precursor (d-3) was obtained as a green solid (obtained amount: 0.54 g, yield: 27%).

Finally, under an argon stream, the precursor (d-3) (522 mg, 0.65 mmol), N,N-diisopropylethylamine (258 mg, 2.0 mmol), and dichloromethane (20 mL) were put into a 100 mL two-neck flask, then, chlorodiphenylborane (600 mg, 3.0 mmol) was added thereto while refluxing, and the resultant product was allowed to react overnight. The reaction liquid was washed with water, and the organic layer was dried over anhydrous magnesium sulfate, and concentrated. The residues were washed with methanol, and purified by column chromatography (eluent: dichloromethane/ethyl acetate = 100/1), whereby a near-infrared fluorescent pigment D was obtained as a green solid (obtained amount: 0.24 g, yield: 32.6%).
¹H-NMR(300MHz,CDCl₃):δ=7.11(m,20H),6.43(m,4H),6.25(s,2H),6.02(m,4H),3 .92(t,4H),2.27(s,6H),1.78(m,10H),1.5-1.2(m,20H),0.85(t,6H)ppm.

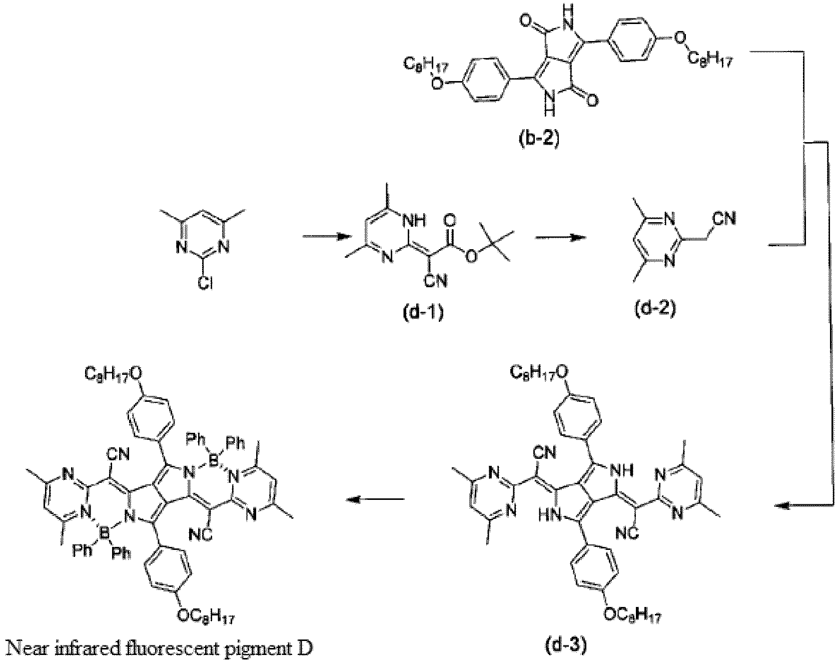

### [Preparation Example 6] Synthesis of Near-infrared Fluorescent Pigment E

Synthesis of a near-infrared fluorescent pigment E was performed in the following manner based on Organic Letters, 2012, Vol. 4, pp. 2670-2673 and Chmestry-A European Journal, 2009, Vol. 15, pp. 4857-4864.

3,6-(4-(2-Ethylhexyl)oxyphenyl)pyrrolo[3,4-c]pyrrole-1,4(2H,5H)-dione (e-2) was obtained as a red solid (obtained amount: 4.6 g) in the same manner as in Preparation Example 3 except that 1-bromo-2-ethylhexane (48 g, 249 mmol) was used instead of 1-bromooctane (48 g, 249 mmol).

Next, 2-amino-4-tert-butylphenol (5.24 g, 31.7 mmol), 2-cyano-acetymytic acid ethyl ester hydrochloride (4.45 g, 33.3 mmol), dichloromethane (30 mL) were put into a 100 mL two-neck flask, followed by refluxing overnight. The reaction liquid was diluted with dichloromethane (100 mL), and the resultant product was washed twice with a 1 mol/L sodium hydroxide aqueous solution. The organic layer was dried over anhydrous magnesium sulfate, and the solvent was removed, whereby (5-tert-butyl-benzoxazol-2-yl)-acetonitrile (e-3) was obtained as a yellow liquid (obtained amount of 6.3 g, yield of 88%).

Subsequently, under an argon stream, the compound (e-2) (1.64 g, 3.0 mmol), the compound (e-3) (1.41 g, 6.6 mmol), and dehydrated toluene (50 mL) were put into a 200 mL three-neck flask, followed by heating to reflux. While heating to reflux, phosphoryl chloride (2.34 mL, 25 mmol) was added dropwise thereto using a syringe, followed by further heating to reflux for 2 hours. After the reaction ended, dichloromethane (40 mL) and a saturated sodium hydrogen carbonate aqueous solution (40 mL) were added thereto while ice-cooling, and the resultant product was extracted with dichloromethane. The organic layer was treated with anhydrous magnesium sulfate, the magnesium sulfate was separated by filtration, the solvent was removed under reduced pressure, and silica gel column chromatography (eluent: hexane/ethyl acetate) was used to roughly remove the impurities in the residues. The residues obtained by distilling off the solvent were purified again by silica gel column chromatography (eluent: dichloromethane), whereby a precursor (e-4) was obtained as a bluish green solid (obtained amount: 0.98 g, yield: 35%).

Finally, under an argon stream, the precursor (e-4) (973 mg, 1.0 mmol), N,N-diisopropylethylamine (387 mg, 3.0 mmol), and dichloromethane (30 mL) were put into a 100 mL two-neck flask, then, chlorodiphenylborane (900 mg, 4.5 mmol) was added thereto while refluxing, and the resultant product was allowed to react overnight. The reaction liquid was washed with water, and the organic layer was dried over anhydrous magnesium sulfate, and concentrated. The residues were washed with methanol, and purified by column chromatography (eluent: dichloromethane), whereby a near-infrared fluorescent pigment E was obtained as a green solid (obtained amount: 0.42 g, yield: 35%).
¹H-NMR(300MHz,CDCl₃):δ=7.11(m,24H),6.62(m,4H),6.32(m,6H),3.8-3.9(m,4 H),2.27(s,6H),1.8(m,2H),1.6-1.3(m,16H),1.38(s,18H),0.9-1.0(m,12H)ppm.

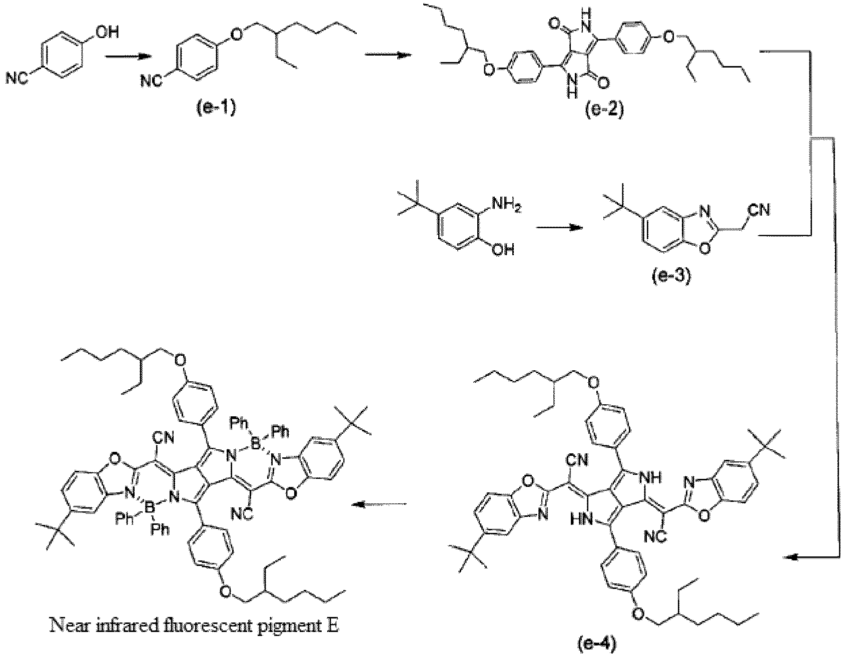

### [Preparation Example 7] Synthesis of Near-infrared Fluorescent Pigment F

A near-infrared fluorescent pigment F was synthesized according to the method described in Journal of Organic Chemistry, 2011, Vol. 76, pp. 4489-4505.

Under an argon stream, 2-ethylthiophene (11.2 g, 100 mmol) and dehydrated THF (80 mL) were put into a 500 mL four-neck flask, followed by stirring at -78°C. n-Butyllithium (68.8 mL, a 1.6 mol/L hexane solution) was added dropwise to this solution, followed by stirring at the same temperature for 1 hour, and a dehydrated THF solution (50 mL) of ethyl chloroformate (10.9 mL, 120 mmol) was added dropwise, followed by further stirring for 1 hour. After the temperature of the reaction liquid was returned to room temperature, a saturated ammonium chloride aqueous solution (110 mL) was added thereto, and the resultant product was extracted with dichloromethane. The organic phase was washed sequentially with water and a saturated saline solution, dried over anhydrous magnesium sulfate, and concentrated. The residues were separated and purified by silica gel chromatography (eluent: dichloromethane/cyclohexane = 6/4 (in volume ratio)), whereby 5-ethylthiophene-2-carboxylate (f-1) was obtained as a colorless liquid (obtained amount: 15.4 g, yield: 83.7%).

Next, the compound (f-1) (15.0 g, 81.5 mmol) and ethanol (40 mL) were put into a 200 mL four-neck flask, and hydrazine monohydrate (12.2 g, 244 mmol) was added dropwise to this solution, followed by refluxing and stirring for 12 hours. After the reaction liquid was cooled, the solvent was distilled off under reduced pressure, and the residues were dissolved in dichloromethane, washed sequentially with water and a saturated saline solution, dried over anhydrous magnesium sulfate, and concentrated. The residues were recrystallized from cyclohexane, collected by filtration, and dried, whereby 5-ethylthiophene-2-carbohydrazine (f-2) was obtained as a white solid (obtained amount: 8.6 g, yield: 62.1%).

Furthermore, the compound (f-2) (8.5 g, 50 mmol) and 4-methoxyacetophenone (7.5 g, 50 mmol) were put into a 50 mL three-neck flask, followed by stirring at 75°C for 1 hour. The residues were recrystallized from dichloromethane/methanol, collected by filtration, and dried, whereby (E)-5-ethyl-N'-(1-(2-hydroxy-4-methoxyphenyl)ethylidene)-thiophene-2-carbohydrazine (f-3) was obtained as a white solid (obtained amount: 12.4 g, yield: 78%).

Subsequently, the compound (f-3) (9.5 g, 29.8 mmol) and THF (300 mL) were put into a 500 mL four-neck flask and dissolved, and lead acetate (15.9 g, 35.9 mmol) was added to this solution, followed by stirring at room temperature for 1 hour. The reaction liquid was filtered, then, the filtrate was concentrated under reduced pressure, and the obtained residues were extracted with water/dichloromethane. The organic phase was washed sequentially with water and a saturated saline solution, dried over anhydrous magnesium sulfate, and concentrated under reduced pressure. The residues were separated and purified by alumina chromatography (eluent: dichloromethane/cyclohexane = 4/6 (in volume ratio)), whereby (5-ethyl-2-thienyl)(2-acetyl-5-methoxy-1-phenyl) ketone (f-4) was obtained as a white solid (obtained amount: 7.6 g, yield: 88.6%).

Furthermore, under an argon stream, the compound (f-4) (6.6 g, 22.8 mmol), acetic acid (48 mL), and ethanol (240 mL) were put into a 500 mL four-neck flask, followed by stirring at 65°C, and ammonium chloride (1.22 g, 22.8 mmol) and ammonium acetate (10.7 g, 139 mmol) were added to this solution, followed by refluxing and stirring for 30 minutes. The reaction liquid was filtered, then, the filtrate was concentrated under reduced pressure, and the obtained residues were extracted with water/dichloromethane. The organic phase was washed sequentially with water and a saturated saline solution, dried over anhydrous magnesium sulfate, and concentrated under reduced pressure. The residues were separated and purified by silica gel chromatography (eluent: dichloromethane), whereby a compound (f-5) was obtained as a dark blue solid (obtained amount: 2.1 g, yield: 35.2%).

Finally, under an argon stream, the compound (f-5) (2.0 g, 3.8 mmol) and dichloromethane (250 mL) were put into a 2 L flask, followed by stirring at room temperature for 5 minutes. N,N-diisopropylethylamine (1.48 g, 11.5 mmol) and boron trifluoride diethylether complex (3.27 g, 23 mmol) were added dropwise thereto, followed by stirring at room temperature for 1 hour. The reaction liquid was concentrated, and the residues were separated and purified by silica gel column chromatography (eluent: dichloromethane), whereby a near-infrared fluorescent pigment F was obtained as a dark green solid (obtained amount: 1.66 g, yield: 76%).
¹H-NMR(300MHz,CDCl₃/CCl₄=1/1):δ=7.85(s,2H),7.64(d,2H),7.39(s,1H),7.29(s ,2H),6.98(m,4H),3.86(s,6H),2.98(q,4H),1.43(t,6H)ppm.

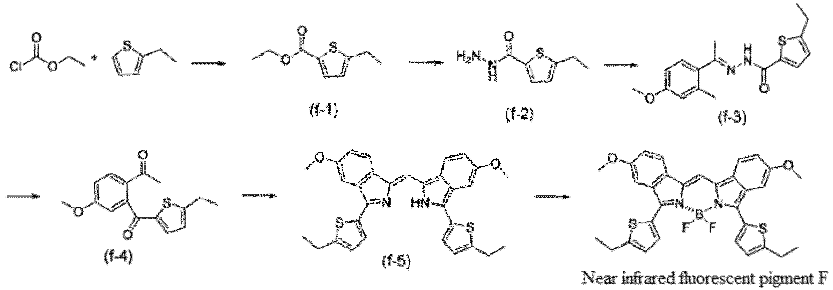

### [Preparation Example 8] Synthesis of Near-infrared Fluorescent Pigment G

A near-infrared fluorescent pigment G was synthesized according to the method described in Chemistry An Asian Journal, 2013, Vol. 8, pp. 3123-3132.

Under an argon stream, 5-bromo-2-thiophenecarboxaldehyde (19.1 g, 0.1 mol) and ethyl azidoacetate (51.6 g, 0.4 mol) were dissolved in ethanol (800 mL) in a 2 L four-neck flask, and a 20% by mass sodium ethoxide ethanol solution (136 g, 0.4 mol) was slowly added dropwise to the obtained solution at 0°C in an ice bath, followed by stirring for 2 hours. After the reaction ended, a saturated ammonium chloride aqueous solution was added thereto to adjust the pH to be weakly acidic. Furthermore, water was added thereto, and the precipitate was collected by filtration, and dried, whereby ethyl 2-azido-3-(5-bromo-thiophen-2-yl)-acrylate (a-2) was obtained as a yellow solid (obtained amount: 18.4 g, yield: 61.3%).

Next, 2-azido-3-(5-bromo-thiophen-2-yl)-acrylate (18.1 g, 60 mmol) was put into a 500 mL egg-plant shaped flask, and dissolved in o-xylene (200 mL), followed by refluxing and stirring for 1.5 hours. After the solution after refluxing and stirring was concentrated under reduced pressure, the obtained crude product was recrystallized (solution: hexane and ethyl acetate), then, the resultant product was subjected to suction filtration, and the obtained filtered material was dried, whereby ethyl 2-bromo-4H- thieno [3.2-b] pyrrole-5-carboxylate (g-1) was obtained (obtained amount: 12.1 g, yield: 73.8%).

Furthermore, the compound (g-1) (6.0 g, 22 mmol) was put into a 500 mL flask, and an aqueous solution obtained by dissolving ethanol (200 mL) and sodium hydroxide (12.4 g, 310 mmol) in water (100 mL) was added thereto, followed by refluxing and stirring for 1 hour. After the solution after refluxing and stirring was cooled, a 6 mol/L hydrochloric acid was added thereto to adjust the solution to be acidic, water was added thereto, suction filtration was performed, and the obtained filtered material was vacuum-dried, whereby 2-bromo-4H-thieno[3.2-b]pyrrole-5-carboxylic acid (g-2) was obtained as a gray solid (obtained amount: 4.1 g, yield: 75.8%).

Subsequently, the compound (g-2) (4.0 g, 16.3 mmol) and trifluoroacetic acid (100 mL) were put into a 300 mL three-neck flask, followed by stirring at 40°C. After the compound (d-2) was dissolved, stirring was performed for 15 minutes until the bubbles subsided. Trifluoroacetic anhydride (36 mL) was added to the solution after stirring, and the resultant product was allowed to react at 80°C for 4 hours. After the reaction ended, the reaction liquid was added to a saturated sodium hydrogen carbonate aqueous solution containing ice to neutralize the solution, then, suction filtration was performed, and the resultant product was vacuum-dried, whereby a compound (g-3) was obtained as a crude product.

Furthermore, under an argon stream, the compound (g-3) and dichloromethane (1 L) were put into a 2 L flask, followed by stirring at room temperature for 5 minutes. Triethylamine (12 mL) and boron trifluoride diethylether complex (16 mL) were added dropwise thereto, followed by stirring at room temperature for 1 hour. The reaction liquid was concentrated, and the residues were separated and purified by silica gel column chromatography (eluent: dichloromethane), whereby 2,8-dibromo-11-trifluoromethyl-dithieno[2,3-b][3,2-g]-5,5-difluoro-5-bora-3a,4a-dithio-s -indacene (g- 4) was obtained as a dark bluish green solid (obtained amount: 580 mg, yield: 13.4%).

Finally, under an argon stream, the compound (g-4) (200 mg, 0.378 mmol), 4-methoxyphenyl boronic acid (240 mg, 1.6 mmol), sodium carbonate (120 mg, 1.2 mmol), toluene/THF/water = 1: 1: 1 (60 mL) were put into a 200 mL three-neck flask, and after bubbling for 30 minutes with argon gas, tetrakis(triphenylphosphine)palladium (0) (22 mg) was added thereto, and the resultant product was subjected to a coupling reaction at 80°C for 4 hours. After cooling, water (10 mL) was added to the reaction liquid, and the resultant product was extracted three times with diethyl ether. The obtained organic phase was washed with water and a saturated saline solution, dried over anhydrous magnesium sulfate, and the solvent was concentrated under reduced pressure. The obtained crude product was separated and purified by silica gel chromatography (eluent: toluene/ethyl acetate = 20/1 (in volume ratio)), whereby a near-infrared fluorescent pigment G was obtained as a dark green crystal (obtained amount: 110 mg, yield: 49.8%).
¹H-NMR(300MHz,CD₂Cl₂):δ=7.76(d,4H),7.34(s,2H),7.32(s,2H),7.03(d,4H),3.9 1(s,6H)ppm.

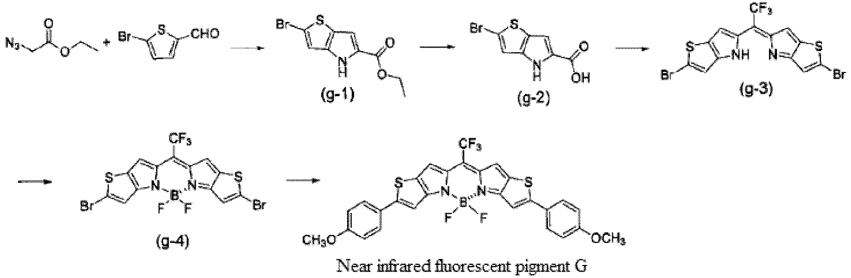

### [Preparation Example 9] Synthesis of Near-infrared Fluorescent Pigment H

A near-infrared fluorescent pigment H was obtained as a dark green crystal (obtained amount: 94 mg, yield: 46.4%) in the same manner as in Preparation Example 7 except that thiophene-2-boronic acid (205 mg, 1.6 mmol) was used instead of 4-methoxyphenyl boronic acid.
¹H-NMR(300MHz,CD₂Cl₂):δ=7.57(m,4H),7.54(d,2H),7.53(s,2H),7.34(s,2H),7.2 4(m,2H)ppm

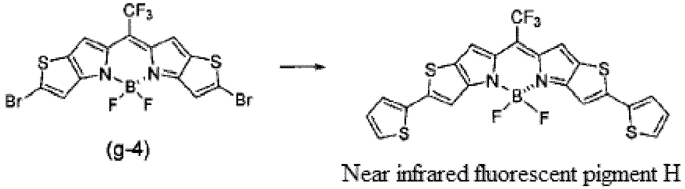

### [Reference Example 1]

55 g of TPU pellets containing 40% by mass of barium sulfate (product name: EG-60D-B40, manufactured by Lubrizol Corp.) and 16.5 mg of Coumarin 6 (a reagent commercially available from Tokyo Chemical Industry Co., Ltd., a visible fluorescent material) were mixed, and a fluorescent material was attached to the pellet surfaces. Next, the pellets were put into Labo Plastomill (manufactured by Toyo Seiki Seisaku-sho, Ltd.), and melt-kneaded at a set temperature of 190°C for 10 minutes. Thereafter, the kneaded fluorescent material-containing resin was taken out, and made to be a film.

The film was obtained in the following manner. First, the melt-kneaded fluorescent material-containing resin was heated for 5 minutes while being sandwiched between iron plates heated to 200°C, and pressed at 5 mPa to 10 mPa while the steel plates were cooled. The film thickness at this time was about 300 um, and the pigment concentration was 0.03 % by mass. In addition, the mixing ratio of the fluorescent material and the radiopaque substance (mass of fluorescent material / mass of radiopaque substance) was 0.00075.

The absorption spectrum of the obtained film was measured using an ultraviolet visible near-infrared spectrophotometer "UV3600" manufactured by Shimadzu Co., and when the emission spectrum was measured using an Absolute PL quantum yields measurement system "Quantaurus-QY C11347" manufactured by Hamamatsu Photonics K.K., it was confirmed that the maximum absorption wavelength was 444 nm, the maximum fluorescence wavelength was around 516 nm, and yellowish green fluorescence was emitted.

In addition, the film can be detected by X-ray photography, and the opaqueness to radiation was the same degree as that of the film obtained from the TPU before a fluorescent material was contained. From the above results, it is apparent that the resin composition according to the present invention containing a fluorescent material and a radiopaque substance can be visualized by using an X-ray detector or a fluorescence detector. The results are summarized in Table 1.

### [Comparative Example 1]

A film was manufactured in the same manner as in Reference Example 1 except that TPU pellets not containing barium sulfate (product name: EG-65D, manufactured by Lubrizol Corp.) were used instead of the pellets containing barium sulfate, and the same evaluation as in Reference Example 1 was performed. As a result, it could be confirmed that the obtained film emittted yellowish green fluorescence, but the film did not have opaqueness to X-rays, and thus, detection using an X-ray detector was not possible. The results are summarized in Table 1.

### [Reference Example 2]

A film having a pigment concentration of 0.03% by mass was manufactured in the same manner as in Reference Example 1 except that Lumogen (registered trademark) Red F305 (a visible light fluorescent material manufactured by BASF Corp.) was used instead of Coumarin 6 as a fluorescent material, and the same evaluation as in Reference Example 1 was performed. The maximum absorption wavelength of the obtained film was 534 nm, and the maximum fluorescence wavelength of the film was around 627 nm. Moreover, the mixing ratio of the fluorescent material and the radiopaque substance was 0.00075.

In addition, when the film was photographed using X-rays, and the opaqueness to radiation was the same degree as that of the film obtained from the TPU before a fluorescent material was contained. From the above results, it is apparent that the resin composition according to the present invention containing a fluorescent material and a radiopaque substance can be visualized by using an X-ray detector or a fluorescence detector. The results are summarized in Table 1.

### [Comparative Example 2]

A film was manufactured in the same manner as in Reference Example 2 except that TPU pellets not containing barium sulfate (product name: EG-65D, manufactured by Lubrizol Corp.) were used instead of the pellets containing barium sulfate, and the same evaluation as in Reference Example 1 was performed. As a result, it could be confirmed that the obtained film emitted red fluorescence, but the film did not have opaqueness to X-rays, and thus, detection using an X-ray detector was not possible. The results are summarized in Table 1.

### [Example 3]

A film having a pigment concentration of 0.03% by mass was manufactured in the same manner as in Reference Example 1 except that the azo-boron complex (near-infrared fluorescent material) synthesized in Preparation Example 1 was used instead of Coumarin 6 as a fluorescent material, and the same evaluation as in Reference Example 1 was performed. The maximum absorption wavelength of the obtained film was 683 nm, and the maximum fluorescence wavelength of the film was around 820 nm. Moreover, the mixing ratio of the fluorescent material and the radiopaque substance was 0.00075.

In addition, when the film was photographed using X-rays, and the opaqueness to radiation was the same degree as that of the film obtained from the TPU before a fluorescent material was contained. From the above results, it is apparent that the resin composition according to the present invention containing a fluorescent material and a radiopaque substance can be visualized by using an X-ray detector or a fluorescence detector. The results are summarized in Table 1.

### [Comparative Example 3]

A film was manufactured in the same manner as in Example 3 except that TPU pellets not containing barium sulfate (product name: EG-65D, manufactured by Lubrizol Corp.) were used instead of the pellets containing barium sulfate, and the same evaluation as in Reference Example 1 was performed. As a result, it could be confirmed that the obtained film emitted near-infrared fluorescence, but the film did not have opaqueness to X-rays, and thus, detection using an X-ray detector was not possible.

As described above, since the resin composition according to the present invention and a molded article obtained from the composition have opaqueness to radiation and contain a light-emitting substance, both of detection by X-ray photography and detection by light-emission are possible. In addition, since the resin composition according to the present invention has stronger emission intensity to the amount of light-emitting substance added than that of a resin composition not containing the radiopaque substance, it is possible to more sensitively detect light emission even by weaker excitation light, and therefore, it is thought that the resin composition according to the present invention is an industrially useful resin composition. The results are summarized in Table 1.

### [Example 4]

A film having a pigment concentration of 0.03% by mass was manufactured in the same manner as in Reference Example 1 except that the near-infrared fluorescent pigment A (near-infrared fluorescent material) synthesized in Preparation Example 2 was used instead of Coumarin 6 as a fluorescent material, and the same evaluation as in Reference Example 1 was performed. The maximum absorption wavelength of the obtained film was 730 nm, the maximum fluorescence wavelength of the film was 765 nm, and a fluorescence peak was observed at 824 nm. Moreover, the mixing ratio of the fluorescent material and the radiopaque substance was 0.00075.

In addition, when the film was photographed using X-rays, and the opaqueness to radiation was the same degree as that of the film obtained from the TPU before a fluorescent material was contained. From the above results, it is apparent that the resin composition according to the present invention containing a fluorescent material and a radiopaque substance can be visualized by using an X-ray detector or a fluorescence detector. The results are summarized in Table 1.

### [Comparative Example 4]

A film was manufactured in the same manner as in Example 4 except that TPU pellets not containing barium sulfate (product name: EG-65D, manufactured by Lubrizol Corp.) were used instead of the pellets containing barium sulfate, and the same evaluation as in Reference Example 1 was performed. As a result, it could be confirmed that the obtained film emits near-infrared fluorescence, but the film did not have opaqueness to X-rays, and thus, detection using an X-ray detector was not possible. The results are summarized in Table 1.

### [Example 5]

110 g of TPU pellets containing 40% by mass of barium sulfate (product name: EG-60D-B40, manufactured by Lubrizol Corp.) and 5.5 mg of the near-infrared fluorescent pigment A synthesized in Preparation Example 2 were mixed, and a fluorescent material was attached to the pellet surfaces. Next, the pellets were put into Labo Plastomill, and melt-kneaded at a set temperature of 190°C for 10 minutes. Thereafter, the kneaded fluorescent material-containing resin was taken out, and a film having a pigment concentration of 0.005% by mass was manufactured in the same manner as in Reference Example 1. Moreover, the mixing ratio of the fluorescent material and the radiopaque substance was 0.000125.

The absorption spectrum of the obtained film was measured using an ultraviolet visible near-infrared spectrophotometer "UV3600" manufactured by Shimadzu Co., and when the emission spectrum was measured using a fluorescence spectrophotometer "FP-8600" manufactured by JASCO Corporation (an excitation wavelength of 740 nm), the maximum absorption wavelength of the obtained film was 738 nm, the film had strong fluorescence at 750 nm or longer, and fluorescence having a peak at 827 nm was observed.

In addition, when the film was photographed using X-rays, the opaqueness to radiation was the same degree as that of the film obtained from the TPU before a fluorescent material was contained. From the above results, it is apparent that the resin composition according to the present invention containing a fluorescent material and a radiopaque substance can be visualized by using an X-ray detector or a fluorescence detector. The results are summarized in Table 1.

### [Comparative Example 5]

A film was manufactured in the same manner as in Example 5 except that TPU pellets not containing barium sulfate (product name: EG-65D, manufactured by Lubrizol Corp.) were used instead of the pellets containing barium sulfate as pellets used, and the same evaluation as in Example 5 was performed. As a result, it could be confirmed that the obtained film emitted near-infrared fluorescence, but the film did not have opaqueness to X-rays, and thus, detection using an X-ray detector was not possible. The results are summarized in Table 1.

### [Example 6]

A film having a pigment concentration of 0.005% by mass was manufactured in the same manner as in Example 5 except that the near-infrared fluorescent pigment B synthesized in Preparation Example 3 was used instead of the near-infrared fluorescent pigment A synthesized in Preparation Example 2 as a fluorescent material, then, the same evaluation as in Example 5 was performed, and the results are summarized in Table 1. Moreover, the maximum absorption wavelength of the obtained film was 738 nm, the maximum fluorescence wavelength of the film was 757 nm, and a fluorescence peak was observed at 832 nm. In addition, the mixing ratio of the fluorescent material and the radiopaque substance was 0.000125.

### [Comparative Example 6]

A film was manufactured in the same manner as in Example 6 except that TPU pellets not containing barium sulfate (product name: EG-65D, manufactured by Lubrizol Corp.) were used instead of the pellets containing barium sulfate as pellets used, and the same evaluation as in Example 6 was performed. As a result, it could be confirmed that the obtained film emitted near-infrared fluorescence, but the film did not have opaqueness to X-rays, and thus, detection using an X-ray detector was not possible. The results are summarized in Table 1.

### [Example 7]

A film having a pigment concentration of 0.00125% by mass was manufactured in the same manner as in Example 5 except that the amount of pellets used was 440 g instead of 110 g, and the near-infrared fluorescent pigment C synthesized in Preparation Example 4 was used instead of the near-infrared fluorescent pigment A synthesized in Preparation Example 2 as a fluorescent material, then, the same evaluation as in Example 5 was performed, and the results are summarized in Table 1. Moreover, the maximum absorption wavelength of the obtained film was 762 nm, the maximum fluorescence wavelength of the film was 772 nm, and a fluorescence peak was observed at 864 nm. In addition, the mixing ratio of the fluorescent material and the radiopaque substance was 0.0000313.

### [Example 8]

A film having a pigment concentration of 0.005% by mass was manufactured in the same manner as in Example 5 except that the near-infrared fluorescent pigment C synthesized in Preparation Example 4 was used instead of the near-infrared fluorescent pigment A synthesized in Preparation Example 2 as a fluorescent material, then, the same evaluation as in Example 5 was performed, and the results are summarized in Table 1. Moreover, the maximum absorption wavelength of the obtained film was 762 nm, the maximum fluorescence wavelength of the film was 784 nm, and a fluorescence peak was observed at 864 nm. In addition, the mixing ratio of the fluorescent material and the radiopaque substance was 0.000125.

### [Comparative Example 7]

A film was manufactured in the same manner as in Example 8 except that TPU pellets not containing barium sulfate (product name: EG-65D, manufactured by Lubrizol Corp.) were used instead of the pellets containing barium sulfate as pellets used, and the same evaluation as in Example 8 was performed. As a result, it could be confirmed that the obtained film emitted near-infrared fluorescence, but the film did not have opaqueness to X-rays, and thus, detection using an X-ray detector was not possible. The results are summarized in Table 1.

### [Example 9]

A film having a pigment concentration of 0.04% by mass was manufactured in the same manner as in Example 5 except that 44 mg of the near-infrared fluorescent pigment C synthesized in Preparation Example 4 was used instead of 5.5 mg of the near-infrared fluorescent pigment A synthesized in Preparation Example 2 as a fluorescent material, then, the same evaluation as in Example 5 was performed, and the results are summarized in Table 1. Moreover, the maximum absorption wavelength of the obtained film was 759 nm, the maximum fluorescence wavelength of the film was 809 nm, and a fluorescence peak was observed at 864 nm. In addition, the mixing ratio of the fluorescent material and the radiopaque substance was 0.001.

### [Example 10]

A film having a pigment concentration of 0.005% by mass was manufactured in the same manner as in Example 5 except that the near-infrared fluorescent pigment D synthesized in Preparation Example 5 was used instead of the near-infrared fluorescent pigment A synthesized in Preparation Example 2 as a fluorescent material, then, the same evaluation as in Example 5 was performed, and the results are summarized in Table 1. Moreover, the maximum absorption wavelength of the obtained film was 743 nm, the maximum fluorescence wavelength of the film was 760 nm, and a fluorescence peak was observed at 852 nm. In addition, the mixing ratio of the fluorescent material and the radiopaque substance was 0.000125.

### [Example 11]

A film having a pigment concentration of 0.005% by mass was manufactured in the same manner as in Example 5 except that the near-infrared fluorescent pigment E synthesized in Preparation Example 6 was used instead of the near-infrared fluorescent pigment A synthesized in Preparation Example 2 as a fluorescent material, then, the same evaluation as in Example 5 was performed, and the results are summarized in Table 1. Moreover, the maximum absorption wavelength of the obtained film was 754 nm, the maximum fluorescence wavelength of the film was 776 nm, and a fluorescence peak was observed at 872 nm. In addition, the mixing ratio of the fluorescent material and the radiopaque substance was 0.000125.

### [Example 12]

A film having a pigment concentration of 0.005% by mass was manufactured in the same manner as in Example 5 except that the near-infrared fluorescent pigment F synthesized in Preparation Example 7 was used instead of the near-infrared fluorescent pigment A synthesized in Preparation Example 2 as a fluorescent material, then, the same evaluation as in Example 5 was performed, and the results are summarized in Table 1. Moreover, the maximum absorption wavelength of the obtained film was 744 nm, and a fluorescence peak at the maximum fluorescence wavelength of 787 nm was observed. In addition, the mixing ratio of the fluorescent material and the radiopaque substance was 0.000125.

### [Example 13]

A film having a pigment concentration of 0.03% by mass was manufactured in the same manner as in Example 5 except that 33 mg of the near-infrared fluorescent pigment G synthesized in Preparation Example 8 was used instead of 5.5 mg of the near-infrared fluorescent pigment A synthesized in Preparation Example 2 as a fluorescent material, then, the same evaluation as in Example 5 was performed, and the results are summarized in Table 1. Moreover, the maximum absorption wavelength of the obtained film was 741 nm, and a fluorescence peak at the maximum fluorescence wavelength of 771 nm was observed. In addition, the mixing ratio of the fluorescent material and the radiopaque substance was 0.00075.

### [Example 14]

A film having a pigment concentration of 0.03% by mass was manufactured in the same manner as in Example 13 except that the near-infrared fluorescent pigment H synthesized in Preparation Example 9 was used instead of the near-infrared fluorescent pigment G synthesized in Preparation Example 8 as a fluorescent material, then, the same evaluation as in Example 13 was performed, and the results are summarized in Table 1. Moreover, the maximum absorption wavelength of the obtained film was 744 nm, and a fluorescence peak at the maximum fluorescence wavelength of 776 nm was observed. In addition, the mixing ratio of the fluorescent material and the radiopaque substance was 0.00075.

### [Example 15]

88 g of TPU pellets (product name: EG-60D, manufactured by Lubrizol Corp.), 22 g of bismuth oxide (manufactured by Sigma-Aldrich Co.), and 5.5 mg of the near-infrared fluorescent pigment B synthesized in Preparation Example 3 were mixed, and a fluorescent material was attached to the pellet surfaces. Next, the pellets were put into Labo Plastomill, and melt-kneaded at a set temperature of 190°C for 10 minutes. Thereafter, the kneaded fluorescent material-containing resin was taken out, and a film having a pigment concentration of 0.005% by mass which contained 20% by mass of bismuth oxide was manufactured in the same manner as in Example 5. The mixing ratio of the fluorescent material and the radiopaque substance at this time was 0.00025. Evaluation was performed on this film in the same manner as in Example 5, and the results are summarized in Table 2. Moreover, the maximum absorption wavelength of the obtained film was 738 nm, the maximum fluorescence wavelength of the film was 756 nm, and a fluorescence peak was observed at 830 nm.

### [Example 16]

104.5 g of TPU pellets (product name: EG-60D, manufactured by Lubrizol Corp.), 5.5 g of calcium carbonate (manufactured by Sigma-Aldrich Co.), and 5.5 mg of the near-infrared fluorescent pigment B synthesized in Preparation Example 3 were mixed, and a fluorescent material was attached to the pellet surfaces. Next, the pellets were put into Labo Plastomill, and melt-kneaded at a set temperature of 190°C for 10 minutes. Thereafter, the kneaded fluorescent material-containing resin was taken out, and a film having a pigment concentration of 0.005% by mass which contained 5% by mass of calcium carbonate was manufactured in the same manner as in Example 5. The mixing ratio of the fluorescent material and the radiopaque substance at this time was 0.001. Evaluation was performed on this film in the same manner as in Example 5, and the results are summarized in Table 2. Moreover, the maximum absorption wavelength of the obtained film was 738 nm, the maximum fluorescence wavelength of the film was 756 nm, and a fluorescence peak was observed at 830 nm.

### [Example 17]

A film having a pigment concentration of 0.005% by mass was manufactured in the same manner as in Example 15 except that the near-infrared fluorescent pigment C synthesized in Preparation Example 4 was used instead of the near-infrared fluorescent pigment B synthesized in Preparation Example 3, then, the same evaluation as in Example 15 was performed, and the results are summarized in Table 2. Moreover, the maximum absorption wavelength of the obtained film was 762 nm, the maximum fluorescence wavelength of the film was 783 nm, and a fluorescence peak was observed at 859 nm. In addition, the mixing ratio of the fluorescent material and the radiopaque substance was 0.00025.

### [Example 18]

A film having a pigment concentration of 0.005% by mass was manufactured in the same manner as in Example 16 except that the near-infrared fluorescent pigment C synthesized in Preparation Example 4 was used instead of the near-infrared fluorescent pigment B synthesized in Preparation Example 3, then, the same evaluation as in Example 16 was performed, and the results are summarized in Table 2. Moreover, the maximum absorption wavelength of the obtained film was 762 nm, the maximum fluorescence wavelength of the film was 779 nm, and a fluorescence peak was observed at 858 nm. In addition, the mixing ratio of the fluorescent material and the radiopaque substance was 0.00025.

### [Example 19]

88 g of PP pellets (product name: B221WA, manufactured by SunAllomer Ltd.), 22 g of barium sulfate (manufactured by Wako Pure Chemical Industries, Ltd.), and 5.5 mg of the near-infrared fluorescent pigment B synthesized in Preparation Example 3 were mixed, and a fluorescent material was attached to the pellet surfaces. Next, the pellets were put into Labo Plastomill, and melt-kneaded at a set temperature of 180°C for 10 minutes. Thereafter, the kneaded fluorescent material-containing resin was taken out, and a PP film having a pigment concentration of 0.005% by mass which contained 20% by mass of barium sulfate was manufactured in the same manner as in Example 5. The mixing ratio of the fluorescent material and the radiopaque substance at this time was 0.00025. Evaluation was performed on this film in the same manner as in Example 5, and the results are summarized in Table 3. Moreover, the maximum absorption wavelength of the obtained film was 737 nm, the maximum fluorescence wavelength of the film was around 750 nm, and a fluorescence peak was observed at 827 nm.

### [Comparative Example 8]

A film was manufactured in the same manner as in Example 19 except that instead of using barium sulfate, PP pellets not containing barium sulfate (product name: B221WA, manufactured by SunAllomer Ltd.) were used as pellet, and the same evaluation as in Example 19 was performed. As a result, it could be confirmed that the obtained film emitted near-infrared fluorescence, but the film did not have opaqueness to X-rays, and thus, detection using an X-ray detector was not possible. The results are summarized in Table 3.

### [Example 20]

A polystyrene film having a pigment concentration of 0.005% by mass was manufactured in the same manner as in Example 19 except that the near-infrared fluorescent pigment A synthesized in Preparation Example 2 was used instead of the near-infrared fluorescent pigment B synthesized in Preparation Example 3, polystyrene (DIC styrene (trade mark) LP-6000, manufactured by DIC Corporation) was used instead of the PP pellets, and the kneading temperature was 230°C, then, the same evaluation as in Example 19 was performed, and the results are summarized in Table 2. Moreover, when the maximum absorption wavelength of the obtained film was 736 nm, the film had strong fluorescence at 750 nm or longer, and a fluorescence peak was observed at 830 nm. In addition, the mixing ratio of the fluorescent material and the radiopaque substance was 0.00025.

### [Example 21]

A PET film having a pigment concentration of 0.005% by mass was manufactured in the same manner as in Example 19 except that the near-infrared fluorescent pigment A synthesized in Preparation Example 2 was used instead of the near-infrared fluorescent pigment B synthesized in Preparation Example 3, PET (Byron (trade mark) SI-173C, manufactured by Toyobo Co., Ltd.) was used instead of the PP pellets, and the kneading temperature was 210°C, then, the same evaluation as in Example 19 was performed, and the results are summarized in Table 2. Moreover, when the maximum absorption wavelength of the obtained film was 738 nm, the film had strong fluorescence at 750 nm or longer, and a fluorescence peak was observed at 827 nm. In addition, the mixing ratio of the fluorescent material and the radiopaque substance was 0.00025.

**[Table 1]**

| | Fluorescent material used | | Concentration of barium sulfate | Light emission | Detection by X-rays |
|---|---|---|---|---|---|
| | Type | Concentration | | | |
| Reference Example 1 | Coumarin 6 | 0.03% | 40% | Present | Possible |
| Reference Example 2 | Lumogen Red 305 | 0.03% | 40% | Present | Possible |
| Example 3 | Azo-boron complex | 0.03% | 40% | Present | Possible |
| Example 4 | Near-infrared fluorescent pigment A | 0.03% | 40% | Present | Possible |
| Example 5 | Near-infrared fluorescent pigment A | 0.005% | 40% | Present | Possible |
| Example 6 | Near-infrared fluorescent pigment B | 0.005% | 40% | Present | Possible |
| Example 7 | Near-infrared fluorescent pigment C | 0.00125% | 40% | Present | Possible |
| Example 8 | Near-infrared fluorescent pigment C | 0.005% | 40% | Present | Possible |
| Example 9 | Near-infrared fluorescent pigment C | 0.04% | 40% | Present | Possible |
| Example 10 | Near-infrared fluorescent pigment D | 0.005% | 40% | Present | Possible |
| Example 11 | Near-infrared fluorescent pigment E | 0.005% | 40% | Present | Possible |
| Example 12 | Near-infrared fluorescent pigment F | 0.005% | 40% | Present | Possible |
| Example 13 | Near-infrared fluorescent pigment G | 0.03% | 40% | Present | Possible |
| Example 14 | Near-infrared fluorescent pigment H | 0.03% | 40% | Present | Possible |
| Comparative Example 1 | Coumarin 6 | 0.03% | 0% | Present | Impossible |
| Comparative Example 2 | Lumogen Red 305 | 0.03% | 0% | Present | Impossible |
| Comparative Example 3 | Azo-boron complex | 0.03% | 0% | Present | Impossible |
| Comparative Example 4 | Near-infrared fluorescent pigment A | 0.03% | 0% | Present | Impossible |
| Comparative Example 5 | Near-infrared fluorescent pigment A | 0.005% | 0% | Present | Impossible |
| Comparative Example 6 | Near-infrared fluorescent pigment B | 0.005% | 0% | Present | Impossible |
| Comparative Example 7 | Near-infrared fluorescent pigment C | 0.005% | 0% | Present | Impossible |

As clearly seen from Table 1, since the film obtained from the resin composition according to the present invention contains a fluorescent material and a radiopaque substance (barium sulfate), the film could be confirmed by both near-infrared fluorescence and X-rays, but the films of Comparative Examples could not be confirmed by X-rays.

**[Table 2]**

| | Fluorescent material used | | Type and concentration of radiopaque substance | Light emission | Detection by X-rays |
|---|---|---|---|---|---|
| | Type | Concentration | | | |
| Example 15 | Near-infrared fluorescent pigment B | 0.005% | Bismuth oxide 20% | Present | Possible |
| Example 16 | Near-infrared fluorescent pigment B | 0.005% | Calcium carbonate 5% | Present | Possible |
| Example 17 | Near-infrared fluorescent pigment C | 0.005% | Bismuth oxide 20% | Present | Possible |
| Example 18 | Near-infrared fluorescent pigment C | 0.005% | Calcium carbonate 5% | Present | Possible |

In addition, from Table 2, the radiopaque substance which can be used in the resin composition according to the present invention is not limited to barium sulfate, and it is found that various materials having opaqueness to radiation are effective.

**[Table 3]**

| | Type of resin | Fluorescent material used | | Light emission | Detection by X-rays |
|---|---|---|---|---|---|
| | | Type | Concentration | | |
| Example 19 | PP | Near-infrared fluorescent pigment B | 0.005% | Present | Possible |
| Example 20 | PS | Near-infrared fluorescent pigment A | 0.005% | Present | Possible |
| Example 21 | PET | Near-infrared fluorescent pigment A | 0.005% | Present | Possible |

Furthermore, from Table 3, the resin which can be used in the resin composition according to the present invention is not limited to TPU, and it is found that various resins are effective.

### [Test Reference Example 1]

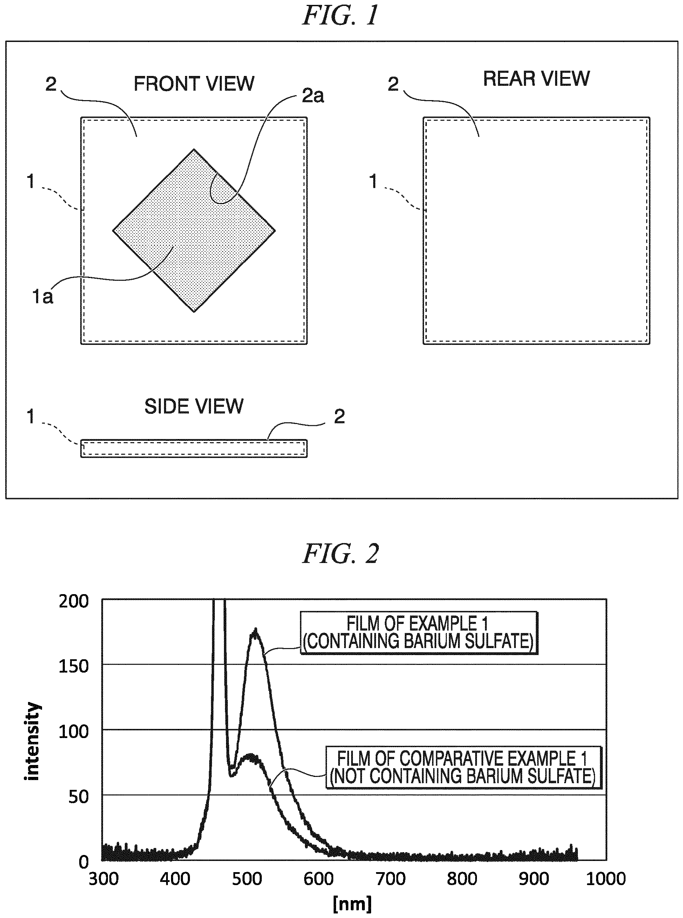

The film (1) manufactured in Reference Example 1 was cut into a size of 1 cm x 1 cm, the cut film was wrapped with aluminum foil (2) of which the inside had been blacked such that an opening (2a) of 5 mm x 5 mm can be formed on only one side thereof, and the part other than the exposed surface (1a) from the opening (2a) was shielded (FIG. 1). Thus, since only the exposed surface (1a) absolved light and only the exposed surface (1a) emitted fluorescence, it was possible to assume the case of actually detecting using a detector such as a camera. The emission spectrum of the film manufactured in this manner, in the case of being irradiated with excitation light of 463 nm was measured using an Absolute PL quantum yields measurement system "Quantaurus-QY C11347" manufactured by Hamamatsu Photonics K.K., and the fluorescence spectrum of the film was measured. In the same manner, the film manufactured in Comparative Example 1 was partially shielded with aluminum foil, and the fluorescence spectrum of the film was measured. As a result, the intensity (fluorescence intensity) at 516 nm which is around the maximum fluorescence wavelength was 170, and this was 115% stronger than the intensity at the maximum fluorescence wavelength of the film of Comparative Example 1 (FIG. 2). The light-emitting efficiency of the film of Reference Example 1 was 0.17, the light-emitting efficiency of the film of Comparative Example 1 was 0.07, and the film of Reference Example 1 had a higher light-emitting efficiency. Therefore, it was found that the film containing barium sulfate had stronger fluorescence intensity, and was easily detected by a detector.

### [Test Reference Example 2]

Each of the films manufactured in Reference Example 2 and Comparative Example 2 was partially exposed in the same manner as in Reference Example 1, and the fluorescence spectrum of the film in the case of being irradiated with excitation light of 582 nm was measured. As a result, the intensity at 627 nm which is around the maximum fluorescence wavelength was 95, and this was about 118% stronger than the intensity at the maximum fluorescence wavelength of the film of Comparative Example 2. Therefore, it was found that the film containing barium sulfate had stronger fluorescence intensity, and was easily detected by a detector.

### [Test Example 3]

Each of the films manufactured in Example 3 and Comparative Example 3 was partially exposed in the same manner as in Example 1, and the fluorescence spectrum of the film in the case of being irradiated with excitation light of 683 nm was measured. As a result, the intensity at 800 nm which is around the maximum fluorescence wavelength was 20, and this was about 430% stronger than the intensity around the maximum fluorescence wavelength of the film of Comparative Example 3. Therefore, it was found that the film containing barium sulfate had stronger fluorescence intensity, and was easily detected by a detector.

### [Test Example 4]

The films manufactured in Example 3 and Comparative Example 3 were irradiated with an LED ring illuminator having excitation light having a center wavelength of 740 nm, and observation was performed using a near-infrared imaging camera having detection sensitivity at 800 nm or longer. As a result, it was confirmed that the film in Example 3 strongly emitted compared to the film not containing barium sulfate manufactured in Comparative Example 3. As described above, it was found that the resin containing the radiopaque substance as represented by barium sulfate strongly emitted compared to the resin not containing the radiopaque substance, and thus, it was thought that a resin containing the radiopaque substance and the light-emitting substance is an industrially useful resin composition.

### [Test Example 5]

Each of the films manufactured in Example 4 and Comparative Example 4 was partially exposed in the same manner as in Example 1, and the fluorescence spectrum of the film in the case of being irradiated with excitation light of 730 nm was measured. As a result, the intensity at 755 nm, which is around the maximum fluorescence wavelength was 70, and this was about 40% stronger than the intensity around the maximum fluorescence wavelength of the film of Comparative Example 4. In addition, the intensity at 822 nm which is around the fluorescence peak wavelength on a longer wavelength side was 43, and this was about 150% stronger than the intensity around the maximum fluorescence wavelength of the film of Comparative Example 4. Therefore, it was found that the film containing barium sulfate had stronger fluorescence intensity, and was easily detected by a detector.

### [Test Example 6]

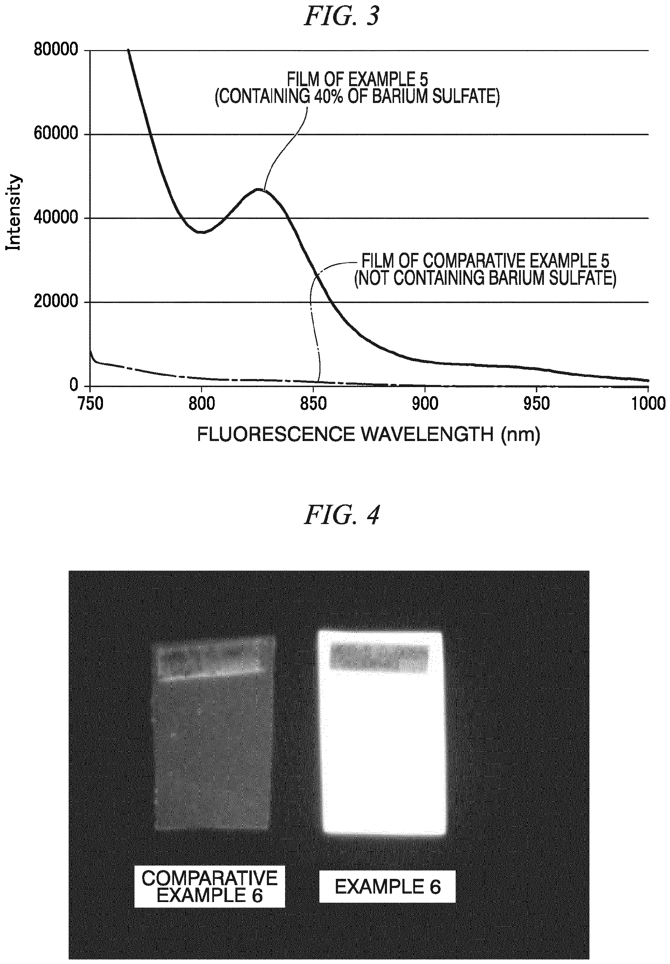

The spectra at an excitation wavelength of 740 nm of the films manufactured in Example 5 and Comparative Example 5 at were measured using a fluorescence spectrophotometer "FP-8600" manufactured by JASCO Corporation. The measurement results are shown in FIG. 3. As a result, the film of Example 5 had a fluorescence peak on a longer wavelength side, the intensity of the film at 827 nm which is around the fluorescence peak wavelength was 47000, and this was about 3200% stronger than the intensity around the maximum fluorescence wavelength of the film of Comparative Example 5.

### [Test Example 7]

When the films manufactured in Example 6 and Comparative Example 6 were photographed by using a near-infrared imaging camera in the same manner as in Test Example 4, the film of Example 6 emitted apparently stronger than the film of Comparative Example 6. The photographs thereof are shown in FIG. 4. From these results, it was found that the film containing barium sulfate had stronger fluorescence intensity, and was easily detected by a detector.

### [Test Example 8]

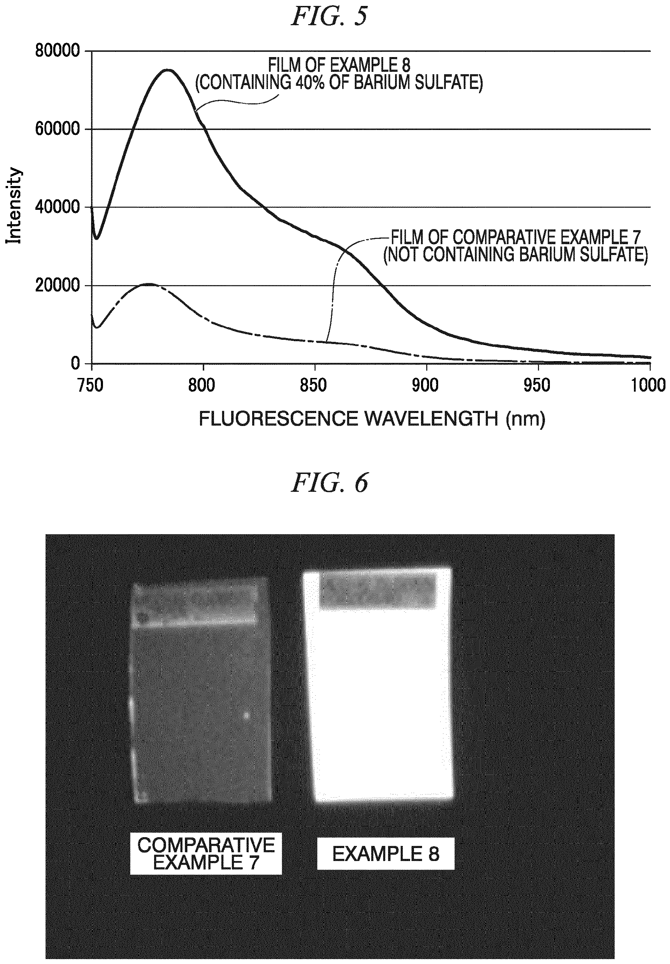

The spectra at an excitation wavelength of 740 nm of the films manufactured in Example 8 and Comparative Example 7 at were measured using a fluorescence spectrophotometer "FP-8600" manufactured by JASCO Corporation. The measurement results are shown in FIG. 5. As a result, the intensity of the film of Example 8 at 784 nm which is around the maximum fluorescence wavelength was 75,000, and this was 275% stronger than the intensity around the maximum fluorescence wavelength of the film of Comparative Example 7. In addition, the intensity of the film of Examples 8 at 864 nm which is around the fluorescence peak wavelength on a longer wavelength side was 31,000, and this was 500% stronger than the intensity around the maximum fluorescence wavelength of the film of Comparative Example 7.

In addition, when the films manufactured in Example 8 and Comparative Example 7 were photographed by using a near-infrared imaging camera in the same manner as in Test Example 4, the film of Example 8 emitted apparently stronger than the film of Comparative Example 7. The photographs thereof are shown in FIG. 6. From these results, it was found that the film containing barium sulfate had stronger fluorescence intensity, and was easily detected by a detector.

### [Test Example 9]

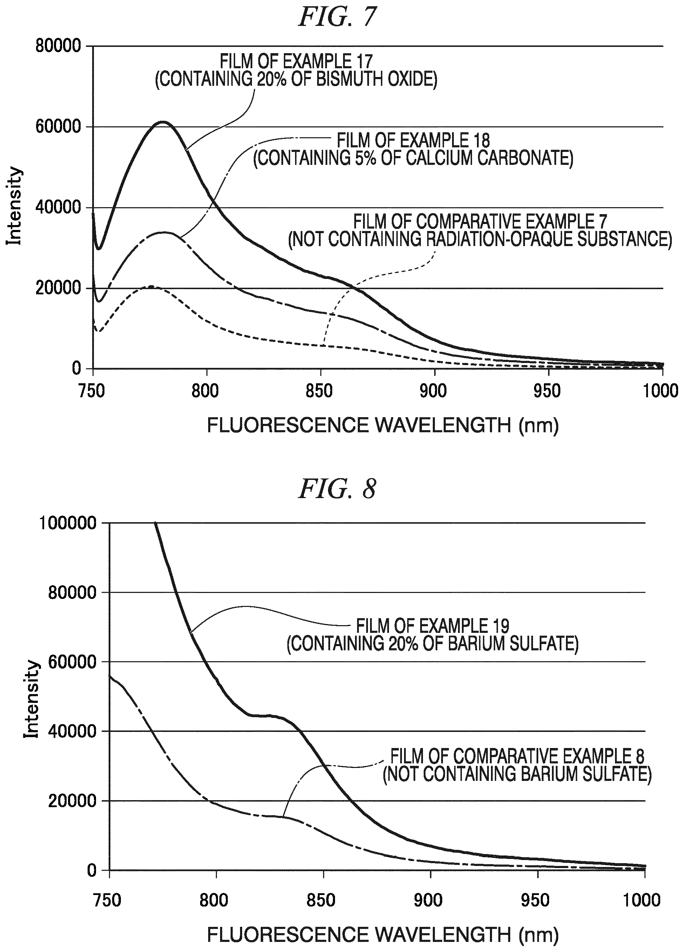

The spectra at an excitation wavelength of 740 nm of the films manufactured in Example 17, Example 18, and Comparative Example 7 at were measured using a fluorescence spectrophotometer "FP-8600" manufactured by JASCO Corporation. The measurement results are shown in FIG. 7. As a result, as the intensity around 780 nm which is the maximum fluorescence wavelength, the intensity in the film of Example 17 was 61,000, and the intensity in the film of Example 18 was 33,000, and these were respectively 200% and 65% stronger than the intensity around the maximum fluorescence wavelength of the film of Comparative Example 7. In addition, as the intensity around 860 nm which is the fluorescence peak on the longer wavelength side, the intensity in the film of Example 17 was 22,000, and the intensity in the film of Example 18 was 13,000, and these were respectively 320% and 150% stronger than the intensity around the maximum fluorescence wavelength of the film of Comparative Example 7. From the above results, it was confirmed that the film obtained from the resin composition according to the present invention had fluorescence intensity stronger than a film to which the radiopaque substance was not added and exhibited sensitizing effects in various radiopaque substances.

### [Test Example 10]

The spectra at an excitation wavelength of 740 nm of the films manufactured in Example 19 and Comparative Example 8 at were measured using a fluorescence spectrophotometer "FP-8600" manufactured by JASCO Corporation. The measurement results are shown in FIG. 8. As a result, the intensity of the film of Example 19 around 827 nm which is the fluorescence peak was 44,000, and this was 190% stronger than the intensity of the fluorescence peak of the film of Comparative Example 8. From the above results, sensitizing effects relating to an increase in fluorescence intensity due to the radiopaque substance were confirmed even in PP.

### [Test Example 11]

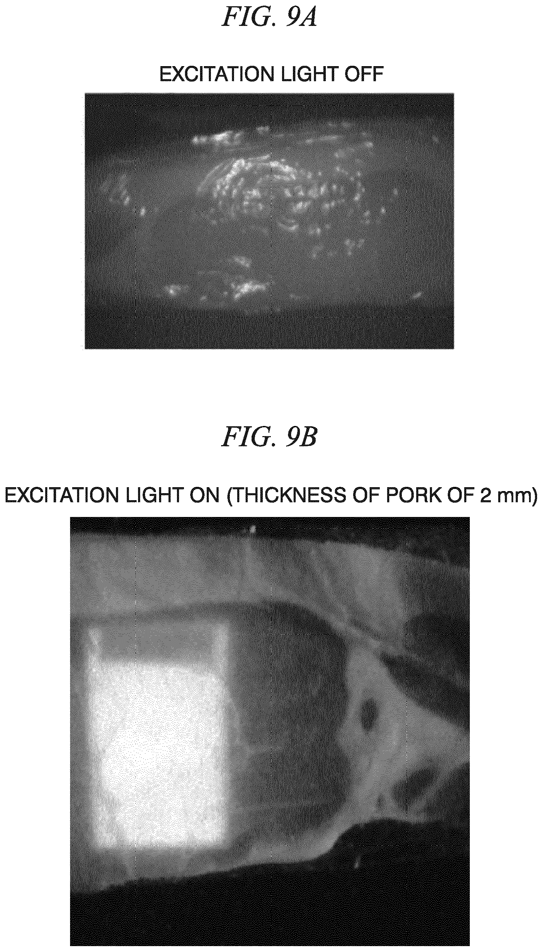

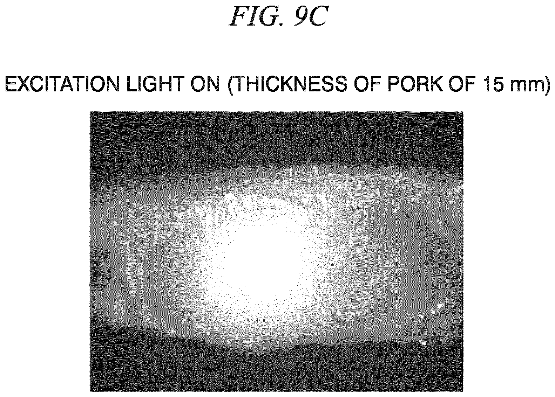

A piece of pork having a thickness of 2 mm or 15 mm was placed on the film manufactured in Example 8, and while being irradiated with an LED ring illuminator having excitation light having a center wavelength of 740 nm, photographs were taken using a near-infrared imaging camera having detection sensitivity at 800 nm or longer. In a case where a photograph was taken without irradiation with excitation light, the film under the piece of pork was not confirmed (FIG. 9A), but in a case where a photograph was taken with irradiation with excitation light, fluorescence could be clearly observed from the film over the piece of pork having a thickness of 2 mm (FIG. 9B), and fluorescence could be clearly observed from the film over the piece of pork having a thickness of 15 mm (FIG. 9C). From these results, it was found that the film can be visualized in the case of being inserted or indwelled in the body since the emission from the film passed through the piece of pork.

As described in these examples and test examples, since the resin composition according to the present invention and a molded article obtained from the composition have opaqueness to radiation and contain a light-emitting substance, both of detection by X-ray irradiation and detection by light-emission are possible. In addition, since the resin composition according to the present invention has sensitizing effects, that is, has stronger emission intensity to the amount of light-emitting substance added than that of a resin composition not containing the radiopaque substance, it is possible to more sensitively detect light emission even by weaker excitation light, and therefore, the resin composition according to the present invention is an industrially useful resin composition.

### Reference Signs List

1 ... film, 1a ... exposed surface, 2 ... aluminum foil of which the inside was blacked, 2a ... opening.

## Claims

1. A resin composition, comprising:
a light-emitting substance;
a radiopaque substance; and
a resin,
wherein the light-emitting substance is a near-infrared fluorescent material.

2. The resin composition according to Claim 1,
wherein the near-infrared fluorescent material is one or more compounds selected from the group consisting of compounds represented by the following General Formula (II₁), (II₂), (II₃), or (II₄) and has a maximum fluorescence wavelength of 650 nm or longer, 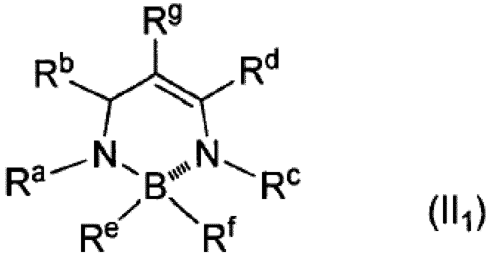 [In Formula (II₁),
R^{a} and R^{b} form an aromatic 5-membered ring, an aromatic 6-membered ring, or a condensed aromatic ring formed by condensation of two or three 5-membered rings or 6-membered rings together with the nitrogen atom to which R^{a} is bonded and the carbon atom to which R^{b} is bonded;
R^{c} and R^{d} form an aromatic 5-membered ring, an aromatic 6-membered ring, or a condensed aromatic ring formed by condensation of two or three 5-membered rings or 6-membered rings together with the nitrogen atom to which R^{c} is bonded and the carbon atom to which R^{d} is bonded;
each of R^{e} and R^{f} represents a halogen atom or an oxygen atom; and
R^{g} represents a hydrogen atom or an electron-withdrawing group.
Here, in a case where R^{e} and R^{f} are oxygen atoms, R^{e}, the boron atom bonded to R^{e}, R^{a}, and the nitrogen atom bonded to R^{a} may together form a ring, and R^{f}, the boron atom bonded to R^{f}, R^{c}, and the nitrogen atom bonded to R^{c} may together form a ring. In a case where R^{e} is an oxygen atom and does not form a ring, R^{e} is an oxygen atom having a substituent, and in a case where R^{f} is an oxygen atom and does not form a ring, R^{f} is an oxygen atom having a substituent.] 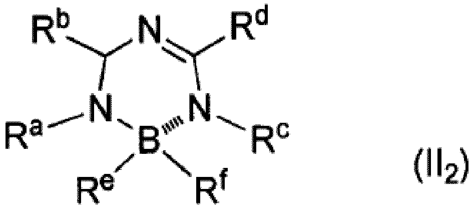 [In Formula (II₂), each of R^{a} to R^{f} is the same as that in Formula (II₁).] 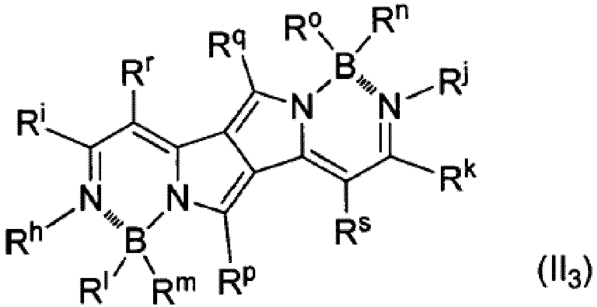 [In Formula (II₃),
R^{h} and Rⁱ form an aromatic 5-membered ring, an aromatic 6-membered ring, or a condensed aromatic ring formed by condensation of two or three 5-membered rings or 6-membered rings together with the nitrogen atom to which R^{h} is bonded and the carbon atom to which Rⁱ is bonded;
R^{j} and R^{k} form an aromatic 5-membered ring, an aromatic 6-membered ring, or a condensed aromatic ring formed by condensation of two or three 5-membered rings or 6-membered rings together with the nitrogen atom to which R^{j} is bonded and the carbon atom to which R^{k} is bonded;
each of R^{l}, R^{m}, Rⁿ, and R° independently represents a halogen atom, a C₁₋₂₀ alkyl group, a C₁₋₂₀ alkoxy group, an aryl group, or a heteroaryl group;
each of R^{p} and R^{q} independently represents a hydrogen atom, a halogen atom, a C₁₋₂₀ alkyl group, a C₁₋₂₀ alkoxy group, an aryl group, or a heteroaryl group; and
each of R^{r} and R^{s} independently represents a hydrogen atom or an electron-withdrawing group.] 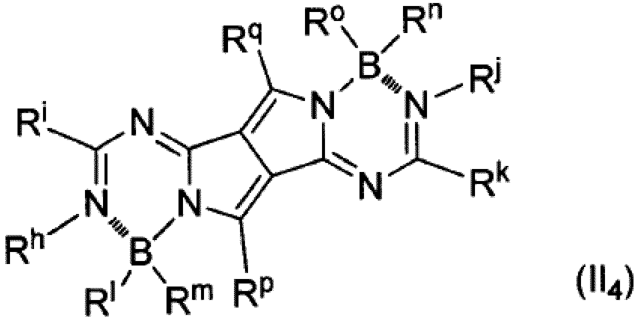 [In Formula (II₄), each of R^{h} to R^{q} is the same as that in Formula (II₃).].

3. The resin composition according to Claim 2, comprising:
one or more compounds selected from the group consisting of compounds represented by the following General Formula (II₁-0) or (II₂-0). 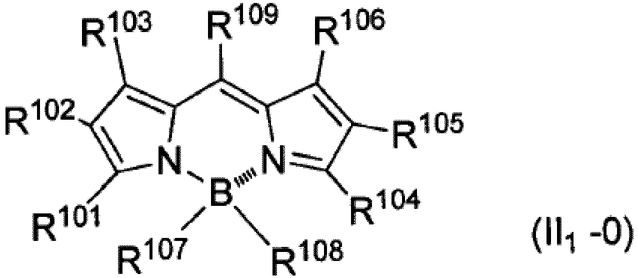 [In Formula (II₁-0),
(p1) each of R¹⁰¹, R¹⁰², and R¹⁰³ independently represents a hydrogen atom, a halogen atom, a C₁₋₂₀ alkyl group, a C₁₋₂₀ alkoxy group, an aryl group, or a heteroaryl group,
(p2) R¹⁰¹ and R¹⁰² together form an aromatic 5-membered ring or an aromatic 6-membered ring, and R¹⁰³ represents a hydrogen atom, a halogen atom, a C₁₋₂₀ alkyl group, a C₁₋₂₀ alkoxy group, an aryl group, or a heteroaryl group, or
(p3) R¹⁰² and R¹⁰³ together form an aromatic 5-membered ring or an aromatic 6-membered ring, and R¹⁰¹ represents a hydrogen atom, a halogen atom, a C₁₋₂₀ alkyl group, a C₁₋₂₀ alkoxy group, an aryl group, or a heteroaryl group, and
(q1) each of R¹⁰⁴, R¹⁰⁵, and R¹⁰⁶ independently represents a hydrogen atom, a halogen atom, a C₁₋₂₀ alkyl group, a C₁₋₂₀ alkoxy group, an aryl group, or a heteroaryl group,
(q2) R¹⁰⁴ and R¹⁰⁵ together form an aromatic 5-membered ring or an aromatic 6-membered ring, and R¹⁰⁶ represents a hydrogen atom, a halogen atom, a C₁₋₂₀ alkyl group, a C₁₋₂₀ alkoxy group, an aryl group, or a heteroaryl group, or
(q3) R¹⁰⁵ and R¹⁰⁶ together form an aromatic 5-membered ring or an aromatic 6-membered ring, and R¹⁰⁴ represents a hydrogen atom, a halogen atom, a C₁₋₂₀ alkyl group, a C₁₋₂₀ alkoxy group, an aryl group, or a heteroaryl group.
Each of R¹⁰⁷ and R¹⁰⁸ represents a halogen atom or an oxygen atom; and
R¹⁰⁹ represents a hydrogen atom or an electron-withdrawing group.
Here, in a case where R¹⁰⁷ and R¹⁰⁸ are oxygen atoms, R¹⁰⁷, the boron atom bonded to R¹⁰⁷, the nitrogen atom bonded to the boron atom, R¹⁰¹, and the carbon atom bonded to R¹⁰¹may together form a ring, and R¹⁰⁸, the boron atom bonded to R¹⁰⁸, the nitrogen atom bonded to the boron atom, R¹⁰⁴, and the carbon atom bonded to R¹⁰⁴ may together form a ring. In a case where R¹⁰⁷ is an oxygen atom and does not form a ring, R¹⁰⁷ is an oxygen atom having a substituent, and in a case where R¹⁰⁸ is an oxygen atom and does not form a ring, R¹⁰⁸ is an oxygen atom having a substituent.], 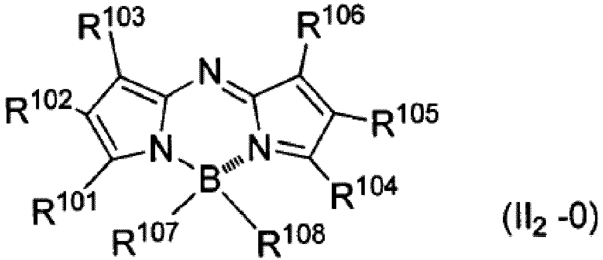 [In Formula (II₂-0), each of R¹⁰¹ to R¹⁰⁸ is the same as that in Formula (II₁-0).].

4. The resin composition according to Claim 3,
wherein, in General Formula (II₁-0) or (II₂-0), R¹⁰¹ and R¹⁰² form a ring, and R¹⁰⁴ and R¹⁰⁵ form a ring, or R¹⁰² and R¹⁰³ form a ring, and R¹⁰⁵ and R¹⁰⁶ form a ring,
and the ring is represented by any one of the following General Formulas (C1 to (C-9), 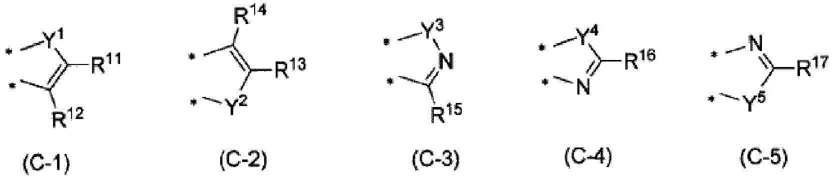 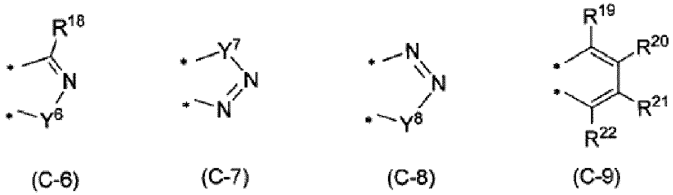 [In Formulas (C-1) to (C-9), each of Y¹ to Y⁸ independently represents a sulfur atom, an oxygen atom, a nitrogen atom, or a phosphorus atom, and each of R¹¹ to R²² independently represents a hydrogen atom or any group which does not inhibit fluorescence of the compound.].

5. The resin composition according to Claim 2, comprising:
one or more compounds selected from the group consisting of compounds represented by any one of the following General Formulas (II₁-1-1) to (II₁-1-6), (II₁-2-1) to (II₁-2-12), (II₂-1-1) to (II₂-1-6), and (II₂-2-1) to (II₂-2-12), 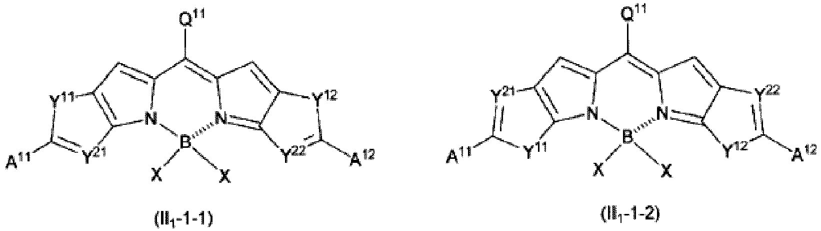 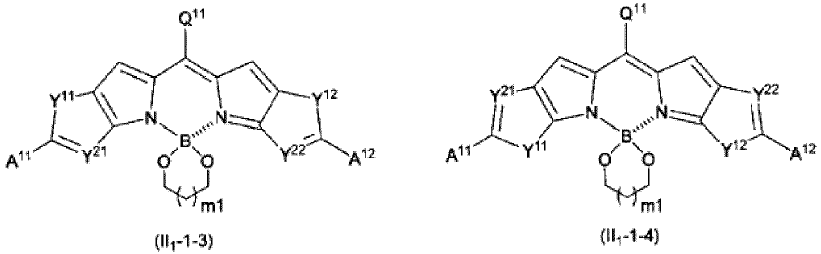 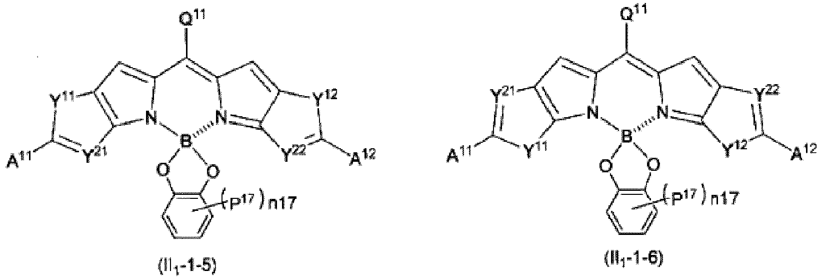 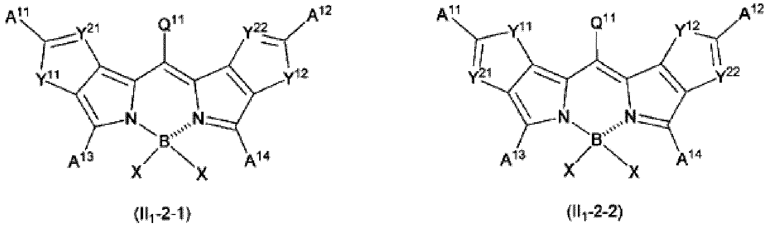 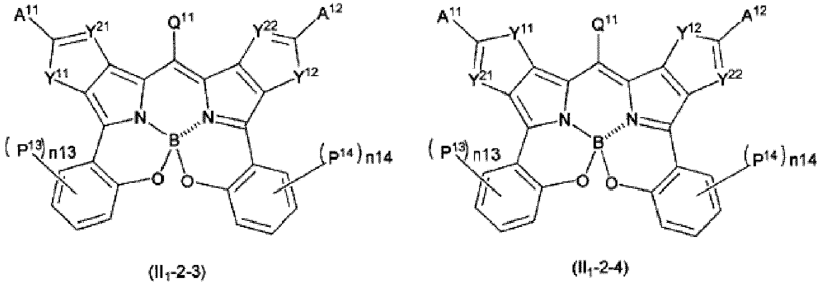 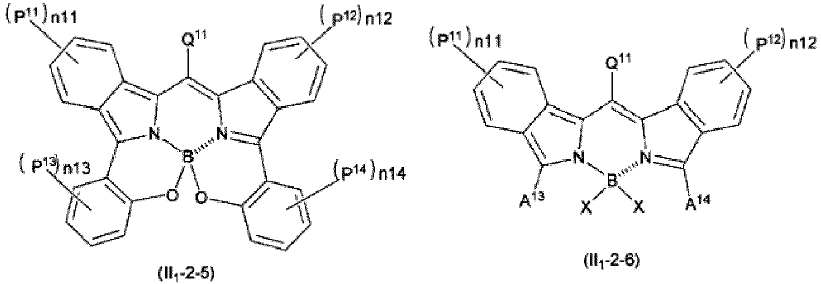 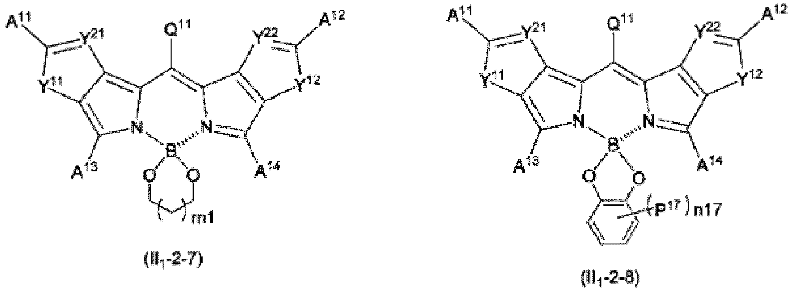 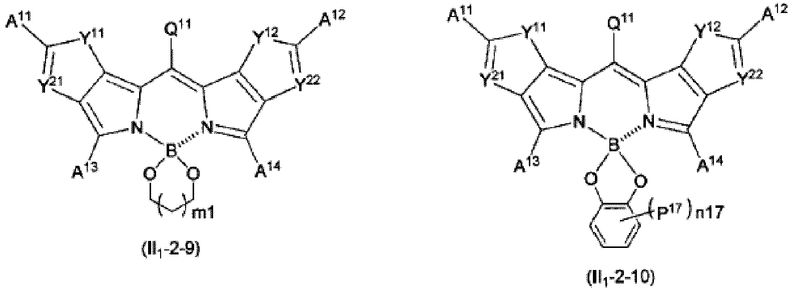 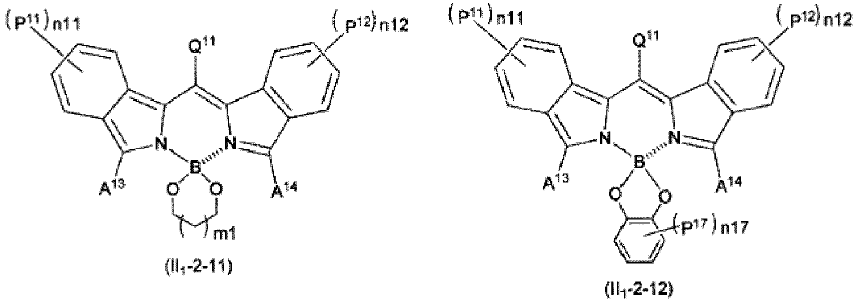 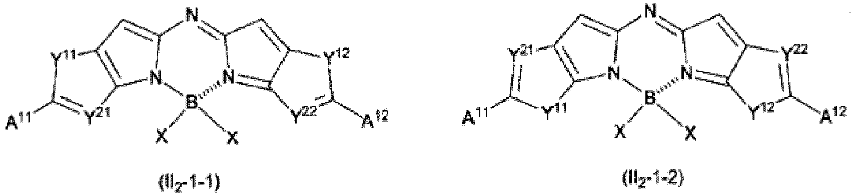 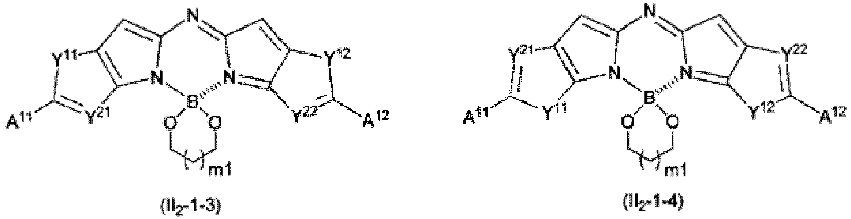 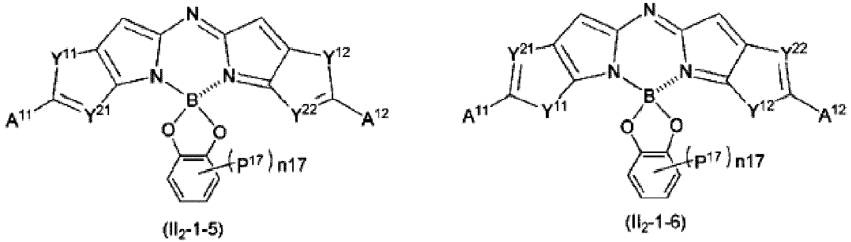 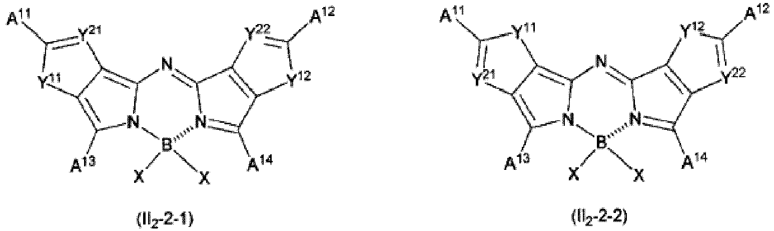 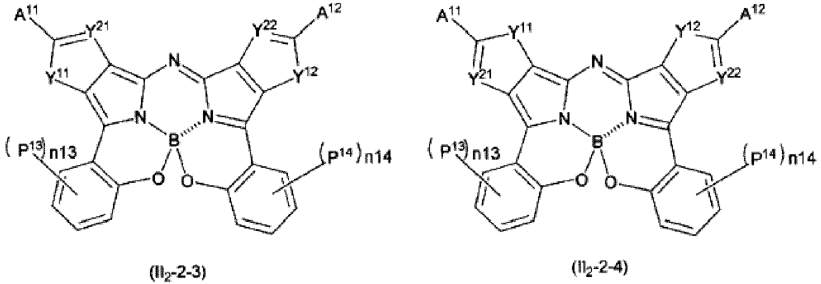 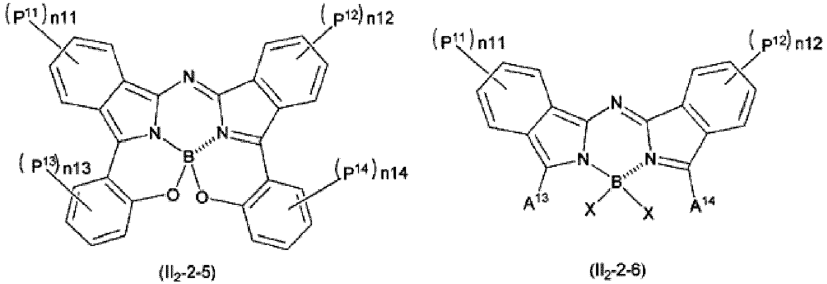 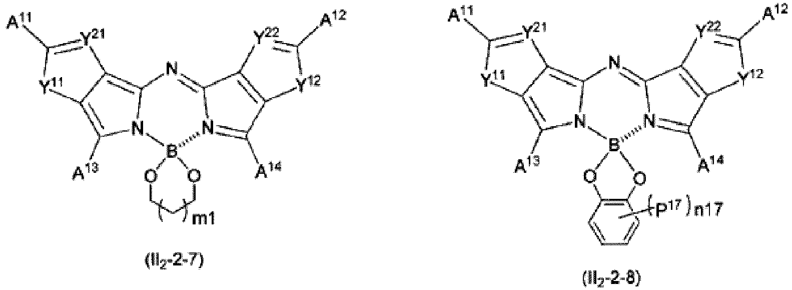 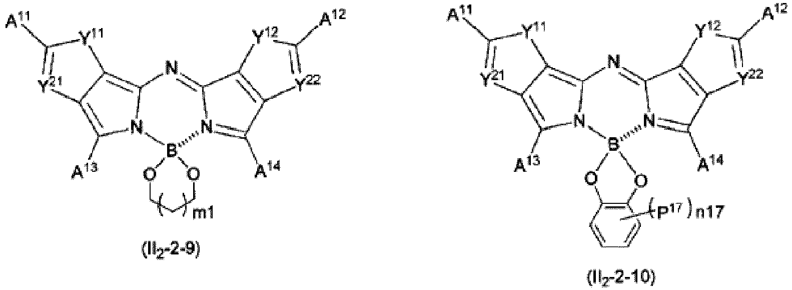 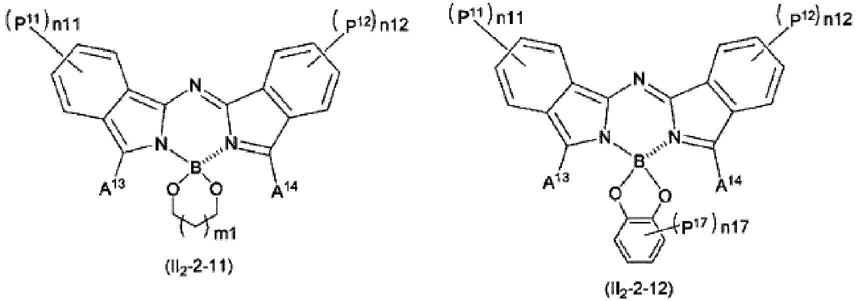
[In the formula, each of Y¹¹ and Y¹² independently represents an oxygen atom or a sulfur atom;
each of Y²¹ and Y²² independently represents a carbon atom or a nitrogen atom;
Q represents a trifluoromethyl group, a cyano group, a nitro group, or a phenyl group;
each of Xs independently represents a halogen atom, a C₁₋₂₀ alkoxy group, an aryloxy group, or an acyloxy group;
each of P¹¹ to P¹⁴ and P¹⁷ independently represents a halogen atom, a C₁₋₂₀ alkyl group, a C₁₋₂₀ alkoxy group, an amino group, a monoalkylamino group, or a dialkylamino group;
each of A¹¹ to A¹⁴ independently represents a phenyl group which may have one to three substituents selected from the group consisting of a halogen atom, a C₁₋₂₀ alkyl group, a C₁₋₂₀ alkoxy group, an amino group, a monoalkylamino group, and a dialkylamino group, or a heteroaryl group which may have one to three substituents selected from the group consisting of a halogen atom, a C₁₋₂₀ alkyl group, a C₁₋₂₀ alkoxy group, an amino group, a monoalkylamino group, and a dialkylamino group;
each of n11 to n14 and n17 independently represents an integer of 0 to 3; and
m1 represents 0 or 1.].

6. The resin composition according to Claim 2, comprising:
one or more compounds selected from the group consisting of compounds represented by any one of the following General Formulas (II₃-7) to (II₃-9) and (II₄-7) to (II₄-9), 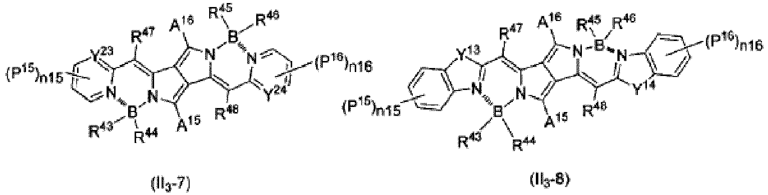 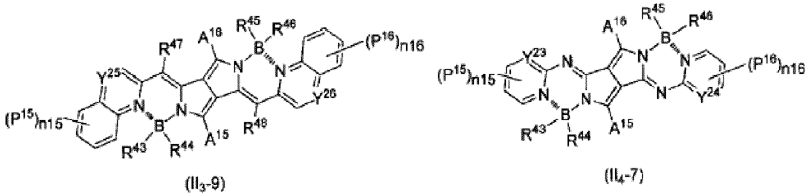 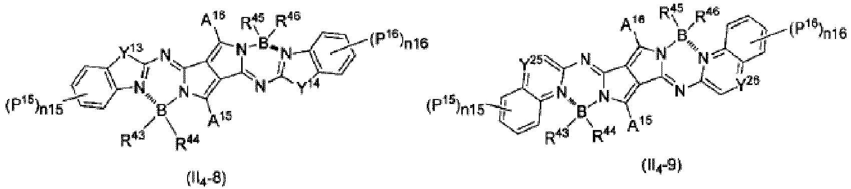
[In the formulas, each of Y²³ and Y²⁴ independently represents a carbon atom or a nitrogen atom;
each of Y¹³ and Y¹⁴ independently represents an oxygen atom or a sulfur atom;
each of Y²⁵ and Y²⁶ independently represents a carbon atom or a nitrogen atom;
each of R⁴⁷ and R⁴⁸ independently represents a hydrogen atom or an electron-withdrawing group;
each of R⁴³, R⁴⁴, R⁴⁵, and R⁴⁶ represents a halogen atom or an aryl group which may have a substituent;
each of P¹⁵ and P¹⁶ independently represents a halogen atom, a C₁₋₂₀ alkyl group, a C₁₋₂₀ alkoxy group, an amino group, a monoalkylamino group, or a dialkylamino group;
each of n15 and n16 independently represents an integer of 0 to 3; and
each of A¹⁵ and A¹⁶ independently represents a phenyl group which may have one to three substituents selected from the group consisting of a hydrogen atom, a halogen atom, a C₁₋₂₀ alkyl group, a C₁₋₂₀ alkoxy group, an amino group, a monoalkylamino group, or a dialkylamino group.].

7. The resin composition according to Claim 2, comprising:
one or more compounds selected from the group consisting of compounds represented by any one of the following General Formulas (II₃-1) to (II₃-6) and (II₄-1) to (II₄-6), 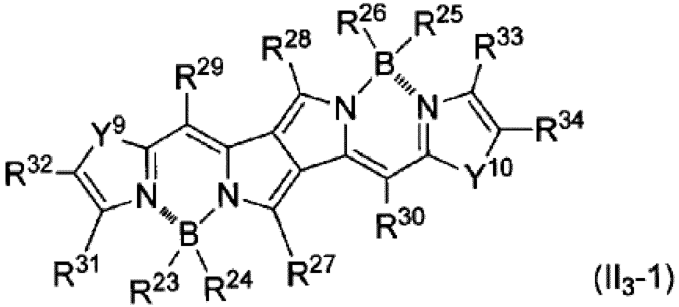 [In Formula (II₃-1),
each of R²³, R²⁴, R²⁵, and R²⁶ independently represents a halogen atom, a C₁₋₂₀ alkyl group, a C₁₋₂₀ alkoxy group, an aryl group, or a heteroaryl group;
each of R²⁷ and R²⁸ independently represents a hydrogen atom, a halogen atom, a C₁₋₂₀ alkyl group, a C₁₋₂₀ alkoxy group, an aryl group, or a heteroaryl group;
each of R²⁹ and R³⁰ independently represents a hydrogen atom or an electron-withdrawing group;
each of Y⁹ and Y¹⁰ independently represents a sulfur atom, an oxygen atom, a nitrogen atom, or a phosphorus atom; and
(p4) each of R³¹ and R³² independently represents a hydrogen atom, a halogen atom, a C₁₋₂₀ alkyl group, a C₁₋₂₀ alkoxy group, an aryl group, or a heteroaryl group, or
(p5) R³¹ and R³² together form an aromatic 5-membered ring which may have a substituent or an aromatic 6-membered ring which may have a substituent;
(q4) each of R³³ and R³⁴ independently represents a hydrogen atom, a halogen atom, a C₁₋₂₀ alkyl group, a C₁₋₂₀ alkoxy group, an aryl group, or a heteroaryl group, or
(q5) R³³ and R³⁴ together form an aromatic 5-membered ring which may have a substituent or an aromatic 6-membered ring which may have a substituent.] 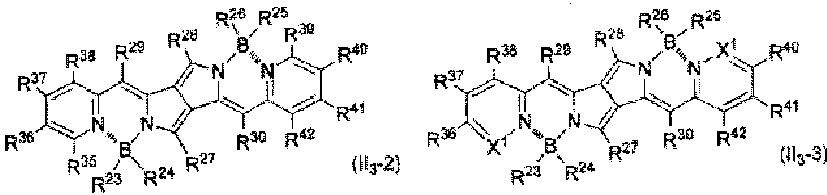 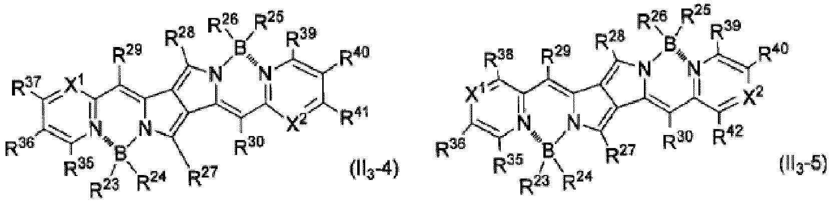 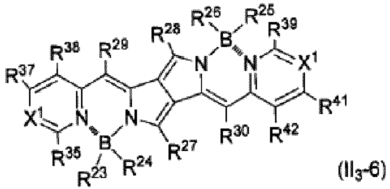
[In Formulas (II₃₋₂) to (II₃-6), each of R²³ to R³⁰ is the same as that in Formula (II₃-1);
each of X¹ and X² independently represents a nitrogen atom or a phosphorus atom;
(p6) each of R³⁵, R³⁶, R³⁷, and R³⁸ independently represents a hydrogen atom, a halogen atom, a C₁₋₂₀ alkyl group, a C₁₋₂₀ alkoxy group, an aryl group, or a heteroaryl group,
(p7) R³⁵ and R³⁶ together form an aromatic 5-membered ring which may have a substituent or an aromatic 6-membered ring which may have a substituent, and each of R³⁷ and R³⁸ independently represents a hydrogen atom, a halogen atom, a C₁₋₂₀ alkyl group, a C₁₋₂₀ alkoxy group, an aryl group, or a heteroaryl group,
(p8) R³⁶ and R³⁷ together form an aromatic 5-membered ring which may have a substituent or an aromatic 6-membered ring which may have a substituent, and each of R³⁵ and R³⁸ independently represents a hydrogen atom, a halogen atom, a C₁₋₂₀ alkyl group, a C₁₋₂₀ alkoxy group, an aryl group, or a heteroaryl group, or
(p9) R³⁷ and R³⁸ together form an aromatic 5-membered ring which may have a substituent or an aromatic 6-membered ring which may have a substituent, and each of R³⁵ and R³⁶ independently represents a hydrogen atom, a halogen atom, a C₁₋₂₀ alkyl group, a C₁₋₂₀ alkoxy group, an aryl group, or a heteroaryl group; and
(q6) each of R³⁹, R⁴⁰, R⁴¹, and R⁴² independently represents a hydrogen atom, a halogen atom, a C₁₋₂₀ alkyl group, a C₁₋₂₀ alkoxy group, an aryl group, or a heteroaryl group,
(q₇) R³⁹ and R⁴⁰ together form an aromatic 5-membered ring which may have a substituent or an aromatic 6-membered ring which may have a substituent, and each of R⁴¹ and R⁴² independently represents a hydrogen atom, a halogen atom, a C₁₋₂₀ alkyl group, a C₁₋₂₀ alkoxy group, an aryl group, or a heteroaryl group,
(q8) R⁴⁰ and R⁴¹ together form an aromatic 5-membered ring which may have a substituent or an aromatic 6-membered ring which may have a substituent, and each of R³⁹ and R⁴² independently represents a hydrogen atom, a halogen atom, a C₁₋₂₀ alkyl group, a C₁₋₂₀ alkoxy group, an aryl group, or a heteroaryl group, or
(q9) R⁴¹ and R⁴² together form an aromatic 5-membered ring which may have a substituent or an aromatic 6-membered ring which may have a substituent, and each of R³⁹ and R⁴⁰ independently represents a hydrogen atom, a halogen atom, a C₁₋₂₀ alkyl group, a C₁₋₂₀ alkoxy group, an aryl group, or a heteroaryl group.] 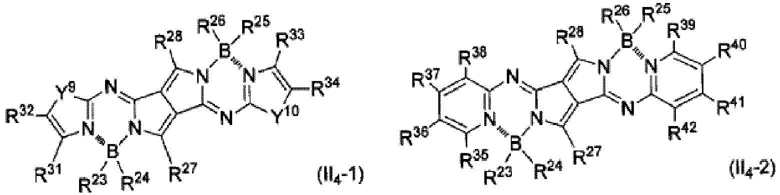 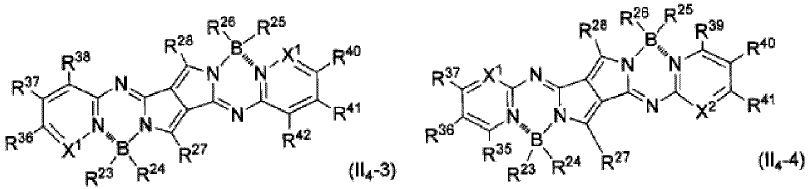 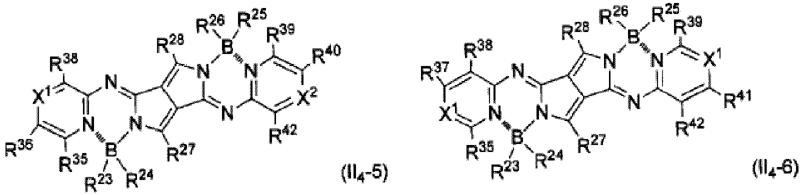 ⁿ Formulas (II₄-1) to (II₄-6), each of R²³ to R²⁸ is the same as that in Formula (II₃-I), and in Formula (II₄-1), each of R³¹ to R³⁴, Y⁹, and Y¹⁰ is the same as that in Formula (II₃-1), in Formulas II₄-2) to (II₄-6), each of R³⁵ R⁴² is the same as that in Formula (II₃₋2, and in Formulas (II₄-3) to (II₄-6), each of X¹ and X² is the same as that in Formula (II₃-3).].

8. The resin composition according to Claim 1,
wherein the infrared fluorescent material is formed of an azo-boron complex compound represented by the following Formula (I) and has a maximum absorption wavelength of 650 nm or longer and a Stokes shift of 50 nm or longer, 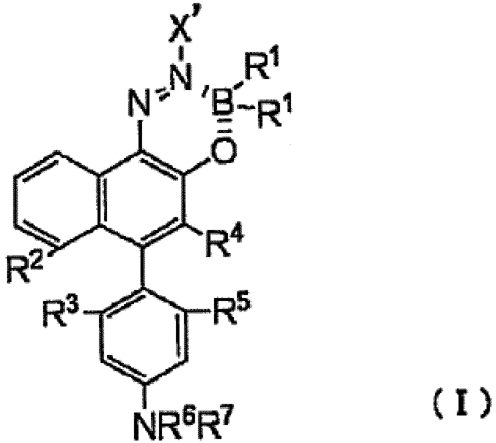 [In Formula (I),
X' represents an aryl group which may have a substituent or a heteroaryl group which may have a substituent;
R¹ represents a C₁₋₁₂ alkyl group, an aryl group, an aryl ethenyl group, an aryl ethynyl group, a C₁₋₁₂ alkoxy group, an aryloxy group, or a halogen atom, or one of R¹s represents an -O-C(=O)- group which is also bonded to X', and forms a 6-membered ring, and the other R¹ independently represents a C₁₋₁₂ alkyl group, an aryl group, an aryl ethenyl group, an aryl ethynyl group, a C₁₋₁₂ alkoxy group, an aryloxy group, or a halogen atom;
R² and R³ together form an -O- group, an -S- group, or an -N(R⁸)-group (here, R⁸ represents a hydrogen atom or a C₁₋₁₂ alkyl group), and each of R⁴ and R⁵ represents a hydrogen atom, or R⁴ and R⁵ together form an -O- group, an -S- group, or an -N(R⁸)- group (R⁸ has the same meaning as that described above), and each of R² and R³ represents a hydrogen atom;
each of R⁶ and R⁷ independently represents a hydrogen atom, a C₁₋₁₂ alkyl group, an aryl group which may have a substituent, or a heteroaryl group which may have a substituent; and
the substituent of the aryl group or the heteroaryl group represents one or more groups selected from the group consisting of a C₁₋₁₂ alkyl group, a mono (C₁₋₁₂ alkyl)amino group, a di (C₁₋₁₂ alkyl)amino group, a hydroxyl group, and a C₁₋₁₂ alkoxy group.].

9. The resin composition according to Claim 8,
wherein the azo-boron complex compound is represented by the following Formula (I₁), 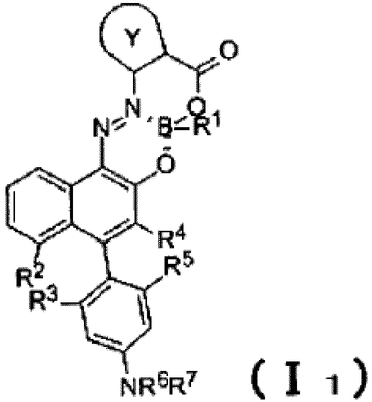 [In Formula (I₁), Y represents an aryl group which may have a substituent or a heteroaryl group which may have a substituent, and each of R¹ to R⁷ has the same meaning as each of R¹ to R⁷ in Formula (I).].

10. The resin composition according any one of Claims 1 to 9,
wherein the radiopaque substance is one or more selected from the group consisting of barium sulfate, bismuth oxide, bismuth subcarbonate, calcium carbonate, aluminum hydroxide, tungsten, zinc oxide, zirconium oxide, zirconium, titanium, platinum, bismuth subnitrate, and bismuth.

11. The resin composition according to any one of Claims 1 to 10,
wherein the content of the radiopaque substance is 5% by mass to 50% by mass.

12. The resin composition according to any one of Claims 1 to 11,
wherein the content of the light-emitting substance is 0.001% by mass to 0.5% by mass.

13. The resin composition according to any one of Claims 1 to 11,
wherein the content of the light-emitting substance is 0.001% by mass to 0.05% by mass.

14. The resin composition according to any one of Claims 1 to 13,
wherein the resin is a thermoplastic resin.

15. The resin composition according to any one of Claims 1 to 14,
wherein the resin is one or more selected from the group consisting of a urethane-based resin, an olefin-based resin, a polystyrene-based resin, a polyester-based resin, and a vinyl chloride-based resin.

16. The resin composition according to any one of Claims 1 to 15, which is used as a medical material.

17. A molded article obtained by processing the resin composition according to any one of Claims 1 to 16.

18. The molded article according to Claim 17,
wherein the article is a medical tool and of which at least a part is used in the body of a patient.

## Patentansprüche

1. Harzzusammensetzung, die umfasst:
eine lichtemittierende Substanz;
eine röntgenopake Substanz; und
ein Harz,
wobei die lichtemittierende Substanz ein im Nahinfrarot fluoreszierendes Material ist.

2. Harzzusammensetzung nach Anspruch 1,
wobei das im Nahinfrarot fluoreszierende Material eine oder mehrere Verbindungen ist, die aus der Gruppe ausgewählt sind, die aus Verbindungen besteht, die durch die folgende allgemeine Formel (II₁), (II₂), (II₃) oder (II₄) dargestellt sind, und eine maximale Fluoreszenzwellenlänge von 650 nm oder länger aufweist, 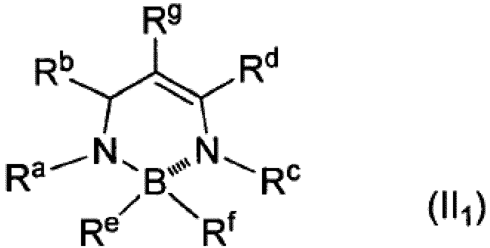 [In Formel (II₁),
bilden R^{a} und R^{b} einen aromatischen 5-gliedrigen Ring, einen aromatischen 6-gliedrigen Ring oder einen kondensierten aromatischen Ring, der durch Kondensation von zwei oder drei 5- oder 6-gliedrigen Ringen zusammen mit dem Stickstoffatom, an das R^{a} gebunden ist, und dem Kohlenstoffatom, an das R^{b} gebunden ist, gebildet wird;
bilden R^{c} und R^{d} einen aromatischen 5-gliedrigen Ring, einen aromatischen-6-gliedrigen Ring oder einen kondensierten aromatischen Ring, der durch Kondensation von zwei oder drei 5- oder 6-gliedrigen Ringen zusammen mit dem Stickstoffatom, an das R^{c} gebunden ist, und dem Kohlenstoffatom, an das R^{d} gebunden ist, gebildet wird;
stellt jedes von R^{e} und R^{f} ein Halogenatom oder ein Sauerstoffatom dar; und
stellt R^{g} ein Wasserstoffatom oder eine elektronenziehende Gruppe dar.
Hier können in einem Fall, in dem R^{e} und R^{f} Sauerstoffatome sind, R^{e}, das an R^{e} gebundene Boratom, R^{a} und das an R^{a} gebundene Stickstoffatom zusammen einen Ring bilden, und R^{f}, das an R^{f} gebundene Boratom, R^{c} und das an R^{c} gebundene Stickstoffatom können zusammen einen Ring bilden. In einem Fall, in dem R^{e} ein Sauerstoffatom ist und keinen Ring bildet, ist R^{e} ein Sauerstoffatom mit einem Substituenten, und in einem Fall, in dem R^{f} ein Sauerstoffatom ist und keinen Ring bildet, ist R^{f} ein Sauerstoffatom mit einem Substituenten]. 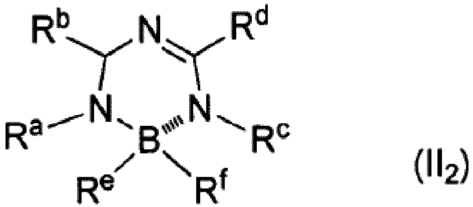 [In der Formel (II₂) ist jedes von R^{a} bis R^{f} dasselbe wie in Formel (II₁)]. 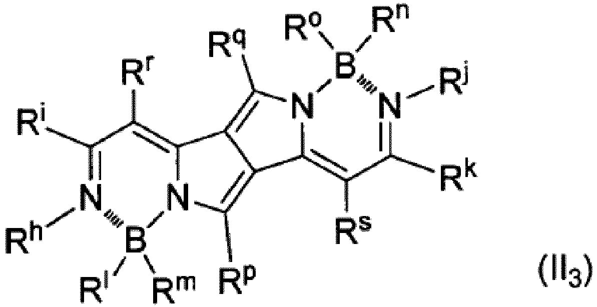 [In Formel (II₃),
bilden R^{h} und Rⁱ einen aromatischen 5-gliedrigen Ring, einen aromatischen 6-gliedrigen Ring oder einen kondensierten aromatischen Ring, der durch Kondensation von zwei oder drei 5- oder 6-gliedrigen Ringen zusammen mit dem Stickstoffatom, an das R^{h} gebunden ist, und dem Kohlenstoffatom, an das Rⁱ gebunden ist, gebildet wird;
bilden R^{j} und R^{k} einen aromatischen 5-gliedrigen Ring, einen aromatischen 6-gliedrigen Ring oder einen kondensierten aromatischen Ring, der durch Kondensation von zwei oder drei 5- oder 6-gliedrigen Ringen zusammen mit dem Stickstoffatom, an das R^{j} gebunden ist, und dem Kohlenstoffatom, an das R^{k} gebunden ist, gebildet wird;
stellen R^{l}, R^{m}, Rⁿ und R^{o} jeweils unabhängig ein Halogenatom, eine C₁₋₂₀-Alkylgruppe, eine C₁₋₂₀-Alkoxygruppe, eine Arylgruppe oder eine Heteroarylgruppe dar;
stellen R^{p} und R^{q} jeweils unabhängig ein Wasserstoffatom, ein Halogenatom, eine C₁₋₂₀-Alkylgruppe, eine C₁₋₂₀-Alkoxygruppe, eine Arylgruppe oder eine Heteroarylgruppe dar; und
stellt jedes von R^{r} und R^{s} unabhängig für ein Wasserstoffatom oder eine elektronenziehende Gruppe dar]. 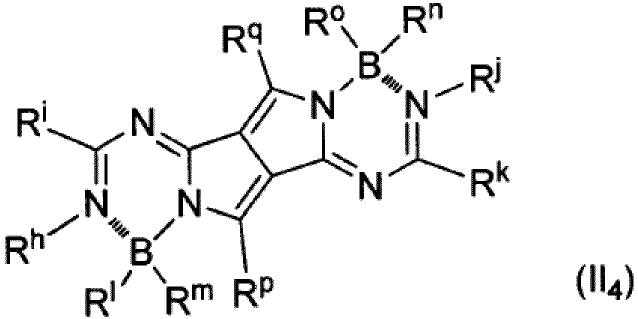 [In der Formel (II₄) ist jedes von R^{h} bis R^{q} dasselbe wie in Formel (II₃)].

3. Harzzusammensetzung nach Anspruch 2, die umfasst:
eine oder mehrere Verbindungen, ausgewählt aus der Gruppe bestehend aus Verbindungen, die durch die folgende allgemeine Formel (II₁-0) oder (II₂-0) dargestellt sind. 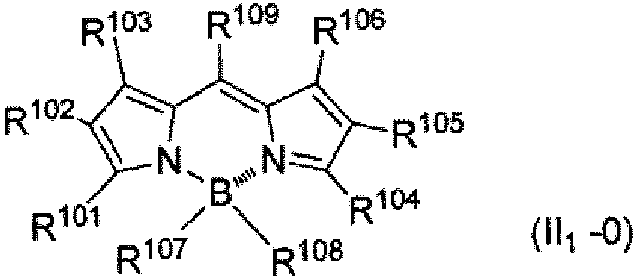 In Formel (II₁-0),
(p1) stellen R¹⁰¹, R¹⁰² und R¹⁰³ jeweils unabhängig ein Wasserstoffatom, ein Halogenatom, eine C₁₋₂₀-Alkylgruppe, eine C₁₋₂₀-Alkoxygruppe, eine Arylgruppe oder eine Heteroarylgruppe dar,
(p2) bilden R¹⁰¹ und R¹⁰² zusammen einen aromatischen 5-gliedrigen Ring oder einen aromatischen 6-gliedrigen Ring, und stellt R¹⁰³ ein Wasserstoffatom, ein Halogenatom, eine C₁₋₂₀-Alkylgruppe, eine C₁₋₂₀-Alkoxygruppe, eine Arylgruppe oder eine Heteroarylgruppe dar, oder
(p3) bilden R¹⁰² und R¹⁰³ zusammen einen aromatischen 5-gliedrigen Ring oder einen aromatischen 6-gliedrigen Ring, und stellt R¹⁰¹ ein Wasserstoffatom, ein Halogenatom, eine C₁₋₂₀-Alkylgruppe, eine C₁₋₂₀-Alkoxygruppe, eine Arylgruppe oder eine Heteroarylgruppe dar, und
(q1) stellen jedes von R¹⁰⁴, R¹⁰⁵ und R¹⁰⁶ unabhängig ein Wasserstoffatom, ein Halogenatom, eine C₁₋₂₀-Alkylgruppe, eine C₁₋₂₀-Alkoxygruppe, eine Arylgruppe oder eine Heteroarylgruppe dar,
(q2) bilden R¹⁰⁴ und R¹⁰⁵ zusammen einen aromatischen 5-gliedrigen Ring oder einen aromatischen 6-gliedrigen Ring, und stellt R¹⁰⁶ ein Wasserstoffatom, ein Halogenatom, eine C₁₋₂₀-Alkylgruppe, eine C₁₋₂₀-Alkoxygruppe, eine Arylgruppe oder eine Heteroarylgruppe dar, oder
(q3) bilden R¹⁰⁵ und R¹⁰⁶ zusammen einen aromatischen 5-gliedrigen Ring oder einen aromatischen 6-gliedrigen Ring, und stellt R¹⁰⁴ ein Wasserstoffatom, ein Halogenatom, eine C₁₋₂₀-Alkylgruppe, eine C₁₋₂₀-Alkoxygruppe, eine Arylgruppe oder eine Heteroarylgruppe dar.
Jedes von R¹⁰⁷ und R¹⁰⁸ stellen ein Halogenatom oder ein Sauerstoffatom dar; und
R¹⁰⁹ stellt ein Wasserstoffatom oder eine elektronenziehende Gruppe dar.
Hier können in einem Fall, in dem R¹⁰⁷ und R¹⁰⁸ Sauerstoffatome sind, R¹⁰⁷, das an R¹⁰⁷ gebundene Boratom, das an das Boratom gebundene Stickstoffatom, R¹⁰¹ und das an R¹⁰¹ gebundene Kohlenstoffatom zusammen einen Ring bilden, und R¹⁰⁸, das an R¹⁰⁸ gebundene Boratom, das an das Boratom gebundene Stickstoffatom, R¹⁰⁴ und das an R¹⁰⁴ gebundene Kohlenstoffatom können zusammen einen Ring bilden. In einem Fall, in dem R¹⁰⁷ ein Sauerstoffatom ist und keinen Ring bildet, ist R¹⁰⁷ ein Sauerstoffatom mit einem Substituenten, und in einem Fall, in dem R¹⁰⁸ ein Sauerstoffatom ist und keinen Ring bildet, ist R¹⁰⁸ ein Sauerstoffatom mit einem Substituenten], 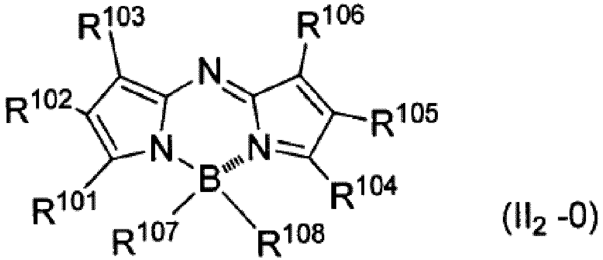 [In der Formel (II₂-0) ist jedes von R¹⁰¹ bis R¹⁰⁸ dasselbe wie in Formel (II₁-0).].

4. Harzzusammensetzung nach Anspruch 3,
wobei in der allgemeinen Formel (II₁-0) oder (II₂-0) R¹⁰¹ und R¹⁰² einen Ring bilden und R¹⁰⁴ und R¹⁰⁵ einen Ring bilden oder R¹⁰² und R¹⁰³ einen Ring bilden und R¹⁰⁵ und R¹⁰⁶ einen Ring bilden,
und der Ring wird durch eine der folgenden allgemeinen Formeln (C-1) bis (C-9)) dargestellt, 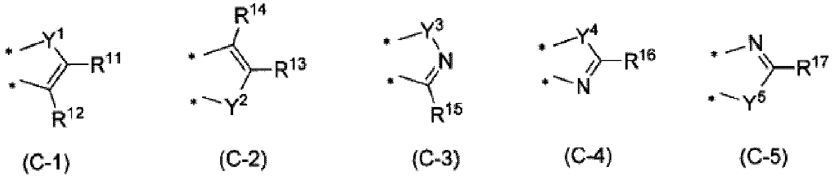 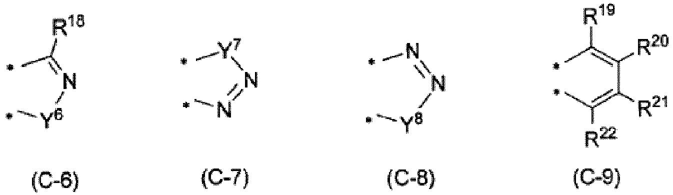 [In den Formeln (C-1) bis (C-9) stellt jedes Y¹ bis Y⁸ unabhängig ein Schwefelatom, ein Sauerstoffatom, ein Stickstoffatom oder ein Phosphoratom dar, und jedes von R¹¹ bis R²² stellt unabhängig ein Wasserstoffatom oder eine Gruppe dar, die die Fluoreszenz der Verbindung nicht hemmt.].

5. Harzzusammensetzung nach Anspruch 2, die umfasst:
eine oder mehrere Verbindungen, ausgewählt aus der Gruppe bestehend aus Verbindungen, die durch eine der folgenden allgemeinen Formeln (II₁-1-1) bis (II₁-1-6), (II₁-2-1) bis (II₁-2-12), (II₂-1-1) bis (II₂-1-6) und (II₂-2-1) bis (II₂-2-12) dargestellt sind, 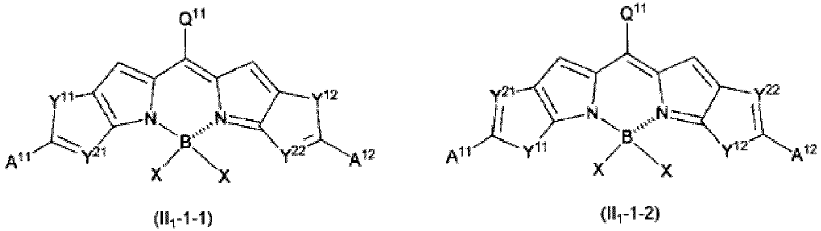 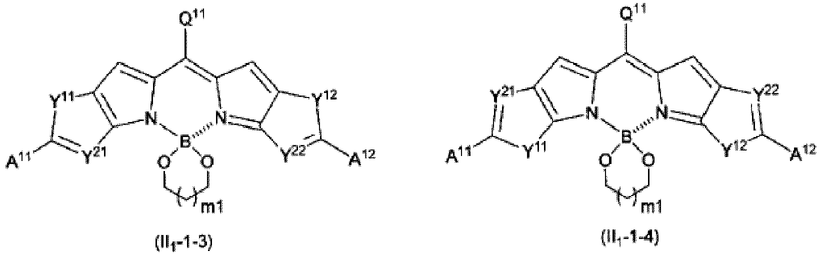 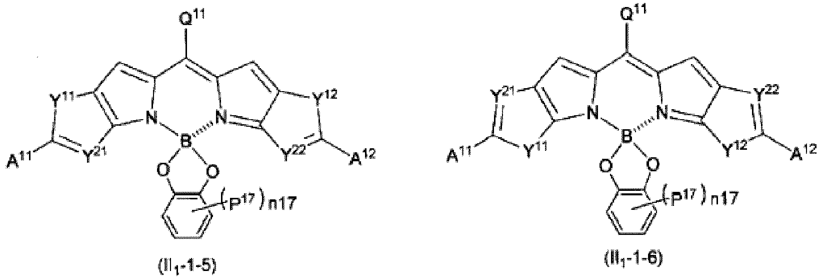 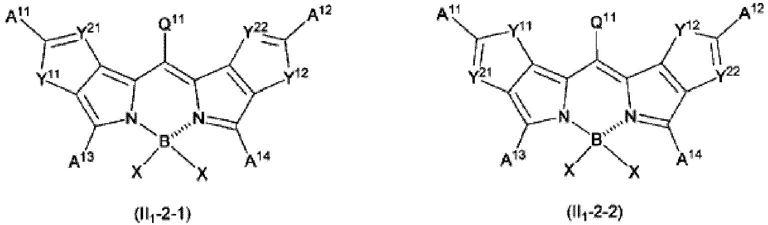 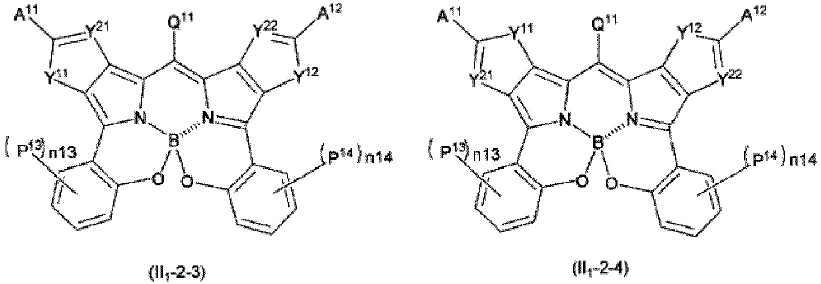 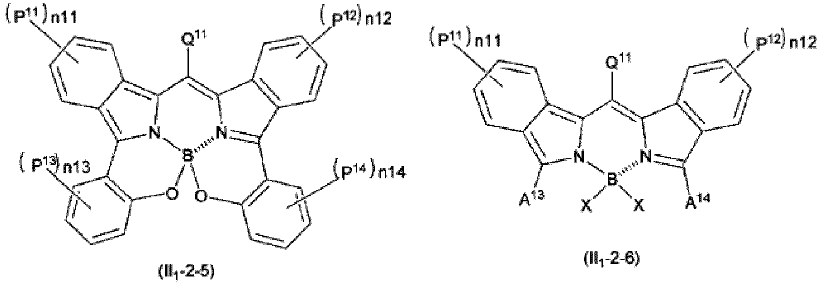 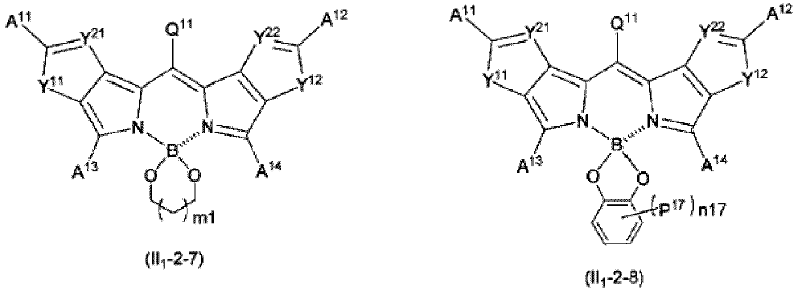 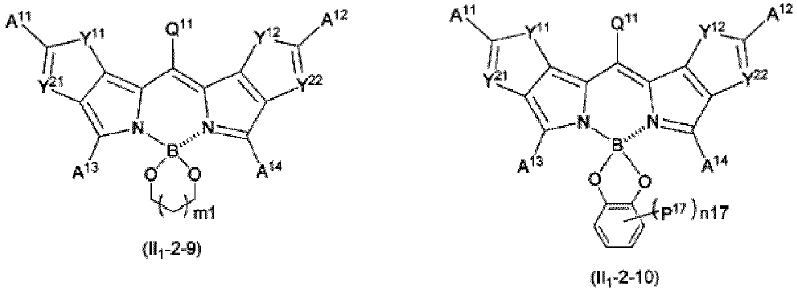 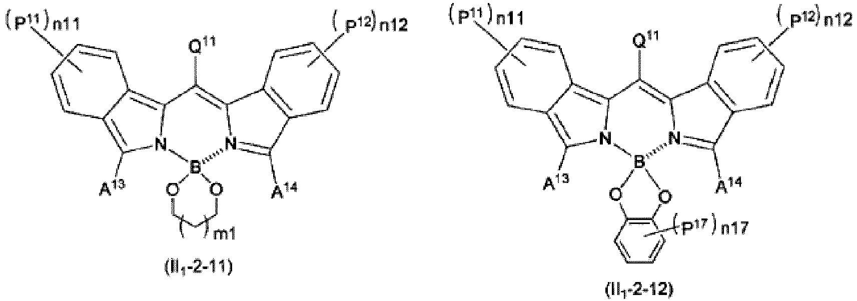 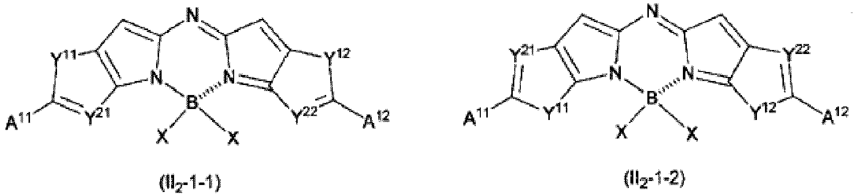 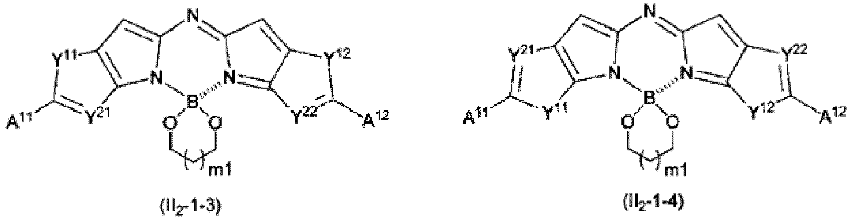 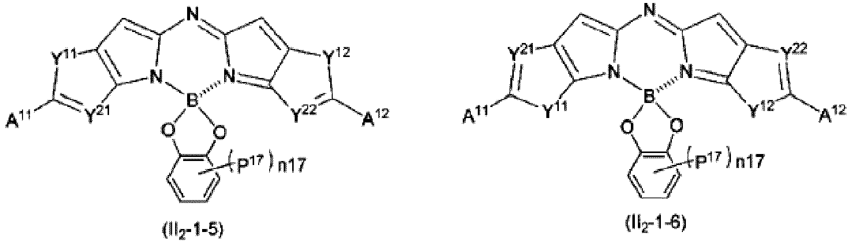 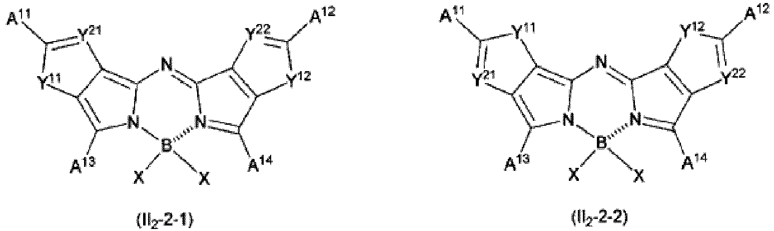 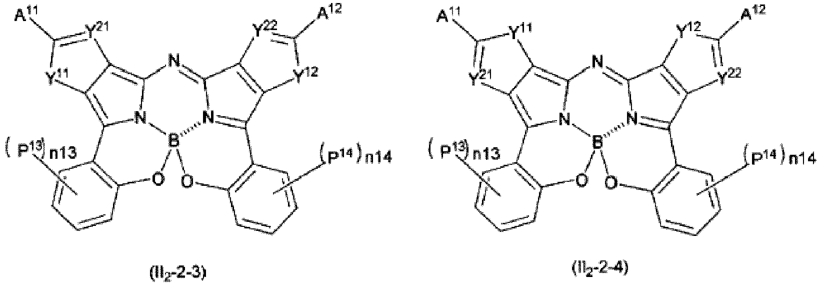 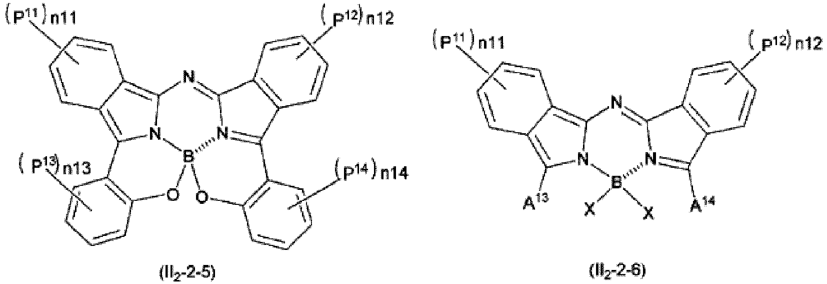 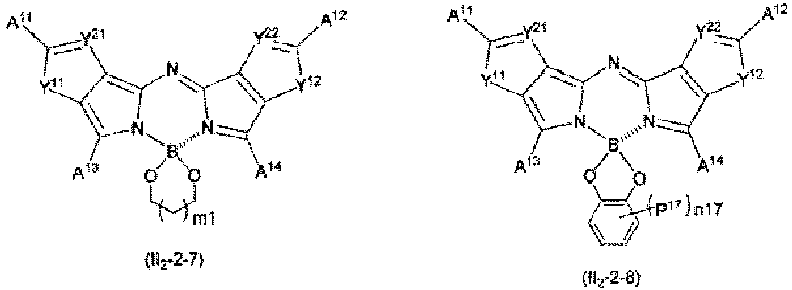 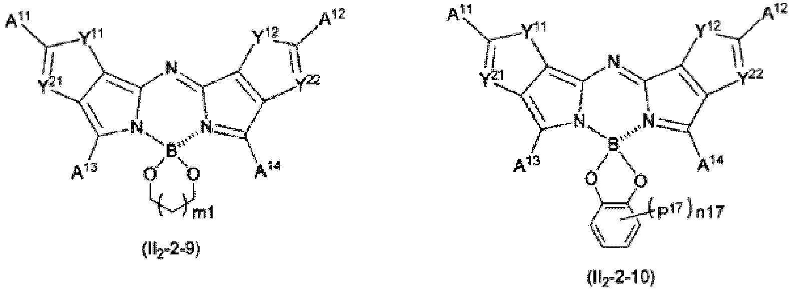 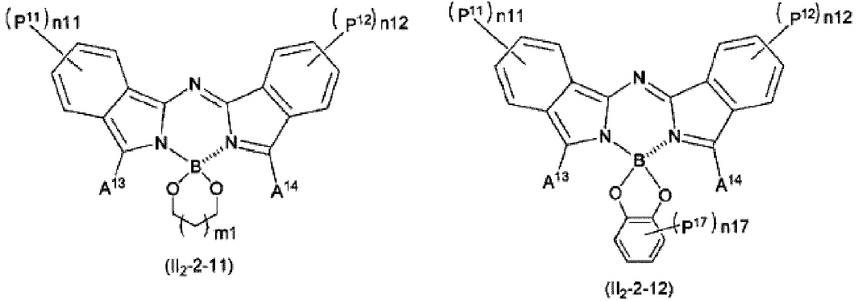
[In der Formel stellen Y¹¹ und Y¹² jeweils unabhängig ein Sauerstoffatom oder ein Schwefelatom dar;
stellt jedes von Y²¹ und Y²² unabhängig ein Kohlenstoffatom oder ein Stickstoffatom dar;
stellt Q eine Trifluormethylgruppe, eine Cyanogruppe, eine Nitrogruppe oder eine Phenylgruppe dar;
stellt jedes X unabhängig ein Halogenatom, eine C₁₋₂₀-Alkoxygruppe, eine Aryloxygruppe oder eine Acyloxygruppe dar;
stellt jedes von P¹¹ bis P¹⁴ und P¹⁷ unabhängig ein Halogenatom, eine C₁₋₂₀-Alkylgruppe, eine C₁₋₂₀-Alkoxygruppe, eine Aminogruppe, eine Monoalkylaminogruppe oder eine Dialkylaminogruppe dar;
stellt jedes von A¹¹ bis A¹⁴ unabhängig eine Phenylgruppe, die ein bis drei Substituenten haben kann, ausgewählt aus der Gruppe, bestehend aus einem Halogenatom, einer C₁₋₂₀-Alkylgruppe, einer C₁₋₂₀-Alkoxygruppe, einer Aminogruppe, einer Monoalkylaminogruppe und einer Dialkylaminogruppe, oder eine Heteroarylgruppe, die ein bis drei Substituenten haben kann, ausgewählt aus der Gruppe, bestehend aus einem Halogenatom, einer C₁₋₂₀-Alkylgruppe, einer C₁₋₂₀-Alkoxygruppe, einer Aminogruppe, einer Monoalkylaminogruppe und einer Dialkylaminogruppe, dar;
stellen jedes von n11 bis n14 und n17 unabhängig eine ganze Zahl von 0 bis 3 dar; und
stellt m1 0 oder 1 dar.].

6. Harzzusammensetzung nach Anspruch 2, die umfasst:
eine oder mehrere Verbindungen, ausgewählt aus der Gruppe, die aus Verbindungen besteht, die durch eine der folgenden allgemeinen Formeln (II₃-7) bis (II₃-9) und (II₄-7) bis (II₄-9) dargestellt sind, 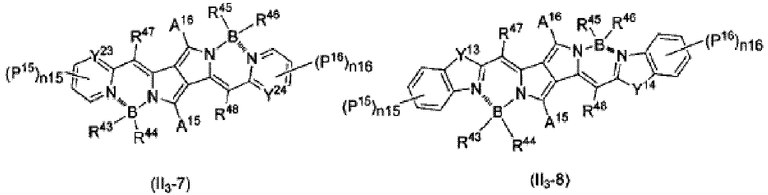 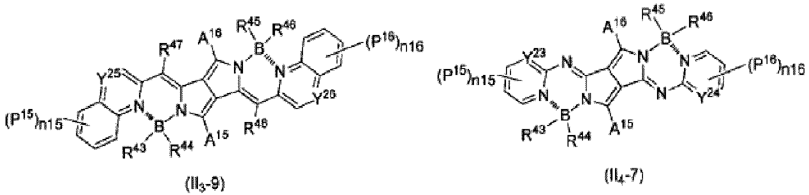 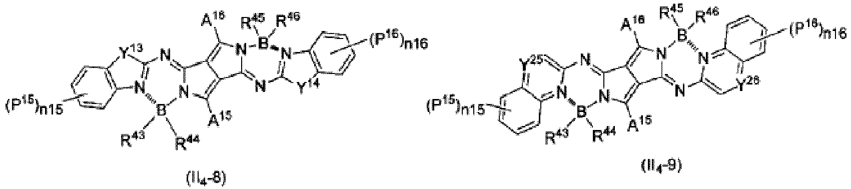
[In den Formeln stellt jedes von Y²³ und Y²⁴ unabhängig ein Kohlenstoffatom oder ein Stickstoffatom dar;
stellt jedes von Y¹³ und Y¹⁴ unabhängig ein Sauerstoffatom oder ein Schwefelatom dar;
stellen Y²⁵ und Y²⁶ jeweils unabhängig ein Kohlenstoffatom oder ein Stickstoffatom dar;
stellt jedes von R⁴⁷ und R⁴⁸ unabhängig ein Wasserstoffatom oder eine elektronenziehende Gruppe dar;
stellt jedes von R⁴³, R⁴⁴, R⁴⁵ und R⁴⁶ ein Halogenatom oder eine Arylgruppe dar, die einen Substituenten haben kann;
stellt jedes von P¹⁵ und P¹⁶ unabhängig ein Halogenatom, eine C₁₋₂₀-Alkylgruppe, eine C₁₋₂₀-Alkoxygruppe, eine Aminogruppe, eine Monoalkylaminogruppe oder eine Dialkylaminogruppe dar;
stellt jedes von n15 und n16 unabhängig eine ganze Zahl von 0 bis 3 dar; und
stellt jedes von A¹⁵ und A¹⁶ unabhängig eine Phenylgruppe dar, die ein bis drei Substituenten haben kann, die aus der Gruppe ausgewählt sind, die aus einem Wasserstoffatom, einem Halogenatom, einer C₁₋₂₀-Alkylgruppe, einer C₁₋₂₀-Alkoxygruppe, einer Aminogruppe, einer Monoalkylaminogruppe oder einer Dialkylaminogruppe besteht.].

7. Harzzusammensetzung nach Anspruch 2, die umfasst:
eine oder mehrere Verbindungen, die aus der Gruppe ausgewählt sind, die aus Verbindungen besteht, die durch eine der folgenden allgemeinen Formeln (II₃-1) bis (II₃-6) und (II₄-1) bis (II₄-6) dargestellt sind, 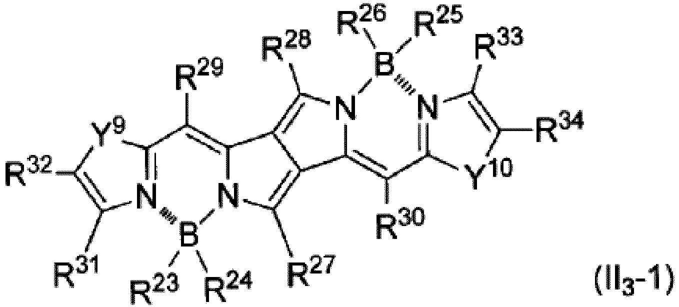 [In Formel (II₃-1),
stellt jedes von R²³, R²⁴, R²⁵ und R²⁶ unabhängig ein Halogenatom, eine C₁₋₂₀-Alkylgruppe, eine C₁₋₂₀-Alkoxygruppe, eine Arylgruppe oder eine Heteroarylgruppe dar;
stellt jedes von R²⁷ und R²⁸ unabhängig ein Wasserstoffatom, ein Halogenatom, eine C₁₋₂₀-Alkylgruppe, eine C₁₋₂₀-Alkoxygruppe, eine Arylgruppe oder eine Heteroarylgruppe dar;
stellt jedes von R²⁹ und R³⁰ unabhängig ein Wasserstoffatom oder eine elektronenziehende Gruppe dar;
stellt jedes von Y⁹ und Y¹⁰ unabhängig ein Schwefelatom, ein Sauerstoffatom, ein Stickstoffatom oder ein Phosphoratom dar; und
(p4) stellt jedes von R³¹ und R³² unabhängig ein Wasserstoffatom, ein Halogenatom, eine C₁₋₂₀-Alkylgruppe, eine C₁₋₂₀-Alkoxygruppe, eine Arylgruppe oder eine Heteroarylgruppe dar, oder
(p5) bilden R³¹ und R³² zusammen einen aromatischen 5-gliedrigen Ring, der einen Substituenten haben kann, oder einen aromatischen 6-gliedrigen Ring, der einen Substituenten haben kann;
(q4) stellt jedes von R³³ und R³⁴ unabhängig ein Wasserstoffatom, ein Halogenatom, eine C₁₋₂₀-Alkylgruppe, eine C₁₋₂₀-Alkoxygruppe, eine Arylgruppe oder eine Heteroarylgruppe dar, oder
(q5) bilden R³³ und R³⁴ zusammen einen aromatischen 5-gliedrigen Ring, der einen Substituenten haben kann, oder einen aromatischen 6-gliedrigen Ring, der einen Substituenten haben kann.] 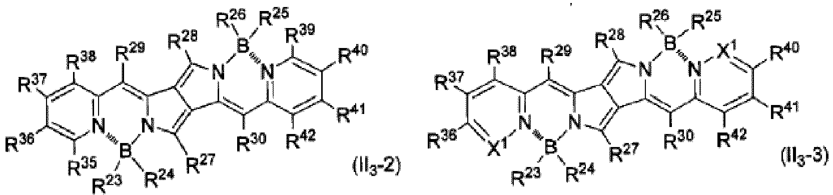 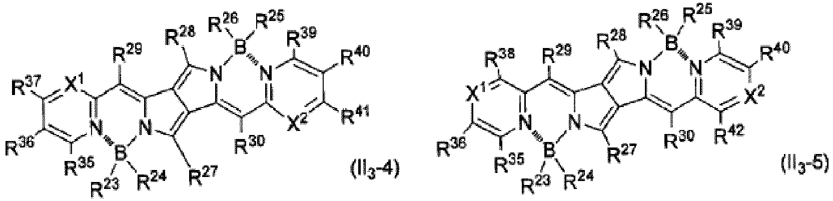 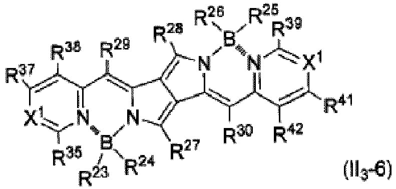
[In den Formeln (II₃-2) bis (II₃-6) ist jedes von R²³ bis R³⁰ dasselbe wie in der Formel (II₃-1);
stellt jedes von X¹ und X² unabhängig ein Stickstoffatom oder ein Phosphoratom dar;
(p6) stellen R³⁵, R³⁶, R³⁷ und R³⁸ jeweils unabhängig ein Wasserstoffatom, ein Halogenatom, eine C₁₋₂₀-Alkylgruppe, eine C₁₋₂₀-Alkoxygruppe, eine Arylgruppe oder eine Heteroarylgruppe dar,
(p7) bilden R³⁵ und R³⁶ zusammen einen aromatischen 5-gliedrigen Ring, der einen Substituenten haben kann, oder einen aromatischen 6-gliedrigen Ring, der einen Substituenten haben kann, und jedes von R³⁷ und R³⁸ stellt unabhängig ein Wasserstoffatom, ein Halogenatom, eine C₁₋₂₀-Alkylgruppe, eine C₁₋₂₀-Alkoxygruppe, eine Arylgruppe oder eine Heteroarylgruppe dar,
(p8) bilden R³⁶ und R³⁷ zusammen einen aromatischen 5-gliedrigen Ring, der einen Substituenten haben kann, oder einen aromatischen 6-gliedrigen Ring, der einen Substituenten haben kann, und jedes von R³⁵ und R³⁸ stellt unabhängig ein Wasserstoffatom, ein Halogenatom, eine C₁₋₂₀-Alkylgruppe, eine C₁₋₂₀-Alkoxygruppe, eine Arylgruppe oder eine Heteroarylgruppe dar, oder
(p9) bilden R³⁷ und R³⁸ zusammen einen aromatischen 5-gliedrigen Ring, der einen Substituenten haben kann, oder einen aromatischen 6-gliedrigen Ring, der einen Substituenten haben kann, und jedes von R³⁵ und R³⁶ stellt unabhängig ein Wasserstoffatom, ein Halogenatom, eine C₁₋₂₀-Alkylgruppe, eine C₁₋₂₀-Alkoxygruppe, eine Arylgruppe oder eine Heteroarylgruppe dar; und
(q6) stellen R³⁹, R⁴⁰, R⁴¹ und R⁴² jeweils unabhängig ein Wasserstoffatom, ein Halogenatom, eine C₁₋₂₀-Alkylgruppe, eine C₁₋₂₀-Alkoxygruppe, eine Arylgruppe oder eine Heteroarylgruppe dar,
(q7) bilden R³⁹ und R⁴⁰ zusammen einen aromatischen 5-gliedrigen Ring, der einen Substituenten haben kann, oder einen aromatischen 6-gliedrigen Ring, der einen Substituenten haben kann, und jedes von R⁴¹ und R⁴² stellt unabhängig ein Wasserstoffatom, ein Halogenatom, eine C₁₋₂₀-Alkylgruppe, eine C₁₋₂₀-Alkoxygruppe, eine Arylgruppe oder eine Heteroarylgruppe dar,
(q8) bilden R⁴⁰ und R⁴¹ zusammen einen aromatischen 5-gliedrigen Ring, der einen Substituenten haben kann, oder einen aromatischen 6-gliedrigen Ring, der einen Substituenten haben kann, und jedes von R³⁹ und R⁴² stellt unabhängig ein Wasserstoffatom, ein Halogenatom, eine C₁₋₂₀-Alkylgruppe, eine C₁₋₂₀-Alkoxygruppe, eine Arylgruppe oder eine Heteroarylgruppe dar, oder
(q9) bilden R⁴¹ und R⁴² zusammen einen aromatischen 5-gliedrigen Ring, der einen Substituenten haben kann, oder einen aromatischen 6-gliedrigen Ring, der einen Substituenten haben kann, und jedes von R³⁹ und R⁴⁰ stellt unabhängig ein Wasserstoffatom, ein Halogenatom, eine C₁₋₂₀-Alkylgruppe, eine C₁₋₂₀-Alkoxygruppe, eine Arylgruppe oder eine Heteroarylgruppe dar.] 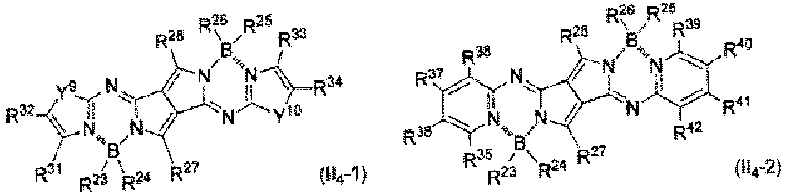 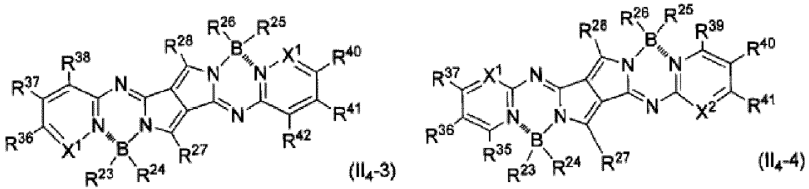 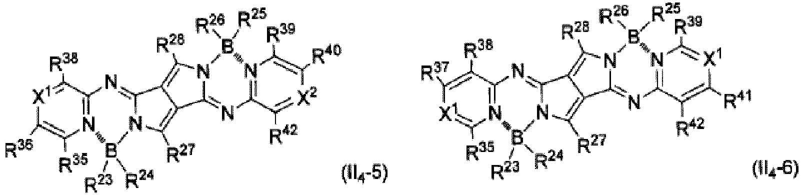 [In den Formeln (II₄-1) bis (II₄-6) ist jedes von R²³ bis R²⁸ derselbe wie in der Formel (II₃-1), und in der Formel (II₄-1) ist jedes von R³¹ bis R³⁴, Y⁹ und Y¹⁰ derselbe wie in der Formel (II₃-1), in den Formeln (II₄-2) bis (II₄-6) ist jedes von R³⁵ bis R⁴² dasselbe wie in der Formel (II₃-2), und in den Formeln (II₄-3) bis (II₄-6) ist jedes von X¹ und X² dasselbe wie in der Formel (II₃-3).].

8. Harzzusammensetzung nach Anspruch 1,
wobei das im Infrarot fluoreszierende Material aus einer Azo-Bor-Komplexverbindung, die durch die folgende Formel (I) dargestellt ist, gebildet ist und eine maximale Absorptionswellenlänge von 650 nm oder länger und eine Stokes-Verschiebung von 50 nm oder länger aufweist, 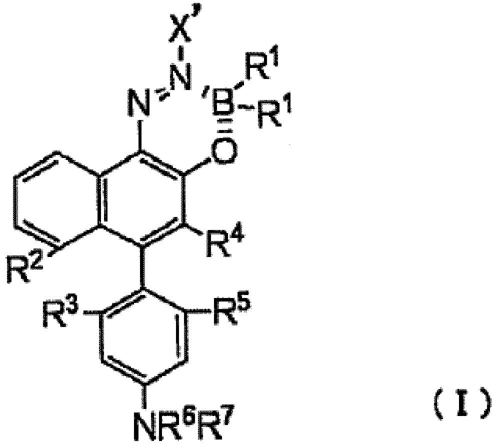 [In Formel (I),
stellt X' eine Arylgruppe, die einen Substituenten haben kann, oder eine Heteroarylgruppe, die einen Substituenten haben kann, dar;
stellt R¹ eine C₁₋₁₂-Alkylgruppe, eine Arylgruppe, eine Arylethenylgruppe, eine Arylethinylgruppe, eine C₁₋₁₂-Alkoxygruppe, eine Aryloxygruppe oder ein Halogenatom dar, oder eines der R¹ stellt eine -O-C(=O)-Gruppe dar, die auch an X' gebunden ist und bildet einen 6-gliedrigen Ring, und das andere R¹ stellt unabhängig eine C₁₋₁₂-Alkylgruppe, eine Arylgruppe, eine Arylethenylgruppe, eine Arylethinylgruppe, eine C₁₋₁₂-Alkoxygruppe, eine Aryloxygruppe oder ein Halogenatom dar;
bilden R² und R³ zusammen eine -O- Gruppe, eine -S- Gruppe oder eine -N(R⁸)- Gruppe (hier stellt R⁸ ein Wasserstoffatom oder eine C₁₋₁₂-Alkylgruppe dar), und jedes von R⁴ und R⁵ stellt ein Wasserstoffatom dar, oder R⁴ und R⁵ bilden zusammen eine -O- Gruppe, eine -S- Gruppe oder eine -N(R⁸)- Gruppe (R⁸ hat die gleiche Bedeutung wie oben beschrieben), und jedes von R² und R³ stellt ein Wasserstoffatom dar;
stellt jedes von R⁶ und R⁷ unabhängig ein Wasserstoffatom, eine C₁₋₁₂-Alkylgruppe, eine Arylgruppe, die einen Substituenten haben kann, oder eine Heteroarylgruppe, die einen Substituenten haben kann, dar; und
der Substituent der Arylgruppe oder der Heteroarylgruppe stellt eine oder mehrere Gruppen dar, die aus der Gruppe ausgewählt sind, die aus einer C₁₋₁₂-Alkylgruppe, einer Mono-(C₁₋₁₂-Alkyl)aminogruppe, einer Di-(C₁₋₁₂-Alkyl)aminogruppe, einer Hydroxylgruppe und einer C₁₋₁₂-Alkoxygruppe besteht.].

9. Harzzusammensetzung nach Anspruch 8,
wobei die Azo-Bor-Komplexverbindung durch die folgende Formel (I₁) dargestellt ist, 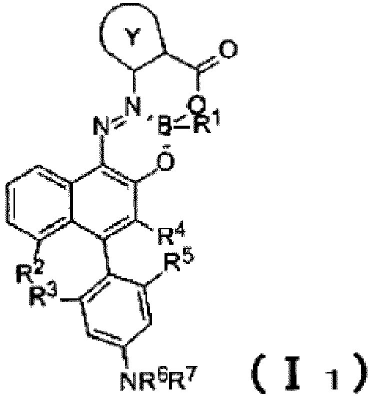 [In Formel (I₁) stellt Y eine Arylgruppe, die einen Substituenten haben kann, oder eine Heteroarylgruppe, die einen Substituenten haben kann, dar, und jedes von R¹ bis R⁷ hat dieselbe Bedeutung wie jedes von R¹ bis R⁷ in der Formel (I)].

10. Harzzusammensetzung nach einem der Ansprüche 1 bis 9,
wobei die röntgenopake Substanz eine oder mehrere aus der Gruppe bestehend aus Bariumsulfat, Bismutoxid, Bismutsubcarbonat, Kalziumcarbonat, Aluminiumhydroxid, Wolfram, Zinkoxid, Zirkoniumoxid, Zirkonium, Titan, Platin, Bismutsubnitrat und Bismut ist.

11. Harzzusammensetzung nach einem der Ansprüche 1 bis 10,
wobei der Gehalt der röntgenopaken Substanz 5 Massen-% bis 50 Massen-% beträgt.

12. Harzzusammensetzung nach einem der Ansprüche 1 bis 11,
wobei der Gehalt der lichtemittierenden Substanz 0,001 Massen-% bis 0,5 Massen-% beträgt.

13. Harzzusammensetzung nach einem der Ansprüche 1 bis 11,
wobei der Gehalt der lichtemittierenden Substanz 0,001 Massen-% bis 0,05 Massen-% beträgt.

14. Harzzusammensetzung nach einem der Ansprüche 1 bis 13,
wobei das Harz ein thermoplastisches Harz ist.

15. Harzzusammensetzung nach einem der Ansprüche 1 bis 14,
wobei das Harz eines oder mehrere ist, die aus der Gruppe ausgewählt sind, die aus einem Harz auf Urethanbasis, einem Harz auf Olefinbasis, einem Harz auf Polystyrolbasis, einem Harz auf Polyesterbasis und einem Harz auf Vinylchloridbasis besteht.

16. Harzzusammensetzung nach einem der Ansprüche 1 bis 15, die als medizinisches Material verwendet wird.

17. Formgegenstand, der durch Verarbeitung der Harzzusammensetzung nach einem der Ansprüche 1 bis 16 erhalten wird.

18. Formgegenstand nach Anspruch 17,
wobei der Gegenstand ein medizinisches Werkzeug ist und von dem zumindest ein Teil im Körper eines Patienten verwendet wird.

## Revendications

1. Composition de résine comprenant :
une substance luminescente ;
une substance radio-opaque ; et
une résine,
dans laquelle la substance luminescente est un matériau fluorescent dans le proche infrarouge.

2. Composition de résine selon la revendication 1, dans laquelle le matériau fluorescent dans le proche infrarouge est un ou plusieurs composés choisis dans l'ensemble constitué par les composés représentés par l'une des formules générales (II₁), (II₂), (II₃) et (II₄) qui suivent et a une longueur d'onde de fluorescence maximale de 650 nm ou plus, 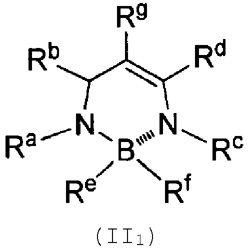 [dans la formule (II₁),
R^{a} et R^{b} forment un cycle aromatique à 5 chaînons, un cycle aromatique à 6 chaînons, ou un cycle aromatique condensé formé par condensation de deux ou trois cycles à 5 chaînons ou cycles à 6 chaînons conjointement avec l'atome d'azote auquel R^{a} est lié et l'atome de carbone auquel R^{b} est lié ;
R^{c} et R^{d} forment un cycle aromatique à 5 chaînons, un cycle aromatique à 6 chaînons, ou un cycle aromatique condensé formé par condensation de deux ou trois cycles à 5 chaînons ou cycles à 6 chaînons conjointement avec l'atome d'azote auquel R^{c} est lié et l'atome de carbone auquel R^{d} est lié ;
chacun de R^{e} et R^{f} représente un atome d'halogène ou un atome d'oxygène ; et
R^{g} représente un atome d'hydrogène ou un groupe extracteur d'électrons ;
ici, dans le cas où R^{e} et R^{f} sont des atomes d'oxygène, R^{e}, l'atome de bore lié à R^{e}, R^{a}, et l'atome d'azote lié à R^{a} peuvent ensemble former un cycle, et R^{f}, l'atome de bore lié à R^{f}, R^{c}, et l'atome d'azote lié à R^{c} peuvent ensemble former un cycle ; dans le cas où R^{e} est un atome d'oxygène et ne forme pas un cycle, R^{e} est un atome d'oxygène portant un substituant, et dans le cas où R^{f} est un atome d'oxygène et ne forme pas un cycle, R^{f} est un atome d'oxygène portant un substituant] 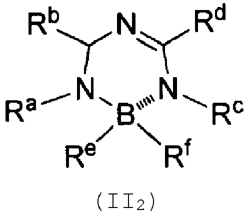 [dans la formule (II₂), chacun de R^{a} à R^{f} est le même que dans la formule (II₁)] 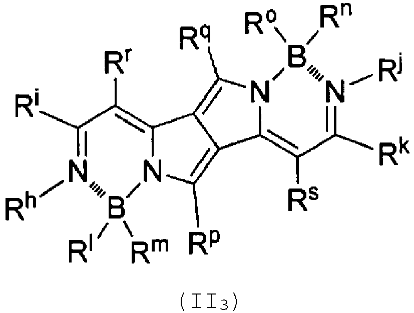 [dans la formule (II₃),
R^{h} et Rⁱ forment un cycle aromatique à 5 chaînons, un cycle aromatique à 6 chaînons, ou un cycle aromatique condensé formé par condensation de deux ou trois cycles à 5 chaînons ou cycles à 6 chaînons conjointement avec l'atome d'azote auquel R^{h} est lié et l'atome de carbone auquel Rⁱ est lié ;
R^{j} et R^{k} forment un cycle aromatique à 5 chaînons, un cycle aromatique à 6 chaînons, ou un cycle aromatique condensé formé par condensation de deux ou trois cycles à 5 chaînons ou cycles à 6 chaînons conjointement avec l'atome d'azote auquel R^{j} est lié et l'atome de carbone auquel R^{k} est lié ;
chacun de R^{l}, R^{m}, Rⁿ et R^{o} représente indépendamment un atome d'halogène, un groupe alkyle en C₁ à C₂₀, un groupe alcoxy en C₁ à C₂₀, un groupe aryle, ou un groupe hétéroaryle ;
chacun de R^{p} et R^{q} représente indépendamment un atome d'hydrogène, un atome d'halogène, un groupe alkyle en C₁ à C₂₀, un groupe alcoxy en C₁ à C₂₀, un groupe aryle, ou un groupe hétéroaryle ; et
chacun de R^{r} et R^{s} représente indépendamment un atome d'hydrogène ou un groupe extracteur d'électrons] 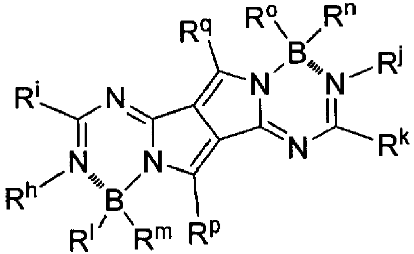 [dans la formule (II₄), chacun de R^{h} à R^{q} est le même que dans la formule (II₃)].

3. Composition de résine selon la revendication 2, comprenant un ou plusieurs composés choisis dans l'ensemble constitué par les composés représentés par l'une des formules générales (II₁-0) et (II₂-0) suivantes 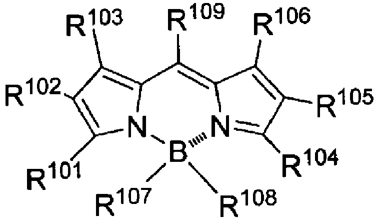 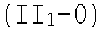 [dans la formule (II₁-0),
(p1) chacun de R¹⁰¹, R¹⁰² et R¹⁰³ représente indépendamment un atome d'hydrogène, un atome d'halogène, un groupe alkyle en C₁ à C₂₀, un groupe alcoxy en C₁ à C₂₀, un groupe aryle, ou un groupe hétéroaryle,
(p2) R¹⁰¹ et R¹⁰² forment ensemble un cycle aromatique à 5 chaînons ou un cycle aromatique à 6 chaînons, et R¹⁰³ représente un atome d'hydrogène, un atome d'halogène, un groupe alkyle en C₁ à C₂₀, un groupe alcoxy en C₁ à C₂₀, un groupe aryle, ou un groupe hétéroaryle, ou
(p3) R¹⁰² et R¹⁰³ forment ensemble un cycle aromatique à 5 chaînons ou un cycle aromatique à 6 chaînons, et R¹⁰¹ représente un atome d'hydrogène, un atome d'halogène, un groupe alkyle en C₁ à C₂₀, un groupe alcoxy en C₁ à C₂₀, un groupe aryle, ou un groupe hétéroaryle, et
(q1) chacun de R¹⁰⁴, R¹⁰⁵ et R¹⁰⁶ représente indépendamment un atome d'hydrogène, un atome d'halogène, un groupe alkyle en C₁ à C₂₀, un groupe alcoxy en C₁ à C₂₀, un groupe aryle, ou un groupe hétéroaryle,
(q2) R¹⁰⁴ et R¹⁰⁵ forment ensemble un cycle aromatique à 5 chaînons ou un cycle aromatique à 6 chaînons, et R¹⁰⁶ représente un atome d'hydrogène, un atome d'halogène, un groupe alkyle en C₁ à C₂₀, un groupe alcoxy en C₁ à C₂₀, un groupe aryle, ou un groupe hétéroaryle, ou
(q3) R¹⁰⁵ et R¹⁰⁶ forment ensemble un cycle aromatique à 5 chaînons ou un cycle aromatique à 6 chaînons, et R¹⁰⁴ représente un atome d'hydrogène, un atome d'halogène, un groupe alkyle en C₁ à C₂₀, un groupe alcoxy en C₁ à C₂₀, un groupe aryle, ou un groupe hétéroaryle,
chacun de R¹⁰⁷ et R¹⁰⁸ représente un atome d'halogène ou un atome d'oxygène ; et
R¹⁰⁹ représente un atome d'hydrogène ou un groupe extracteur d'électrons ;
ici, dans le cas où R¹⁰⁷ et R¹⁰⁸ sont des atomes d'oxygène, R¹⁰⁷, l'atome de bore lié à R¹⁰⁷, l'atome d'azote lié à l'atome de bore, R¹⁰¹, et l'atome d'azote lié à R¹⁰¹ peuvent ensemble former un cycle, et R¹⁰⁸, l'atome de bore lié à R¹⁰⁸, l'atome d'azote lié à l'atome de bore, R¹⁰⁴, et l'atome d'azote lié à R¹⁰⁴ peuvent ensemble former un cycle ; dans le cas où R¹⁰⁷ est un atome d'oxygène et ne forme pas un cycle, R¹⁰⁷ est un atome d'oxygène portant un substituant, et dans le cas où R¹⁰⁸ est un atome d'oxygène et ne forme pas un cycle, R¹⁰⁸ est un atome d'oxygène portant un substituant] 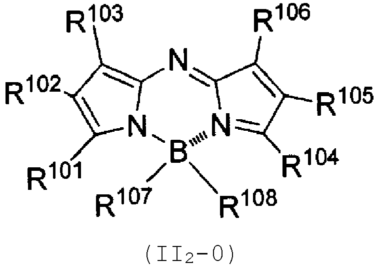 [dans la formule (II₂-0), chacun de R¹⁰¹ à R¹⁰⁸ est le même que dans la formule (II₁-0)].

4. Composition de résine selon la revendication 3, dans laquelle, dans la formule générale (II₁-0) ou (II₂-0), R¹⁰¹ et R¹⁰² forment un cycle, et R¹⁰⁴ et R¹⁰⁵ forment un cycle, ou R¹⁰² et R¹⁰³ forment un cycle, et R¹⁰⁵ et R¹⁰⁶ forment un cycle, et le cycle est représenté par l'une quelconque des formules chimiques (C-1) à (C-9) suivantes, 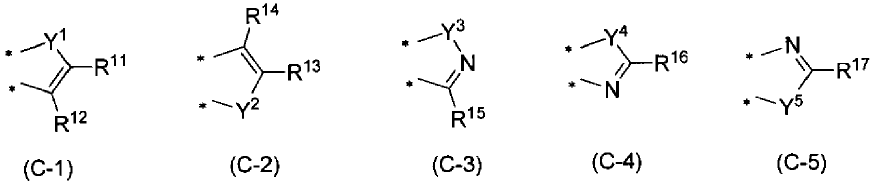 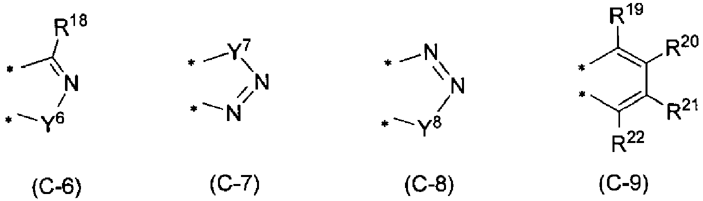 [dans les formules (C-1) à (C-9), chacun de Y¹ à Y⁸ représente indépendamment un atome de soufre, un atome d'oxygène, un atome d'azote, ou un atome de phosphore, et chacun de R¹¹ à R²² représente indépendamment un atome d'hydrogène ou n'importe quel groupe qui n'inhibe pas la fluorescence du composé].

5. Composition de résine selon la revendication 2, comprenant un ou plusieurs composés choisis dans l'ensemble constitué par les composés représentés par l'une quelconque des formules générales (II₁-1-1) à (II₁-1-6), (II₂-2-1) à (II₂-2-12), (II₂-1-1) à (II₂-1-6), et (II₂-2-1) à (II₂-2-12), 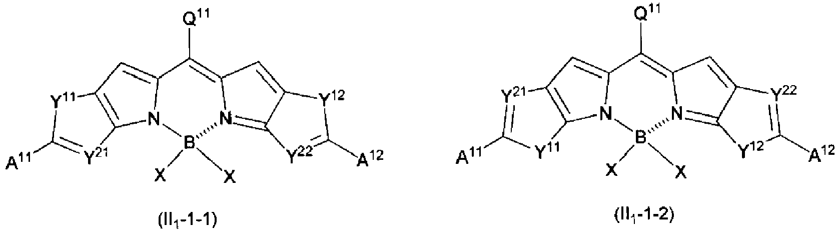 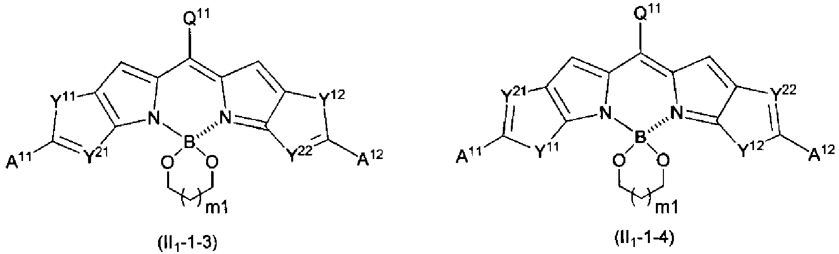 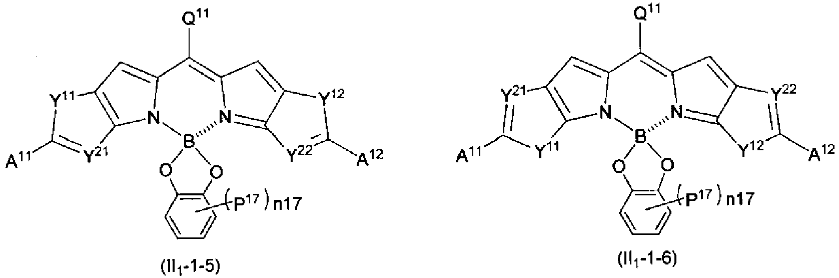 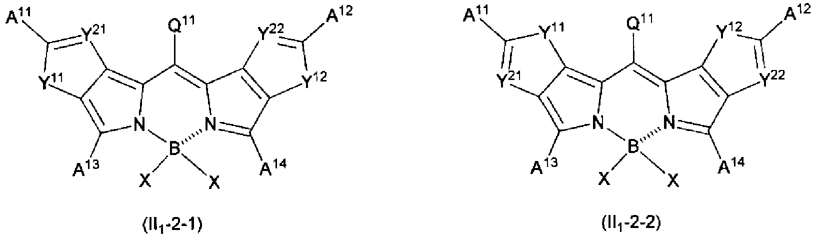 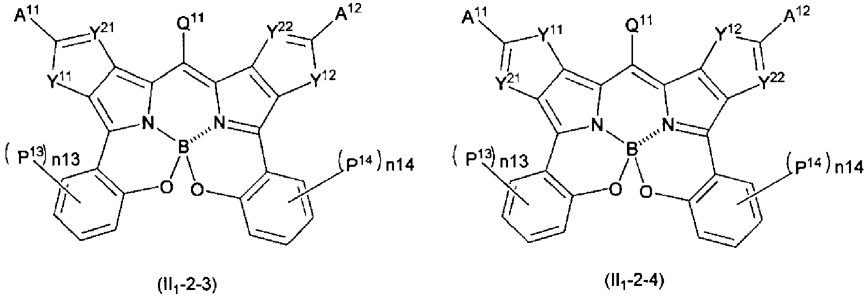 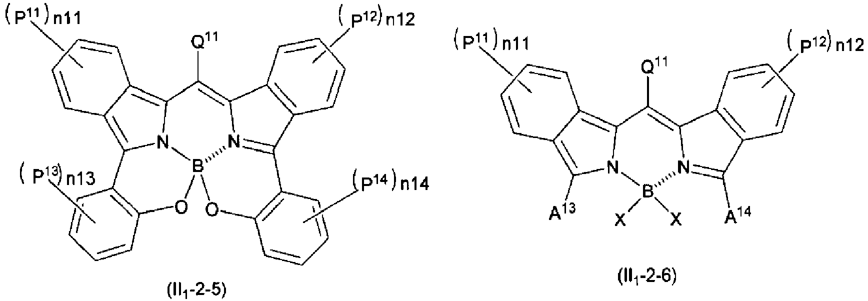 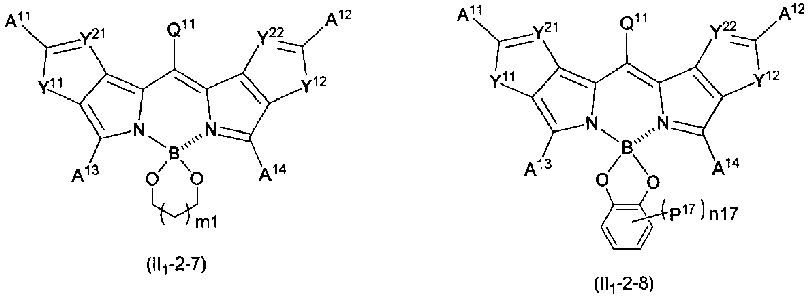 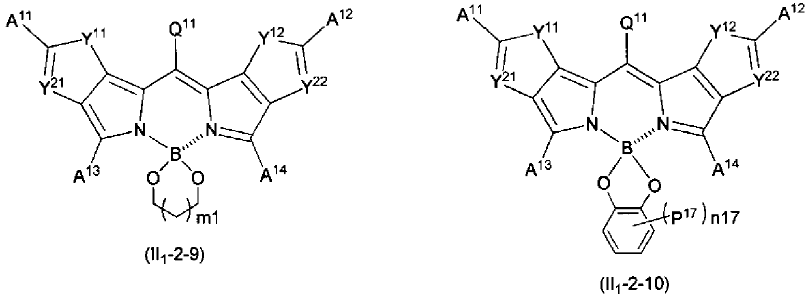 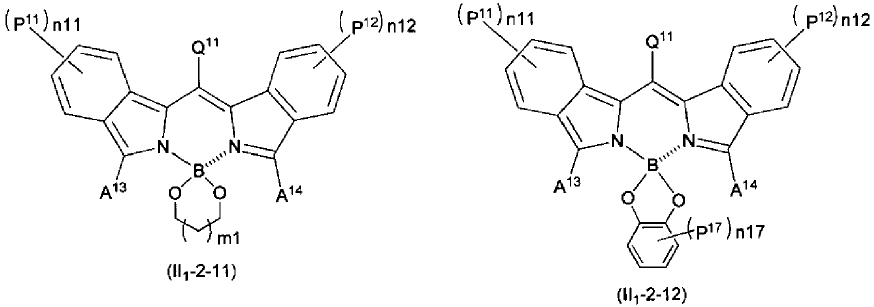 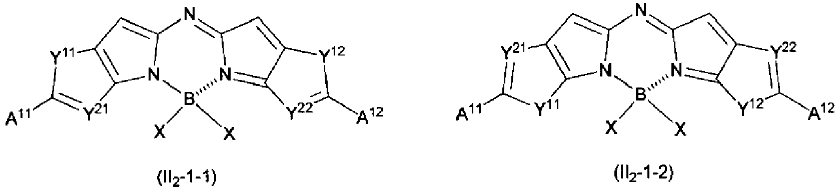 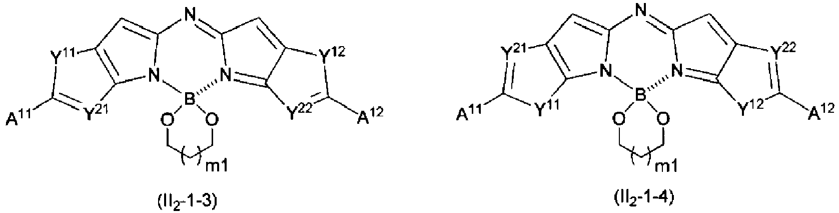 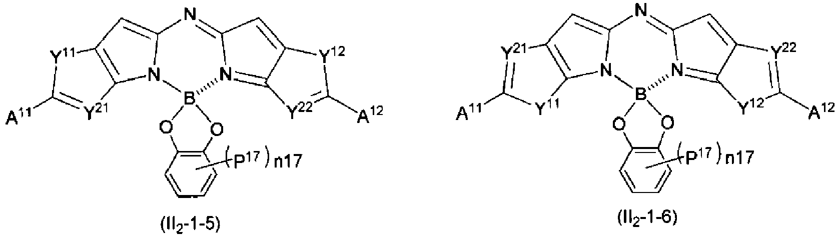 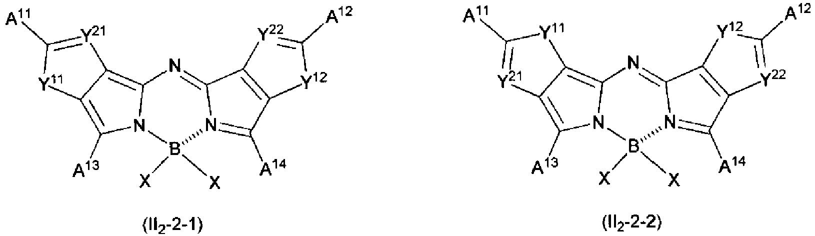 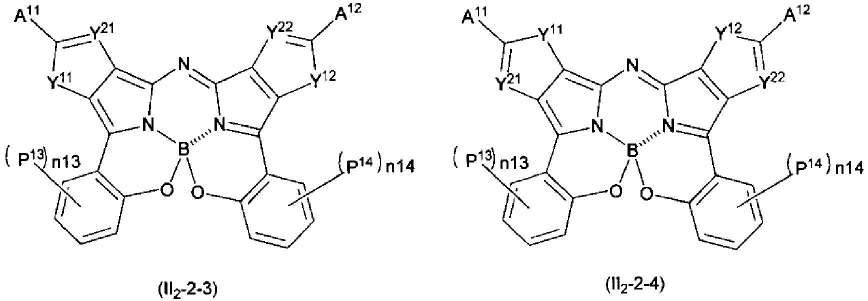 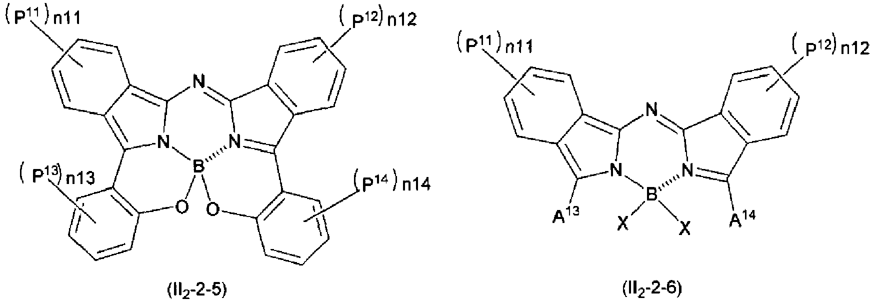 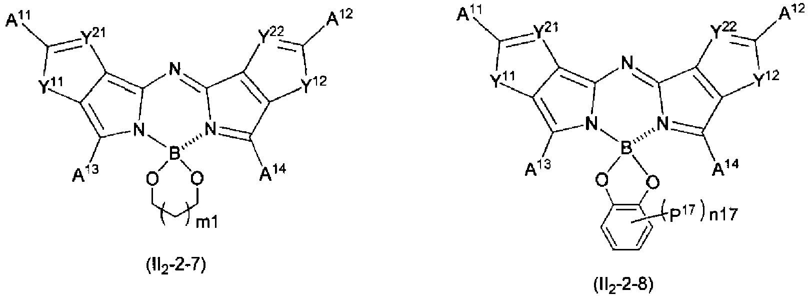 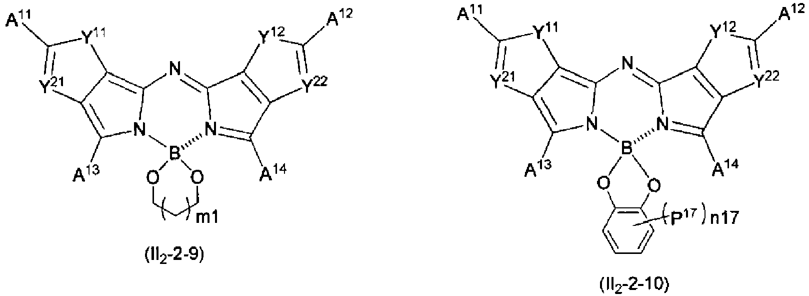 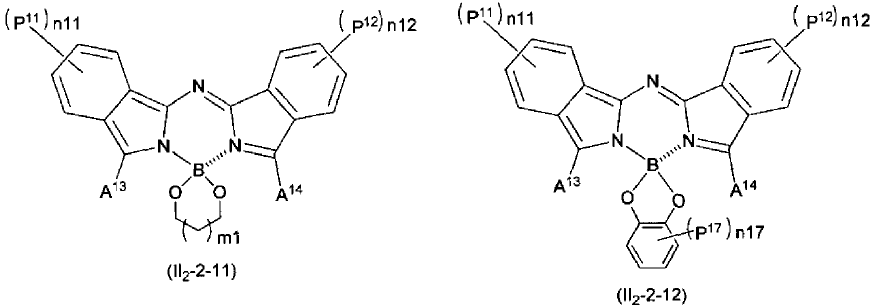 [dans les formules,
chacun de Y¹¹ et Y¹² représente indépendamment un atome d'oxygène ou un atome de soufre ;
chacun de Y²¹ et Y²² représente indépendamment un atome de carbone ou un atome d'azote ;
Q représente un groupe trifluorométhyle, un groupe cyano, un groupe nitro, ou un groupe phényle ;
chaque X représente indépendamment un atome d'halogène, un groupe alcoxy en C₁ à C₂₀, un groupe aryloxy, ou un groupe acyloxy ;
chacun de P¹¹ à P¹⁴ et P¹⁷ représente indépendamment un atome d'halogène, un groupe alkyle en C₁ à C₂₀, un groupe alcoxy en C₁ à C₂₀, un groupe amino, un groupe monoalkylamino, ou un groupe dialkylamino ;
chacun de A¹¹ à A¹⁴ représente indépendamment un groupe phényle qui peut porter un à trois substituants choisis dans l'ensemble constitué par un atome d'halogène, un groupe alkyle en C₁ à C₂₀, un groupe alcoxy en C₁ à C₂₀, un groupe amino, un groupe monoalkylamino, et un groupe dialkylamino, ou un groupe hétéroaryle qui peut porter un à trois substituants choisis dans l'ensemble constitué par un atome d'halogène, un groupe alkyle en C₁ à C₂₀, un groupe alcoxy en C₁ à C₂₀, un groupe amino, un groupe monoalkylamino, et un groupe dialkylamino ;
chacun de n11 à n14 et n17 représente indépendamment un entier de 0 à 3 ; et
m1 vaut 0 ou 1].

6. Composition de résine selon la revendication 2, comprenant un ou plusieurs composés choisis dans l'ensemble constitué par les composés représentés par l'une quelconque des formules générales (II₃-7) à (II₃-9) et (II₄-7) à (II₄-9) suivantes, 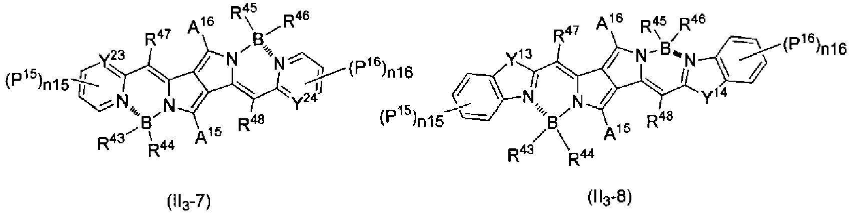 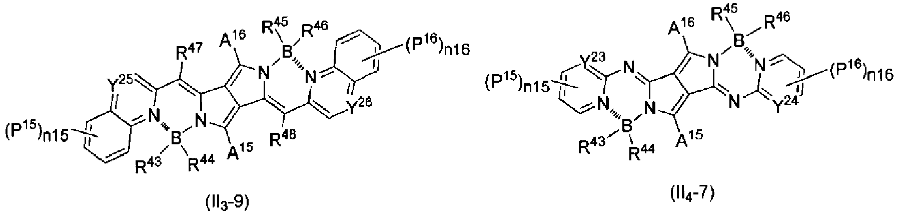 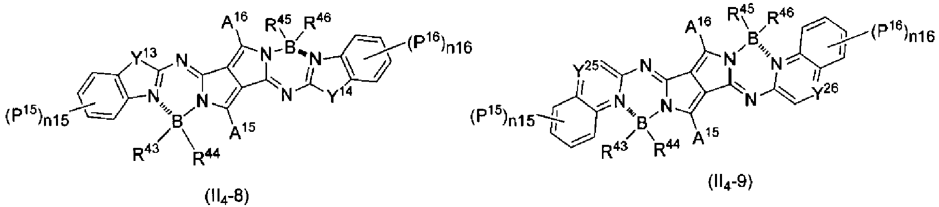 [dans les formules,
chacun de Y²³ et Y²⁴ représente indépendamment un atome de carbone ou un atome d'azote ;
chacun de Y¹³ et Y¹⁴ représente indépendamment un atome d'oxygène ou un atome de soufre ;
chacun de Y²⁵ et Y²⁶ représente indépendamment un atome de carbone ou un atome d'azote ;
chacun de R⁴⁷ et R⁴⁸ représente indépendamment un atome d'hydrogène ou un groupe extracteur d'électrons ;
chacun de R⁴³, R⁴⁴, R⁴⁵ et R⁴⁶ représente un atome d'halogène ou un groupe aryle qui peut porter un substituant ;
chacun de P¹⁵ et P¹⁶ représente indépendamment un atome d'halogène, un groupe alkyle en C₁ à C₂₀, un groupe alcoxy en C₁ à C₂₀, un groupe amino, un groupe monoalkylamino, ou un groupe dialkylamino ;
chacun de n15 et n16 représente indépendamment un entier de 0 à 3 ; et
chacun de A¹⁵ et A¹⁶ représente indépendamment un groupe phényle qui peut porter un à trois substituants choisis dans l'ensemble constitué par un atome d'hydrogène, un atome d'halogène, un groupe alkyle en C₁ à C₂₀, un groupe alcoxy en C₁ à C₂₀, un groupe amino, un groupe monoalkylamino, ou un groupe dialkylamino].

7. Composition de résine selon la revendication 2, comprenant un ou plusieurs composés choisis dans l'ensemble constitué par les composés représentés par l'une quelconque des formules générales (II₃-1) à (II₃-6) et (II₄-1) à (II₄-6) suivantes, 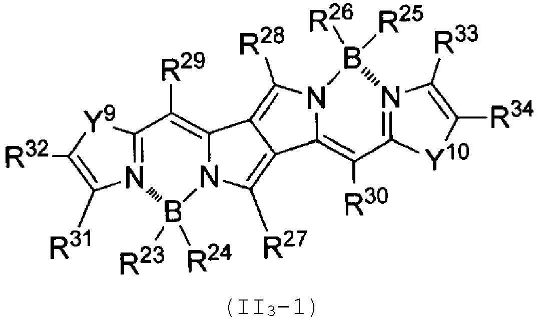 [dans la formule (II₃-1),
chacun de R²³, R²⁴, R²⁵ et R²⁶ représente indépendamment un atome d'halogène, un groupe alkyle en C₁ à C₂₀, un groupe alcoxy en C₁ à C₂₀, un groupe aryle, ou un groupe hétéroaryle ;
chacun de R²⁷ et R²⁸ représente indépendamment un atome d'hydrogène, un atome d'halogène, un groupe alkyle en C₁ à C₂₀, un groupe alcoxy en C₁ à C₂₀, un groupe aryle, ou un groupe hétéroaryle ;
chacun de R²⁹ et R³⁰ représente indépendamment un atome d'hydrogène ou un groupe extracteur d'électrons ;
chacun de Y⁹ et Y¹⁰ représente indépendamment un atome de soufre, un atome d'oxygène, un atome d'azote, ou un atome de phosphore ; et
(p4) chacun de R³¹ et R³² représente indépendamment un atome d'hydrogène, un atome d'halogène, un groupe alkyle en C₁ à C₂₀, un groupe alcoxy en C₁ à C₂₀, un groupe aryle, ou un groupe hétéroaryle, ou
(p5) R³¹ et R³² forment ensemble un cycle aromatique à 5 chaînons qui peut porter un substituant ou un cycle aromatique à 6 chaînons qui peut porter un substituant ;
(q4) chacun de R³³ et R³⁴ représente indépendamment un atome d'hydrogène, un atome d'halogène, un groupe alkyle en C₁ à C₂₀, un groupe alcoxy en C₁ à C₂₀, un groupe aryle, ou un groupe hétéroaryle, ou
(q5) R³³ et R³⁴ forment ensemble un cycle aromatique à 5 chaînons qui peut porter un substituant ou un cycle aromatique à 6 chaînons qui peut porter un substituant] 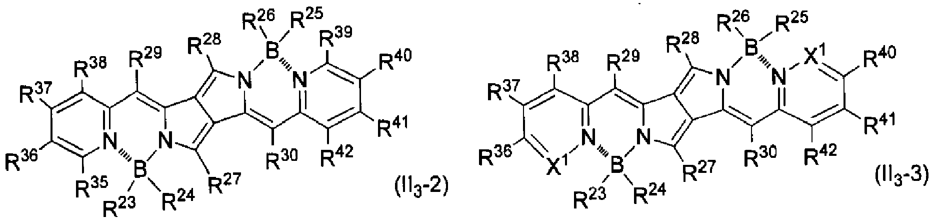 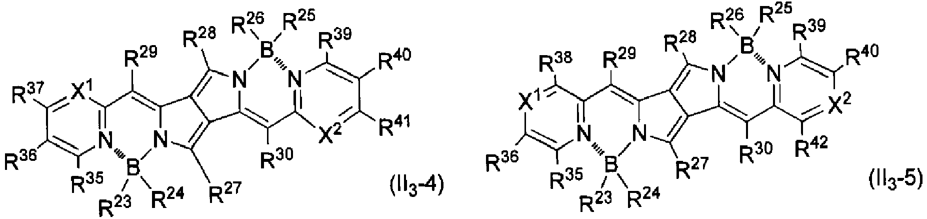 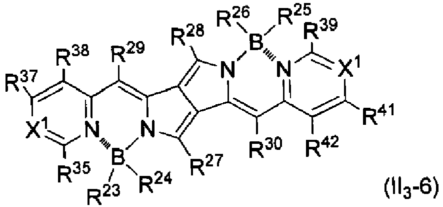 [dans les formules (II₃-2) à (II₃-6),
chacun de R²³ à R³⁰ est le même que dans la formule (II₃-1) ;
chacun de X¹ et X² représente indépendamment un atome d'azote ou un atome de phosphore ;
(p6) chacun de R³⁵, R³⁶, R³⁷ et R³⁸ représente indépendamment un atome d'hydrogène, un atome d'halogène, un groupe alkyle en C₁ à C₂₀, un groupe alcoxy en C₁ à C₂₀, un groupe aryle, ou un groupe hétéroaryle, ou
(p7) R³⁵ et R³⁶ forment ensemble un cycle aromatique à 5 chaînons qui peut porter un substituant ou un cycle aromatique à 6 chaînons qui peut porter un substituant, et chacun de R³⁷ et R³⁸ représente indépendamment un atome d'hydrogène, un atome d'halogène, un groupe alkyle en C₁ à C₂₀, un groupe alcoxy en C₁ à C₂₀, un groupe aryle, ou un groupe hétéroaryle,
(p8) R³⁶ et R³⁷ forment ensemble un cycle aromatique à 5 chaînons qui peut porter un substituant ou un cycle aromatique à 6 chaînons qui peut porter un substituant, et chacun de R³⁵ et R³⁸ représente indépendamment un atome d'hydrogène, un atome d'halogène, un groupe alkyle en C₁ à C₂₀, un groupe alcoxy en C₁ à C₂₀, un groupe aryle, ou un groupe hétéroaryle, ou
(p9) R³⁷ et R³⁸ forment ensemble un cycle aromatique à 5 chaînons qui peut porter un substituant ou un cycle aromatique à 6 chaînons qui peut porter un substituant, et chacun de R³⁵ et R³⁶ représente indépendamment un atome d'hydrogène, un atome d'halogène, un groupe alkyle en C₁ à C₂₀, un groupe alcoxy en C₁ à C₂₀, un groupe aryle, ou un groupe hétéroaryle ; et
(q6) chacun de R³⁹, R⁴⁰, R⁴¹ et R⁴² représente indépendamment un atome d'hydrogène, un atome d'halogène, un groupe alkyle en C₁ à C₂₀, un groupe alcoxy en C₁ à C₂₀, un groupe aryle, ou un groupe hétéroaryle,
(q7) R⁴⁰ et R⁴¹ forment ensemble un cycle aromatique à 5 chaînons qui peut porter un substituant ou un cycle aromatique à 6 chaînons qui peut porter un substituant, et chacun de R³⁹ et R⁴² représente indépendamment un atome d'hydrogène, un atome d'halogène, un groupe alkyle en C₁ à C₂₀, un groupe alcoxy en C₁ à C₂₀, un groupe aryle, ou un groupe hétéroaryle, ou
(q8) R⁴⁰ et R⁴¹ forment ensemble un cycle aromatique à 5 chaînons qui peut porter un substituant ou un cycle aromatique à 6 chaînons qui peut porter un substituant, et chacun de R³⁹ et R⁴² représente indépendamment un atome d'hydrogène, un atome d'halogène, un groupe alkyle en C₁ à C₂₀, un groupe alcoxy en C₁ à C₂₀, un groupe aryle, ou un groupe hétéroaryle, ou
(q9) R⁴¹ et R⁴² forment ensemble un cycle aromatique à 5 chaînons qui peut porter un substituant ou un cycle aromatique à 6 chaînons qui peut porter un substituant, et chacun de R³⁹ et R⁴⁰ représente indépendamment un atome d'hydrogène, un atome d'halogène, un groupe alkyle en C₁ à C₂₀, un groupe alcoxy en C₁ à C₂₀, un groupe aryle, ou un groupe hétéroaryle] 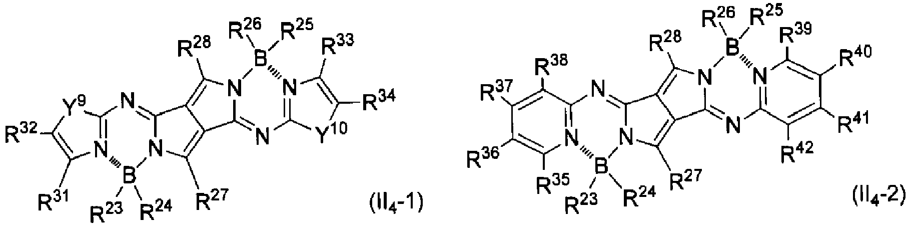 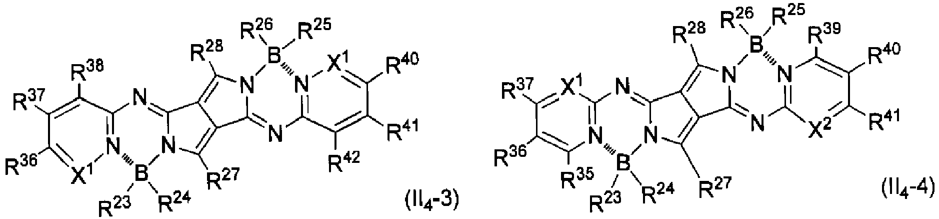 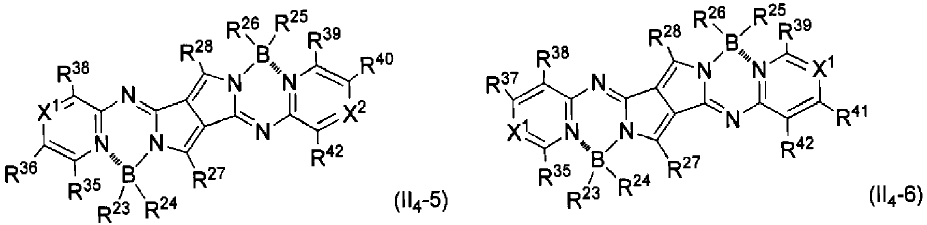 [dans les formules (II₄-1) à (II₄-6), chacun de R²³ à R²⁸ est le même que dans la formule (II₃-1) et, dans la formule (II₄-1), chacun de R³¹ à R³⁴, Y⁹ et Y¹⁰ est le même que dans la formule (II₃-1), dans les formules (II₄-2) à (II₄-6), chacun de R³⁵ à R⁴² est le même que dans la formule (II₃-2) et, dans les formules (II₄-3) à (II₄-6), chacun de X¹ et X² est le même que dans la formule (II₃-3)].

8. Composition de résine selon la revendication 1, dans laquelle le matériau fluorescent infrarouge est formé d'un composé complexe azo-bore représenté par la formule (I) qui suit et a une longueur d'onde d'absorption maximale de 650 nm ou plus et un décalage Stokes de 50 nm ou plus, 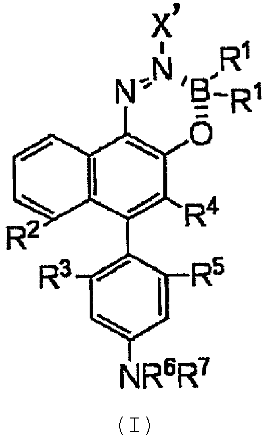 [dans la formule (I),
X' représente un groupe aryle qui peut porter un substituant ou un groupe hétéroaryle qui peut porter un substituant ;
R¹ représente un groupe alkyle en C₁ à C₁₂, un groupe aryle, un groupe aryléthényle, un groupe aryléthynyle, un groupe alcoxy en C₁ à C₁₂, un groupe aryloxy, ou un atome d'halogène, ou bien un R¹ représente un groupe -O-C(=O)- qui est aussi lié à X' et forme un cycle à 6 chaînons, et l'autre R¹ représente indépendamment un groupe alkyle en C₁ à C₁₂, un groupe aryle, un groupe aryléthényle, un groupe aryléthynyle, un groupe alcoxy en C₁ à C₁₂, un groupe aryloxy, ou un atome d'halogène ;
R² et R³ forment ensemble un groupe -O-, un groupe -S-, ou un groupe -N(R⁸)- (ici, R⁸ représente un atome d'hydrogène ou un groupe alkyle en C₁ à C₁₂), et chacun de R⁴ et R⁵ représente un atome d'hydrogène, ou bien R⁴ et R⁵ forment ensemble un groupe -O-, un groupe -S-, ou un groupe -N(R⁸)- (ici, R⁸ a la même signification que celle décrite ci-dessus), et chacun de R² et R³ représente un atome d'hydrogène ;
chacun de R⁶ et R⁷ représente indépendamment un atome d'hydrogène, un groupe alkyle en C₁ à C₁₂, un groupe aryle qui peut porter un substituant, ou un groupe hétéroaryle qui peut porter un substituant ; et
le substituant du groupe aryle ou du groupe hétéroaryle est un ou plusieurs groupes choisis dans l'ensemble constitué par un groupe alkyle en C₁ à C₁₂, un groupe mono (alkyle en C₁ à C₁₂) amino, un groupe di (alkyle en C₁ à C₁₂) amino, un groupe hydroxyle, et un groupe alcoxy en C₁ à C₁₂].

9. Composition de résine selon la revendication 8, dans laquelle le composé complexe azo-bore est représenté par la formule (I₁) suivante, 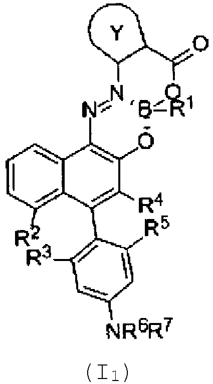 [dans la formule (I₁), Y représente un groupe aryle qui peut porter un substituant ou un groupe hétéroaryle qui peut porter un substituant, et chacun de R¹ à R⁷ a la même signification que chacun de R¹ à R⁷ dans la formule (I)].

10. Composition de résine selon l'une quelconque des revendications 1 à 9, dans laquelle la substance radio-opaque est un ou plusieurs choisis dans l'ensemble constitué par le sulfate de baryum, l'oxyde de bismuth, le sub-carbonate de bismuth, le carbonate de calcium, l'hydroxyde d'aluminium, le tungstène, l'oxyde de zinc, l'oxyde de zirconium, le zirconium, le titane, le platine, le sub-nitrate de bismuth, et le bismuth.

11. Composition de résine selon l'une quelconque des revendications 1 à 10, dans laquelle la teneur en la substance radio-opaque est de 5 % en masse à 50 % en masse.

12. Composition de résine selon l'une quelconque des revendications 1 à 11, dans laquelle la teneur en la substance luminescente est de 0,001 % en masse à 0,5 % en masse.

13. Composition de résine selon l'une quelconque des revendications 1 à 11, dans laquelle la teneur en la substance luminescente est de 0,001 % en masse à 0,05 % en masse.

14. Composition de résine selon l'une quelconque des revendications 1 à 13, dans laquelle la résine est une résine thermoplastique.

15. Composition de résine selon l'une quelconque des revendications 1 à 14, dans laquelle la résine est une ou plusieurs choisies dans l'ensemble constitué par une résine à base d'uréthane, une résine à base d'oléfine, une résine à base de polystyrène, une résine à base de polyester, et une résine à base de chlorure de vinyle.

16. Composition de résine selon l'une quelconque des revendications 1 à 15, qui est utilisée en tant que matériel médical.

17. Article moulé obtenu par traitement de la composition de résine de l'une quelconque des revendications 1 à 16.

18. Article moulé selon la revendication 17, lequel article est un outil médical dont au moins une partie est utilisée dans le corps d'un patient.
